# EUROPEAN PATENT SPECIFICATION

(11) **EP 3 063 153 B1**
(45) Date of publication and mention of the grant of the patent: **07.03.2018**
(21) Application number: 14790074.0
(22) Date of filing: 28.10.2014
(51) Int. Cl.: C07D 495/04, C07D 519/00, H01L 51/00

(54) **AZADIBENZOTHIOPHENES FOR ELECTRONIC APPLICATIONS**
AZADIBENZOTHIOPHENE FÜR ELECTRONISCHE ANWENDUNGEN
AZA-DIBENZOTHIOPHÈNES POUR DES APPLICATIONS ÉLECTRONIQUES

(30) Priority: 31.10.2013 EP 13191100
(43) Date of publication of application: 07.09.2016
(73) Proprietor: Idemitsu Kosan Co., Ltd., Chiyoda-ku Tokyo 100-8321 (JP)
(72) Inventor: WOLLEB, Annemarie, 4232 Fehren (CH); SCHÄFER, Thomas, 4410 Liestal (CH); HEINEMEYER, Ute, 67433 Neustadt (DE); NAGASHIMA, Hideaki, 4057 Basel (CH); RAIMANN, Thomas, 4334 Sisseln (CH); WAGENBLAST, Gerhard, 67157 Wachenheim (DE)
(74) Representative: Hollah, Dorothee
(86) International application number: PCT/EP2014/073038
(87) International publication number: WO 2015/063046

(56) References cited:
- WO-A1-2012/130709
- WO-A1-2013/068376
- US-A1- 2012 241 681

## Description

The present invention relates to compounds of formula I, a process for their production and their use in electronic devices, especially electroluminescent devices. When used as hole transport material in electroluminescent devices, the compounds of formula I may provide improved efficiency, stability, manufacturability, or spectral characteristics of electroluminescent devices.

Khan, Misbahul Ain; Ribeiro, Vera Lucia Teixeira, Pakistan Journal of Scientific and Industrial Research 43 (2000) 168-170 describes the synthesis of benzimidazo[1,2-a]benzimadozoles 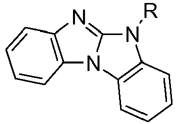 (R = H, Me, Et) by trialkyl phosphite-induced deoxygenation and thermolysis of 1-(o-nitrophenyl)- and 1-(o-azidophenyl)benzimidazoles.

Pedro Molina et al. Tetrahedron (1994) 10029-10036 reports that aza Wittig-type reaction of bis(iminophosphoranes), derived from bis(2-aminophenyl)amine with two equivalents of isocyanate directly provided benzimidazo[1,2,a]benzimidazole derivatives. 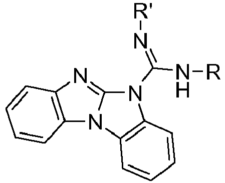 (R = R' = 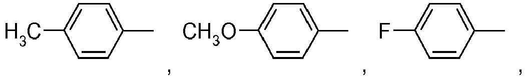 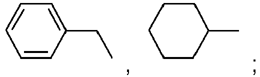 R = 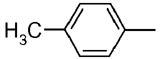 and R' = 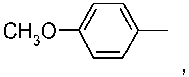 R = iso-propyl and R' = ethyl)
Kolesnikova, I. V.; Zhurnal Organicheskoi Khimii 25 (1989) 1689-95 describes the synthesis of 5H-benzimidazo[1,2-a]benzimidazole 1,2,3,4,7,8,9,10-octafluoro-5-(2,3,4,5,6-pentafluorophenyl). 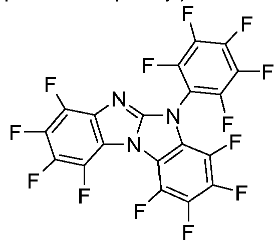 Achour, Reddouane; Zniber, Rachid, Bulletin des Societes Chimiques Belges 96 (1987) 787-92 describes the synthesis of benzimidazobenzimidazoles 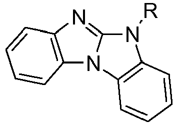 (R = H, -CH(CH₃)₂) which were prepared from benzimidazolinone derivatives.

Hubert, Andre J.; Reimlinger, Hans, Chemische Berichte 103 (1970) 2828-35 describes the synthesis of benzimidazobenzimidazoles 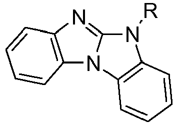 (R = H, CH₃, 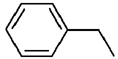 ).

WO2011160757 relates to an electronic device comprising an anode, cathode and at least one organic layer which contains a compound of formulae 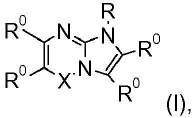 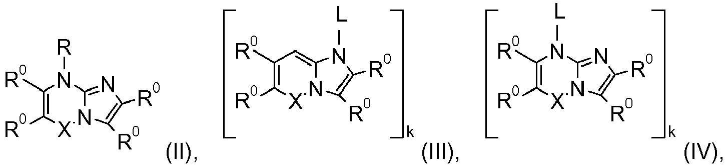 wherein X may be a single bond and L may be a divalent group. The following 4H-Imidazo[1,2-a]imidazole compounds are explicitly disclosed: 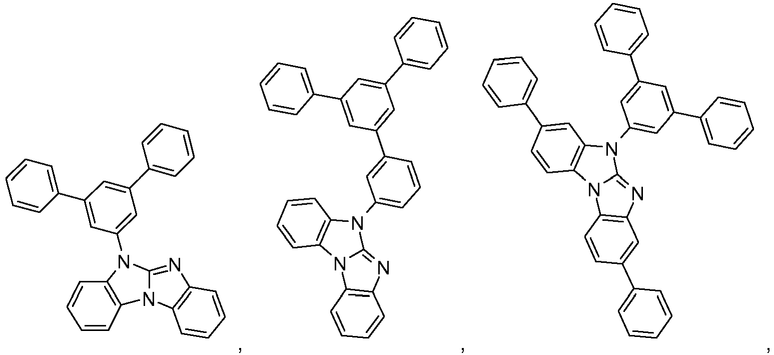 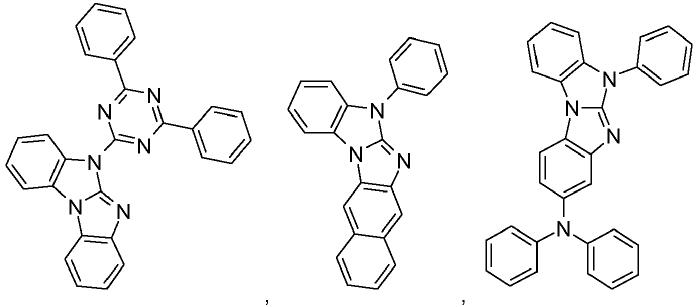 and 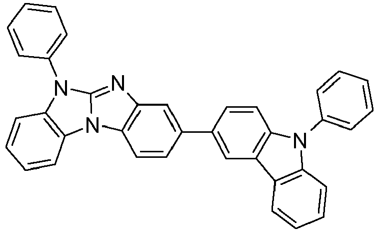 X. Wang et al. Org. Lett. 14 (2012) 452-455 discloses a highly efficient copper-catalyzed synthesis for compounds of formula 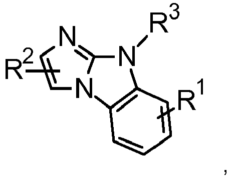 wherein compounds of formula 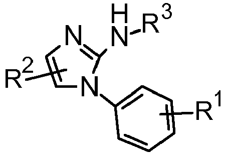 are reacted in the presence of copper acetate (Cu(OAc)₂)/PPh₃/1,10-phenathroline/sodium acetate and oxygen in m-xylene (1 atm) at elevated temperature [published on web: December 29, 2011]. Among others the following compounds can be prepared by the described synthesis method: 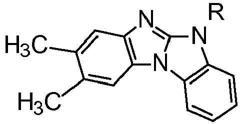 (R = 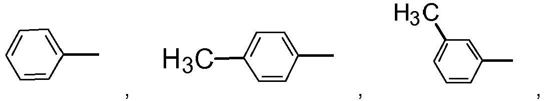 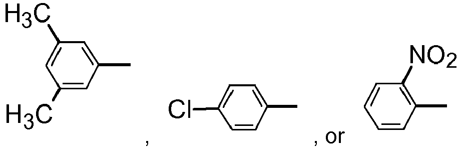 ).

WO2012/130709 relates to 4H-Imidazo[1,2-a]imidazoles, 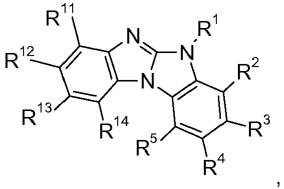 such as, for example, 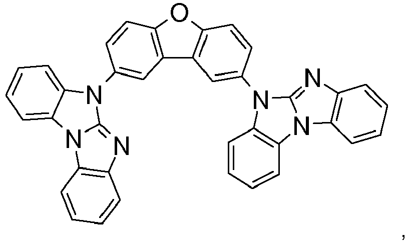 a process for their production and their use in electronic devices, especially electroluminescent devices.

WO2013/068376 describes 4H-imidazo[1,2-a]imidazoles of formula 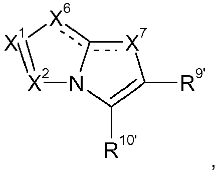 wherein X⁶ is -N= and X⁷ is -NR⁶-, or X⁷ is =N- and X⁶ is -NR⁶-, R⁶ is a group of formula 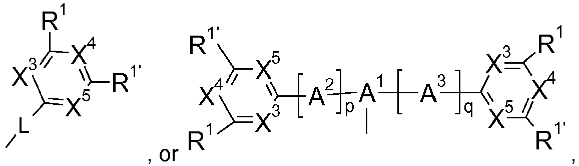 such as, for example, 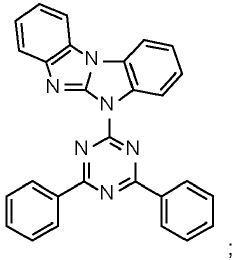 a process for their production and their use in electronic devices, especially electroluminescent devices.

WO2014/009317 relates to compounds of formula 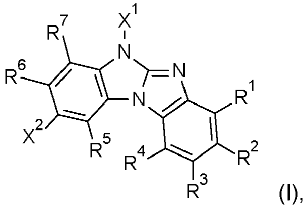 a process for their production and their use in electronic devices, especially electroluminescent devices. The 2,5-disubstituted benzimidazo[1,2-a]benzimidazole derivatives are suitable hole transporting materials, or host materials for phosphorescent emitters.

WO2014/044722 relates to compounds of formula 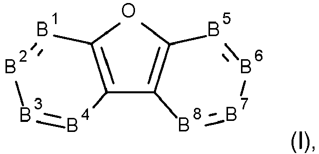 which are characterized in that they substituted by benzimidazo[1,2-a]benzimidazo-5-yl and/or benzimidazo[1,2-a]benzimidazo-2,5-ylene groups and in that at least one of the substituents B¹, B², B³, B⁴, B⁵, B⁶, B⁷ and B⁸ represents N, a process for their production and their use in electronic devices, especially electroluminescent devices. The compounds of WO2014/044722 may be used host material for phosphorescent emitters in electroluminescent devices.

JP2011084531, JP2008074939, WO2011/137072, WO2012/048266, WO2009/086028, WO2013/105206, WO2012/090967, WO2013/069242, WO2012/102967, WO2012/162325 and EP2551932 disclose azadibenzothiophene derivatives.

None of the above references disclose benzimidazo[1,2-a]benzimidazo-5-yl and/or benzimidazo[1,2-a]benzimidazo-2,5-ylene substituted aza-dibenzothiophene compounds.

Notwithstanding these developments, there remains a need for organic light emitting devices comprising new hole transport materials to provide improved efficiency, stability, manufacturability, and/or spectral characteristics of electroluminescent devices.

Accordingly, it is an object of the present invention, with respect to the aforementioned prior art, to provide further materials suitable for use in OLEDs and further applications in organic electronics. More particularly, it should be possible to provide electron transport materials, hole transport materials, electron/exciton blocker materials, hole/exciton blocker materials and matrix materials for use in OLEDs. The materials should be suitable especially for OLEDs which comprise at least one phosphorescence emitter, especially at least one green emitter or at least one blue emitter. Furthermore, the materials should be suitable for providing OLEDs which ensure good efficiencies, good operative lifetimes and a high stability to thermal stress, and a low use and operating voltage of the OLEDs.

Certain aza-dibenzothiophene (benzothiophenopyridine) derivatives substituted with a benzimidazo[1,2-a]benzimidazo-5-yl group and/or a benzimidazo[1,2-a]benzimidazo-2,5-ylene group are found to be suitable for use in organo-electroluminescent devices. In particular, said derivatives are suitable hole and electron transporting materials, or host materials for phosphorescent emitters with good efficiency and durability.
Said object has been solved by compounds of the formula 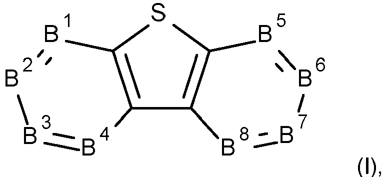 wherein
B¹ is N, or CR⁸¹,
B² is N, or CR⁸²,
B³ is N, or CR⁸³,
B⁴ is N, or CR⁸⁴,
B⁵ is N, or CR⁸⁵,
B⁶ is N, or CR⁸⁶,
B⁷ is N, or CR⁸⁷,
B⁸ is N, or CR⁸⁸,
R⁸¹, R⁸², R⁸³, R⁸⁴, R⁸⁵, R⁸⁶, R⁸⁷ and R⁸⁸ are independently of each other H, F, a cyano group, a C₁-C₂₅alkyl group, which can optionally be substituted by E and or interupted by D;
a C₁-C₂₅alkoxy group; a C₃-C₁₈cycloalkyl group, which can optionally be substituted by E; a C₆-C₂₄aryl group, which can optionally be substituted by G, a C₂-C₃₀heteroaryl group, which can optionally be substituted by G; or a group of formula --(A¹)ₒ-(A²)ₚ-(A³)_{q}-(A⁴)ᵣ-R¹⁶,
o is 0, or 1, p is 0, or 1, q is 0, or 1, r is 0, or 1,
A¹, A², A³ and A⁴ are independently of each other a C₆-C₂₄arylen group, which can optionally be substituted by G, or a C₂-C₃₀heteroarylen group, which can optionally be substituted by G;
R¹⁶ is -NR¹⁰R¹¹, or -Si(R¹²)(R¹³)(R¹⁴), a C₆-C₂₄aryl group, which can optionally be substituted by G; or a C₂-C₃₀heteroaryl group, which can optionally be substituted by G;
R¹⁰ and R¹¹ are independently of each other a C₆-C₂₄aryl group, which can optionally be substituted by G; or a C₂-C₃₀heteroaryl group, which can optionally be substituted by G;
R¹², R¹³ and R¹⁴ are independently of each other a C₁-C₂₅alkyl group, which can optionally be substituted by E and or interupted by D; C₆-C₂₄aryl group, which can optionally be substituted by G; or a C₂-C₃₀heteroaryl group, which can optionally be substituted by G;
D is -CO-, -COO-, -S-, -SO-, -SO₂-, -O-, -NR⁶⁵-, -SiR⁷⁰R⁷¹-, -POR⁷²-, -CR⁶³=CR⁶⁴-, or -C≡C-,
E is -OR⁶⁹, -SR⁶⁹, -NR⁶⁵R⁶⁶, -COR⁶⁸, -COOR⁶⁷, -CONR⁶⁵R⁶⁶, -CN, or F,
G is E, or a C₁-C₁₈alkyl group, a C₃-C₁₈cycloalkyl group, a C₆-C₂₄aryl group, a C₆-C₂₄aryl group, which is substituted by F, C₁-C₁₈alkyl, or C₁-C₁₈alkyl which is interrupted by O; a C₂-C₃₀heteroaryl group, or a C₂-C₃₀heteroaryl group, which is substituted by F, C₁-C₁₈alkyl, or C₁-C₁₈alkyl which is interrupted by O;
R⁶³ and R⁶⁴ are independently of each other H, C₆-C₁₈aryl; C₆-C₁₈aryl which is substituted by C₁-C₁₈alkyl, or C₁-C₁₈alkoxy; C₁-C₁₈alkyl; or C₁-C₁₈alkyl which is interrupted by -O-;
R⁶⁵ and R⁶⁶ are independently of each other a C₆-C₁₈aryl group; a C₆-C₁₈aryl which is substituted by C₁-C₁₈alkyl, or C₁-C₁₈alkoxy; a C₁-C₁₈alkyl group; or a C₁-C₁₈alkyl group, which is interrupted by -O-; or
R⁶⁵ and R⁶⁶ together form a five or six membered ring,
R⁶⁷ is a C₆-C₁₈aryl group; a C₆-C₁₈aryl group, which is substituted by C₁-C₁₈alkyl, or C₁-C₁₈alkoxy; a C₁-C₁₈alkyl group; or a C₁-C₁₈alkyl group, which is interrupted by -O-,
R⁶⁸ is H; a C₆-C₁₈aryl group; a C₆-C₁₈aryl group, which is substituted by C₁-C₁₈alkyl, or C₁-C₁₈alkoxy; a C₁-C₁₈alkyl group; or a C₁-C₁₈alkyl group, which is interrupted by -O-,
R⁶⁹ is a C₆-C₁₈aryl; a C₆-C₁₈aryl, which is substituted by C₁-C₁₈alkyl, or C₁-C₁₈alkoxy; a C₁-C₁₈alkyl group; or a C₁-C₁₈alkyl group, which is interrupted by -O-,
R⁷⁰ and R⁷¹ are independently of each other a C₁-C₁₈alkyl group, a C₆-C₁₈aryl group, or a C₆-C₁₈aryl group, which is substituted by C₁-C₁₈alkyl, and
R⁷² is a C₁-C₁₈alkyl group, a C₆-C₁₈aryl group, or a C₆-C₁₈aryl group, which is substituted by C₁-C₁₈alkyl, with the proviso that
at least one of the substituents B¹, B², B³, B⁴, B⁵, B⁶, B⁷ and B⁸ represents N;
not more than two of the groups B¹, B², B³ and B⁴ represent N; and
not more than two of the groups B⁵, B⁶, B⁷ and B⁸ represent N; and
with the further proviso that at least one of the substituents R⁸¹, R⁸², R⁸³, R⁸⁴, R⁸⁵, R⁸⁶, R⁸⁷ and R⁸⁸ represent a group of formula -(A¹)ₒ-(A²)ₚ-(A³)_{q}-(A⁴)ᵣ-R¹⁶, wherein R¹⁶ represents a benzimidazo[1,2-a]benzimidazo-5-yl group, which can optionally be substituted by G;
and/or at least one of the groups A¹, A², A³ and A⁴ respresents a benzimidazo[1,2-a]benzimidazo-2,5-ylene group, which can optionally be substituted by G.

In a preferred embodiment the present invention is directed to compounds of formula (I), wherein A¹, A², A³ and A⁴ are different from benzofuro[2,3-b]pyridylene ( 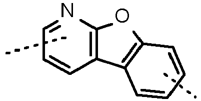 ), benzofuro[2,3-c]pyridylene ( 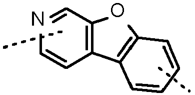 ), furo[3,2-b:4,5-b']dipyridylene ( 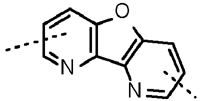 ); and
R¹⁶ and R^{16'} are different from a benzofuro[2,3-b]pyridyl, benzothiopheno[2,3-b]pyridyl, 9H-pyrido[2,3-c]indolyl, benzofuro[2,3-c]pyridyl, benzothiopheno[2,3-c]pyridyl, furo[3,2-b:4,5-b']dipyridyl, pyrrolo[3,2-b:4,5-b']dipyridyl, thieno[3,2-b:4,5-b']dipyridyl.
In said embodiment the group of the formula -(A¹)ₒ-(A²)ₚ-(A³)_{q}-(A⁴)ᵣ-R¹⁶ is preferably a group of formula (**XIIa**), (**XIIb**), (**XIIc**), (**XIIg**), (**XIIj**), (**XIIn**), (**XIIs**), (X**IIt**), (**XIIw**), (**XIIx**), (**XIIIb**), (**XIIIc**), (**XIIId**), (**XIIIf**), or (**XIIIg**).
The group of the formula -(A¹)ₒ-(A²)ₚ-(A³)_{q}-(A⁴)ᵣ-R^{16'} is preferably H, or a group of formula (**XIIa**), (**XIIb**), (**XIIc**), (**XIIg**), (**XIIj**), (**XIIn**), (**XIIs**), (**XIIt**), (**XIIw**), (**XIIx**), (**XIIIb**), (**XIIIc**), (**XIIId**), (**XIIIf**), or (**XIIIg**); or a group of formula (**XIVa**), (**XIVb**), (**XIVc**), (**XIVi**), (**XIVo**), (**XIVp**), (**XIVq**), (**XIVt**), (**XIVw**), (**XIVz**), (**XVc**), (**XVd**), (**XVh**), (**XVl**), (**XVm**), (**XVo**), (**XVp**), (**XVq**), (**XVr**), (**XVt**), (**XVx**), (**XVy**), (**XVIc**), or (**XVId**).

Certain compounds of the present invention have a LUMO-Level of 1.8 [calculated] - 2.5 eV and show, when used as host in combination with phosphorescent emitters, excellent power efficiencies, in particular. electroluminescent (EL) devices comprising the compounds of the present invention exhibit reduced drive voltage while maintaining excellent luminance properties.

The compounds of the present invention may be used for electrophotographic photoreceptors, photoelectric converters, organic solar cells (organic photovoltaics), switching elements, such as organic transistors, for example, organic FETs and organic TFTs, organic light emitting field effect transistors (OLEFETs), image sensors, dye lasers and electroluminescent devices, such as, for example, organic light-emitting diodes (OLEDs).

Accordingly, a further subject of the present invention is directed to an electronic device, comprising a compound according to the present invention. The electronic device is preferably an electroluminescent device.

The compounds of formula I can in principal be used in any layer of an EL device, but are preferably used as host, hole transport, electron transport, hole blocking and electron blocking material. Particularly, the compounds of formula I are used as host material for blue light emitting phosphorescent emitters.

Hence, a further subject of the present invention is directed to a hole transport layer and an electron transport layer comprising a compound of formula I according to the present invention.

A further subject of the present invention is directed to an emitting layer, comprising a compound of formula I according to the present invention. In said embodiment a compound of formula I is preferably used as host material in combination with a phosphorescent emitter.

The compounds of formula I have preferably a molecular weight below 1500 g/mol.

A further subject of the present invention is directed to an electron blocking layer, or hole blcking layer, comprising a compound of formula I according to the present invention.

D is preferably -CO-, -COO-, -S-, -SO-, -SO₂-, -O-, -NR⁶⁵-, wherein R⁶⁵ is C₁-C₁₈alkyl, such as methyl, ethyl, n-propyl, iso-propyl, n-butyl, isobutyl, or sec-butyl, or C₆-C₁₄aryl, such as phenyl, tolyl, naphthyl, or biphenylyl, or C₂-C₃₀heteroaryl, such as, for example, benzimidazo[1,2-a]benzimidazo-5-yl ( 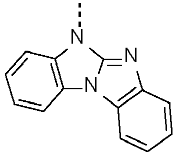 ), benzimidazo[1,2-a]benzimidazo-2-yl ( 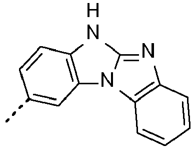 ), carbazolyl, dibenzofuranyl, which can be unsubstituted or substituted especially by C₆-C₁₀aryl, or C₆-C₁₀aryl, which is substituted by C₁-C₄alkyl; or C₂-C₁₄heteroaryl.

E is preferably -OR⁶⁹; -SR⁶⁹; -NR⁶⁵R⁶⁵; -COR⁶⁸; -COOR⁶⁷; -CONR⁶⁵R⁶⁵; or -CN; wherein R⁶⁵, R⁶⁷, R⁶⁸ and R⁶⁹ are independently of each other C₁-C₁₈alkyl, such as methyl, ethyl, n-propyl, iso-propyl, n-butyl, isobutyl, sec-butyl, hexyl, octyl, or 2-ethyl-hexyl, or C₆-C₁₄aryl, such as phenyl, tolyl, naphthyl, or biphenylyl.

Among the compounds of formula (I) compounds of formula 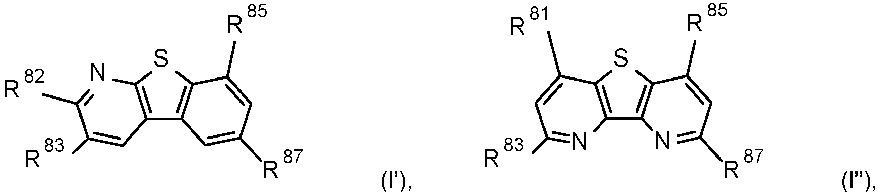 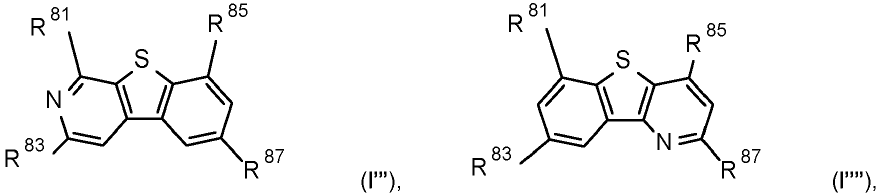 and 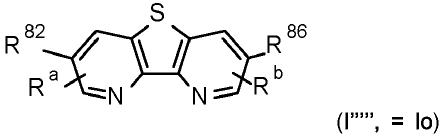 are preferred, where R⁸¹, R⁸², R⁸³, R⁸⁵, R⁸⁶ and R⁸⁷ have the (preferred) meanings given above and below, respectively.

In a preferred embodiment the present invention is directed to compounds of formula 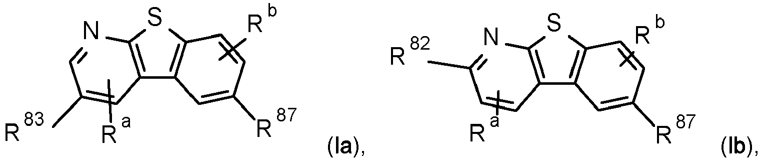 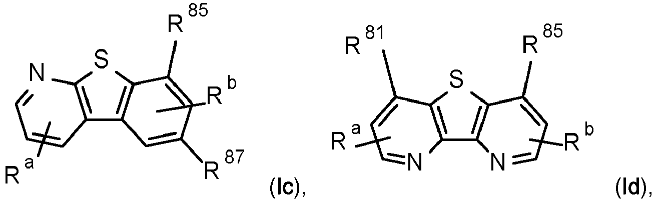 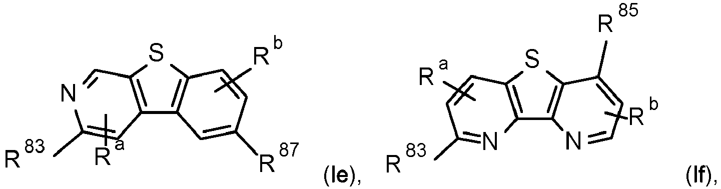 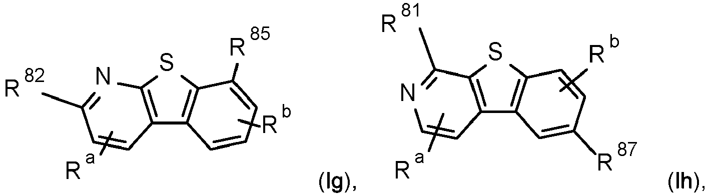 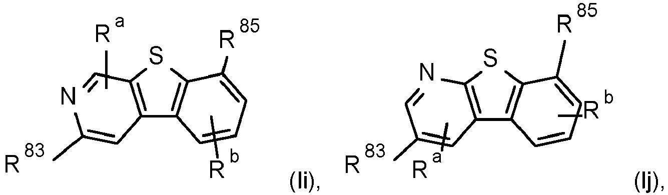 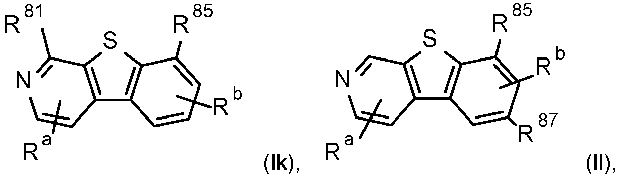 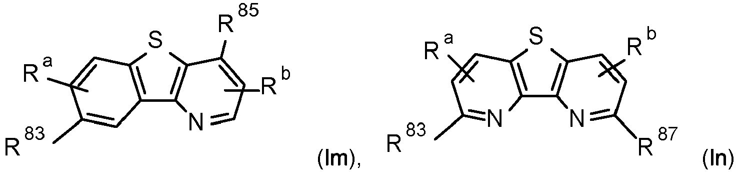 and 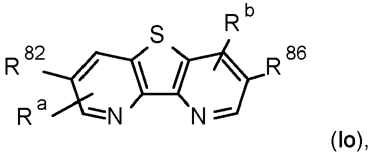 wherein R⁸¹, R⁸², R⁸³, R⁸⁵, R⁸⁶ and R⁸⁷ are as defined above. R^{a} and R^{b} are independently of each other H, a C₁-C₂₅alkyl group, or a C₃-C₁₈cycloalkyl group. R^{a} and R^{b} are preferably H.

Compounds of formula (**Ia**), (**Ib**), (**Ic**), (**Id**), (**Ie**), (**If**), (**Ii**), (**Ij**), (**Il**), (**Im**) and (**In**) are preferred. Compounds of formula (**Ia**), (**Ib**), (**Ic**), (**Id**), (**Ie**), (**If**), (**Ij**), (**Im**) and (**In**) are especially preferred.

R^{16'} (R¹⁶) may be a C₆-C₂₄aryl group, which can optionally be substituted by G, or a C₂-C₃₀heteroaryl group, which can optionally be substituted by G.

The C₆-C₂₄aryl group, which optionally can be substituted by G, is typically phenyl, 4-methylphenyl, 4-methoxyphenyl, naphthyl, especially 1-naphthyl, or 2-naphthyl, biphenylyl, terphenylyl, pyrenyl, 2- or 9-fluorenyl, phenanthryl, or anthryl, which may be unsubstituted or substituted.

The C₂-C₃₀heteroaryl group R^{16'} (R¹⁶), which optionally can be substituted by G, represent a ring with five to seven ring atoms or a condensed ring system, wherein nitrogen, oxygen or sulfur are the possible hetero atoms, and is typically a heterocyclic group with five to 30 atoms having at least six conjugated *π*-etectrons such as 9H-pyrido[2,3-b]indolyl, benzofuro[2,3-b]pyridyl, benzothiopheno[2,3-b]pyridyl, 9H-pyrido[2,3-c]indolyl, benzofuro[2,3-c]pyridyl, benzothiopheno[2,3-c]pyridyl, furo[3,2-b:4,5-b']dipyridyl, pyrrolo[3,2-b:4,5-b']dipyridyl, thieno[3,2-b:4,5-b']dipyridyl, thienyl, benzothiophenyl, dibenzothiophenyl, thianthrenyl, furyl, furfuryl, 2H-pyranyl, benzofuranyl, isobenzofuranyl, dibenzofuranyl, phenoxythienyl, pyrrolyl, imidazolyl, pyrazolyl, pyridyl, bipyridyl, triazinyl, pyrimidinyl, pyrazinyl, pyridazinyl, indolizinyl, isoindolyl, indolyl, indazolyl, purinyl, quinolizinyl, chinolyl, isochinolyl, phthalazinyl, naphthyridinyl, chinoxalinyl, chinazolinyl, cinnolinyl, pteridinyl, carbolinyl, benzotriazolyl, benzoxazolyl, phenanthridinyl, acridinyl, pyrimidinyl, phenanthrolinyl, phenazinyl, isothiazolyl, phenothiazinyl, isoxazolyl, furazanyl, benzimidazo[1,2-a]benzimidazo-5-yl, benzimidazo[1,2-a]benzimidazo-2-yl, carbazolyl, or phenoxazinyl, which can be unsubstituted or substituted.

The C₆-C₂₄aryl and C₂-C₃₀heteroaryl groups may be substituted by G.

G has the same preferences as E, or is C₁-C₁₈alkyl, such as methyl, ethyl, n-propyl, iso-propyl, n-butyl, isobutyl, sec-butyl, hexyl, octyl, or 2-ethyl-hexyl, or is C₁-C₁₈perfluoroalkyl, such, for example, -CF₃.

Prefered C₂-C₃₀heteroaryl groups are pyridyl, triazinyl, pyrimidinyl, especially 9H-pyrido[2,3-b]indolyl, benzofuro[2,3-b]pyridyl, benzothiopheno[2,3-b]pyridyl, 9H-pyrido[2,3-c]indolyl, benzofuro[2,3-c]pyridyl, benzothiopheno[2,3-c]pyridyl, furo[3,2-b:4,5-b']dipyridyl, pyrrolo[3,2-b:4,5-b']dipyridyl, thieno[3,2-b:4,5-b']dipyridyl, benzimidazo[1,2-a]benzimidazo-5-yl ( 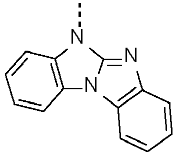 ), benzimidazo[1,2-a]benzimidazo-2-yl ( 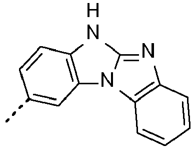 ), carbazolyl, dibenzofuranyl, and dibenzothiophenyl, which can be unsubstituted or substituted especially by C₆-C₁₀aryl, or C₆-C₁₀aryl, which is substituted by C₁-C₄alkyl; or C₂-C₁₄heteroaryl.

R¹⁶ is a group of the formula 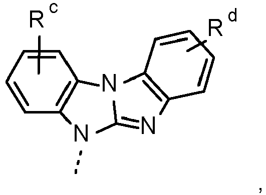 or R¹⁶ has the meaning of R^{16'}, if at least one of the groups A¹, A², A³ and A⁴ respresent a group of formula

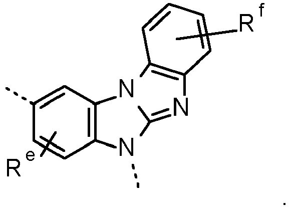

R^{16'} is preferably H, or a group of the formula -Si(R¹²)(R¹³)(R¹⁴), especially

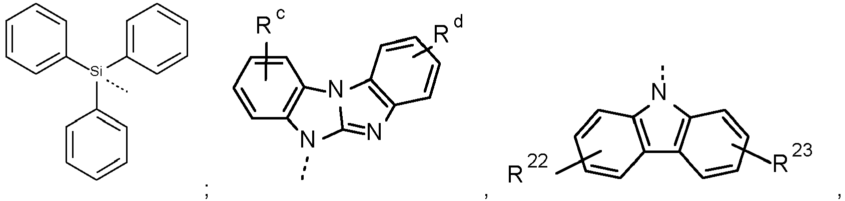

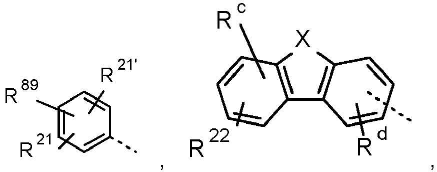

especially 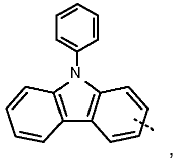 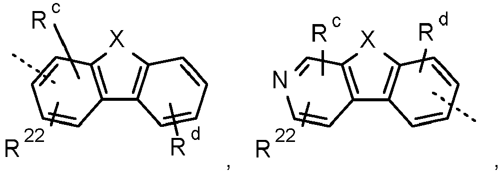 especially 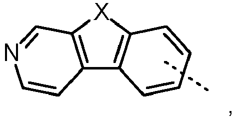 very especially 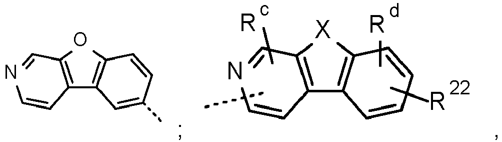 especially 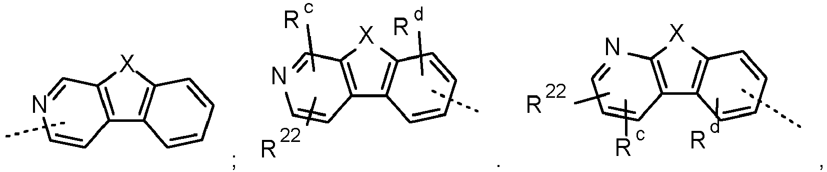 especially 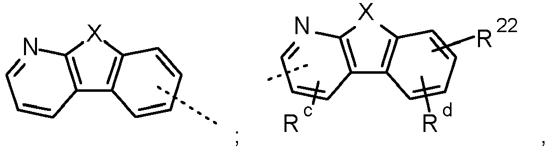 or 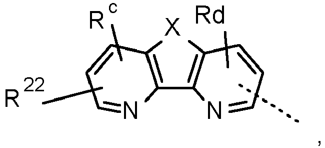 especially 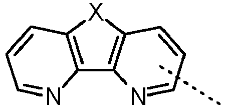 R¹², R¹³ and R¹⁴ are independently of each other a phenyl group, which can optionally be substituted by one, or more C₁-C₁₈alkyl groups;
R²¹ and R^{21'} are independently of each other H, a phenyl group, or a C₁-C₁₈alkyl group;
R²² and R²³ are independently of each other H, or a group of the formula 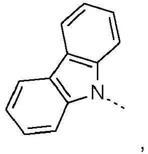 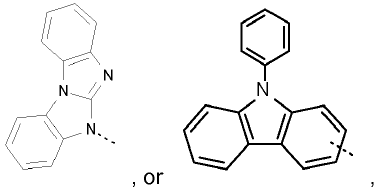 especially 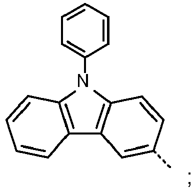 X is O, S, or NR²⁴, R²⁴ is a C₆-C₂₄aryl group, or a C₂-C₃₀heteroaryl group, which can optionally be substituted by G, wherein G is as defined in above; and R⁸⁹ is H, a group of formula 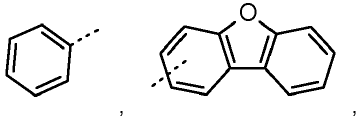 especially 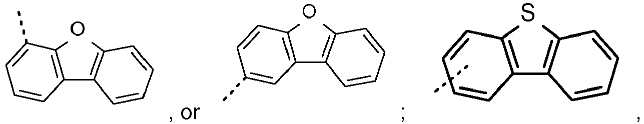 especially 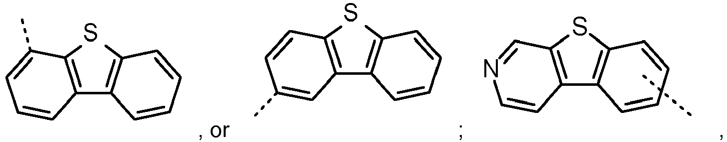 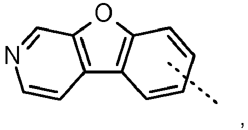 especially 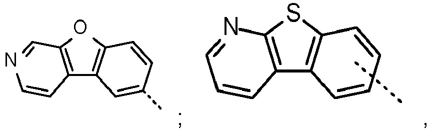 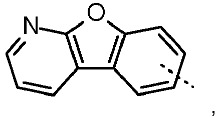 especially 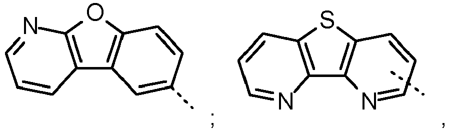 or

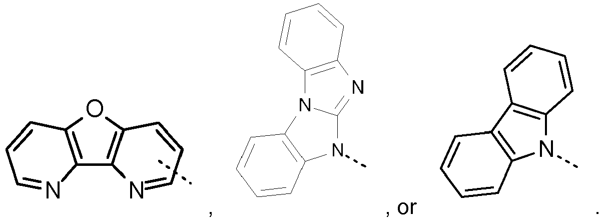

R^{c} and R^{d} are independently of each other H, a C₁-C₂₅alkyl group, or a C₃-C₁₈cycloalkyl group. R^{c} and R^{d} are preferably H. R^{e} and R^{f} are independently of each other H, a C₁-C₂₅alkyl group, or a C₃-C₁₈cycloalkyl group. R^{e} and R^{f} are preferably H.

A¹, A², A³ and A⁴ are independently of each other a C₆-C₂₄arylen group, which can optionally be substituted by G, or a C₂-C₃₀heteroarylen group, which can optionally be substituted by G. The C₆-C₂₄arylen groups A¹, A², A³ and A⁴ which optionally can be substituted by G, are typically phenylene, 4-methylphenylene, 4-methoxyphenylene, naphthylene, especially 1-naphthylene, or 2-naphthylene, biphenylylene, terphenylylene, pyrenylene, 2- or 9-fluorenylene, phenanthrylene, or anthrylene, which may be unsubstituted or substituted.

The C₂-C₃₀heteroarylen groups A¹, A², A³ and A⁴, which optionally can be substituted by G, represent a ring with five to seven ring atoms or a condensed ring system, wherein nitrogen, oxygen or sulfur are the possible hetero atoms, and is typically a heterocyclic group with five to 30 atoms having at least six conjugated -electrons such as benzofuro[2,3-b]pyridylene ( 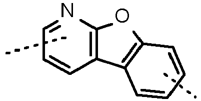 ), benzothiopheno[2,3-b]pyridylene ( 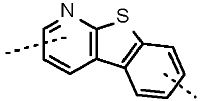 ), pyrido[2,3-b]indolylene ( 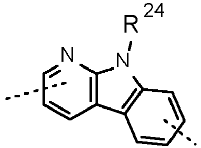 ), benzofuro[2,3-c]pyridylene ( 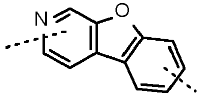 ), benzothiopheno[2,3-c]pyridylene ( 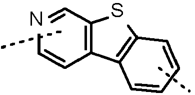 ), pyrido[2,3-c]indolylene ( 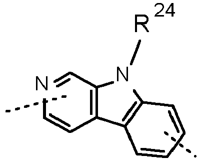 ), furo[3,2-b:4,5-b']dipyridylene ( 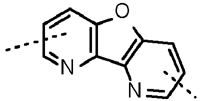 ), thieno[3,2-b:4,5-b']dipyridylene ( 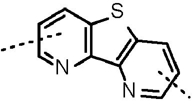 ), pyrrolo[3,2-b:4,5-b']dipyridylene ( 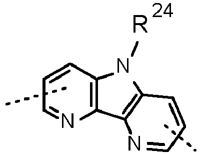 ), thienylene, benzothiophenylene, thianthrenylene, furylene, furfurylene, 2H-pyranylene, benzofuranylene, isobenzofuranylene, dibenzofuranylene ( 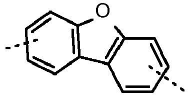 ), dibenzothiophenylene ( 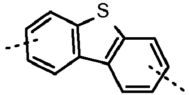 ), phenoxythienylene, pyrrolylene, imidazolylene, pyrazolylene, pyridylene, bipyridylene, triazinylene, pyrimidinylene, pyrazinylene, pyridazinylene, indolizinylene, isoindolylene, indolylene, indazolylene, purinylene, quinolizinylene, chinolylene, isochinolylene, phthalazinylene, naphthyridinylene, chinoxalinylene, chinazolinylene, cinnolinylene, pteridinylene, carbolinylene, benzotriazolylene, benzoxazolylene, phenanthridinylene, acridinylene, pyrimidinylene, phenanthrolinylene, phenazinylene, isothiazolylene, phenothiazinylene, isoxazolylene, furazanylene, carbazolylene ( 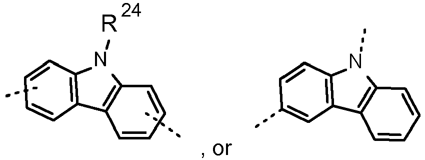 ), benzimidazo[1,2-a]benzimidazo-2,5-ylene ( 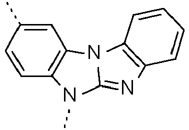 ), or phenoxazinylene, which can be unsubstituted or substituted.

Preferred C₆-C₂₄arylen groups are 1,3-phenylene, 3,3'-biphenylylene, 3,3'-m-terphenylene, 2- or 9-fluorenylene, phenanthrylene, which may be unsubstituted or substituted, especially by C₆-C₁₀aryl, C₆-C₁₀aryl which is substituted by C₁-C₄alkyl; or C₂-C₁₄heteroaryl.

Preferred C₂-C₃₀heteroarylen groups are pyridylene, triazinylene, pyrimidinylene, especially benzofuro[2,3-b]pyridylene, benzothiopheno[2,3-b]pyridylene , pyrido[2,3-b]indolylene , benzofuro[2,3-c]pyridylene, benzothiopheno[2,3-c]pyridylene , pyrido[2,3-c]indolylene furo[3,2-b:4,5-b']dipyridylene, thieno[3,2-b:4,5-b']dipyridylene, pyrrolo[3,2-b:4,5-b']dipyridylene, dibenzofuranylene, dibenzothiophenylene , carbazolylene and benzimidazo[1,2-a]benzimidazo-2,5-ylene , which can be unsubstituted or substituted, especially by C₆-C₁₀aryl, C₆-C₁₀aryl which is substituted by C₁-C₄alkyl; or C₂-C₁₄heteroaryl.

Benzimidazo[1,2-a]benzimidazo-5-yl, benzimidazo[1,2-a]benzimidazo-2-yl, carbazolyl and dibenzofuranyl are examples of a C₂-C₁₄heteroaryl group. Phenyl, 1-naphthyl and 2-naphthyl are examples of a C₆-C₁₀aryl group.

The C₆-C₂₄arylen and C₂-C₃₀heteroarylen groups may be substituted by G.

A¹, A², A³ and A⁴ are preferably a group of the formula 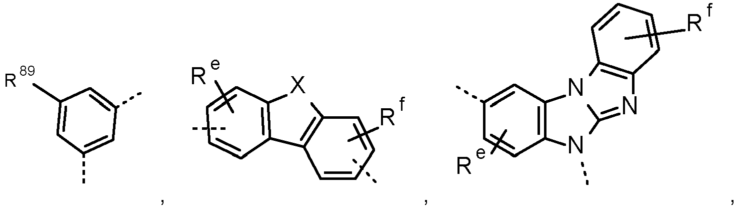 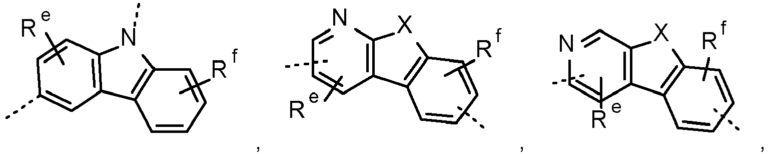 or 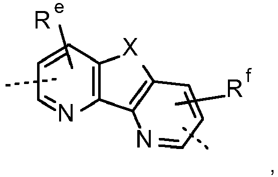 wherein R⁸⁹ and X are as defined below. X is preferably O. R^{e} and R^{f} are independently of each other H, a C₁-C₂₅alkyl group, or a C₃-C₁₈cycloalkyl group. R^{e} and R^{f} are preferably H.

The aza-dibenzofuran derivatives of the present invention are characterized in that they are substituted with at least one benzimidazo[1,2-a]benzimidazo-5-yl group and/or at least one benzimidazo[1,2-a]benzimidazo-2,5-ylene group.

In particular, the aza-dibenzofuran derivatives of the present invention are characterized in that at least one of the substituents R⁸¹, R⁸², R⁸³, R⁸⁴, R⁸⁵, R⁸⁶, R⁸⁷ and R⁸⁸ is a group of formula --(A¹)ₒ-(A²)ₚ-(A³)_{q}-(A⁴)ᵣ-R¹⁶, wherein R¹⁶ is a group of the formula 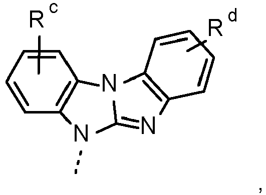 especially 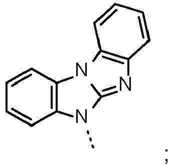 and/or at least one of the substituents R⁸¹, R⁸², R⁸³, R⁸⁴, R⁸⁵, R⁸⁶, R⁸⁷ and R⁸⁸ is a group of formula -(A¹)ₒ-(A²)ₚ-(A³)_{q}-(A⁴)ᵣ-R^{16'}, wherein at least one of the groups A¹, A², A³ and A⁴ represent a group of formula 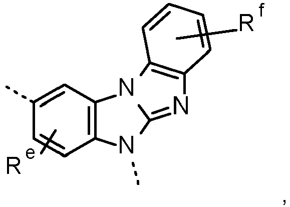 especially

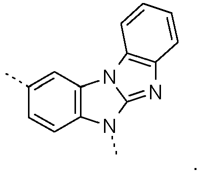

In a preferred embodiment the present invention is directed to compounds of formula (**Ia**)**,** (**Ib**), (**Ic**), (**Id**), (**Ie**), (**If**), (**Ig**), (**Ih**), (**Ii**), (**Ij**), (**Ik**), (**Il**), (**Im**), (**In**) and (**Io**), wherein
in the compounds of formula (**Ia**)
R⁸³ is a group of the formula -(A¹)ₒ-(A²)ₚ-(A³)_{q}-(A⁴)ᵣ-R¹⁶; and
R⁸⁷ is H, a C₁-C₂₅alkyl group, or a group of the formula -(A¹)ₒ-(A²)ₚ-(A³)_{q}-(A⁴)ᵣ-R^{16'}; or
R⁸³ is H, a C₁-C₂₅alkyl group, or a group of the formula -(A¹)ₒ-(A²)ₚ-(A³)_{q}-(A⁴)ᵣ-R^{16'}; and
R⁸⁷ is a group of the formula -(A¹)ₒ-(A²)ₚ-(A³)_{q}-(A⁴)ᵣ-R¹⁶;
in the compounds of formula (**Ib**)
R⁸² is a group of the formula -(A¹)ₒ-(A²)ₚ-(A³)_{q}-(A⁴)ᵣ-R¹⁶; and
R⁸⁷ is H, a C₁-C₂₅alkyl group, or a group of the formula -(A¹)ₒ-(A²)ₚ-(A³)_{q}-(A⁴)ᵣ-R^{16'}; or
R⁸² is H, a C₁-C₂₅alkyl group, or a group of the formula -(A¹)ₒ-(A²)ₚ-(A³)_{q}-(A⁴)ᵣ-R^{16'}; and
R⁸⁷ is a group of the formula -(A¹)ₒ-(A²)ₚ-(A³)_{q}-(A⁴)ᵣ-R¹⁶;
in the compounds of formula (**Ic**)
R⁸⁵ is a group of the formula -(A¹)ₒ-(A²)ₚ-(A³)_{q}-(A⁴)ᵣ-R¹⁶; and
R⁸⁷ is H, a C₁-C₂₅alkyl group, or a group of the formula -(A¹)ₒ-(A²)ₚ-(A³)_{q}-(A⁴)ᵣ-R^{16'}; or
R⁸⁵ is H, a C₁-C₂₅alkyl group, or a group of the formula -(A¹)ₒ-(A²)ₚ-(A³)_{q}-(A⁴)ᵣ-R^{16'}; and
R⁸⁷ is a group of the formula -(A¹)ₒ-(A²)ₚ-(A³)_{q}-(A⁴)ᵣ-R¹⁶;
in the compounds of formula (**Id**)
R⁸¹ is a group of the formula -(A¹)ₒ-(A²)ₚ-(A³)_{q}-(A⁴)ᵣ-R¹⁶; and
R⁸⁵ is H, a C₁-C₂₅alkyl group, or a group of the formula -(A¹)ₒ-(A²)ₚ-(A³)_{q}-(A⁴)ᵣ-R^{16'}; or
R⁸¹ is H, a C₁-C₂₅alkyl group, or a group of the formula -(A¹)ₒ-(A²)ₚ-(A³)_{q}-(A⁴)ᵣ-R^{16'}; and
R⁸⁵ is a group of the formula -(A¹)ₒ-(A²)ₚ-(A³)_{q}-(A⁴)ᵣ-R¹⁶;
in the compounds of formula (**Ie**)
R⁸³ is a group of the formula -(A¹)ₒ-(A²)ₚ-(A³)_{q}-(A⁴)ᵣ-R¹⁶; and
R⁸⁷ is H, a C₁-C₂₅alkyl group, or a group of the formula -(A¹)ₒ-(A²)ₚ-(A³)_{q}-(A⁴)ᵣ-R^{16'}; or
R⁸³ is H, a C₁-C₂₅alkyl group, or a group of the formula -(A¹)ₒ-(A²)ₚ-(A³)_{q}-(A⁴)ᵣ-R^{16'}; and
R⁸⁷ is a group of the formula -(A¹)ₒ-(A²)ₚ-(A³)_{q}-(A⁴)ᵣ-R¹⁶;
in the compounds of formula (**If**)
R⁸³ is a group of the formula -(A¹)ₒ-(A²)ₚ-(A³)_{q}-(A⁴)ᵣ-R¹⁶; and
R⁸⁵ is H, a C₁-C₂₅alkyl group, or a group of the formula -(A¹)ₒ-(A²)ₚ-(A³)_{q}-(A⁴)ᵣ-R^{16'}; or
R⁸³ is H, a C₁-C₂₅alkyl group, or a group of the formula -(A¹)ₒ-(A²)ₚ-(A³)_{q}-(A⁴)ᵣ-R^{16'}; and
R⁸⁵ is a group of the formula -(A¹)ₒ-(A²)ₚ-(A³)_{q}-(A⁴)ᵣ-R¹⁶;
in the compounds of formula (**Ig**)
R⁸² is a group of the formula -(A¹)ₒ-(A²)ₚ-(A³)_{q}-(A⁴)ᵣ-R¹⁶; and
R⁸⁵ is H, a C₁-C₂₅alkyl group, or a group of the formula -(A¹)ₒ-(A²)ₚ-(A³)_{q}-(A⁴)ᵣ-R^{16'}; or
R⁸² is H, a C₁-C₂₅alkyl group, or a group of the formula -(A¹)ₒ-(A²)ₚ-(A³)_{q}-(A⁴)ᵣ-R^{16'}; and
R⁸⁵ is a group of the formula -(A¹)ₒ-(A²)ₚ-(A³)_{q}-(A⁴)ᵣ-R¹⁶;
in the compounds of formula (**Ih**)
R⁸¹ is a group of the formula -(A¹)ₒ-(A²)ₚ-(A³)_{q}-(A⁴)ᵣ-R¹⁶; and
R⁸⁷ is H, a C₁-C₂₅alkyl group, or a group of the formula -(A¹)ₒ-(A²)ₚ-(A³)_{q}-(A⁴)ᵣ-R^{16'}; or
R⁸¹ is H, a C₁-C₂₅alkyl group, or a group of the formula -(A¹)ₒ-(A²)ₚ-(A³)_{q}-(A⁴)ᵣ-R^{16'}; and
R⁸⁷ is a group of the formula -(A¹)ₒ-(A²)ₚ-(A³)_{q}-(A⁴)ᵣ-R¹⁶;
in the compounds of formula (**Ii**)
R⁸³ is a group of the formula -(A¹)ₒ-(A²)ₚ-(A³)_{q}-(A⁴)ᵣ-R¹⁶; and
R⁸⁵ is H, a C₁-C₂₅alkyl group, or a group of the formula -(A¹)ₒ-(A²)ₚ-(A³)_{q}-(A⁴)ᵣ-R^{16'}; or
R⁸³ is H, a C₁-C₂₅alkyl group, or a group of the formula -(A¹)ₒ-(A²)ₚ-(A³)_{q}-(A⁴)ᵣ-R^{16'}; and
R⁸⁵ is a group of the formula -(A¹)ₒ-(A²)ₚ-(A³)_{q}-(A⁴)ᵣ-R¹⁶;
in the compounds of formula (**Ij**)
R⁸³ is a group of the formula -(A¹)ₒ-(A²)ₚ-(A³)_{q}-(A⁴)ᵣ-R¹⁶; and
R⁸⁵ is H, a C₁-C₂₅alkyl group, or a group of the formula -(A¹)ₒ-(A²)ₚ-(A³)_{q}-(A⁴)ᵣ-R^{16'}; or
R⁸³ is H, a C₁-C₂₅alkyl group, or a group of the formula -(A¹)ₒ-(A²)ₚ-(A³)_{q}-(A⁴)ᵣ-R^{16'}; and
R⁸⁵ is a group of the formula -(A¹)ₒ-(A²)ₚ-(A³)_{q}-(A⁴)ᵣ-R¹⁶;
in the compounds of formula (**Ik**)
R⁸¹ is a group of the formula -(A¹)ₒ-(A²)ₚ-(A³)_{q}-(A⁴)ᵣ-R¹⁶; and
R⁸⁵ is H, a C₁-C₂₅alkyl group, or a group of the formula -(A¹)ₒ-(A²)ₚ-(A³)_{q}-(A⁴)ᵣ-R^{16'}; or
R⁸¹ is H, a C₁-C₂₅alkyl group, or a group of the formula -(A¹)ₒ-(A²)ₚ-(A³)_{q}-(A⁴)ᵣ-R^{16'}; and
R⁸⁵ is a group of the formula -(A¹)ₒ-(A²)ₚ-(A³)_{q}-(A⁴)ᵣ-R¹⁶;
in the compounds of formula (**Il**)
R⁸⁵ is a group of the formula -(A¹)ₒ-(A²)ₚ-(A³)_{q}-(A⁴)ᵣ-R¹⁶; and
R⁸⁷ is H, a C₁-C₂₅alkyl group, or a group of the formula -(A¹)ₒ-(A²)ₚ-(A³)_{q}-(A⁴)ᵣ-R^{16'}; or
R⁸⁵ is H, a C₁-C₂₅alkyl group, or a group of the formula -(A¹)ₒ-(A²)ₚ-(A³)_{q}-(A⁴)ᵣ-R^{16'}; and
R⁸⁷ is a group of the formula -(A¹)ₒ-(A²)ₚ-(A³)_{q}-(A⁴)ᵣ-R¹⁶;
in the compounds of formula (**Im**)
R⁸³ is a group of the formula -(A¹)ₒ-(A²)ₚ-(A³)_{q}-(A⁴)ᵣ-R¹⁶; and
R⁸⁵ is H, a C₁-C₂₅alkyl group, or a group of the formula -(A¹)ₒ-(A²)ₚ-(A³)_{q}-(A⁴)ᵣ-R^{16'}; or
R⁸³ is H, a C₁-C₂₅alkyl group, or a group of the formula -(A¹)ₒ-(A²)ₚ-(A³)_{q}-(A⁴)ᵣ-R^{16'}; and
R⁸⁵ is a group of the formula -(A¹)ₒ-(A²)ₚ-(A³)_{q}-(A⁴)ᵣ-R¹⁶;
in the compounds of formula (**In**)
R⁸³ is a group of the formula -(A¹)ₒ-(A²)ₚ-(A³)_{q}-(A⁴)ᵣ-R¹⁶; and
R⁸⁷ is H, a C₁-C₂₅alkyl group, or a group of the formula -(A¹)ₒ-(A²)ₚ-(A³)_{q}-(A⁴)ᵣ-R^{16'}; or
R⁸³ is H, a C₁-C₂₅alkyl group, or a group of the formula -(A¹)ₒ-(A²)ₚ-(A³)_{q}-(A⁴)ᵣ-R^{16'}; and
R⁸⁷ is a group of the formula -(A¹)ₒ-(A²)ₚ-(A³)_{q}-(A⁴)ᵣ-R¹⁶.
o is 0, or 1, p is 0, or 1, q is 0, or 1, r is 0, or 1;
in the compounds of formula (**Io**)
R⁸² is a group of the formula -(A¹)ₒ-(A²)ₚ-(A³)_{q}-(A⁴)ᵣ-R¹⁶; and
R⁸⁶ is H, a C₁-C₂₅alkyl group, or a group of the formula -(A¹)ₒ-(A²)ₚ-(A³)_{q}-(A⁴)ᵣ-R^{16'}; or
R⁸² is H, a C₁-C₂₅alkyl group, or a group of the formula -(A¹)ₒ-(A²)ₚ-(A³)_{q}-(A⁴)ᵣ-R^{16'}; and
R⁸⁶ is a group of the formula -(A¹)ₒ-(A²)ₚ-(A³)_{q}-(A⁴)ᵣ-R¹⁶; wherein
A¹, A², A³ and A⁴ are independently of each other a group of the formula 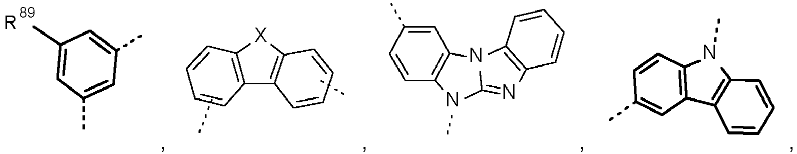 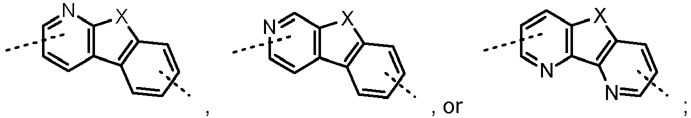 R¹⁶ is a group of the formula 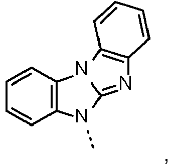 or R¹⁶ has the meaning of R^{16'}, if at least one of the groups A¹, A², A³ and A⁴ respresent a group of formula 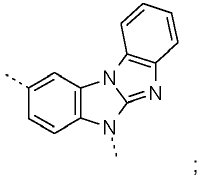 R^{16'} is H, or a group of the formula -Si(R¹²)(R¹³)(R¹⁴), 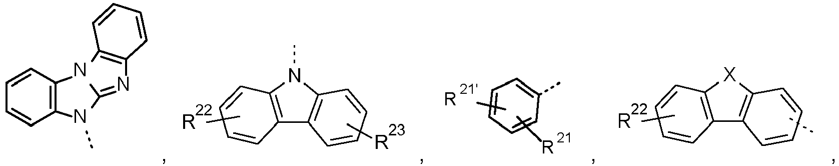 especially 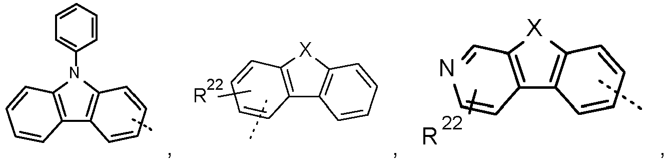 especially 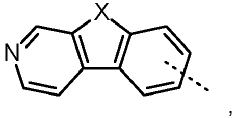 very especially 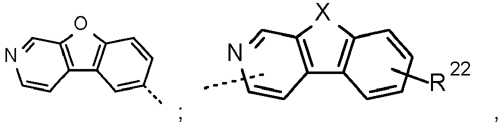 especially 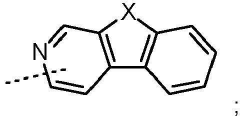 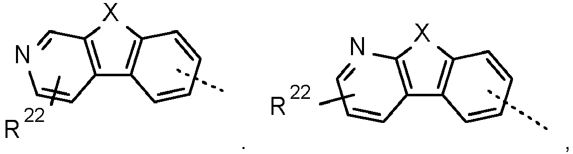 especially 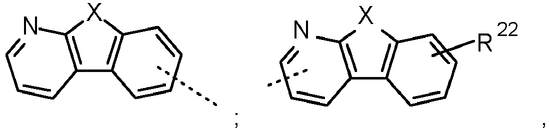 or 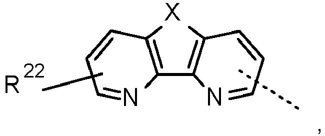 especially 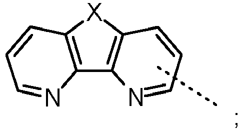 R¹², R¹³ and R¹⁴ are independently of each other a phenyl group, which can optionally be substituted by one, or more alkyl groups, especially C₁-C₁₈alkyl groups;
R²¹ and R^{21'} are independently of each other H, a phenyl group, or a C₁-C₁₈alkyl group; R²² and R²³ are independently of each other H, or a group of the formula 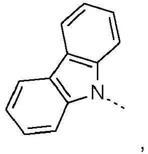 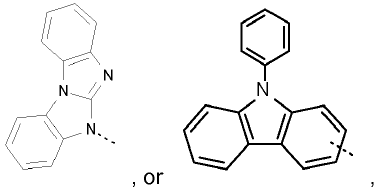 especially 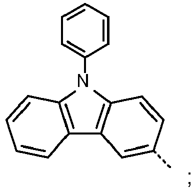 X is O, S, or NR²⁴,
R²⁴ is a C₆-C₂₄aryl group, or a C₂-C₃₀heteroaryl group, which can optionally be substituted by G, wherein G is as defined in above; and
R⁸⁹ is H, a group of formula 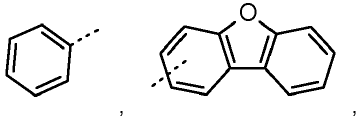 especially 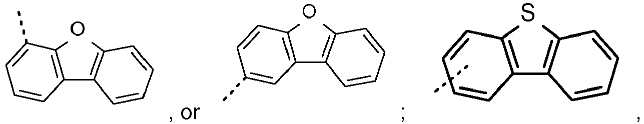 especially 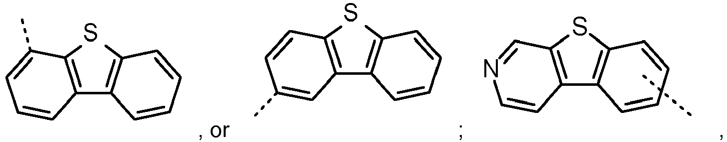 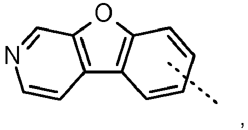 especially 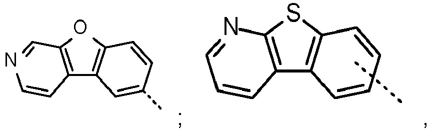 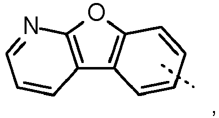 especially 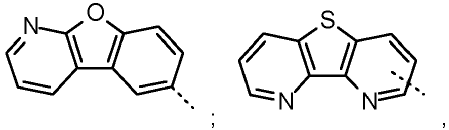 or 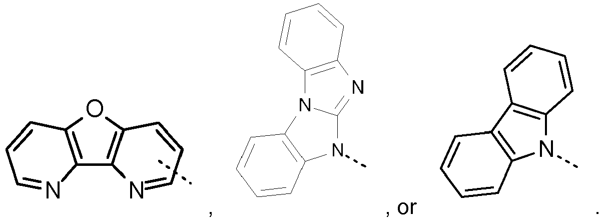 More preferred, A¹, A², A³ and A⁴ are independently of each other a group of the formula 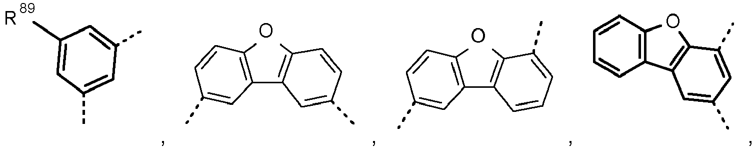 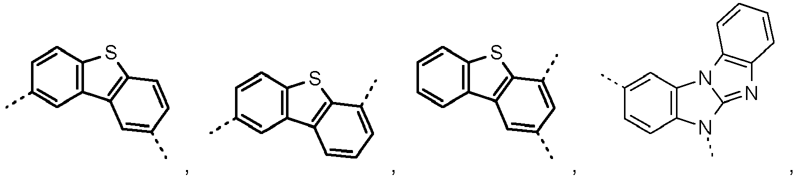 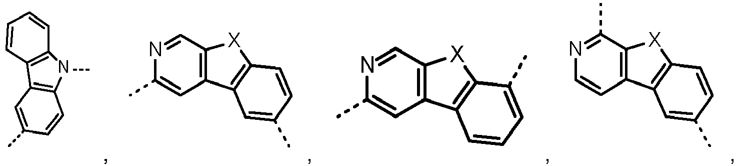 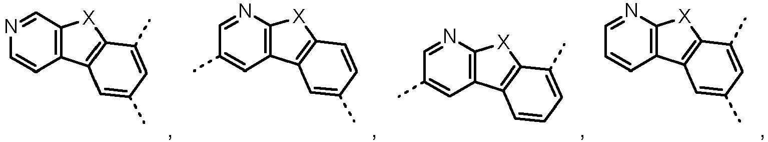 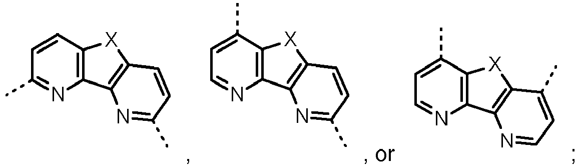 R¹⁶ is a group of the formula 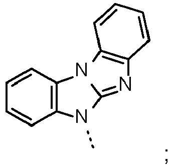 or R¹⁶ has the meaning of R^{16'}, if at least one of the groups A¹, A², A³ and A⁴ respresent a group of formula 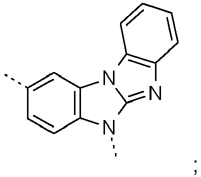 R^{16'} is H, or a group of the formula 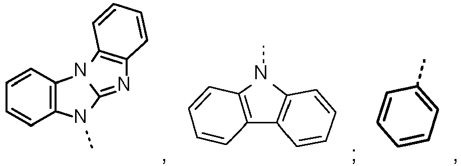 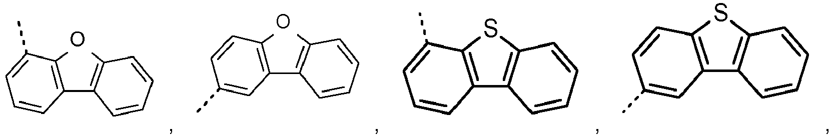 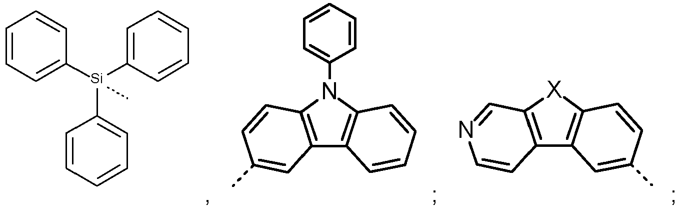 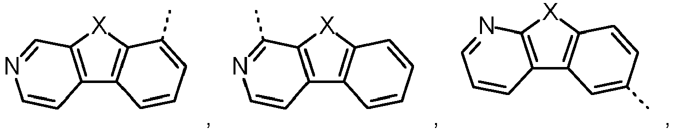 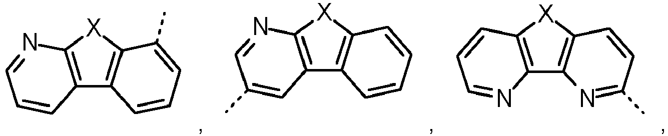 or 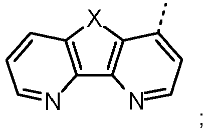 and R⁸⁹ is H, a group of formula 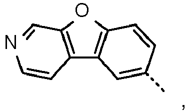 or 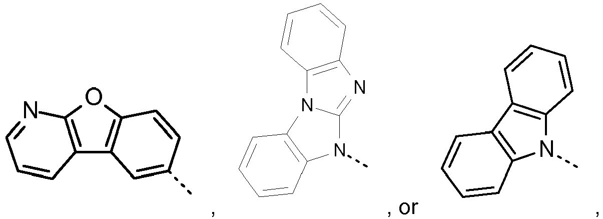 wherein X is O, S, or NR²⁴, wherein R²⁴ is 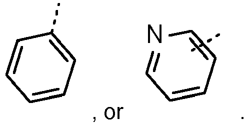 R²⁴ is preferably 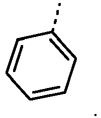 X is preferably O.

The group of the formula -(A¹)ₒ-(A²)ₚ-(A³)_{q}-(A⁴)ᵣ-R¹⁶ is preferably a group of formula

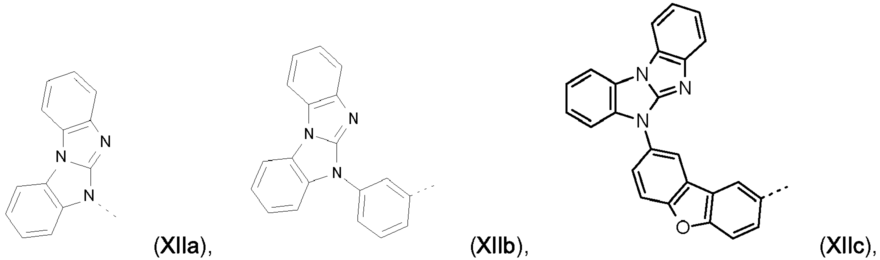

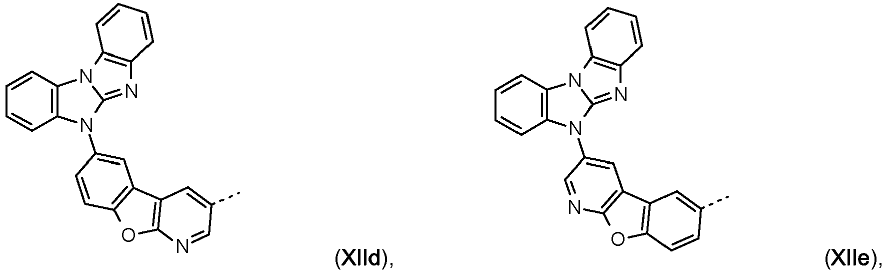

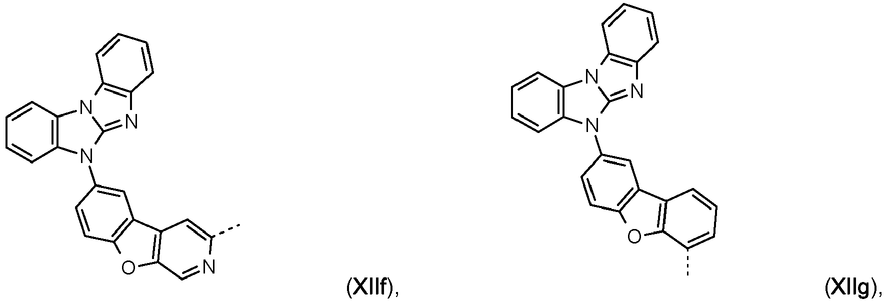

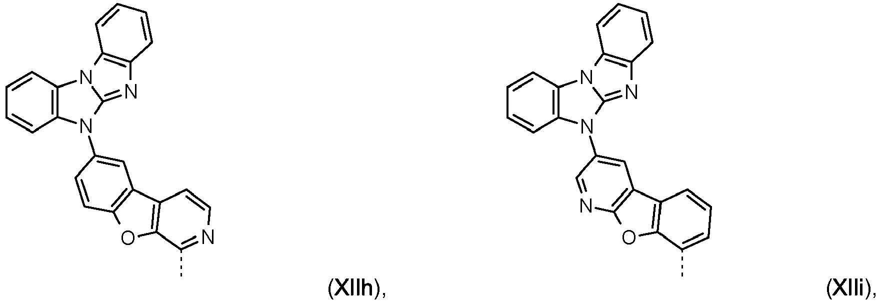

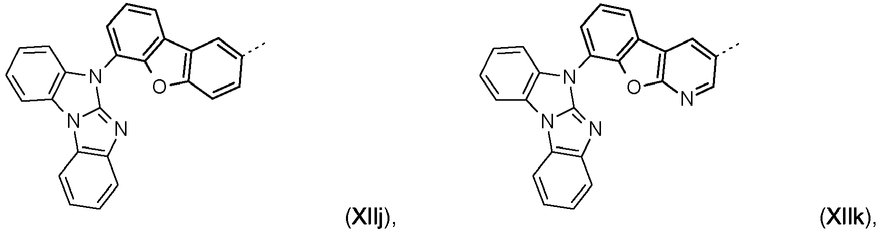

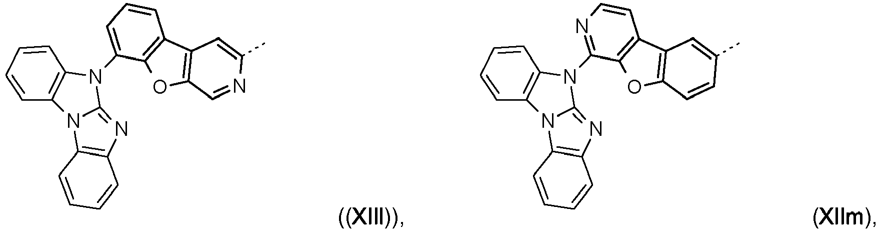

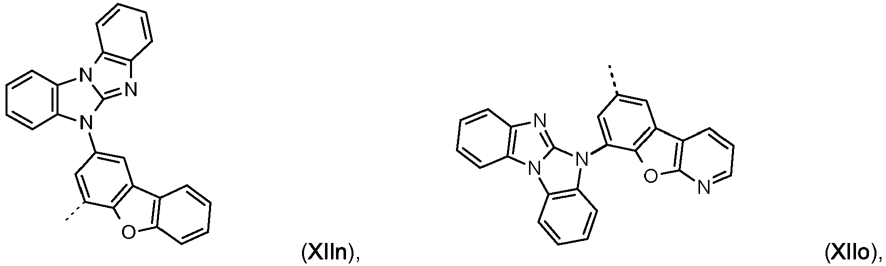

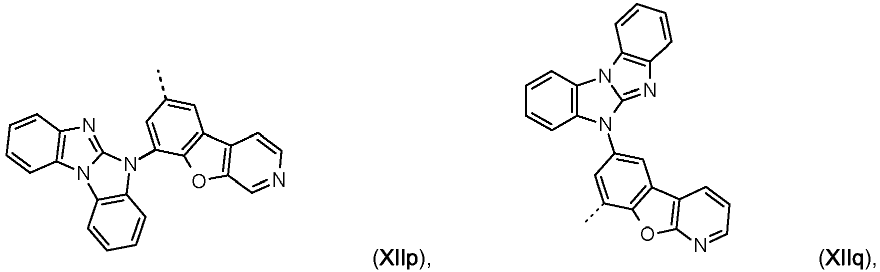

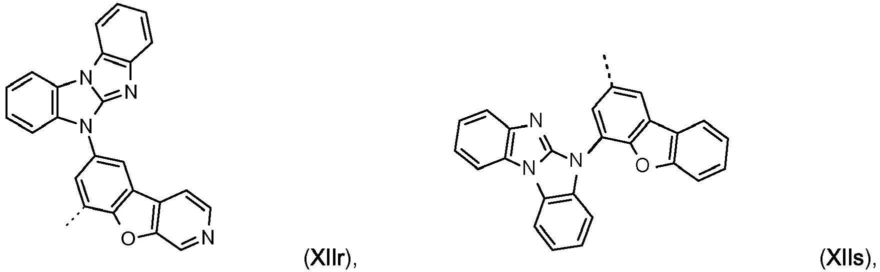

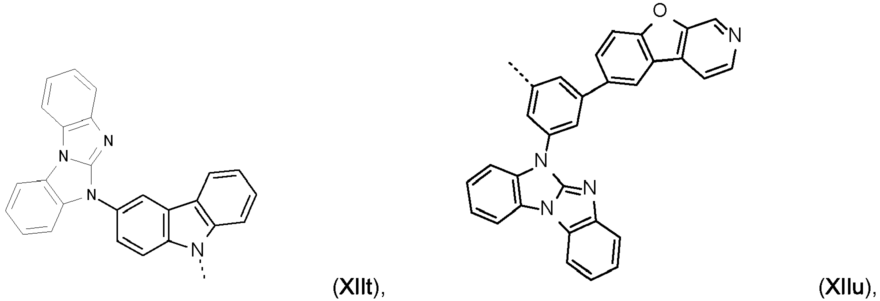

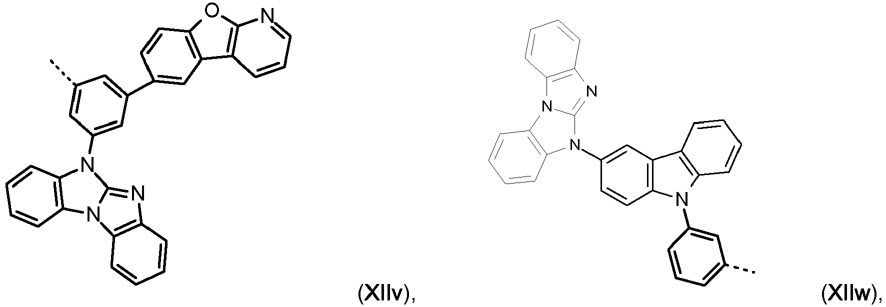

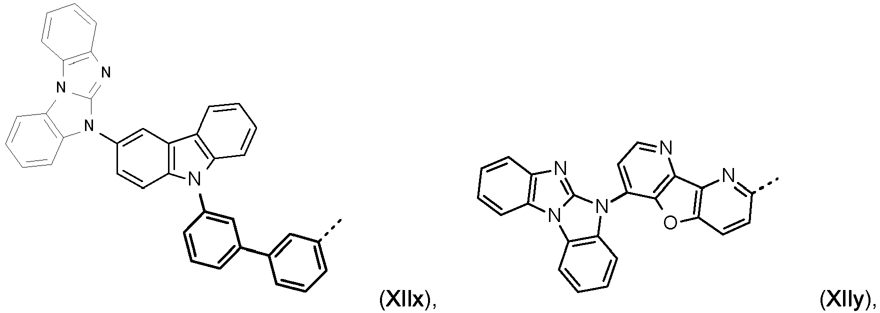

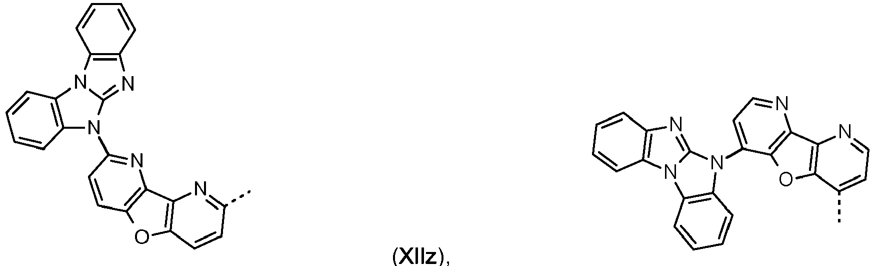

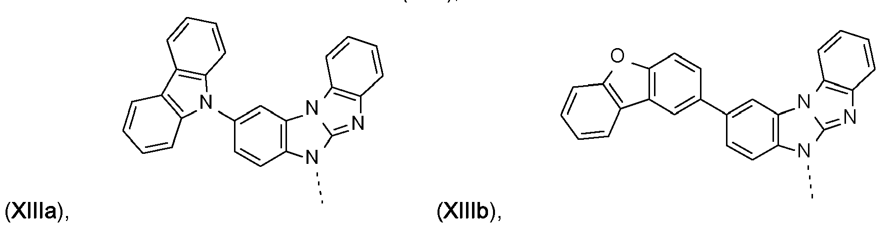

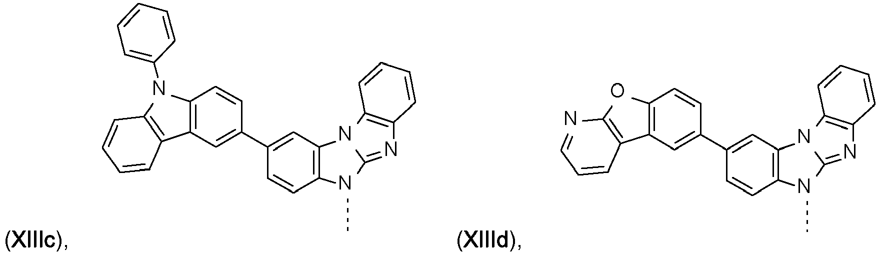

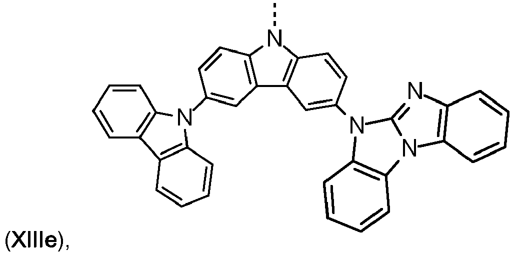

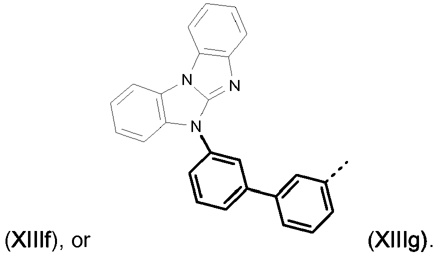

The group of the formula -(A¹)ₒ-(A²)ₚ-(A³)_{q}-(A⁴)ᵣ-R^{16'} is preferably H, a C₁-C₂₅alkyl group, or a group of formula (**XIIa**), (**XIIb**), (**XIIc**), (**XIId**), (**XIIe**), (**XIIf**), (**XIIg**), (**XIIh**), (**XIIi**), (**XIIj**), (**XIIk**), ((**XIIl**)), (**XIIm**), (**XIIn**), (**XIIo**), (**XIIp**), (**XIIq**), (**XIIr**), (**XIIs**), (**XIIt**), (**XIIu**), (**XIIv**), (**XIIw**), (**XIIx**), (**XIIy**), (**XIIz**), (**XIIIa**), (**XIIIb**), (**XIIIc**), (**XIIId**), (**XIIIe**), (**XIIIf**), or (**XIIIg**), as defined above; or a group of formula

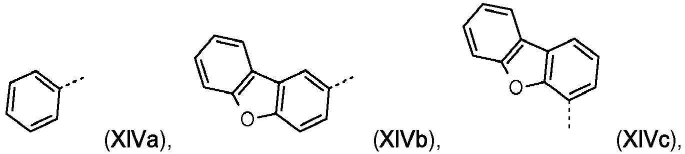

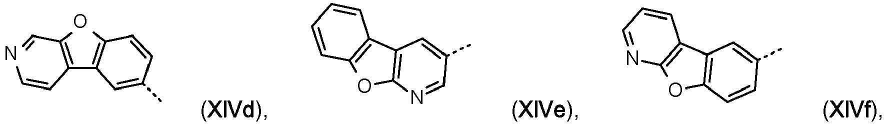

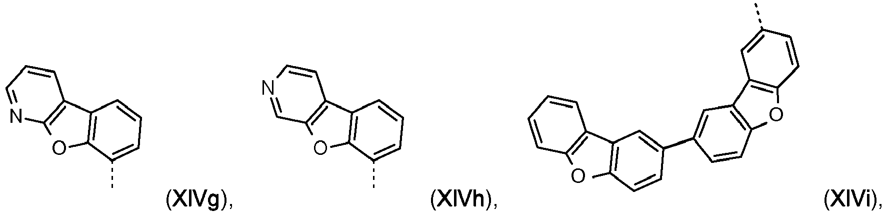

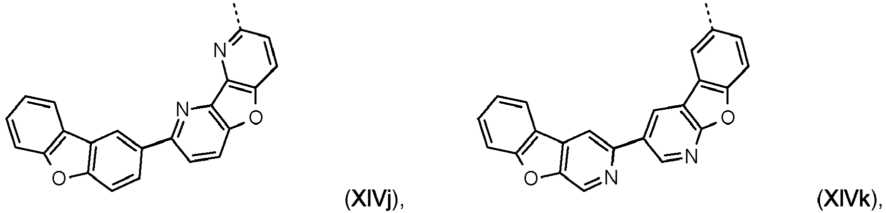

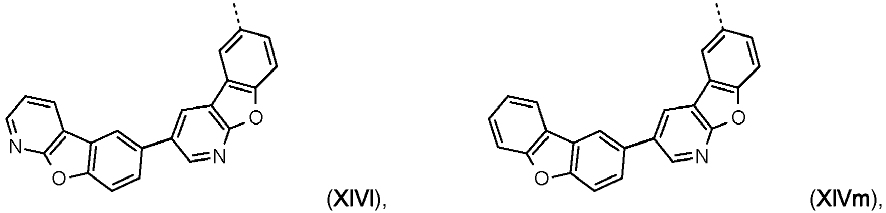

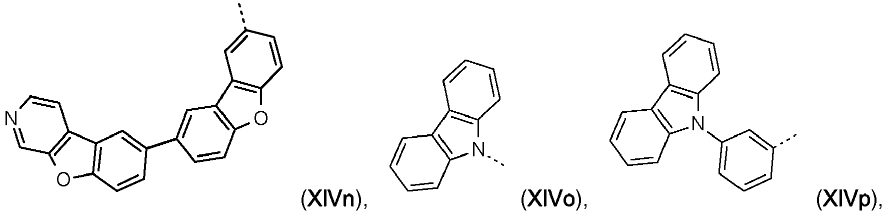

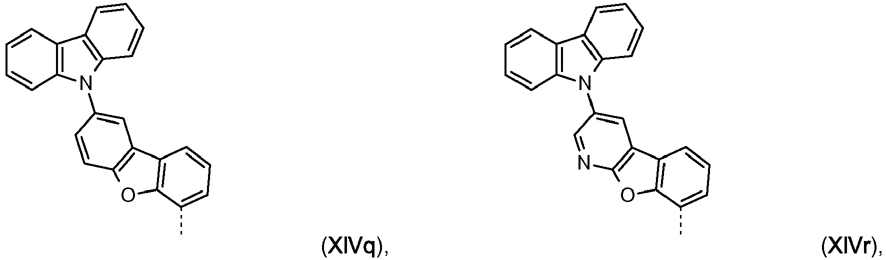

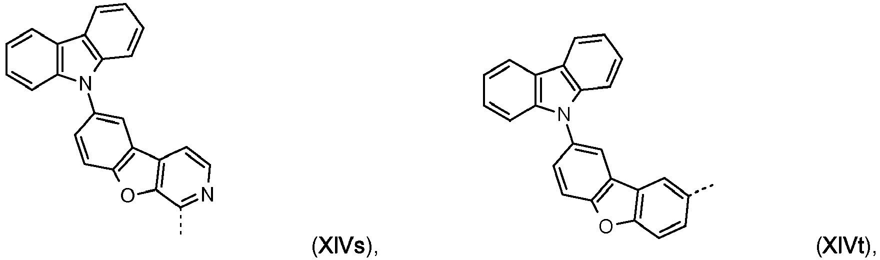

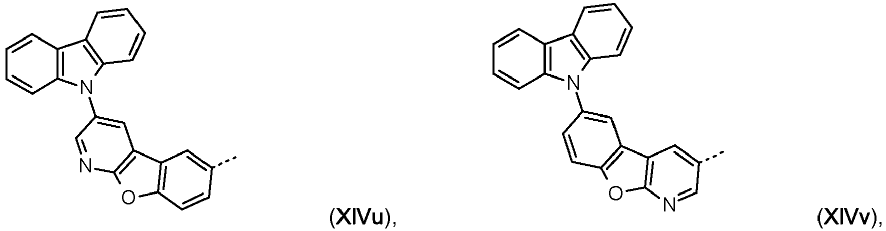

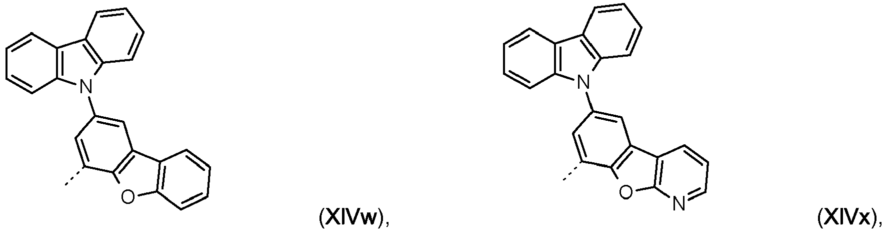

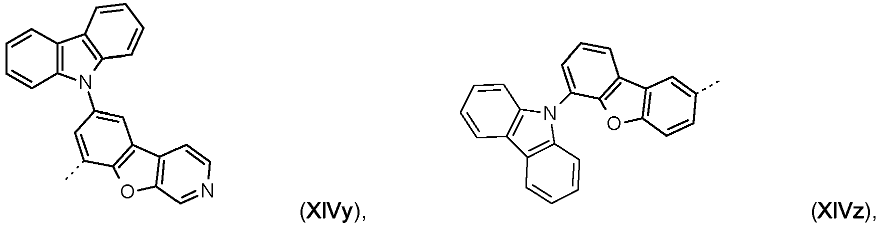

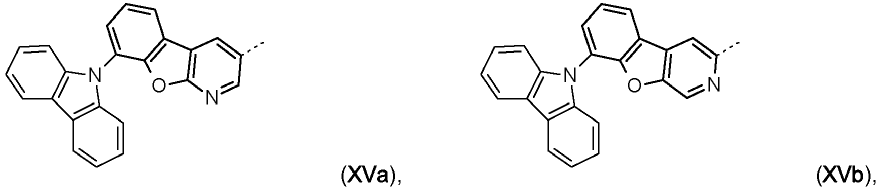

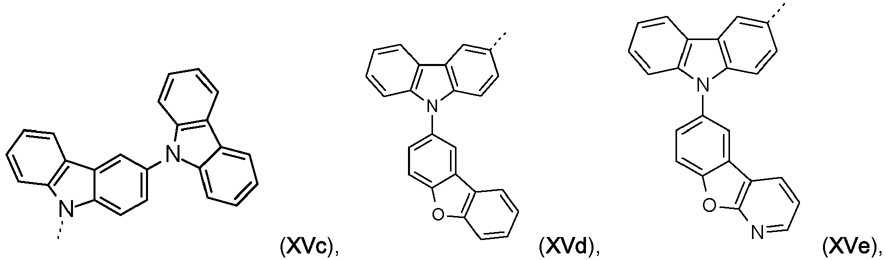

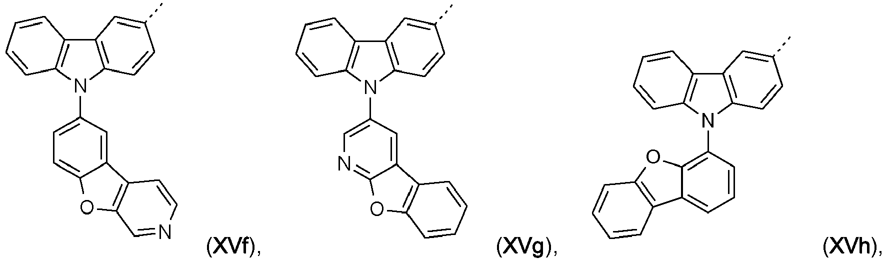

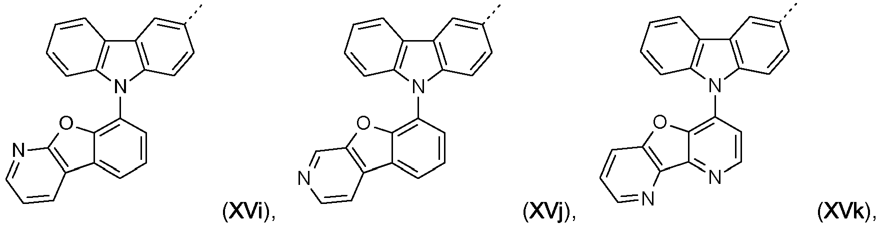

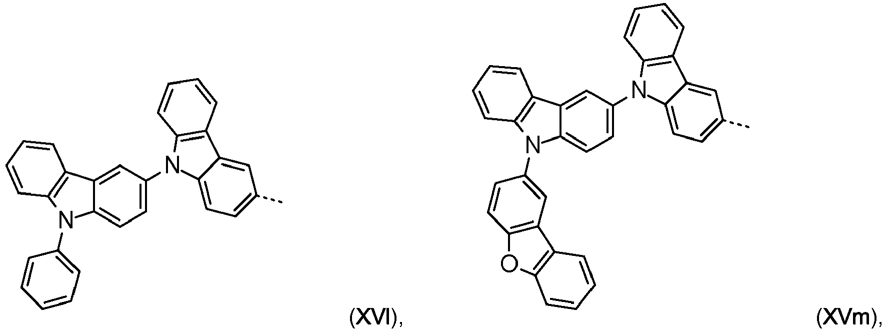

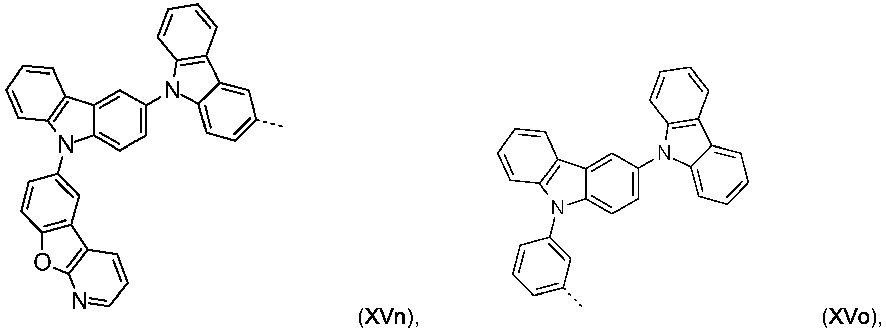

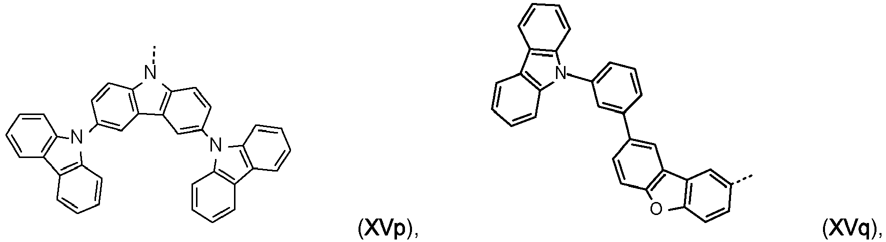

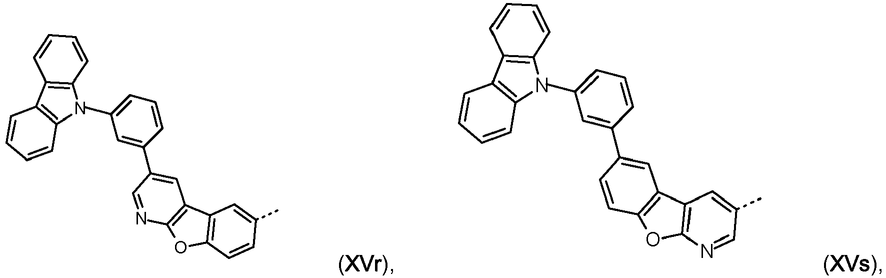

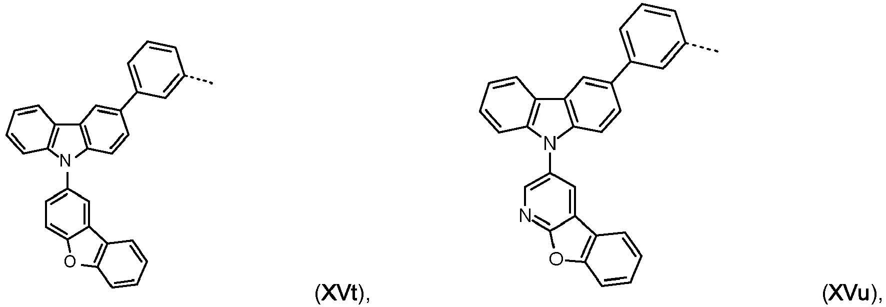

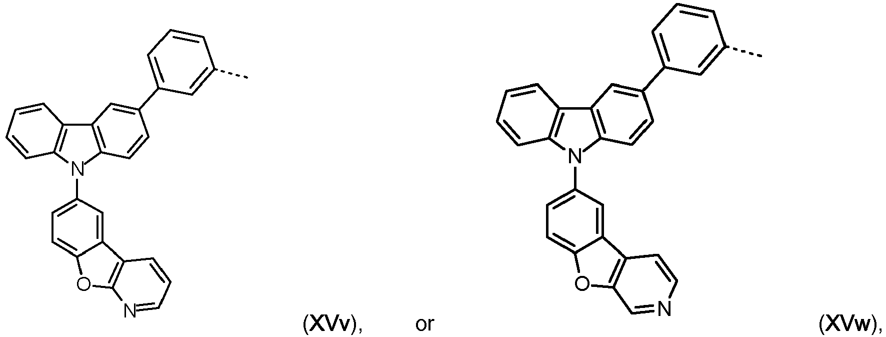

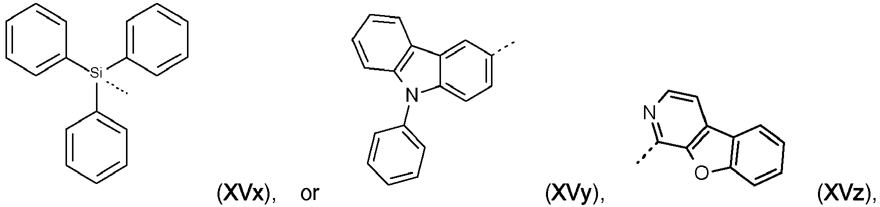

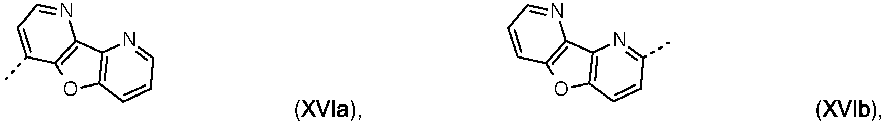

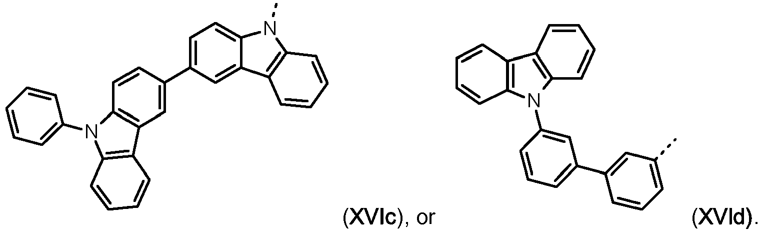

In a preferred embodiment the present invention is directed to compounds of formula (**Ia**), wherein is a group of formula (**XIIa**), (**XIIb**), (**XIIc**), (**XIId**), (**XIIe**), (**XIIf**), (**XIIg**), (**XIIh**), (**XIIi**), (**XIIj**), (**XIIk**), (**XIIl**), (**XIIm**), (**XIIn**), (**XIIo**), (**XIIp**), (**XIIq**), (**XIIr**), (**XIIs**), (**XIIt**), (**XIIu**), (**XIIv**), (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined above, and
R⁸⁷ is H, a C₁-C₂₅alkyl group, a group of formula (**XIIa**), (**XIIb**), (**XIIc**), (**XIId**), (**XIIe**), (**XIIf**), (**XIIg**), (**XIIh**), (**XIIi**), (**XIIj**), (**XIIk**), ((**XIIl**)), (**XIIm**), (**XIIn**), (**XIIo**), (**XIIp**), (**XIIq**), (**XIIr**), (**XIIs**), (**XIIt**), (**XIIu**), (**XIIv**) (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined above, or a group of formula (**XIVb**), (**XIVc**), (**XIVd**), (**XIVe**), (**XIVf**), (**XIVg**), (**XIVh**), (**XIVo**), (**XIVp**), (**XIVq**), (**XIVr**), (**XIVs**), (**XIVt**), (**XIVu**), (**XIVv**), (**XIVw**), (**XIVx**),(**XIVy**), (**XIVz**), (**XVa**), (**XVb**), (**XVc**), (**XVd**), (**XVe**), (**XVf**), (**XVg**), (**XVh**), (**XVi**), (**XVj**), (**XVk**), (**XVl**), (**XVm**), (**XVn**), (**XVo**), (**XVp**), (**XVq**), (**XVr**), (**XVs**), (**XVt**), (**XVu**), (**XVv**), (**XVw**), (**XVx**), (**XVy**), (**XVz**), (**XVIa**), (**XVIb**), (**XVIc**) or (**XVId**) as defined above; or a compound of formula (**Ia**), wherein R⁸⁷ is a group of formula (**XIIa**), (**XIIb**), (**XIIc**), (**XIId**), (**XIIe**), (**XIIf**), (**XIIg**), (**XIIh**), (**XIIi**), (**XIIj**), (**XIIk**), ((**XIIl**)), (**XIIm**), (**XIIn**), (**XIIo**), (**XIIp**), (**XIIq**), (**XIIr**), (**XIIs**), (**XIIt**), (**XIIu**), (**XIIv**), (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined above, and
is H, a C₁-C₂₅alkyl group, a group of formula (**XIIa**), (**XIIb**), (**XIIc**), (**XIId**), (**XIIe**), (**XIIf**), (**XIIg**), (**XIIh**), (**XIIi**), (**XIIj**), (**XIIk**), ((**XIIl**)), (**XIIm**), (**XIIn**), (**XIIo**), (**XIIp**), (**XIIq**), (**XIIr**), (**XIIs**), (**XIIt**), (**XIIu**), (**XIIv**) (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined above, or a group of formula (**XIVb**), (**XIVc**), (**XIVd**), (**XIVe**), (**XIVf**), (**XIVg**), (**XIVh**), (**XIVo**), (**XIVp**), (**XIVq**), (**XIVr**), (**XIVs**), (**XIVt**), (**XIVu**), (**XIVv**), (**XIVw**), (**XIVx**),(**XIVy**)**,** (**XIVz**), (**XVa**), (**XVb**), (**XVc**), (**XVd**), (**XVe**), (**XVf**), (**XVg**), (**XVh**), (**XVi**), (**XVj**), (**XVk**), (**XVl**), (**XVm**), (**XVn**), (**XVo**), (**XVp**), (**XVq**), (**XVr**), (**XVs**), (**XVt**), (**XVu**), (**XVv**), (**XVw**), (**XVx**), (**XVy**), (**XVz**), (**XVIa**), (**XVIb**), (**XVIc**) or (**XVId**) as defined above.
In said embodiment compounds of formula (**Ia**), wherein is a group of formula (**XIIa**), (**XIIb**), (**XIIc**), (**XIId**), (**XIIe**), (**XIIf**), (**XIIg**), (**XIIj**), (**XIIk**), (**XIIn**), (**XIIo**), (**XIIq**), (**XIIs**), (**XIIt**), (**XIIu**), (**XIIv**), (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined above, and
R⁸⁷ is H, a C₁-C₂₅alkyl group, a group of formula (**XIIa**), (**XIIb**), (**XIIc**), (**XIId**), (**XIIe**), (**XIIf**), (**XIIg**), (**XIIj**), (**XIIk**), (**XIIn**), (**XIIo**), (**XIIq**), (**XIIs**), (**XIIt**), (**XIIu**), (**XIIv**) (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined above, or a group of formula (**XIVb**), (**XIVc**), (**XIVd**), (**XIVe**), (**XIVf**), (**XIVg**), (**XIVo**), (**XIVp**), (**XIVq**), (**XIVr**), (**XIVt**),
(**XIVu**), (**XIVv**), (**XIVw**), (**XIVx**), (**XIVz**), (**XVa**), (**XVb**), (**XVc**), (**XVk**), (**XVl**), (**XVo**), (**XVp**), (**XVs**), (**XVw**), (**XVx**), (**XVy**), (**XVIa**), (**XVIb**), (**XVIc**) or (**XVId**) as defined above; or compounds of formula (**Ia**), wherein R⁸⁷ is a group of formula (**XIIa**), (**XIIb**), (**XIIc**)**,** (**XIId**), (**XIIe**), (**XIIf**), (**XIIg**), (**XIIj**), (**XIIk**), (**XIIn**), (**XIIo**), (**XIIq**), (**XIIs**), (**XIIt**), (**XIIu**), (**XIIv**), (**XIIw**), (**XIIx**), (**XIIy**), (**XIIz**), **(XIIa),** (**XIII**b), **(XIIIc),** (**XIIId**), (**XIIIe**), **(XIIIf)** or (**XIIIg**) as defined above, and
R⁸³ is H, a C₁-C₂₅alkyl group, a group of formula (**XIIa**), (**XIIb**), (**XIIc**), (**XIId**), (**XIIe**), (**XIIf**), (**XIIg**), (**XIIj**), (**XIIk**), (**XIIn**), (**XIIo**), (**XIIq**), (**XIIs**), (**XIIt**), (**XIIu**), (**XIIv**) (**XIIw**), (**XIIx**), (**XIIy**), (**XIIz**), (**XIIIa**), (**XIII**b), **(XIIIc),** (**XIIId**), (**XIIIe**), (**XIII**f) or (**XIIIg**) as defined above, or a group of formula (**XIVb**), (**XIVc**), (**XIVd**), (**XIVe**), (**XIVf**), (**XIVg**), (**XIVo**), (**XIVp**), (**XIVq**), (**XIVr**), (**XIVt**), (**XIVu**), (**XIVv**), (**XIVw**), (**XIVx**), (**XIVz**), (**XVa**), (**XVb**), (**XVc**), (**XVk**), (**XVl**), (**XVo**), (**XVp**), (**XVs**), (**XVw**), (**XVx**), (**XVy**), (**XVIa**), (**XVIb**), (**XVIc**) or (**XVId**) as defined above; are more preferred.

In a preferred embodiment the present invention is directed to compounds of formula (**Ib**), wherein R⁸² is a group of formula (**XIIa**), (**XIIb**), (**XIIc**), (**XIId**), (**XIIe**), (**XIIf**), (**XIIg**), (**XIIh**), (**XIIi**), (**XIIj**), (**XIIk**), ((**XIIl**)), (**XIIm**), (**XIIn**), (**XIIo**), (**XIIp**), (**XIIq**), (**XIIr**), (**XIIs**), (**XIIt**), (**XIIu**), (**XIIv**), (**XIIw**), (**XIIx**), (**XIIy**), (**XIIz**), (**XIIIa**), (**XIII**b), **(XIIIc),** (**XIIId**), (**XIIIe**), (**XIIIf**) or (**XIIIg**) as defined above, and
R⁸⁷ is H, a group of formula (**XIIa**), (**XIIb**), (**XIIc**), (**XIId**), (**XIIe**), (**XIIf**), (**XIIg**), (**XIIh**), (**XIIi**), (**XIIj**), (**XIIk**), ((**XIIl**)), (**XIIm**), (**XIIn**), (**XIIo**), (**XIIp**), (**XIIq**), (**XIIr**), (**XIIs**), (**XIIt**), (**XIIu**), (**XIIv**) (**XIIw**), (**XIIx**), (**XIIy**), (**XIIz**), (**XIIIa**), (**XIII**b), (**XIIIc**), (**XIIId**), (**XIIIe**), (**XIIIf**) or (**XIIIg**) as defined above, or a group of formula (**XIVb**), (**XIVc**), (**XIVd**), (**XIVe**), (**XIVf**), (**XIVg**), (**XIVh**), (**XIVo**), (**XIVp**), (**XIVq**), (**XIVr**), (**XIVs**), (**XIVt**), **(XIVu),** (**XIVv**), **(XIVw),** (**XIVx**),(**XIVy**), (**XIVz**), (**XVa**), (**XVb**), (**XVc**), (**XVd**), (**XVe**), (**XVf**), (**XVg**), (**XVh**), (**XVi**), (**XVj**), (**XVk**), (**XVl**), (**XVm**), (**XVn**), (**XVo**), (**XVp**), (**XVq**), (**XVr**), (**XVs**), (**XVt**), (**XVu**), (**XVv**), (**XVw**), (**XVx**), (**XVy**), (**XVz**), **(XVIa),** (**XVIb**), (**XVIc**) or (**XVId**) as defined above; or
compounds of formula (**Ib**), wherein R⁸⁷ is a group of formula (**XIIa**), (**XIIb**), (**XIIc**), (**XIId**), (**XIIe**), (**XIIf**), (**XIIg**), (**XIIh**), (**XIIi**), (**XIIj**), (**XIIk**), ((**XIIl**)), (**XIIm**), (**XIIn**), (**XIIo**), (**XIIp**), (**XIIq**), (**XIIr**), (**XIIs**), (**XIIt**), (**XIIu**), (**XIIv**), (**XIIw**), (**XIIx**), (**XIIy**), (**XIIz**), (**XIIIa**), (**XIIIb**), **(XIIIc),** (**XIIId**), (**XIIIe**), (**XIII**f) or (**XIIIg**) as defined above, and
R⁸² is H, a group of formula (**XIIa**), (**XIIb**), (**XIIc**), (**XIId**), (**XIIe**), (**XIIf**), (**XIIg**), (**XIIh**), (**XIIi**), (**XIIj**), (**XIIk**), ((**XIIl**)), (**XIIm**), (**XIIn**), (**XIIo**), (**XIIp**), (**XIIq**), (**XIIr**), (**XIIs**), (**XIIt**), (**XIIu**), (**XIIv**) (**XIIw**), (**XIIx**), (**XIIy**), (**XIIz**), (**XIIIa**), (**XIIIb**), **(XIIIc),** (**XIIId**), (**XIIIe**), (**XIIIf**) or (**XIIIg**) as defined above, or a group of formula (**XIVb**), (**XIVc**), (**XIVd**), (**XIVe**), (**XIVf**), (**XIVg**), (**XIVh**), (**XIVo**), (**XIVp**), (**XIVq**), (**XIVr**), (**XIVs**), (**XIVt**), **(XIVu),** (**XIVv**), **(XIVw),** (**XIVx**),(**XIVy**), (**XIVz**), (**XVa**), (**XVb**), (**XVc**), (**XVd**), (**XVe**), (**XVf**), (**XVg**), (**XVh**), (**XVi**), (**XVj**), (**XVk**), (**XVl**), (**XVm**), (**XVn**), (**XVo**), (**XVp**), (**XVq**), (**XVr**), (**XVs**), (**XVt**), (**XVu**), (**XVv**), (**XVw**), (**XVx**), (**XVy**), (**XVz**), **(XVIa), (XVIb), (XVIc)** or **(XVId)** as defined above.
In said embodiment compounds of formula (**Ib**), wherein R⁸² is a group of formula (**XIIa**), (**XIIb**), (**XIIc**, (**XIId**), (**XIIe**), (**XIIf**), (**XIIg**), (**XIIj**), (**XIIk**), (**XIIn**), (**XIIo**), (**XIIq**), (**XIIs**), (**XIIt**), (**XIIu**), (**XIIv**), (**XIIw**), (**XIIx**), (**XIIy**), (**XIIz**), (**XIIIa**), (**XIIIb**), **(XIIIc),** (**XIIId**), (**XIIIe**), (**XIIIf**) or (**XIIIg**) as defined above, and
R⁸⁷ is H, a group of formula (**XIIa**), (**XIIb**), (**XIIc**), (**XIId**), (**XIIe**), (**XIIf**), (**XIIg**), (**XIIj**), (**XIIk**), (**XIIn**), (**XIIo**), (**XIIq**), (**XIIs**), (**XIIt**), (**XIIu**), (**XIIv**) (**XIIw**), (**XIIx**), (**XIIy**), (**XIIz**), (**XIIIa**), (**XIIIb**), (**XIIIc**), (**XIIId**), (**XIIIe**), (**XIII**f) or (**XIIIg**) as defined above, or a group of formula (**XIVb**), (**XIVc**), (**XIVd**), (**XIVe**), (**XIVf**), (**XIVg**), (**XIVo**), (**XIVp**), (**XIVq**), (**XIVr**), (**XIVt**), **(XIVu),** (**XIVv**), (**XIVw**), (**XIVx**), (**XIVz**), (**XVa**), (**XVb**), (**XVc**), (**XVk**), (**XVl**), (**XVo**), (**XVp**), (**XVs**), (**XVw**), (**XVx**), (**XVy**), (**XVIa**), (**XVIb**), (**XVIc**) or (**XVId**) as defined above; or
compounds of formula (**Ib**), wherein R⁸⁷ is a group of formula (**XIIa**), (**XIIb**), (**XIIc**), (**XIId**), (**XIIe**), (**XIIf**), (**XIIg**), (**XIIj**), (**XIIk**), (**XIIn**), (**XIIo**), (**XIIq**), (**XIIs**), (**XIIt**), (**XIIu**), (**XIIv**), (**XIIw**), (**XIIx**), (**XIIy**), (**XIIz**), (**XIIIa**), (**XIIIb**), **(XIIIc),** (**XIIId**), (**XIIIe**), (**XIIIf**) or (**XIIIg**) as defined above, and
R⁸² is H, a group of formula (**XIIa**), (**XIIb**), (**XIIc**), (**XIId**), (**XIIe**), (**XIIf**), (**XIIg**), (**XIIj**), (**XIIk**), (**XIIn**), (**XIIo**), (**XIIq**), (**XIIs**), (**XIIt**), (**XIIu**), (**XIIv**) (**XIIw**), (**XIIx**), (**XIIy**), (**XIIz**), (**XIIIa**), (**XIII**b), **(XIIIc),** (**XIIId**), (**XIIIe**), (**XIII**f) or (**XIIIg**) as defined above, or a group of formula (**XIVb**), (**XIVc**), (**XIVd**), (**XIVe**), (**XIVf**), (**XIVg**), (**XIVo**), (**XIVp**), (**XIVq**), (**XIVr**), (**XIVt**), **(XIVu),** (**XIVv**), **(XIVw), (XIVx), (XIVz),** (**XVa**), (**XVb**), (**XVc**), (**XVk**), (**XVl**), (**XVo**), (**XVp**), (**XVs**), (**XVw**), (**XVx**), (**XVy**), (**XVIa**), (**XVIb**), (**XVIc**) or (**XVId**) as defined above; are more preferred.

In a preferred embodiment the present invention is directed to compounds of formula (Ic), wherein R⁸⁵ is a group of formula (**XIIa**), (**XIIb**), (**XIIc**), (**XIId**), (**XIIe**), (**XIIf**), (**XIIg**), (**XIIh**), (**XIIi**), (**XIIj**), (**XIIk**), ((**XIII**)), (**XIIm**), (**XIIn**), (**XIIo**), (**XIIp**), (**XIIq**), (**XIIr**), (**XIIs**), (**XIIt**), (**XIIu**), (**XIIv**), (**XIIw**), (**XIIx**), (**XIIy**), (**XIIz**), (**XIIIa**), (**XIIIb**), (**XIIIc**)**,** (**XIIId**), (**XIIIe**), (**XIIIf**) or (**XIIIg**) as defined above, and
R⁸⁷ is H, a C₁-C₂₅alkyl group, a group of formula (**XIIa**), (**XIIb**), (**XIIc**), (**XIId**), (**XIIe**), (**XIIf**), (**XIIg**), (**XIIh**), (**XIIi**), (**XIIj**), (**XIIk**), ((**XIII**)), (**XIIm**), (**XIIn**), (**XIIo**), (**XIIp**), (**XI**I**q**), (**XIIr**), (**XIIs**), (**XIIt**), (**XIIu**), (**XIIv**) (**XIIw**), (**XIIx**), (**XIIy**), (**XIIz**), (**XIIIa**), (**XIIIb**), **(XIIIc),** (**XIIId**), (**XIIIe**), (**XIIIf**) or (**XIIIg**) as defined above, or a group of formula (**XIVb**), (**XIVc**), (**XIVd**), (**XIVe**), (**XIVf**), (**XIVg**), (**XIVh**), (**XIVo**), (**XIVp**), (**XIVq**), (**XIVr**), (**XIVs**), (**XIVt**), **(XIVu),** (**XIVv**), (**XIVw**), (**XIVx**),(**XIVy**), (**XIVz**), (**XVa**), (**XVb**), (**XVc**), (**XVd**), (**XVe**), (**XVf**), (**XVg**), (**XVh**), (**XVi**), (**XVj**), (**XVk**), (**XVl**), (**XVm**), (**XVn**), (**XVo**), (**XVp**), (**XVq**), (**XVr**), (**XVs**), (**XVt**), (**XVu**), (**XVv**), (**XVw**), (**XVx**), (**XVy**), (**XVz**), (**XVIa**), (**XVIb**), (**XVIc**) or (**XVId**) as defined above; or compounds of formula (Ic), wherein R⁸⁷ is a group of formula (**XIIa**), (**XIIb**), (**XIIc**), (**XIId**), (**XIIe**), (**XIIf**), (**XIIg**), (**XIIh**), (**XIIi**), (**XIIj**), (**XIIk**), ((**XIIl**)), (**XIIm**), (**XIIn**), (**XIIo**), (**XIIp**), (**XIIq**), (**XIIr**), (**XIIs**), (**XIIt**), (**XIIu**), (**XIIv**), (**XIIw**), (**XIIx**), (**XIIy**), (**XIIz**), (**XIIIa**), (**XIII**b), **(XIIIc),** (**XIIId**), (**XIIIe**), (**XIII**f) or (**XIIIg**) as defined above; and
R⁸⁵ is H, a C₁-C₂₅alkyl group, a group of formula (**XIIa**), (**XIIb**), (**XIIc**), (**XIId**), (**XIIe**), (**XIIf**), (**XIIg**), (**XIIh**), (**XIIi**), (**XIIj**), (**XIIk**), ((**XIIl**)), (**XIIm**), (**XIIn**), (**XIIo**), (**XIIp**), (**XIIq**), (**XIIr**), (**XIIs**), (**XIIt**), (**XIIu**), (**XIIv**) (**XIIw**), (**XIIx**), (**XIIy**), (**XIIz**), (**XIIIa**), (**XIIIb**), **(XIIIc),** (**XIIId**), (**XIIIe**), (**XIIIf**) or (**XIIIg**) as defined above, or a group of formula (**XIVb**), (**XIVc**), (**XIVd**), (**XIVe**), (**XIVf**), (**XIVg**), (**XIVh**), (**XIVo**), (**XIVp**), (**XIVq**), (**XIVr**), (**XIVs**), (**XIVt**), (**XIVu**), (**XIVv**), (**XIVw**), (**XIVx**),(**XIVy**), (**XIVz**), (**XVa**), (**XVb**), (**XVc**), (**XVd**), (**XVe**), (**XVf**), (**XVg**), (**XVh**), (**XVi**), (**XVj**), (**XVk**), (**XVl**), (**XVm**), (**XVn**), (**XVo**), (**XVp**), (**XVq**), (**XVr**), (**XVs**), (**XVt**), (**XVu**), (**XVv**), (**XVw**), (**XVx**), (**XVy**), (**XVz**), **(XVIa), (XVIb), (XVIc)** or **(XVId)** as defined above.
In said embodiment compounds of formula (**Ic**), wherein R⁸⁵ is a group of formula (**XIIa**), (**XIIb**), (**XIIc**), (**XIId**), (**XIIe**), (**XIIf**), (**XIIg**), (**XIIj**), (**XIIk**), (**XIIn**), (**XIIo**), (**XIIq**), (**XIIs**), (**XIIt**), (**XIIu**), (**XIIv**), (**XIIw**), (**XIIx**), (**XIIy**), (**XIIz**), (**XIIIa**), (**XIIIb**), **(XIIIc),** (**XIIId**), (**XIIIe**), (**XIIIf**) or (**XIIIg**) as defined above, and
R⁸⁷ is H, a C₁-C₂₅alkyl group, a group of formula (**XIIa**), (**XIIb**), (**XIIc**), (**XIId**), (**XIIe**), (**XIIf**), (**XIIg**), (**XIIj**), (**XIIk**), (**XIIn**), (**XIIo**), (**XIIq**), (**XIIs**), (**XIIt**), (**XIIu**), (**XIIv**) (**XIIw**), (**XIIx**), (**XIIy**), (**XIIz**), (**XIIIa**), (**XIIIb**), (**XIIIc**), (**XIIId**), (**XIIIe**), (**XIII**f) or (**XIIIg**) as defined above, or a group of formula (**XIVb**), (**XIVc**), (**XIVd**), (**XIVe**), (**XIVf**), (**XIVg**), (**XIVo**), (**XIVp**), (**XIVq**), (**XIVr**), (**XIVt**), **(XIVu),** (**XIVv**), **(XIVw), (XIVx), (XIVz),** (**XVa**), (**XVb**), (**XVc**), (**XVk**), (**XVl**), (**XVo**), (**XVp**), (**XVs**), (**XVw**), (**XVx**), (**XVy**), **(XVIa), (XVIb), (XVIc)** or **(XVId)** as defined above; or compounds of formula (**Ic**), wherein R⁸⁷ is a group of formula (**XIIa**), (**XIIb**), (**XIIc**), (**XIId**), (**XIIe**), (**XIIf**), (**XIIg**), (**XIIj**), (**XIIk**), (**XIIn**), (**XIIo**), (**XIIq**), (**XIIs**), (**XIIt**), (**XIIu**), (**XIIv**), (**XIIw**), (**XIIx**), (**XIIy**), (**XIIz**), (**XIIIa**), (**XIII**b), **(XIIIc),** (**XIIId**), (**XIIIe**), (**XIII**f) or (**XIIIg**) as defined above, and
R⁸⁵ is H, a C₁-C₂₅alkyl group, a group of formula (**XIIa**), (**XIIb**), (**XIIc**), (**XIId**), (**XIIe**), (**XIIf**), (**XIIg**), (**XIIj**), (**XIIk**), (**XIIn**), (**XIIo**), (**XIIq**), (**XIIs**), (**XIIt**), (**XIIu**), (**XIIv**) (**XIIw**), (**XIIx**), (**XIIy**), (**XIIz**), (**XIIIa**), (**XIII**b), **(XIIIc),** (**XIIId**), (**XIIIe**), (**XIII**f) or (**XIIIg**) as defined above, or a group of formula (**XIVb**), (**XIVc**), (**XIVd**), (**XIVe**), (**XIVf**), (**XIVg**), (**XIVo**), (**XIVp**), (**XIVq**), (**XIVr**), (**XIVt**), **(XIVu),** (**XIVv**), **(XIVw), (XIVx), (XIVz),** (**XVa**), (**XVb**), (**XVc**), (**XVk**), (**XVl**), (**XVo**), (**XVp**), (**XVs**), (**XVw**), (**XVx**), (**XVy**), **(XVIa), (XVIb), (XVIc)** or **(XVId)** as defined above; are more preferred.

In a preferred embodiment the present invention is directed to compounds of formula (**Id**), wherein R⁸¹ is a group of formula (**XIIa**), (**XIIb**), (**XIIc**), (**XIId**), (**XIIe**), (**XIIf**), (**XIIg**), (**XIIh**), (**XIIi**), (**XIIj**), (**XIIk**), ((**XIIl**)), (**XIIm**), (**XIIn**), (**XIIo**), (**XIIp**), (**XIIq**), (**XIIr**), (**XIIs**), (**XIIt**), (**XIIu**), (**XIIv**), (**XIIw**), (**XIIx**), (**XIIy**), (**XIIz**), (**XIIIa**), (**XIIIb**), **(XIIIc),** (**XIIId**), (**XIIIe**), (**XIIIf**) or (**XIIIg**) as defined above, and
R⁸⁵ is H, a group of formula (**XIIa**), (**XIIb**), (**XIIc**), (**XIId**), (**XIIe**), (**XIIf**), (**XIIg**), (**XIIh**), (**XIIi**), (**XIIj**), (**XIIk**), ((**XIIl**)), (**XIIm**), (**XIIn**), (**XIIo**), (**XIIp**), (**XIIq**), (**XIIr**), (**XIIs**), (**XIIt**), (**XIIu**), (**XIIv**) (**XIIw**), (**XIIx**), (**XIIy**), (**XIIz**), (**XIIIa**), (**XIII**b), **(XIIIc),** (**XIIId**), (**XIIIe**), (**XIII**f) or (**XIIIg**) as defined above; or a group of formula (**XIVb**), (**XIVc**), (**XIVd**), (**XIVe**), (**XIVf**), (**XIVg**), (**XIVh**), (**XIVo**), (**XIVp**), (**XIVq**), (**XIVr**), (**XIVs**), (**XIVt**), **(XIVu),** (**XIVv**), **(XIVw),** (**XIVx**),(**XIVy**), (**XIVz**), (**XVa**), (**XVb**), (**XVc**), (**XVd**), (**XVe**), (**XVf**), (**XVg**), (**XVh**), (**XVi**), (**XVj**), (**XVk**), (**XVl**), (**XVm**), (**XVn**), (**XVo**), (**XVp**), (**XVq**), (**XVr**), (**XVs**), (**XVt**), (**XVu**), (**XVv**), (**XVw**), (**XVx**), (**XVy**), (**XVz**), **(XVIa), (XVIb), (XVIc)** or **(XVId)** as defined above.
In said embodiment compounds of formula (Id), wherein R⁸¹ is a group of formula (**XIIa**), (**XIIb**), (**XIIc**), (**XIId**), (**XIIe**), (**XIIf**), (**XIIg**), (**XIIj**), (**XIIk**), (**XIIn**), (**XIIo**), (**XIIq**), (**XIIs**), (**XIIt**), (**XIIu**), (**XIIv**), (**XIIw**), (**XIIx**), (**XIIy**), (**XIIz**), (**XIIIa**), (**XIIIb**), **(XIIIc),** (**XIIId**), (**XIIIe**), (**XIIIf**) or (**XIIIg**) as defined above, and
R⁸⁵ is H, a group of formula (**XIIa**), (**XIIb**), (**XIIc**), (**XIId**), (**XIIe**), (**XIIf**), (**XIIg**), (**XIIj**), (**XIIk**), (**XIIn**), (**XIIo**), (**XIIq**), (**XIIs**), (**XIIt**), (**XIIu**), (**XIIv**) (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined above, or a group of formula (**XIVb**), (**XIVc**), (**XIVd**), (**XIVe**), (**XIVf**), (**XIVg**), (**XIVo**), (**XIVp**), (**XIVq**), (**XIVr**), (**XIVt**), (**XIVu**), (**XIVv**), (**XIVw**), (**XIVx**), (**XIVz**), (**XVa**), (**XVb**), (**XVc**), (**XVk**), (**XVl**), (**XVo**), (**XVp**), (**XVs**), (**XVw**), (**XVx**), (**XVy**), (**XVIa**), (**XVIb**), (**XVIc**) or (**XVId**) as defined above.

In a preferred embodiment the present invention is directed to compounds of formula (**Ie**), wherein R⁸³ is a group of formula (**XIIa**), (**XIIb**), (**XIIc**), (**XIId**), (**XIIe**), (**XIIf**), (**XIIg**), (**XIIh**), (**XIIi**), (**XIIj**), (**XIIk**), ((**XIII**)), (**XIIm**), (**XIIn**), (**XIIo**), (**XIIp**), (**XIIq**), (**XIIr**), (**XIIs**), (**XIIt**), (**XIIu**), (**XIIv**), (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined above, and
R⁸⁷ is H, a C₁-C₂₅alkyl group, a group of formula (**XIIa**), (**XIIb**), (**XIIc**), (**XIId**), (**XIIe**), (**XIIf**), (**XIIg**), (**XIIh**), (**XIIi**), (**XIIj**), (**XIIk**), ((**XIIl**)), (**XIIm**), (**XIIn**), (**XIIo**), (**XIIp**), (**XIIq**), (**XIIr**), (**XIIs**), (**XIIt**), (**XIIu**), (**XIIv**) (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined above, or a group of formula (**XIVb**), (**XIVc**), (**XIVd**), (**XIVe**), (**XIVf**), (**XIVg**), (**XIVh**), (**XIVo**), (**XIVp**), (**XIVq**), (**XIVr**), (**XIVs**), (**XIVt**), **(XIVu),** (**XIVv**), (**XIVw**), (**XIVx**),(**XIVy**), (**XIVz**), (**XVa**), (**XVb**), (**XVc**), (**XVd**), (**XVe**), (**XVf**), (**XVg**), (**XVh**), (**XVi**), (**XVj**), (**XVk**), (**XVl**), (**XVm**), (**XVn**), (**XVo**), (**XVp**), (**XVq**), (**XVr**), (**XVs**), (**XVt**), (**XVu**), (**XVv**), (**XVw**), (**XVx**), (**XVy**), (**XVz**), (**XVIa**), (**XVIb**), (**XVIc**) or (**XVId**) as defined above; or
compounds of formula (**Ie**), wherein R⁸⁷ is a group of formula (**XIIa**), (**XIIb**), (**XIIc**), (**XIId**), (**XIIe**), (**XIIf**), (**XIIg**), (**XIIh**), (**XIIi**), (**XIIj**), (**XIIk**), ((**XIII**)), (**XIIm**), (**XIIn**), (**XIIo**), (**XIIp**), (**XIIq**), (**XIIr**), (**XIIs**), (**XIIt**), (**XIIu**), (**XIIv**), (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined above, and
R⁸³ is H, a C₁-C₂₅alkyl group, a group of formula (**XIIa**), (**XIIb**), (**XIIc**), (**XIId**), (**XIIe**), (**XIIf**), (**XIIg**), (**XIIh**), (**XIIi**), (**XIIj**), (**XIIk**), ((**XIIl**)), (**XIIm**), (**XIIn**), (**XIIo**), (**XIIp**), (**XIIq**), (**XIIr**), (**XIIs**), (**XIIt**), (**XIIu**), (**XIIv**) (**XIIw**), (**XIIx**), (**XIIy**), (**XIIz**), (**XIIIa**), (**XIIIb**), **(XIIIc),** (**XIIId**), (**XIIIe**), (**XIIIf**) or (**XIIIg**) as defined above, or a group of formula (**XIVb**), (**XIVc**), (**XIVd**), (**XIVe**), (**XIVf**), (**XIVg**), (**XIVh**), (**XIVo**), (**XIVp**), (**XIVq**), (**XIVr**), (**XIVs**), (**XIVt**), **(XIVu),** (**XIVv**), (**XIVw**), (**XIVx**),(**XIVy**), (**XIVz**), (**XVa**), (**XVb**), (**XVc**), (**XVd**), (**XVe**), (**XVf**), (**XVg**), (**XVh**), (**XVi**), (**XVj**), (**XVk**), (**XVl**), (**XVm**), (**XVn**), (**XVo**), (**XVp**), (**XVq**), (**XVr**), (**XVs**), (**XVt**), (**XVu**), (**XVv**), (**XVw**), (**XVx**), (**XVy**), (**XVz**), (**XVIa**), (**XVIb**), (**XVIc**) or (**XVId**) as defined above.
In said embodiment compounds of formula (**Ie**), wherein R⁸³ is a group of formula (**XIIa**), (**XIIb**), (**XIIc**), (**XIId**), (**XIIe**), (**XIIf**), (**XIIg**), (**XIIj**), (**XIIk**), (**XIIn**), (**XIIo**), (**XIIq**), (**XIIs**), (**XIIt**), (**XIIu**), (**XIIv**), (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined above, and
R⁸⁷ is H, a C₁-C₂₅alkyl group, a group of formula (**XIIa**), (**XIIb**), (**XIIc**), (**XIId**), (**XIIe**), (**XIIf**), (**XIIg**), (**XIIj**), (**XIIk**), (**XIIn**), (**XIIo**), (**XIIq**), (**XIIs**), (**XIIt**), (**XIIu**), (**XIIv**) (**XIIw**), (**XIIx**), (**XIIy**), (**XIIz**), (**XIIIa**), (**XIII**b), **(XIIIc),** (**XIIId**), (**XIIIe**), (**XIII**f) or (**XIIIg**) as defined above, or a group of formula (**XIVb**), (**XIVc**), (**XIVd**), (**XIVe**), (**XIVf**), (**XIVg**), (**XIVo**), (**XIVp**), (**XIVq**), (**XIVr**), (**XIVt**), (**XIVu**), (**XIVv**), (**XIVw**), (**XIVx**), (**XIVz**), (**XVa**), (**XVb**), (**XVc**), (**XVk**), (**XVl**), (**XVo**), (**XVp**), (**XVs**), (**XVw**), (**XVx**), (**XVy**), (**XVIa**), (**XVIb**), (**XVIc**) or (**XVId**) as defined above; or compounds of formula (**Ie**), wherein R⁸⁷ is a group of formula (**XIIa**), (**XIIb**), (**XIIc**), (**XIId**), (**XIIe**), (**XIIf**), (**XIIg**), (**XIIj**), (**XIIk**), (**XIIn**), (**XIIo**), (**XIIq**), (**XIIs**), (**XIIt**), (**XIIu**), (**XIIv**), (**XIIw**), (**XIIx**), (**XIIy**), (**XIIz**), (**XIIIa**), (**XIII**b), **(XIIIc),** (**XIIId**), (**XIIIe**), (**XIIIf**) or (**XIIIg**) as defined above, and R⁸³ is H, a C₁-C₂₅alkyl group, a group of formula (**XIIa**), (**XIIb**), (**XIIc**), (**XIId**), (**XIIe**), (**XIIf**), (**XIIg**), (**XIIj**), (**XIIk**), (**XIIn**), (**XIIo**), (**XIIq**), (**XIIs**), (**XIIt**), (**XIIu**), (**XIIv**) (**XIIw**), (**XIIx**), (**XIIy**), (**XIIz**), (**XIIa**), (**XIIIb**), (**XIIIc**), (**XIIId**), (**XIIIe**), (**XIIIf**) or (**XIIIg**) as defined above, or a group of formula (**XIVb**), (**XIVc**), (**XIVd**), (**XIVe**), (**XIVf**), (**XIVg**), (**XIVo**), (**XIVp**), (**XIVq**), (**XIVr**), (**XIVt**), (**XIVu**), (**XIVv**), (**XIVw**), (**XIVx**), (**XIVz**), (**XVa**), (**XVb**), (**XVc**), (**XVk**), (**XVl**), (**XVo**), (**XVp**), (**XVs**), (**XVw**), (**XVx**), (**XVy**), (**XVIa**), (**XVIb**), (**XVIc**) or (**XVId**) as defined above; are more preferred.

In a preferred embodiment the present invention is directed to compounds of formula (**If**), wherein R⁸³ is a group of formula (**XIIa**), (**XIIb**), (**XIIc**), (**XIId**), (**XIIe**), (**XIIf**), (**XIIg**), (**XIIh**), **(**XIIi**), (**XIIj**),** (**XIIk**), ((**XIIl**)), (**XIIm**), (**XIIn**), (**XIIo**), (**XIIp**), (**XIIq**), (**XIIr**), (**XIIs**), (**XIIt**), (**XIIu**), (**XIIv**), (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined above, and
R⁸⁵ is H, a group of formula (**XIIa**), (**XIIb**), (**XIIc**), (**XIId**), (**XIIe**), (**XIIf**), (**XIIg**), (**XIIh**), (**XIIi**), (**XIIj**), (**XIIk**), ((**XIIl**)), (**XIIm**), (**XIIn**), (**XIIo**), (**XIIp**), (**XIIq**), (**XIIr**), (**XIIs**), (**XIIt**), (**XIIu**), (**XIIv**) (**Xllw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined above, or a group of formula (**XIVb**), (**XIVc**), (**XIVd**), (**XIVe**), (**XIVf**), (**XIVg**), (**XIVh**), (**XIVo**), (**XIVp**), (**XIVq**), (**XIVr**), (**XIVs**), (**XIVt**), (**XIVu**), (**XIVv**), (**XIVw**), (**XIVx**),(**XIVy**), (**XIVz**), (**XVa**), (**XVb**), (**XVc**), (**XVd**), (**XVe**), (**XVf**), (**XVg**), (**XVh**), (**XVi**), (**XVj**), (**XVk**), (**XVl**), (**XVm**), (**XVn**), (**XVo**), (**XVp**), (**XVq**), (**XVr**), (**XVs**), (**XVt**), (**XVu**), (**XVv**), (**XVw**), (**XVx**), (**XVy**), (**XVz**), (**XVIa**), (**XVIb**), (**XVIc**) or (**XVId**) as defined above; or
compounds of formula (**If**), wherein R⁸⁵ is a group of formula (**XIIa**), (**XIIb**), (**XIIc**), (**XIId**), (**XIIe**), (**XIIf**), (**XIIg**), (**XIIh**), (**XIIi**), (**XIIj**), (**XIIk**), ((**XIIl**)), (**XIIm**), (**XIIn**), (**XIIo**), (**XIIp**), (**XIIq**), (**XIIr**), (**XIIs**), (**XIIt**), (**XIIu**), (**XIIv**), (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined above, and
R⁸³ is H, a group of formula (**XIIa**), (**XIIb**), (**XIIc**), (**XIId**), (**XIIe**), (**XIIf**), (**XIIg**), (**XIIh**), (**XIIi**), (**XIIj**), (**XIIk**), ((**XIIl**)), (**XIIm**), (**XIIn**), (**XIIo**), (**XIIp**), (**XIIq**), (**XIIr**), (**XIIs**), (**XIIt**), (**XIIu**), (**XIIv**) (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined above, or a group of formula (**XIVb**), (**XIVc**), (**XIVd**), (**XIVe**), (**XIVf**), (**XIVg**), (**XIVh**), (**XIVo**), (**XIVp**), (**XIVq**), (**XIVr**), (**XIVs**), (**XIVt**), (**XIVu**), (**XIVv**), (**XIVw**), (**XIVx**),(**XIVy**), (**XIVz**), (**XVa**), (**XVb**), (**XVc**), (**XVd**), (**XVe**), (**XVf**), (**XVg**), (**XVh**), (**XVi**), (**XVj**), (**XVk**), (**XVl**), (**XVm**), (**XVn**), (**XVo**), (**XVp**), (**XVq**), (**XVr**), (**XVs**), (**XVt**), (**XVu**), (**XVv**), (**XVw**), (**XVx**), (**XVy**), (**XVz**), (**XVIa**), (**XVIb**), (**XVIc**) or (**XVId**) as defined above.
In said embodiment compounds of formula (If), wherein R⁸³ is a group of formula (**XIIa**), (**XIIb**), (**XIIc**), (**XIId**), (**XIIe**), (**XIIf**), (**XIIg**), (**XIIj**), (**XIIk**), (**XIIn**), (**XIIo**), (**XIIq**), (**XIIs**), (**XIIt**), (**XIIu**), (**XIIv**), (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined above, and
R⁸⁵ is H, a group of formula (**XIIa**), (**XIIb**), (**XIIc**), (**XIId**), (**XIIe**), (**XIIf**), (**XIIg**), (**XIIj**), (**XIIk**), (**XIIn**), (**XIIo**), (**XIIq**), (**XIIs**), (**XIIt**), (**XIIu**), (**XIIv**) (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined above, or a group of formula (**XIVb**), (**XIVc**), (**XIVd**), (**XIVe**), (**XIVf**), (**XIVg**), (**XIVo**), (**XIVp**), (**XIVq**), (**XIVr**), (**XIVt**), (**XIVu**), (**XIVv**), (**XIVw**), (**XIVx**),(**XIVz**), (**XVa**), (**XVb**), (**XVc**), (**XVk**), (**XVl**), (**XVo**), (**XVp**), (**XVs**), (**XVw**), (**XVx**), (**XVy**), (**XVIa**), (**XVIb**), (**XVIc**) or (**XVId**) as defined above; or
compounds of formula (If), wherein R⁸⁵ is a group of formula (**XIIa**), (**XIIb**), (**XIIc**), (**XIId**), (**XIIe**), (**XIIf**), (**XIIg**), (**XIIj**), (**XIIk**), (**XIIn**), (**XIIo**), (**XIIq**), (**XIIs**), (**XIIt**), (**XIIu**), (**XIIv**), (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined above, and
R⁸³ is H, a group of formula (**XIIa**), (**XIIb**), (**XIIc**), (**XIId**), (**XIIe**), (**XIIf**), (**XIIg**), (**XIIj**), (**XIIk**), (**XIIn**), (**XIIo**), (**XI**I**q**), (**XIIs**), (**XIIt**), (**XIIu**), (**XIIv**) (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined above, or a group of formula (**XIVb**), (**XIVc**), (**XIVd**), (**XIVe**), (**XIVf**), (**XIVg**), (**XIVo**), (**XIVp**), (**XIVq**), (**XIVr**), (**XIVt**), (**XIVu**), (**XIVv**), (**XIVw**), (**XIVx**),(**XIVz**), (**XVa**), (**XVb**), (**XVc**), (**XVk**), (**XVl**), (**XVo**), (**XVp**), (**XVs**), (**XVw**), (**XVx**), (**XVy**), (**XVIa**), (**XVIb**), (**XVIc**) or (**XVId**) as defined above; are more preferred.

In a preferred embodiment the present invention is directed to compounds of formula (**Ij**), wherein R⁸³ is a group of formula (**XIIa**), (**XIIb**), (**XIIc**), (**XIId**), (**XIIe**), (**XIIf**), (**XIIg**), (**XIIh**), (**XIIi**), (**XIIj**), (**XIIk**), ((**XIIl**)), (**XIIm**), (**XIIn**), (**XIIo**), (**XIIp**), (**XIIq**), (**XIIr**), (**XIIs**), (**XIIt**), (**XIIu**), (**XIIv**), (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined above, and
R⁸⁵ is H, a C₁-C₂₅alkyl group, a group of formula (**XIIa**), (**XIIb**), (**XIIc**), (**XIId**), (**XIIe**), (**XIIf**), (**XIIg**), (**XIIh**), (**XIIi**), (**XIIj**), (**XIIk**), ((**XIIl**)), (**XIIm**), (**XIIn**), (**XIIo**), (**XIIp**), (**XIIq**), (**XIIr**), (**XIIs**), (**XIIt**), (**XIIu**), (**XIIv**) (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined above, or a group of formula (**XIVb**), (**XIVc**), (**XIVd**), (**XIVe**), (**XIVf**), (**XIVg**), (**XIVh**), (**XIVo**), (**XIVp**), (**XIVq**), (**XIVr**), (**XIVs**), (**XIVt**), (**XIVu**), (**XIVv**), (**XIVw**), (**XIVx**),(**XIVy**), (**XIVz**), (**XVa**), (**XVb**), (**XVc**), (**XVd**), (**XVe**), (**XVf**), (**XVg**), (**XVh**), (**XVi**), (**XVj**), (**XVk**), (**XVl**), (**XVm**), (**XVn**), (**XVo**), (**XVp**), (**XVq**), (**XVr**), (**XVs**), (**XVt**), (**XVu**), (**XVv**), (**XVw**), (**XVx**), (**XVy**), (**XVz**), (**XVIa**), (**XVIb**), (**XVIc**) or (**XVId**) as defined above; or
compounds of formula (**Ij**), wherein R⁸⁵ is a group of formula (**XIIa**), (**XIIb**), (**XIIc**), (**XIId**), (**XIIe**), (**XIIf**), (**XIIg**), (**XIIh**), (**XIIi**), (**XIIj**), (**XIIk**), ((**XIIl**)), (**XIIm**), (**XIIn**), (**XIIo**), (**XIIp**), (**XIIq**), (**XIIr**), (**XIIs**), (**XIIt**), (**XIIu**), (**XIIv**), (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined above, and
R⁸³ is H, a C₁-C₂₅alkyl group, a group of formula (**XIIa**), (**XIIb**), (**XIIc**), (**XIId**), (**XIIe**), (**XIIf**), (**XIIg**), (**XIIh**), (**XIIi**), (**XIIj**), (**XIIk**), ((**XIIl**)), (**XIIm**), (**XIIn**), (**XIIo**), (**XIIp**), (**XIIq**), (**XIIr**), (**XIIs**), (**XIIt**), (**XIIu**), (**XIIv**) (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined above, or a group of formula (**XIVb**), (**XIVc**), (**XIVd**), (**XIVe**), (**XIVf**), (**XIVg**), (**XIVh**), (**XIVo**), (**XIVp**), (**XIVq**), (**XIVr**), (**XIVs**), (**XIVt**), (**XIVu**), (**XIVv**), (**XIVw**), (**XIVx**),(**XIVy**), (**XIVz**), (**XVa**), (**XVb**), (**XVc**), (**XVd**), (**XVe**), (**XVf**), (**XVg**), (**XVh**), (**XVi**), (**XVj**), (**XVk**), (**XVl**), (**XVm**), (**XVn**), (**XVo**), (**XVp**), (**XVq**), (**XVr**), (**XVs**), (**XVt**), (**XVu**), (**XVv**), (**XVw**), (**XVx**), (**XVy**), (**XVz**), (**XVIa**), (**XVIb**), (**XVIc**) or (**XVId**) as defined above.
In said embodiment compounds of formula compounds of formula (**Ij**), wherein R⁸³ is a group of formula (**XIIa**), (**XIIb**), (**XIIc**), (**XIId**), (**XIIe**), (**XIIf**), (**XIIg**), (**XIIj**), (**XIIk**), (**XIIn**), (**XIIo**), (**XIIq**), (**XIIs**), (**XIIt**), (**XIIu**), (**XIIv**), (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined above, and
R⁸⁵ is H, a C₁-C₂₅alkyl group, a group of formula (**XIIa**), (**XIIb**), (**XIIc**), (**XIId**), (**XIIe**), (**XIIf**), (**XIIg**), (**XIIj**), (**XIIk**), (**XIIn**), (**XIIo**), (**XIIq**), (**XIIs**), (**XIIt**), (**XIIu**), (**XIIv**) (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined above, or a group of formula (**XIVb**), (**XIVc**), (**XIVd**), (**XIVe**), (**XIVf**), (**XIVg**), (**XIVo**), (**XIVp**), (**XIVq**), (**XIVr**), (**XIVt**), (**XIVu**), (**XIVv**), (**XIVw**), (**XIVx**),(**XIVz**), (**XVa**), (**XVb**), (**XVc**), (**XVk**), (**XVl**), (**XVo**), (**XVp**), (**XVs**), (**XVw**), (**XVx**), (**XVy**), (**XVIa**), (**XVIb**), (**XVIc**) or (**XVId**) as defined above; or
compounds of formula (Ij), wherein R⁸⁵ is a group of formula (**XIIa**), (**XIIb**), (**XIIc**), (**XIId**), (**XIIe**), (**XIIf**), (**XIIg**), (**XIIj**), (**XIIk**), (**XIIn**), (**XIIo**), (**XIIq**), (**XIIs**), (**XIIt**), (**XIIu**), (**XIIv**), (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined above, and
R⁸³ is H, a C₁-C₂₅alkyl group, a group of formula (**XIIa**), (**XIIb**), (**XIIc**), (**XIId**), (**XIIe**), (**XIIf**), (**XIIg**), (**XIIj**), (**XIIk**), (**XIIn**), (**XIIo**), (**XIIq**), (**XIIs**), (**XIIt**), (**XIIu**), (**XIIv**) (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined above, or a group of formula (**XIVb**), (**XIVc**), (**XIVd**), (**XIVe**), (**XIVf**), (**XIVg**), (**XIVo**), (**XIVp**), (**XIVq**), (**XIVr**), (**XIVt**), (**XIVu**), (**XIVv**), (**XIVw**), (**XIVx**),(**XIVz**), (**XVa**), (**XVb**), (**XVc**), (**XVk**), (**XVl**), (**XVo**), (**XVp**), (**XVs**), (**XVw**), (**XVx**), (**XVy**), (**XVIa**), (**XVIb**), (**XVIc**) or (**XVId**) as defined above; are more preferred.

In a preferred embodiment the present invention is directed to compounds of formula (**II**), wherein R⁸⁵ is a group of formula (**XIIa**), (**XIIb**), (**XIIc**), (**XIId**), (**XIIe**), (**XIIf**), (**XIIg**), (**XIIh**), (**XIIi**), (**XIIj**), (**XIIk**), ((**XIIl**)), (**XIIm**), (**XIIn**), (**XIIo**), (**XIIp**), (**XIIq**), (**XIIr**), (**XIIs**), (**XIIt**), (**XIIu**), (**XIIv**), (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined above, and
R⁸⁷ is H, a group of formula (**XIIa**), (**XIIb**), (**XIIc**), (**XIId**), (**XIIe**), (**XIIf**), (**XIIg**), (**XIIh**), (**XIIi**), (XI**I**j), (**XIIk**), ((**XIIl**)), (**XIIm**), (**XIIn**), (**XIIo**), (**XIIp**), (**XIIq**), (**XIIr**), (**XIIs**), (**XIIt**), (**XIIu**), (**XIIv**) (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined above, or a group of formula (**XIVb**), (**XIVc**), (**XIVd**), (**XIVe**), (**XIVf**), (**XIVg**), (**XIVh**), (**XIVo**), (**XIVp**), (**XIVq**), (**XIVr**), (**XIVs**), (**XIVt**), (**XIVu**), (**XIVv**), (**XIVw**), (**XIVx**),(**XIVy**), (**XIVz**), (**XVa**), (**XVb**), (**XVc**), (**XVd**), (**XVe**), (**XVf**), (**XVg**), (**XVh**), (**XVi**), (**XVj**), (**XVk**), (**XVl**), (**XVm**), (**XVn**), (**XVo**), (**XVp**), (**XVq**), (**XVr**), (**XVs**), (**XVt**), (**XVu**), (**XVv**), (**XVw**), (**XVx**), (**XVy**), (**XVz**), (**XVIa**), (**XVIb**), (**XVIc**) or (**XVId**) as defined above; or
compounds of formula (**II**), wherein R⁸⁷ is a group of formula (**XIIa**), (**XIIb**), (**XIIc**), (**XIId**), (**XIIe**), (**XIIf**), (**XIIg**), (**XIIh**), (**XIIi**), (**XIIj**), (**XIIk**), ((**XIIl**)), (**XIIm**), (**XIIn**), (**XIIo**), (**XIIp**), (**XIIq**), (**XIIr**), (**XIIs**), (**XIIt**), (**XIIu**), (**XIIv**), (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined above, and
R⁸⁵ is H, a group of formula (**XIIa**), (**XIIb**), (**XIIc**), (**XIId**), (**XIIe**), (**XIIf**), (**XIIg**), (**XIIh**), (**XIIi**), (**XIIj**), (**XIIk**), ((**XIIl**)), (**XIIm**), (**XIIn**), (**XIIo**), (**XIIp**), (**XIIq**), (**XIIr**), (**XIIs**), (**XIIt**), (**XIIu**), (**XIIv**) (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined above, or a group of formula (**XIVb**), (**XIVc**), (**XIVd**), (**XIVe**), (**XIVf**), (**XIVg**), (**XIVh**), (**XIVo**), (**XIVp**), (**XIVq**), (**XIVr**), (**XIVs**), (**XIVt**), (**XIVu**), (**XIVv**), (**XIVw**), (**XIVx**),(**XIVy**), (**XIVz**), (**XVa**), (**XVb**), (**XVc**), (**XVd**), (**XVe**), (**XVf**), (**XVg**), (**XVh**), (**XVi**), (**XVj**), (**XV**k), (**XVl**), (**XVm**), (**XVn**), (**XVo**), (**XVp**), (**XVq**), (**XVr**), (**XVs**), (**XVt**), (**XVu**), (**XVv**), (**XVw**), (**XVx**), (**XVy**), (**XVz**), (**XVIa**), (**XVIb**), (**XVIc**) or (**XVId**) as defined above.

In a preferred embodiment the present invention is directed to compounds of formula (**Im**), wherein R⁸³ is a group of formula (**XIIa**), (**XIIb**), (**XIIc**), (**XIId**), (**XIIe**), (**XIIf**), (**XIIg**), (**XIIh**), (**XIIi**), (**XIIj**), (**XIIk**), ((**XIIl**)), (**XIIm**), (**XIIn**), (**XIIo**), (**XIIp**), (**XIIq**), (**XIIr**), (**XIIs**), (**XIIt**), (**XIIu**), (**XIIv**), (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined above, and
R⁸⁵ is H, a C₁-C₂₅alkyl group, a group of formula (**XIIa**), (**XIIb**), (**XIIc**), (**XIId**), (**XIIe**), (**XIIf**), (**XIIg**), (**XIIh**), (**XIIi**), (**XIIj**), (**XIIk**), ((**XIIl**)), (**XIIm**), (**XIIn**), (**XIIo**), (**XIIp**), (**XIIq**), (**XIIr**), (**XIIs**), (**XIIt**), (**XIIu**), (**XIIv**) (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined in claim 5, or a group of formula (**XIVb**), (**XIVc**), (**XIVd**), (**XIVe**), (**XIVf**), (**XIVg**), (**XIVh**), (**XIVo**), (**XIVp**), (**XIVq**), (**XIVr**), (**XIVs**), (**XIVt**), (**XIVu**), (**XIVv**), (**XIVw**), (**XIVx**),(**XIVy**), (**XIVz**), (**XVa**), (**XVb**), (**XVc**), (**XVd**), (**XVe**), (**XVf**), (**XVg**), (**XVh**), (**XVi**), (**XVj**), (**XVk**), (**XVl**), (**XVm**), (**XVn**), (**XVo**), (**XVp**), (**XVq**), (**XVr**), (**XVs**), (**XVt**), (**XVu**), (**XVv**), (**XVw**), (**XVx**), (**XVy**), (**XVz**), (**XVIa**), (**XVIb**), (**XVIc**) or (**XVId**) as defined above; or
compounds of formula (Im), wherein R⁸⁵ is a group of formula (**XIIa**), (**XIIb**), (**XIIc**), (**XIId**), (**XIIe**), (**XIIf**), (**XIIg**), (**XIIh**), (**XIIi**), (**XIIj**), (**XIIk**), ((**XIIl**)), (**XIIm**), (**XIIn**), (**XIIo**), (**XIIp**), (**XIIq**), (**XIIr**), (**XIIs**), (**XIIt**), (**XIIu**), (**XIIv**), (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined above, and
R⁸³ is H, a C₁-C₂₅alkyl group, a group of formula (**XIIa**), (**XIIb**), (**XIIc**), (**XIId**), (**XIIe**), (**XIIf**), (**XIIg**), (**XIIh**), (**XIIi**), (**XIIj**), (**XIIk**), ((**XIIl**)), (**XIIm**), (**XIIn**), (**XIIo**), (**XIIp**), (**XIIq**), (**XIIr**), (**XIIs**), (**XIIt**), (**XIIu**), (**XIIv**) (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined above, or a group of formula (**XIVb**), (**XIVc**), (**XIVd**), (**XIVe**), (**XIVf**), (**XIVg**), (**XIVh**), (**XIVo**), (**XIVp**), (**XIVq**), (**XIVr**), (**XIVs**), (**XIVt**), (**XIVu**), (**XIVv**), (**XIVw**), (**XIVx**),(**XIVy**), (**XIVz**), (**XVa**), (**XVb**), (**XVc**), (**XVd**), (**XVe**), (**XVf**), (**XVg**), (**XVh**), (**XVi**), (**XVj**), (**XVk**), (**XVl**), (**XVm**), (**XVn**), (**XVo**), (**XVp**), (**XVq**), (**XVr**), (**XVs**), (**XVt**), (**XVu**), (**XVv**), (**XVw**), (**XVx**), (**XVy**), (**XVz**), (**XVIa**), (**XVIb**), (**XVIc**) or (**XVId**) as defined above.
In said embodiment compounds of formula (Im), wherein R⁸³ is a group of formula (**XIIa**), (**XIIb**), (**XIIc**), (**XIId**), (**XIIe**), (**XIIf**), (**XIIg**), (**XIIj**), (**XIIk**), (**XIIn**), (**XIIo**), (**XIIq**), (**XIIs**), (**XIIt**), (**XIIu**), (**XIIv**), (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined above, and
R⁸⁵ is H, a C₁-C₂₅alkyl group, a group of formula (**XIIa**), (**XIIb**), (**XIIc**), (**XIId**), (**XIIe**), (**XIIf**), (**XIIg**), (**XIIj**), (**XIIk**), (**XIIn**), (**XIIo**), (**XIIq**), (**XIIs**), (**XIIt**), (**XIIu**), (**XIIv**) (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined above, or a group of formula (**XIVb**), (**XIVc**), (**XIVd**), (**XIVe**), (**XIVf**), (**XIVg**), (**XIVo**), (**XIVp**), (**XIVq**), (**XIVr**), (**XIVt**), (**XIVu**), (**XIVv**), (**XIVw**), (**XIVx**),(**XIVz**), (**XVa**), (**XVb**), (**XVc**), (**XVk**), (**XVl**), (**XVo**), (**XVp**), (**XVs**), (**XVw**), (**XVx**), (**XVy**), (**XVIa**), (**XVIb**), (**XVIc**) or (**XVId**) as defined above; or
compounds of formula (**Im**), wherein R⁸⁵ is a group of formula (**XIIa**), (**XIIb**), (**XIIc**), (**XIId**), (**XIIe**), (**XIIf**), (**XIIg**), (**XIIj**), (**XIIk**), (**XIIn**), (**XIIo**), (**XIIq**), (**XIIs**), (**XIIt**), (**XIIu**), (**XIIv**), (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined above, and
R⁸³ is H, a C₁-C₂₅alkyl group, a group of formula (**XIIa**), (**XIIb**), (**XIIc**), (**XIId**), (**XIIe**), (**XIIf**), (**XIIg**), (**XIIj**), (**XIIk**), (**XIIn**), (**XIIo**), (**XIIq**), (**XIIs**), (**XIIt**), (**XIIu**), (**XIIv**) (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined above, or a group of formula (**XIVb**), (**XIVc**), (**XIVd**), (**XIVe**), (**XIVf**), (**XIVg**), (**XIVo**), (**XIVp**), (**XIVq**), (**XIVr**), (**XIVt**), (**XIVu**), (**XIVv**), (**XIVw**), (**XIVx**),(**XIVz**), (**XVa**), (**XVb**), (**XVc**), (**XVk**), (**XVl**), (**XVo**), (**XVp**), (**XVs**), (**XVw**), (**XVx**), (**XVy**), (**XVIa**), (**XVIb**), (**XVIc**) or (**XVId**) as defined above; are more preferred.

In a preferred embodiment the present invention is directed to compounds of formula (**In**), wherein R⁸³ is a group of formula (**XIIa**), (**XIIb**), (**XIIc**), (**XIId**), (**XIIe**), (**XIIf**), (**XIIg**), (**XIIh**), (**XIIi**), (**XIIj**), (**XIIk**), ((**XIIl**)), (**XIIm**), (**XIIn**), (**XIIo**), (**XIIp**), (**XIIq**), (**XIIr**), (**XIIs**), (**XIIt**), (**XIIu**), (**XIIv**), (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as above, and
R⁸⁷ is H, a group of formula (**XIIa**), (**XIIb**), (**XIIc**), (**XIId**), (**XIIe**), (**XIIf**), (**XIIg**), (**XIIh**), (**XIIi**), (**XIIj**), (**XIIk**), ((**XIIl**)), (**XIIm**), (**XIIn**), (**XIIo**), (**XIIp**), (**XIIq**), (**XIIr**), (**XIIs**), (**XIIt**), (**XIIu**), (**XIIv**) (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined above, or a group of formula (**XIVb**), (**XIVc**), (**XIVd**), (**XIVe**), (**XIVf**), (**XIVg**), (**XIVh**), (**XIVo**), (**XIVp**), (**XIVq**), (**XIVr**), (**XIVs**), (**XIVt**), (**XIVu**), (**XIVv**), (**XIVw**), (**XIVx**),(**XIVy**), (**XIVz**), (**XVa**), (**XVb**), (**XVc**), (**XVd**), (**XVe**), (**XVf**), (**XVg**), (**XVh**), (**XVi**), (**XVj**), (**XVk**), (**XVl**), (**XVm**), (**XVn**), (**XVo**), (**XVp**), (**XVq**), (**XVr**), (**XVs**), (**XVt**), (**XVu**), (**XVv**), (**XVw**), (**XVx**), (**XVy**), (**XVz**), (**XVIa**), (**XVIb**), (**XVIc**) or (**XVId**) as defined above.

In said embodiment compounds of formula (**In**), wherein R⁸³ is a group of formula (**XIIa**), (**XIIb**), (**XIIc**), (**XIId**), (**XIIe**), (**XIIf**), (**XIIg**), (**XIIj**), (**XIIk**), (**XIIn**), (**XIIo**), (**XIIq**), (**XIIs**), (**XIIt**), (**XIIu**), (**XIIv**), (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined above, and
R⁸⁷ is H, a group of formula (**XIIa**), (**XIIb**), (**XIIc**), (**XIId**), (**XIIe**), (**XIIf**), (**XIIg**), (**XIIj**), (**XIIk**), (**XIIn**), (**XIIo**), (**XIIq**), (**XIIs**), (**XIIt**), (**XIIu**), (**XIIv**) (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined above, or a group of formula (**XIVb**), (**XIVc**), (**XIVd**), (**XIVe**), (**XIVf**), (**XIVg**), (**XIVo**), (**XIVp**), (**XIVq**), (**XIVr**), (**XIVt**), (**XIVu**), (**XIVv**), (**XIVw**), (**XIVx**),(**XIVz**), (**XVa**), (**XVb**), (**XVc**), (**XVk**), (**XVI**), (**XVo**), (**XVp**), (**XVs**), (**XVw**), (**XVx**), (**XVy**), (**XVIa**), (**XVIb**), (**XVIc**) or (**XVId**) as defined above-are more preferred.

Examples of preferred compounds are compounds **A-1** to **A-75, B-1** to **B-8, C-1** to **C-75, D-1** to **D-8, E-1** to **E-75, F-1** to **F-75** and **G-1** to **G-75** as shown below:

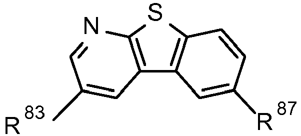

| **Compound** | **R⁸³** | **R⁸⁷** |
|---|---|---|
| **A-1** | 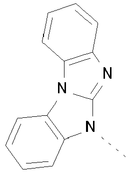 | 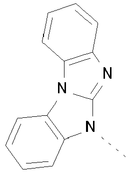 |
| **A-2** | 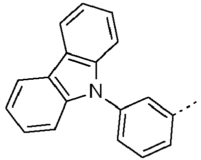 | 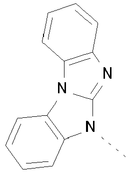 |
| **A-3** | 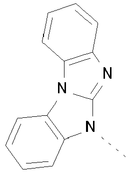 | 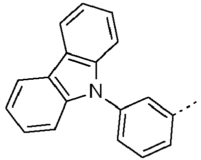 |
| **A-4** | 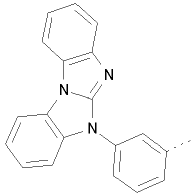 | 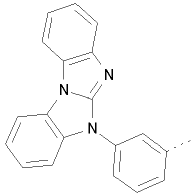 |
| **A-5** | H | 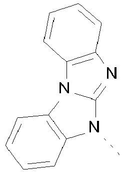 |
| **A-6** | 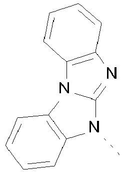 | H |
| **A-7** | H | 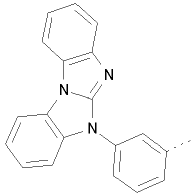 |
| **A-8** | 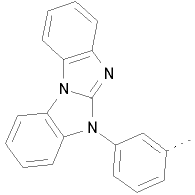 | H |
| **A-9** | H | 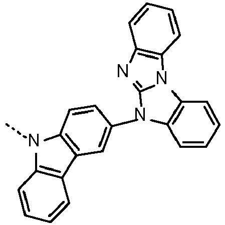 |
| **A-10** | 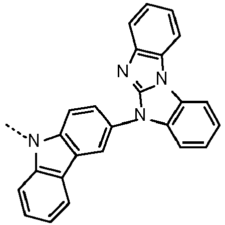 | H |
| **A-11** | 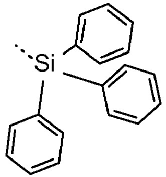 | 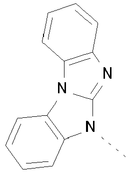 |
| **A-12** | 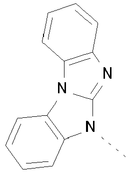 | 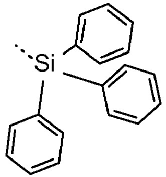 |
| **A-13** | 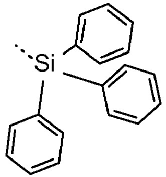 | 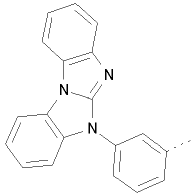 |
| **A-14** | 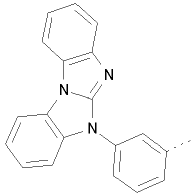 | 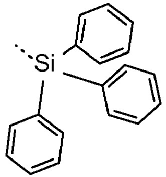 |
| **A-15** | 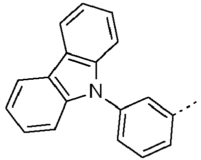 | 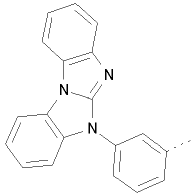 |
| **A-16** | 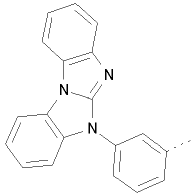 | 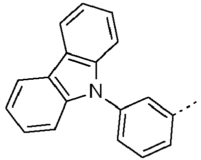 |
| **A-17** | 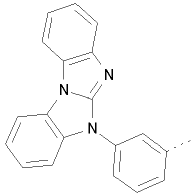 | 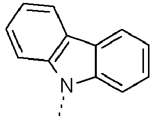 |
| **A-18** | 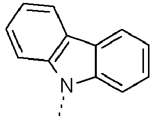 | 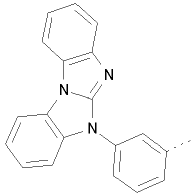 |
| **A-19** | 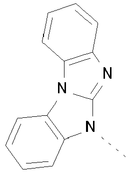 | 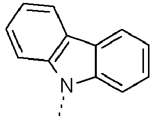 |
| **A-20** | 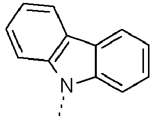 | 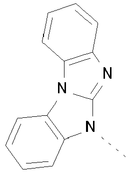 |
| **A-21** | 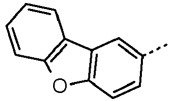 | 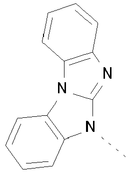 |
| **A-22** | 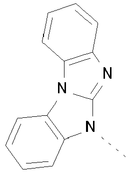 | 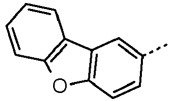 |
| **A-23** | 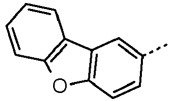 | 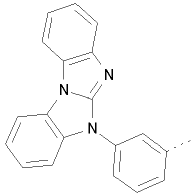 |
| **A-24** | 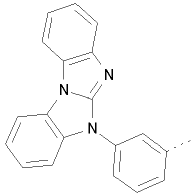 | 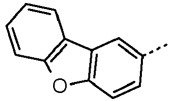 |
| **A-25** | 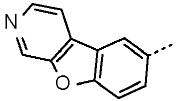 | 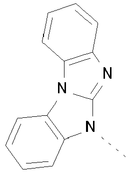 |
| **A-26** | 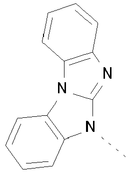 | 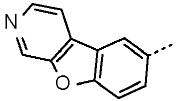 |
| **A-27** | 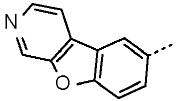 | 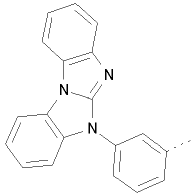 |
| **A-28** | 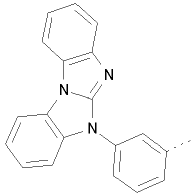 | 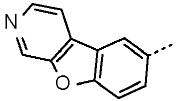 |
| **A-29** | 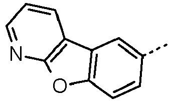 | 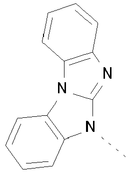 |
| **A-30** | 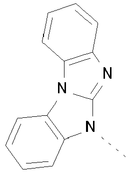 | 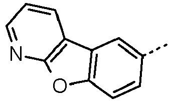 |
| **A-31** | 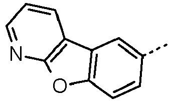 | 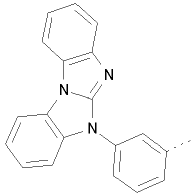 |
| **A-32** | 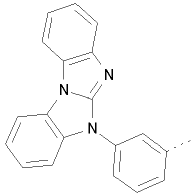 | 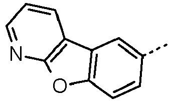 |
| **A-33** | 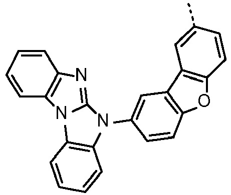 | 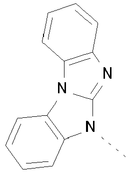 |
| **A-34** | 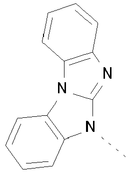 | 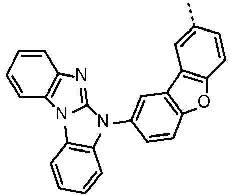 |
| **A-35** | 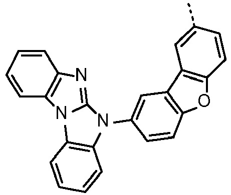 | H |
| **A-36** | H | 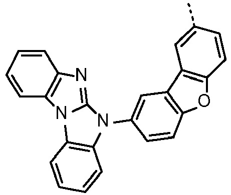 |
| **A-37** | 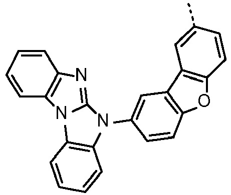 | 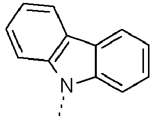 |
| **A-38** | 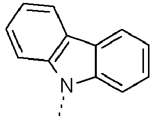 | 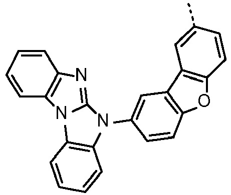 |
| **A-39** | 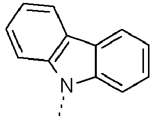 | 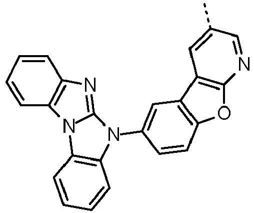 |
| **A-40** | 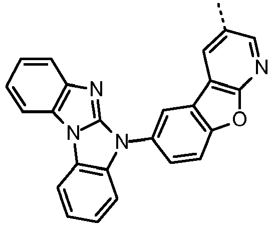 | 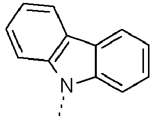 |
| **A-41** | 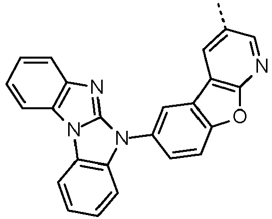 | H |
| **A-42** | H | 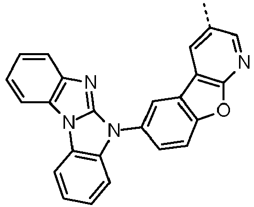 |
| **A-43** | 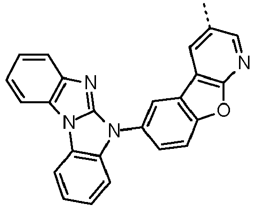 | 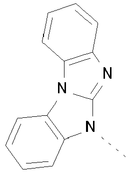 |
| **A-44** | 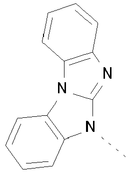 | 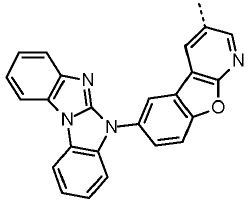 |
| **A-45** | 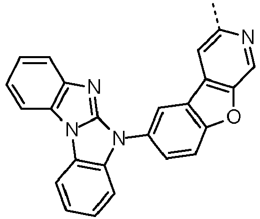 | 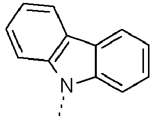 |
| **A-46** | 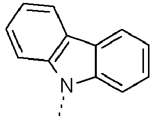 | 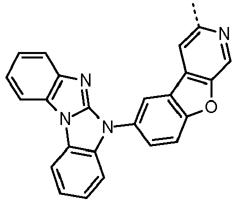 |
| **A-47** | 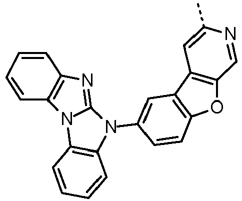 | H |
| **A-48** | H | 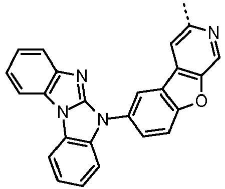 |
| **A-49** | 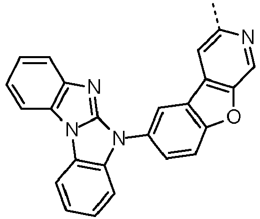 | 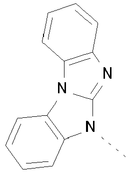 |
| **A-50** | 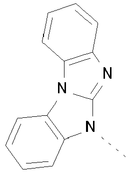 | 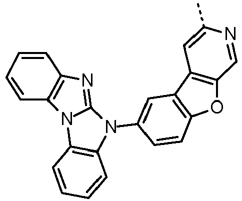 |
| **A-51** | 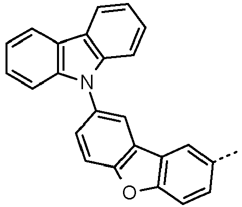 | 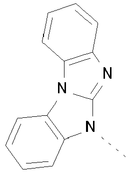 |
| **A-52** | 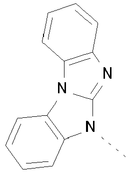 | 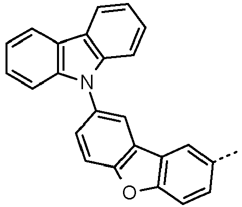 |
| **A-53** | 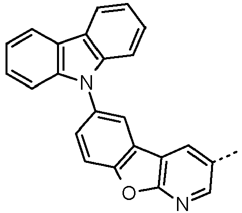 | 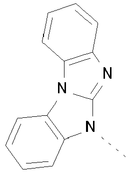 |
| **A-54** | 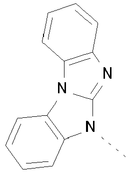 | 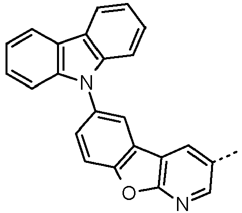 |
| **A-55** | 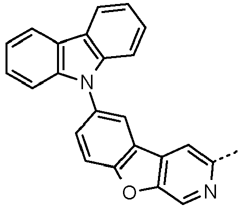 | 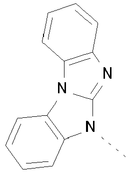 |
| **A-56** | 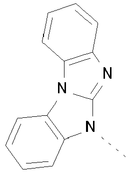 | 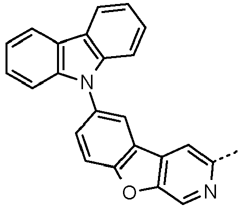 |
| **A-57** | H | 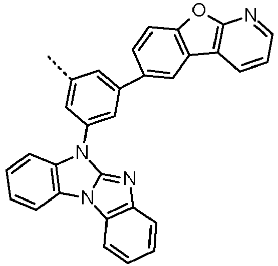 |
| **A-58** | 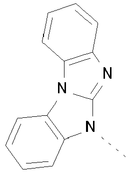 | 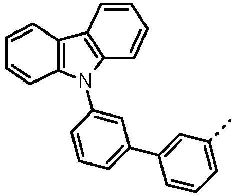 |
| **A-59** | 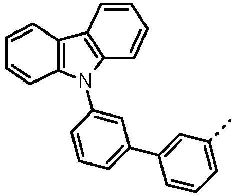 | 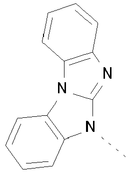 |
| **A-60** | 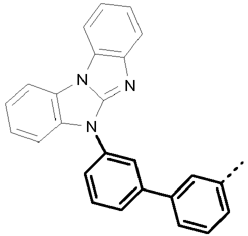 | H |
| **A-61** | H | 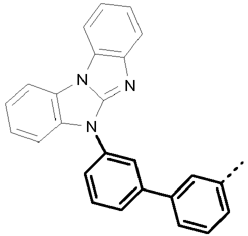 |
| **A-62** | H | 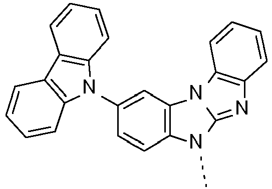 |
| **A-63** | H | 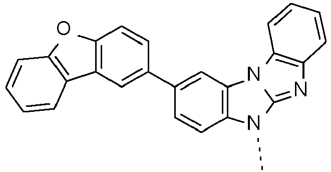 |
| **A-64** | H | 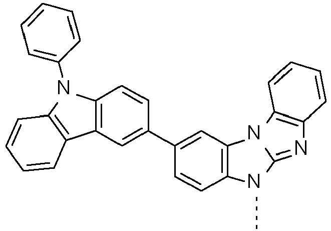 |
| **A-65** | H | 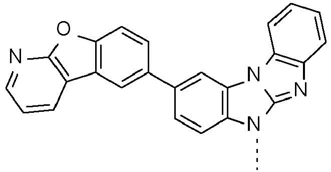 |
| **A-66** | 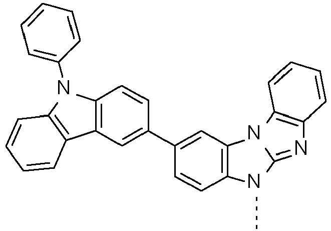 | 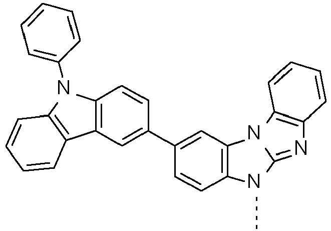 |
| **A-67** | 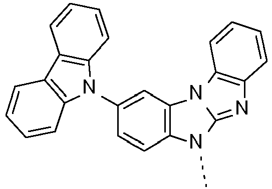 | 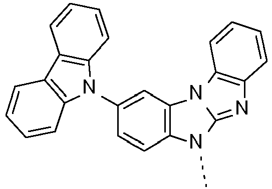 |
| **A-68** | 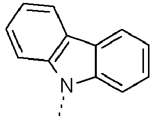 | 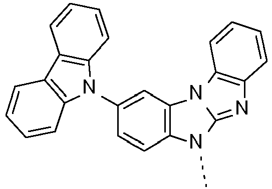 |
| **A-69** | 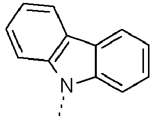 | 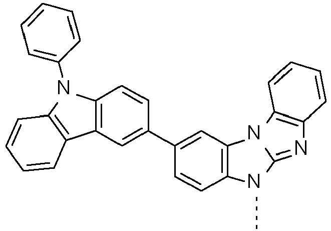 |
| **A-70** | 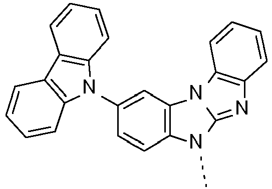 | 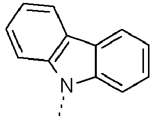 |
| **A-71** | 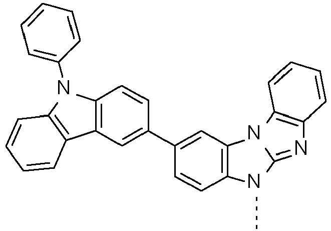 | 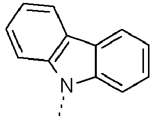 |
| **A-72** | -C₈H₁₇ | 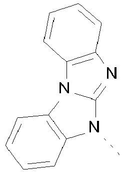 |
| **A-73** | -C₈H₁₇ | 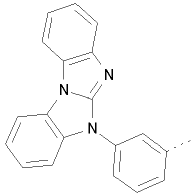 |
| **A-74** | 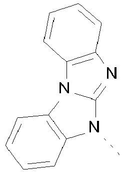 | -C₈H₁₇ |
| **A-75** | 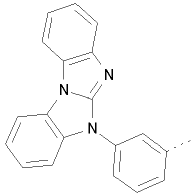 | -C₈H₁₇ |

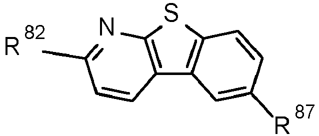

| **Compound** | **R⁸²** | **R⁸⁷** |
|---|---|---|
| | | |
| **B-1** | 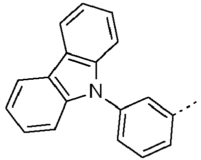 | 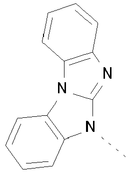 |
| **B-2** | 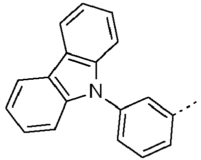 | 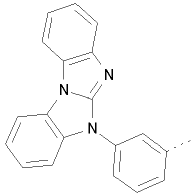 |
| **B-3** | 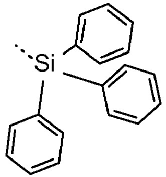 | 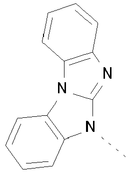 |
| **B-4** | 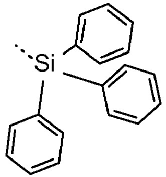 | 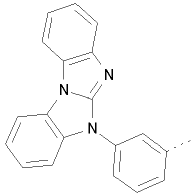 |
| **B-5** | 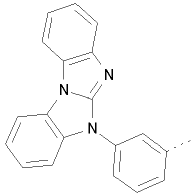 | 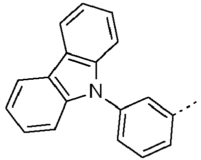 |
| **B-6** | 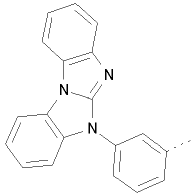 | 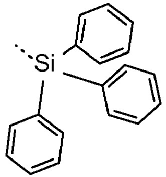 |
| **B-7** | 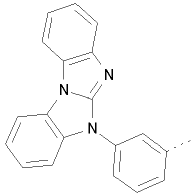 | 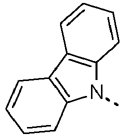 |
| **B-8** | 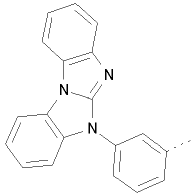 | 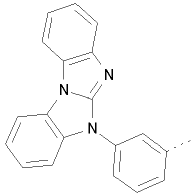 |

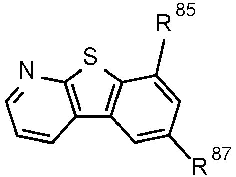

| **Compound** | **R⁸⁵** | **R⁸⁷** |
|---|---|---|
| **C-1** | 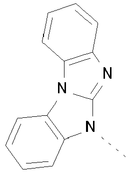 | 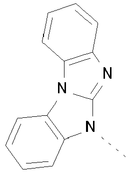 |
| **C-2** | 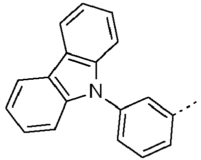 | 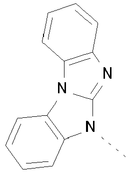 |
| **C-3** | 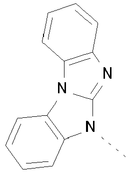 | 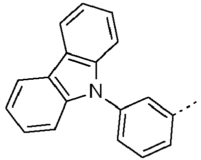 |
| **C-4** | 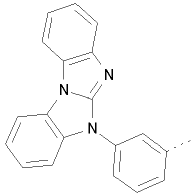 | 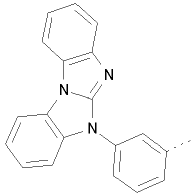 |
| **C-5** | H | 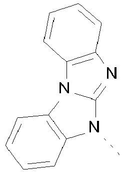 |
| **C-6** | 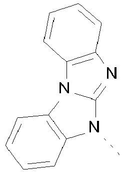 | H |
| **C-7** | H | 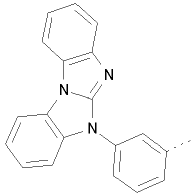 |
| **C-8** | 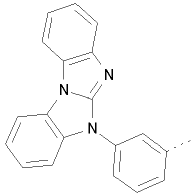 | H |
| **C-9** | H | 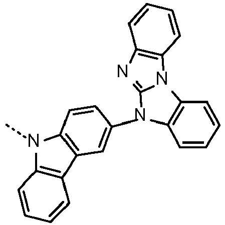 |
| **C-10** | 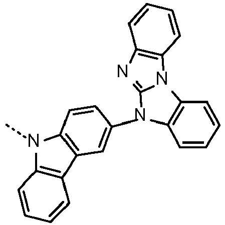 | H |
| **C-11** | 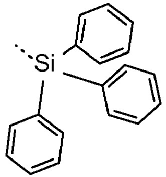 | 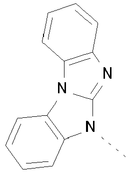 |
| **C-12** | 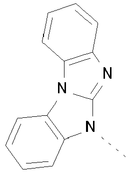 | 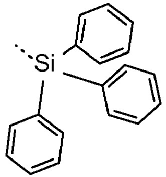 |
| **C-13** | 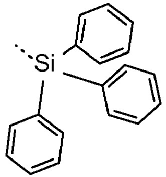 | 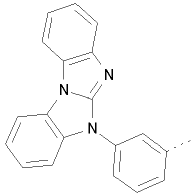 |
| **C-14** | 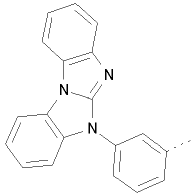 | 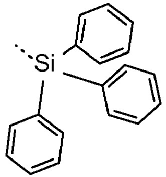 |
| **C-15** | 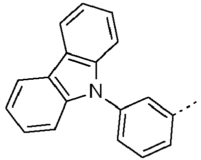 | 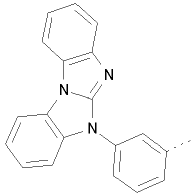 |
| **C-16** | 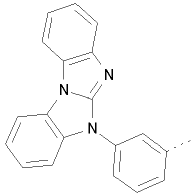 | 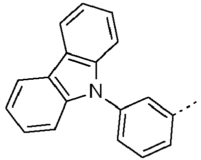 |
| **C-17** | 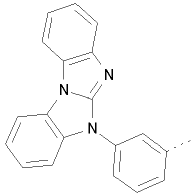 | 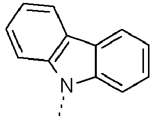 |
| **C-18** | 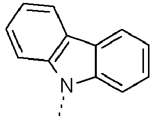 | 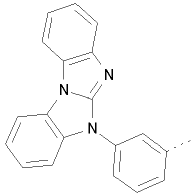 |
| **C-19** | 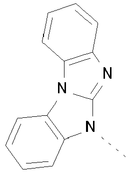 | 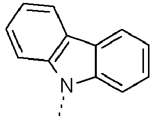 |
| **C-20** | 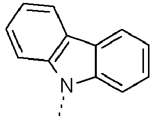 | 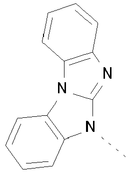 |
| **C-21** | 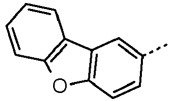 | 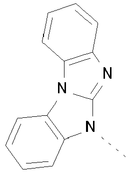 |
| **C-22** | 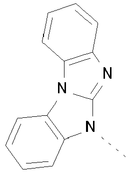 | 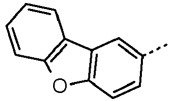 |
| **C-23** | 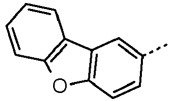 | 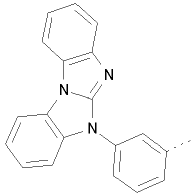 |
| **C-24** | 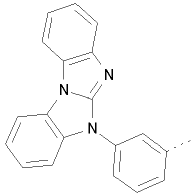 | 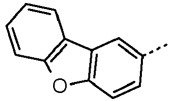 |
| **C-25** | 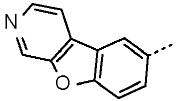 | 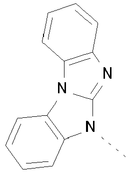 |
| **C-26** | 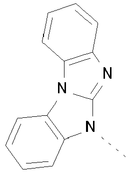 | 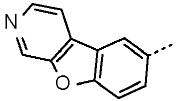 |
| **C-27** | 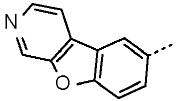 | 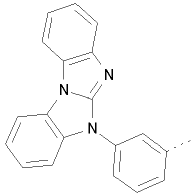 |
| **C-28** | 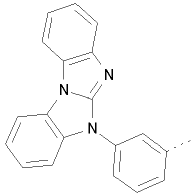 | 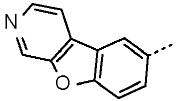 |
| **C-29** | 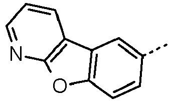 | 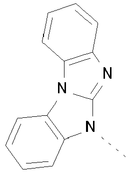 |
| **C-30** | 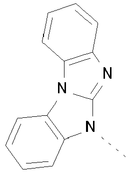 | 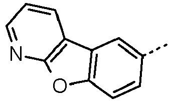 |
| **C-31** | 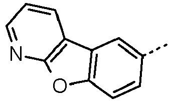 | 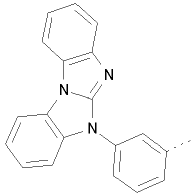 |
| **C-32** | 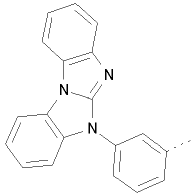 | 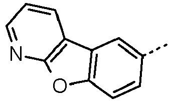 |
| **C-33** | 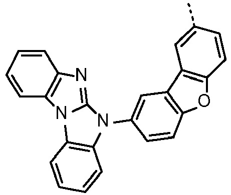 | 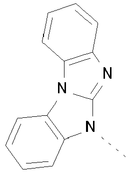 |
| **C-34** | 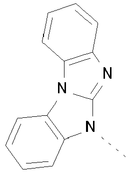 | 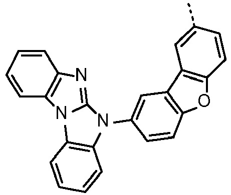 |
| **C-35** | 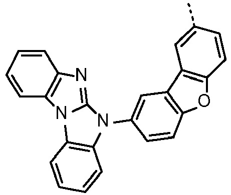 | H |
| **C-36** | H | 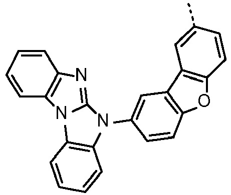 |
| **C-37** | 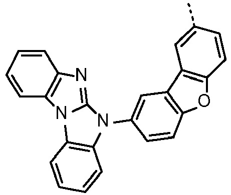 | 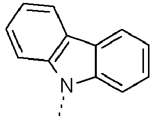 |
| **C-38** | 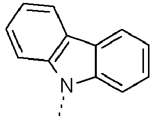 | 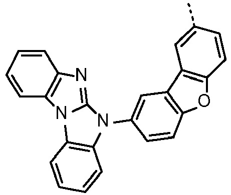 |
| **C-39** | 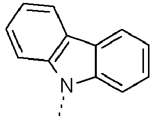 | 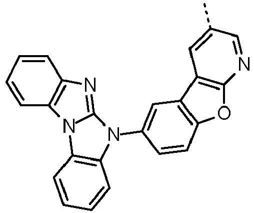 |
| **C-40** | 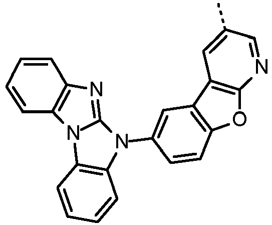 | 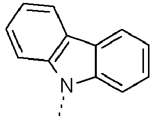 |
| **C-41** | 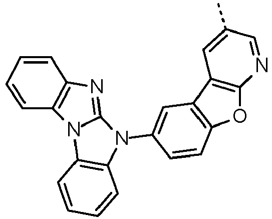 | H |
| **C-42** | H | 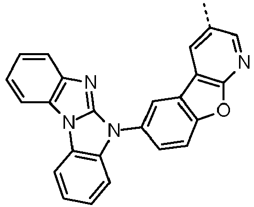 |
| **C-43** | 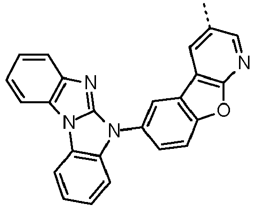 | 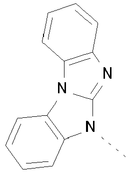 |
| **C-44** | 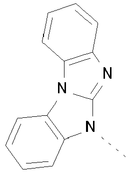 | 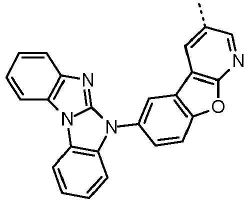 |
| **C-45** | 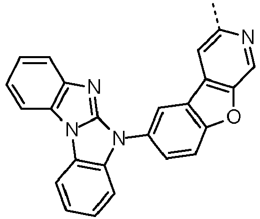 | 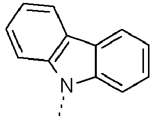 |
| **C-46** | 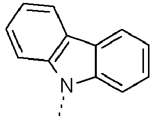 | 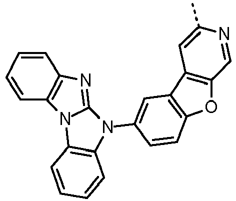 |
| **C-47** | 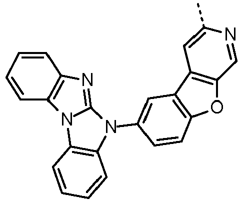 | H |
| **C-48** | H | 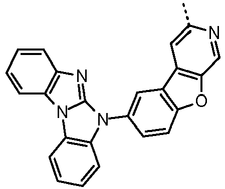 |
| **C-49** | 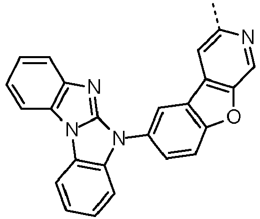 | 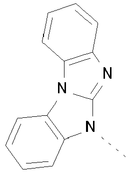 |
| **C-50** | 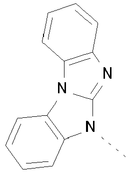 | 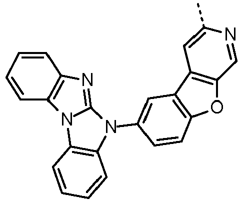 |
| **C-51** | 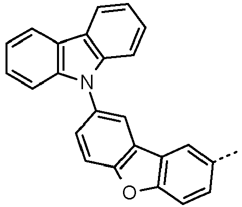 | 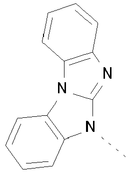 |
| **C-52** | 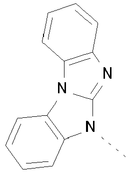 | 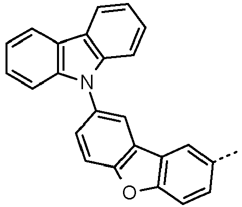 |
| **C-53** | 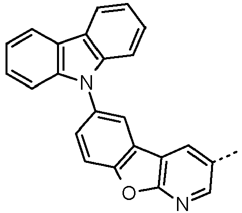 | 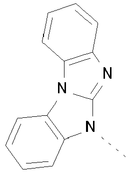 |
| **C-54** | 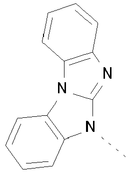 | 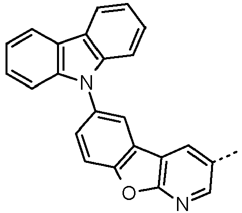 |
| **C-55** | 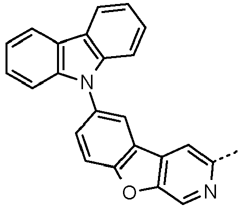 | 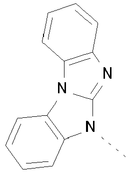 |
| **C-56** | 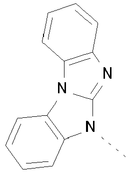 | 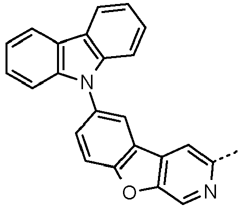 |
| **C-57** | H | 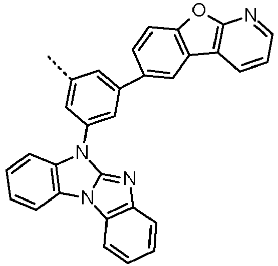 |
| **C-58** | 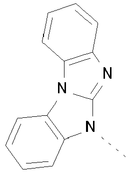 | 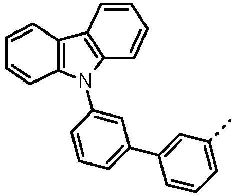 |
| **C-59** | 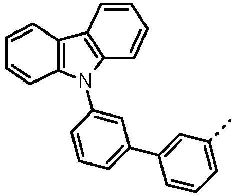 | 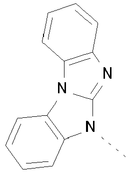 |
| **C-60** | 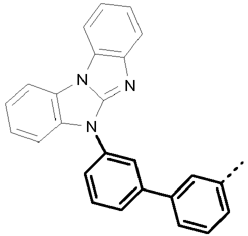 | H |
| **C-61** | H | 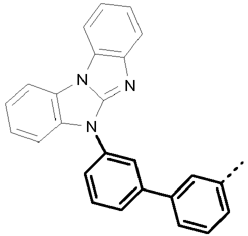 |
| **C-62** | H | 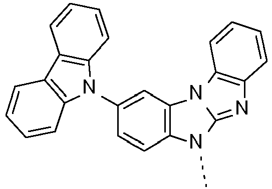 |
| **C-63** | H | 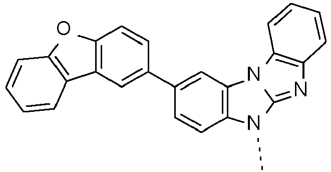 |
| **C-64** | H | 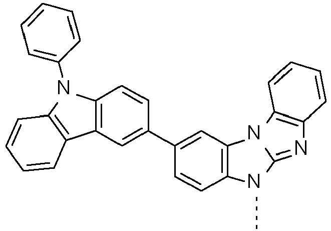 |
| **C-65** | H | 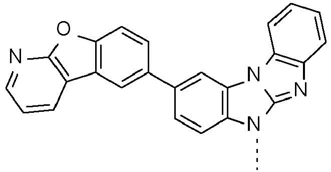 |
| **C-66** | 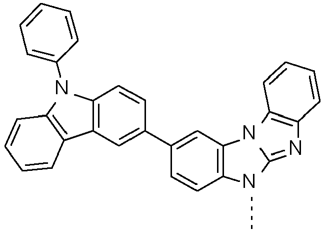 | 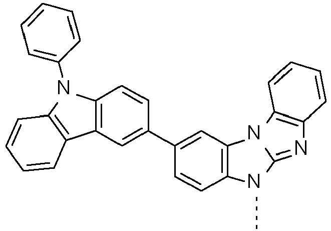 |
| **C-67** | 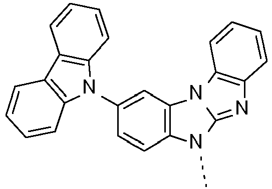 | 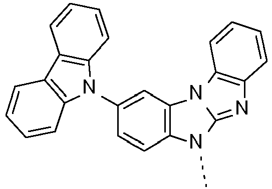 |
| **C-68** | 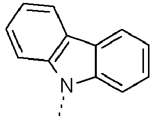 | 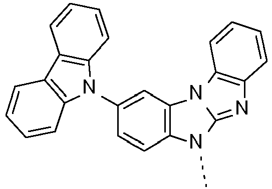 |
| **C-69** | 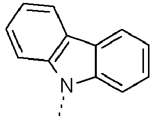 | 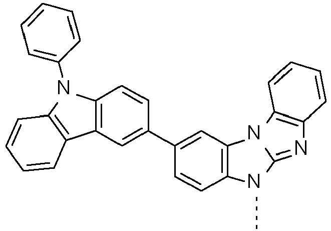 |
| **C-70** | 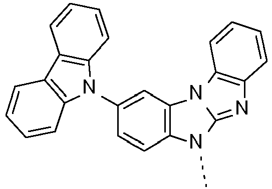 | 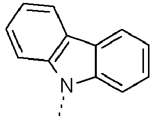 |
| **C-71** | 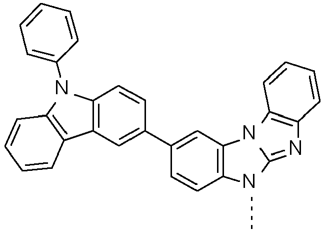 | 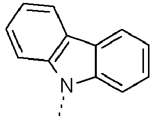 |
| **C-72** | -C₈H₁₇ | 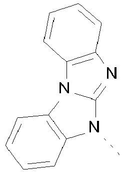 |
| **C-73** | -C₈H₁₇ | 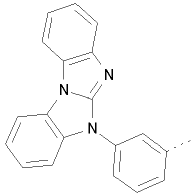 |
| **C-74** | 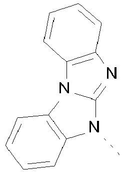 | -C₈H₁₇ |
| **C-75** | 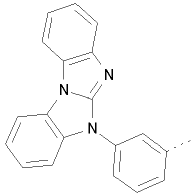 | -C₈H₁₇ |

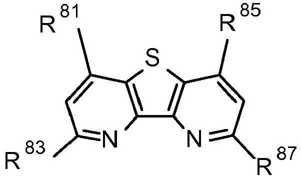

| **Cmp.** | **R⁸¹** | **R⁸³** | **R⁸⁵** | **R⁸⁷** |
|---|---|---|---|---|
| **D-1** | 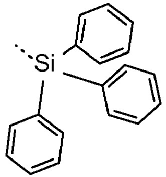 | 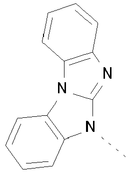 | 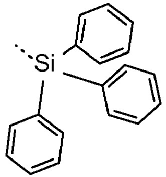 | 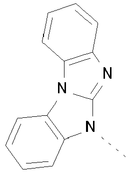 |
| **D-2** | H | 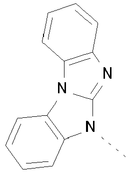 | H | 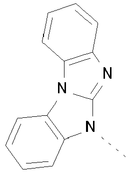 |
| **D-3** | H | 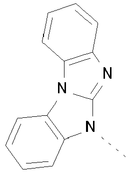 | H | H |
| **D-4** | H | 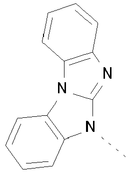 | 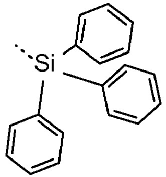 | 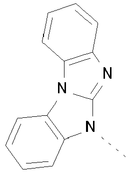 |
| **D-5** | 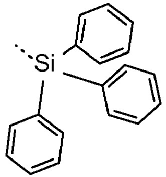 | 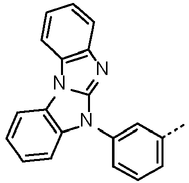 | 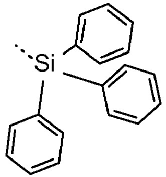 | 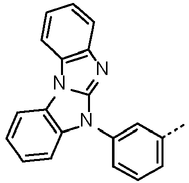 |
| **D-6** | H | 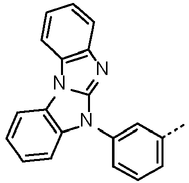 | H | 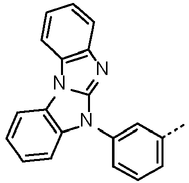 |
| **D-7** | H | 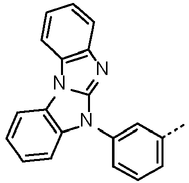 | H | |
| **D-8** | H | 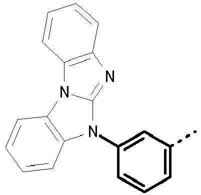 | 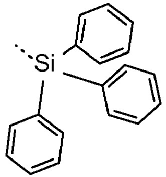 | 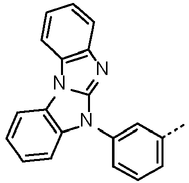 |
| **D-9** | 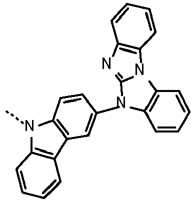 | H | H | H |

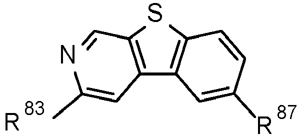

| **Compound** | **R⁸³** | **R⁸⁷** |
|---|---|---|
| **E-1** | 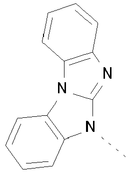 | 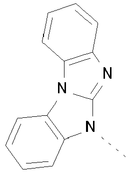 |
| **E-2** | 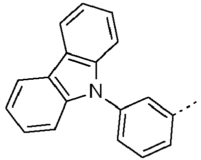 | 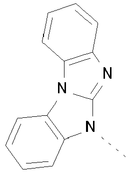 |
| **E-3** | 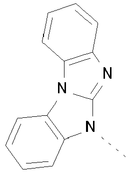 | 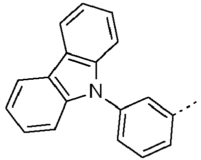 |
| **E-4** | 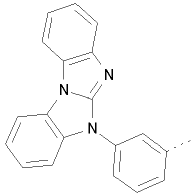 | 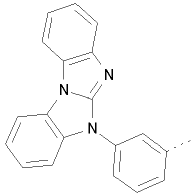 |
| **E-5** | H | 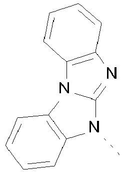 |
| **E-6** | 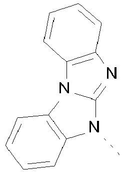 | H |
| **E-7** | H | 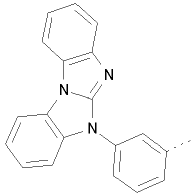 |
| **E-8** | 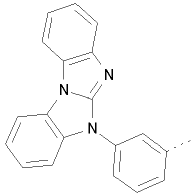 | H |
| **E-9** | H | 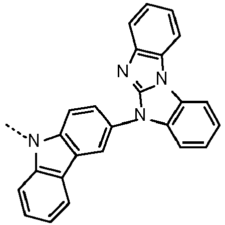 |
| **E-10** | 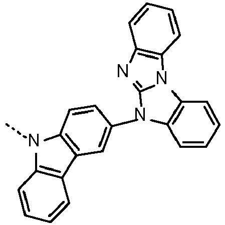 | H |
| **E-11** | 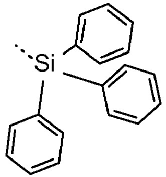 | 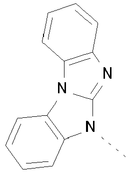 |
| **E-12** | 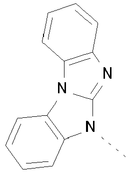 | 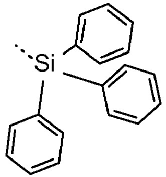 |
| **E-13** | 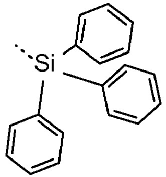 | 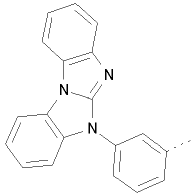 |
| **E-14** | 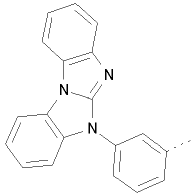 | 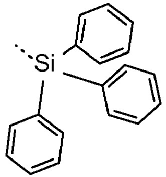 |
| **E-15** | 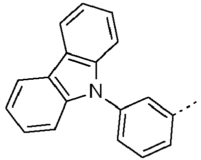 | 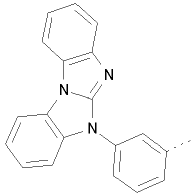 |
| **E-16** | 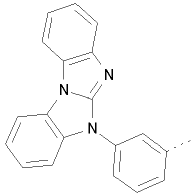 | 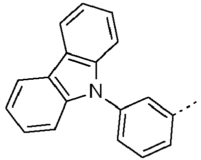 |
| **E-17** | 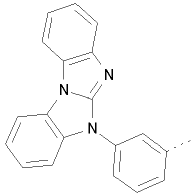 | 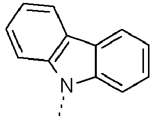 |
| **E-18** | 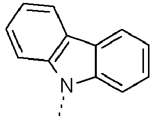 | 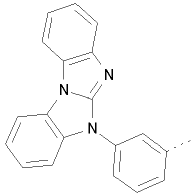 |
| **E-19** | 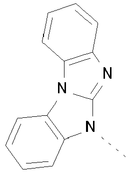 | 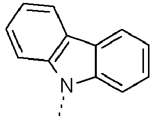 |
| **E-20** | 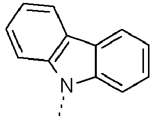 | 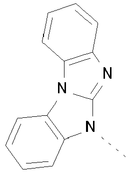 |
| **E-21** | 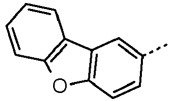 | 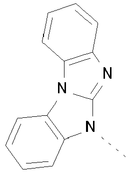 |
| **E-22** | 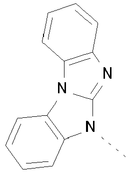 | 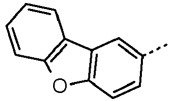 |
| **E-23** | 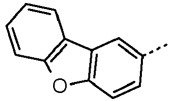 | 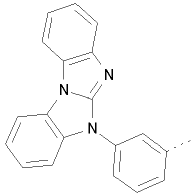 |
| **E-24** | 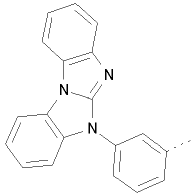 | 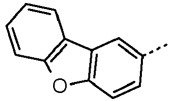 |
| **E-25** | 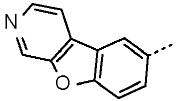 | 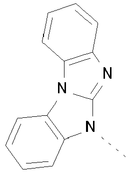 |
| **E-26** | 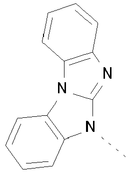 | 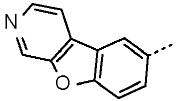 |
| **E-27** | 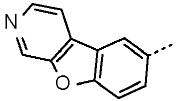 | 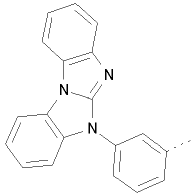 |
| **E-28** | 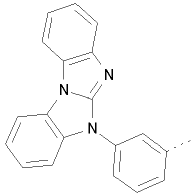 | 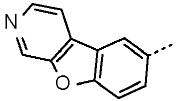 |
| **E-29** | 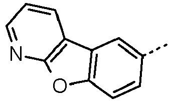 | 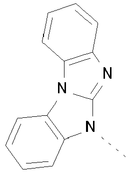 |
| **E-30** | 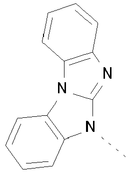 | 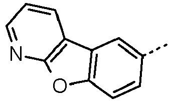 |
| **E-31** | 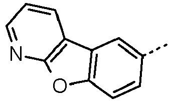 | 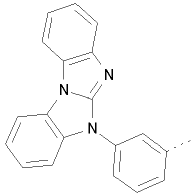 |
| **E-32** | 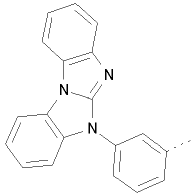 | 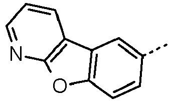 |
| **E-33** | 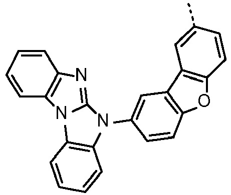 | 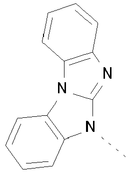 |
| **E-34** | 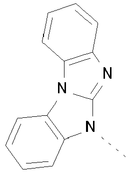 | 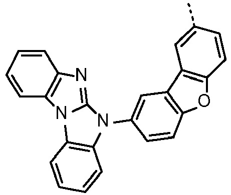 |
| **E-35** | 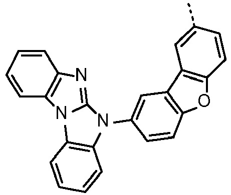 | H |
| **E-36** | H | 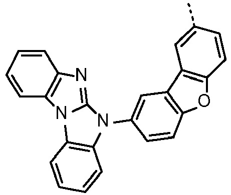 |
| **E-37** | 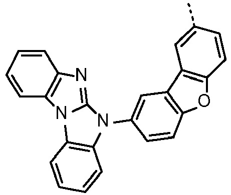 | 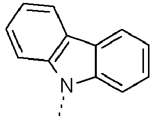 |
| **E-38** | 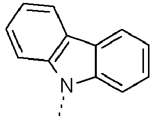 | 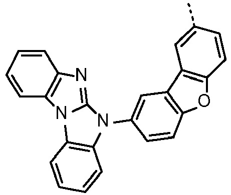 |
| **E-39** | 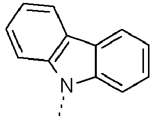 | 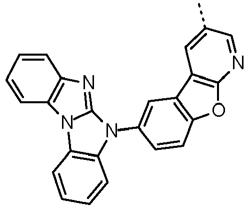 |
| **E-40** | 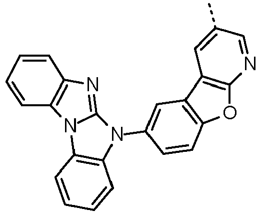 | 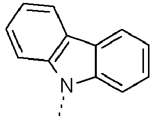 |
| **E-41** | 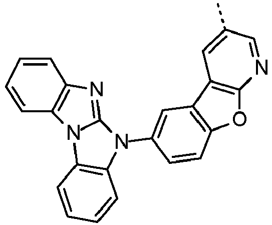 | H |
| **E-42** | H | 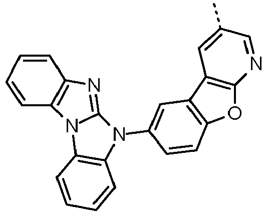 |
| **E-43** | 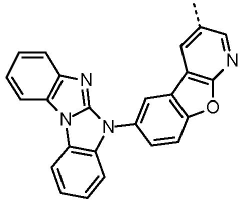 | 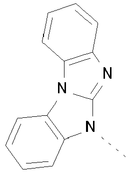 |
| **E-44** | 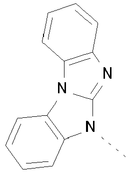 | 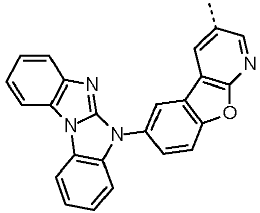 |
| **E-45** | 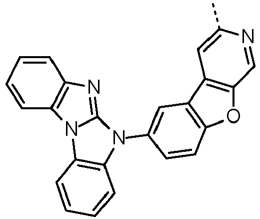 | 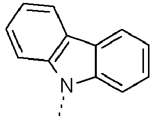 |
| **E-46** | 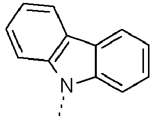 | 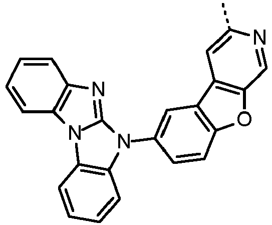 |
| **E-47** | 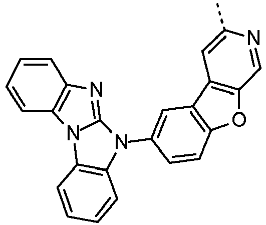 | H |
| **E-48** | H | 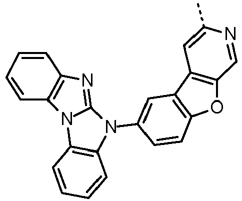 |
| **E-49** | 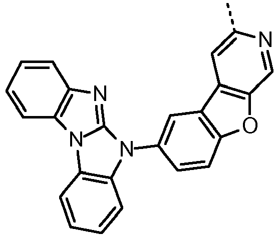 | 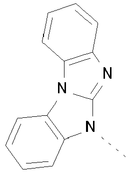 |
| **E-50** | 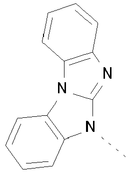 | 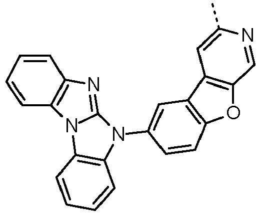 |
| **E-51** | 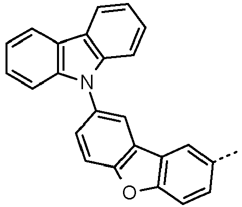 | 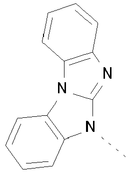 |
| **E-52** | 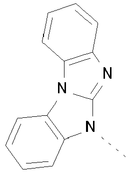 | 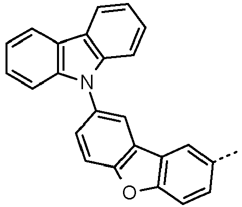 |
| **E-53** | 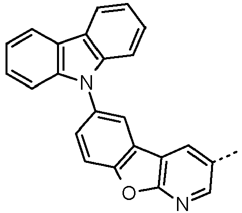 | 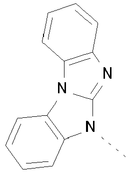 |
| **E-54** | 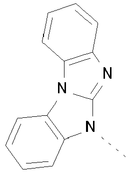 | 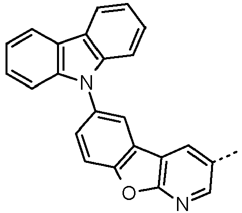 |
| **E-55** | 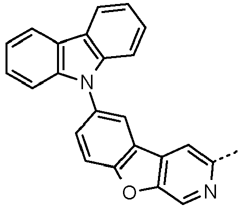 | 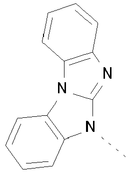 |
| **E-56** | 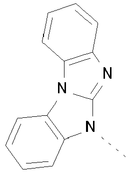 | 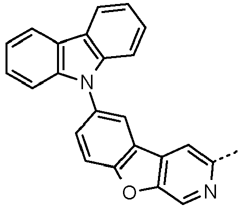 |
| **E-57** | H | 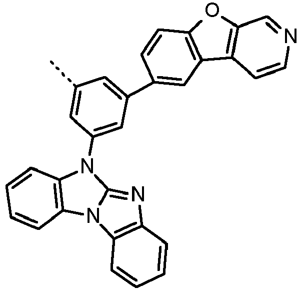 |
| **E-58** | 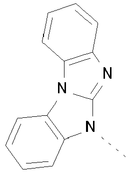 | 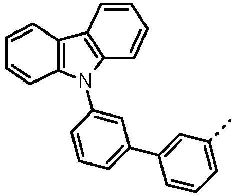 |
| **E-59** | 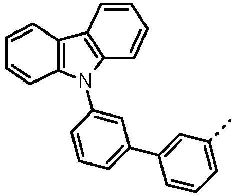 | 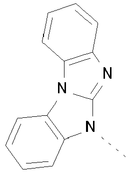 |
| **E-60** | 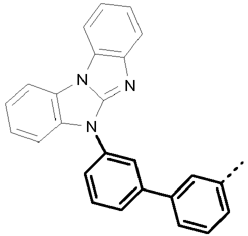 | H |
| **E-61** | H | 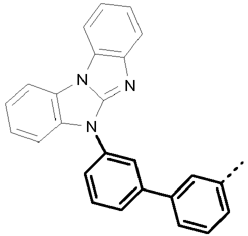 |
| **E-62** | H | 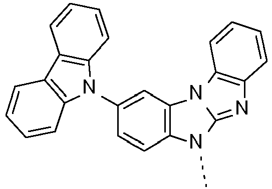 |
| **E-63** | H | 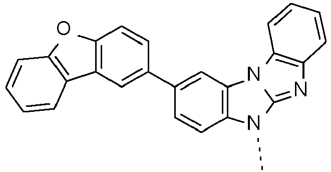 |
| **E-64** | H | 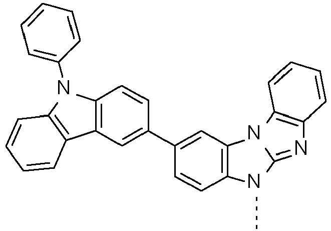 |
| **E-65** | H | 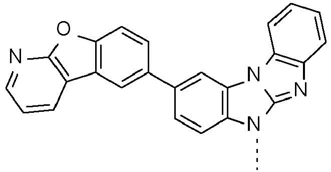 |
| **E-66** | 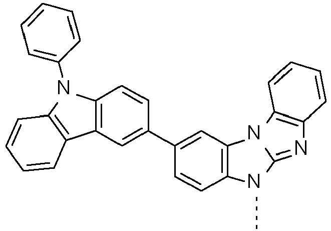 | 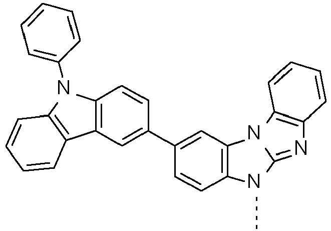 |
| **E-67** | 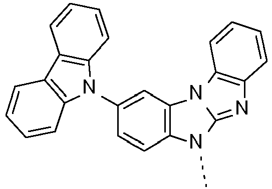 | 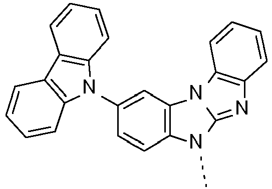 |
| **E-68** | 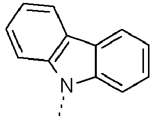 | 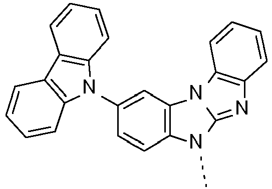 |
| **E-69** | 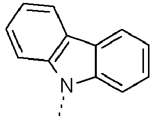 | 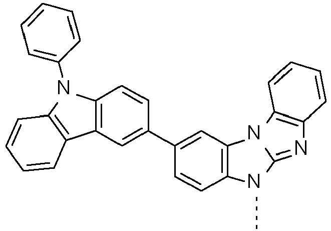 |
| **E-70** | 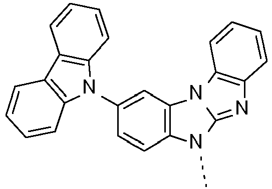 | 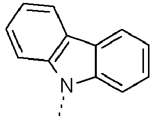 |
| **E-71** | 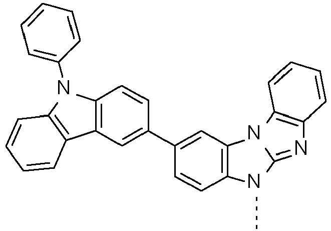 | 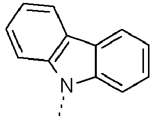 |
| **E-72** | -C₈H₁₇ | 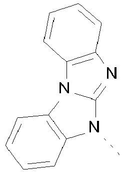 |
| **E-73** | -C₈H₁₇ | 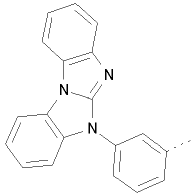 |
| **E-74** | 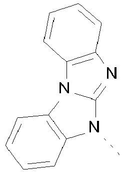 | -C₈H₁₇ |
| **E-75** | 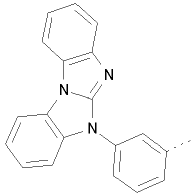 | -C₈H₁₇ |

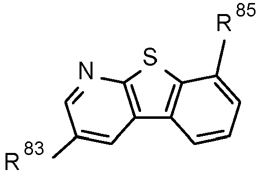

| **Compound** | **R⁸³** | **R⁸⁵** |
|---|---|---|
| **F-1** | 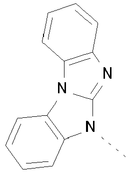 | 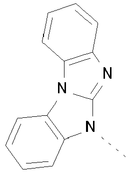 |
| **F-2** | 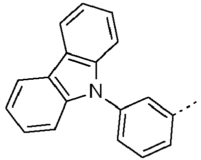 | 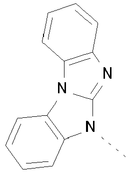 |
| **F-3** | 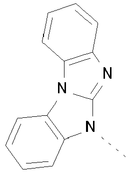 | 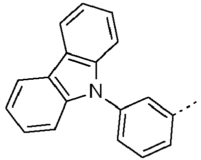 |
| **F-4** | 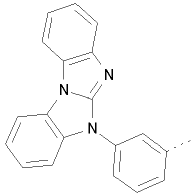 | 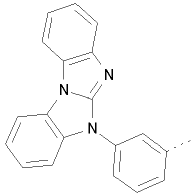 |
| **F-5** | H | 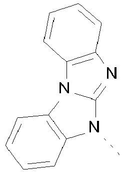 |
| **F-6** | 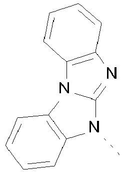 | H |
| **F-7** | H | 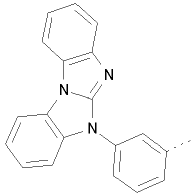 |
| **F-8** | 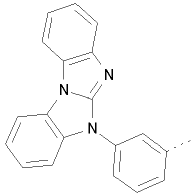 | H |
| **F-9** | H | 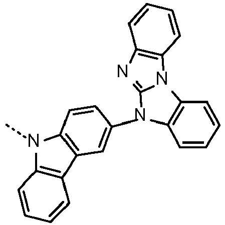 |
| **F-10** | 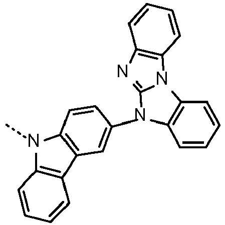 | H |
| **F-11** | 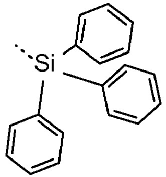 | 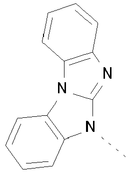 |
| **F-12** | 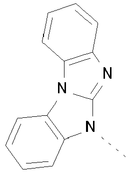 | 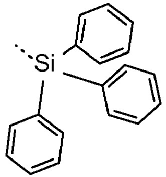 |
| **F-13** | 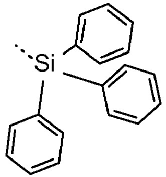 | 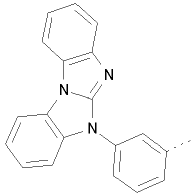 |
| **F-14** | 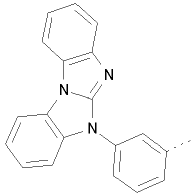 | 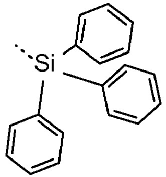 |
| **F-15** | 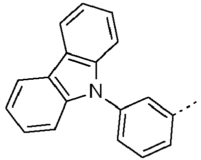 | 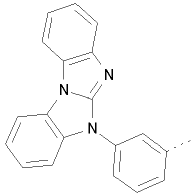 |
| **F-16** | 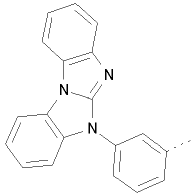 | 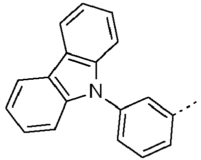 |
| **F-17** | 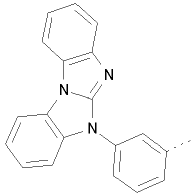 | 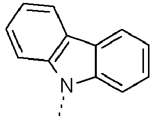 |
| **F-18** | 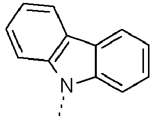 | 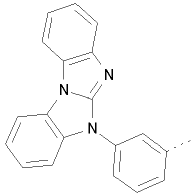 |
| **F-19** | 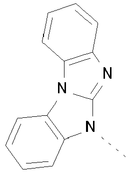 | 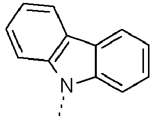 |
| **F-20** | 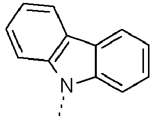 | 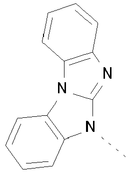 |
| **F-21** | 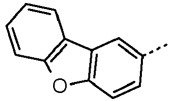 | 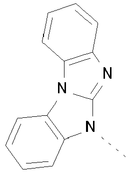 |
| **F-22** | 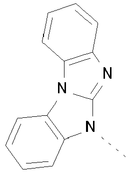 | 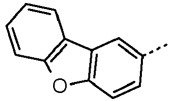 |
| **F-23** | 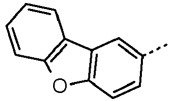 | 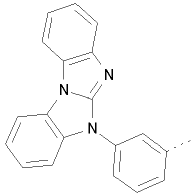 |
| **F-24** | 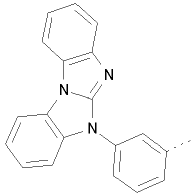 | 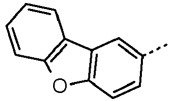 |
| **F-25** | 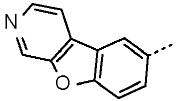 | 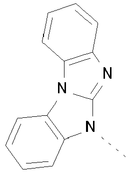 |
| **F-26** | 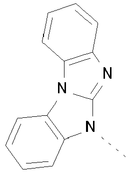 | 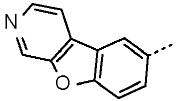 |
| **F-27** | 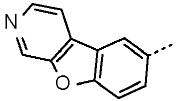 | 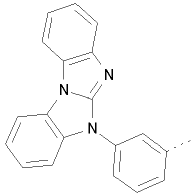 |
| **F-28** | 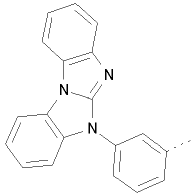 | 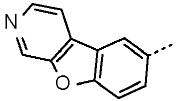 |
| **F-29** | 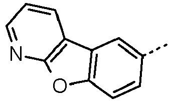 | 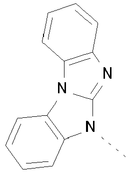 |
| **F-30** | 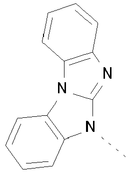 | 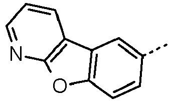 |
| **F-31** | 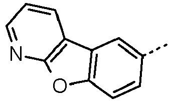 | 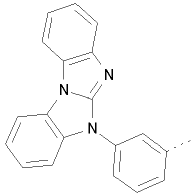 |
| **F-32** | 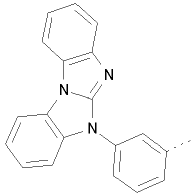 | 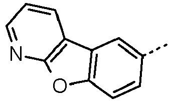 |
| **F-33** | 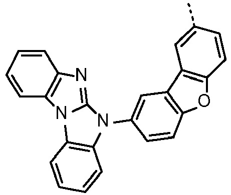 | 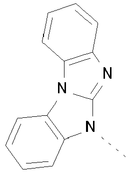 |
| **F-34** | 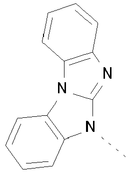 | 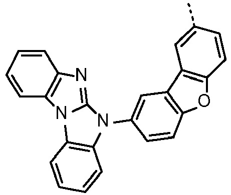 |
| **F-35** | 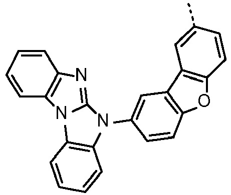 | H |
| **F-36** | H | 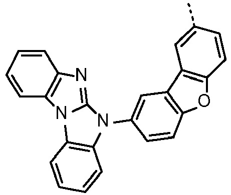 |
| **F-37** | 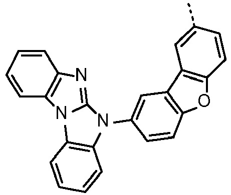 | 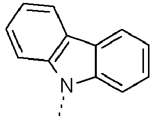 |
| **F-38** | 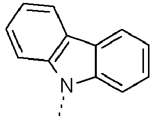 | 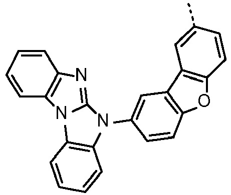 |
| **F-39** | 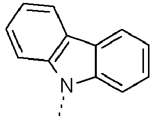 | 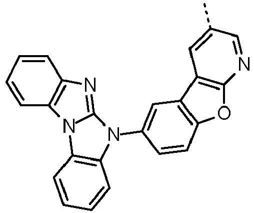 |
| **F-40** | 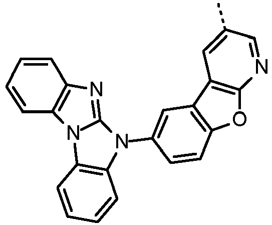 | 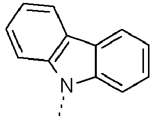 |
| **F-41** | 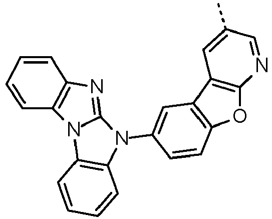 | H |
| **F-42** | H | 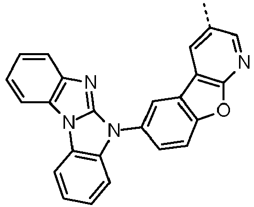 |
| **F-43** | 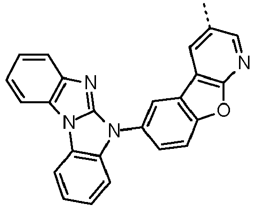 | 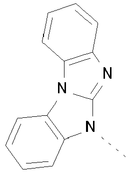 |
| **F-44** | 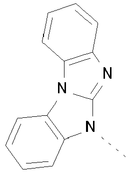 | 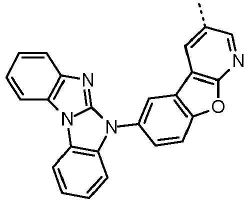 |
| **F-45** | 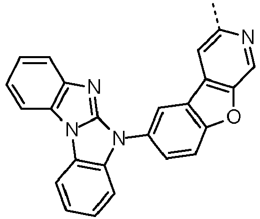 | 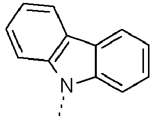 |
| **F-46** | 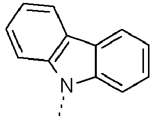 | 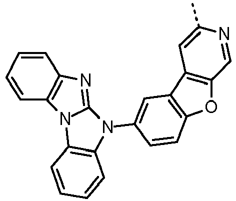 |
| **F-47** | 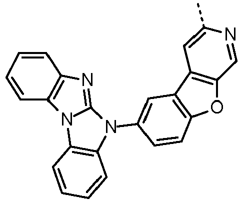 | H |
| **F-48** | H | 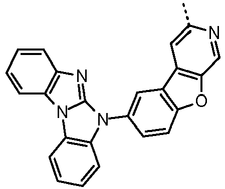 |
| **F-49** | 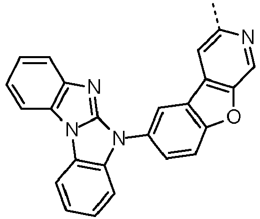 | 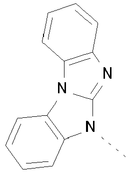 |
| **F-50** | 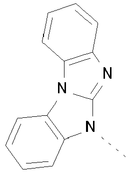 | 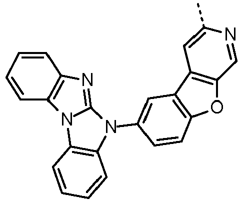 |
| **F-51** | 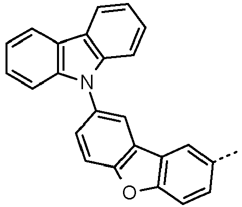 | 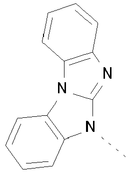 |
| **F-52** | 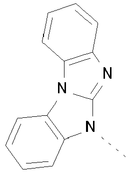 | 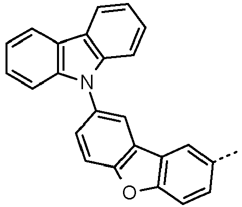 |
| **F-53** | 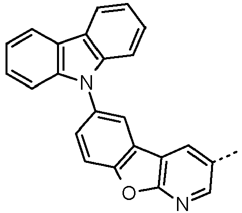 | 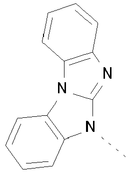 |
| **F-54** | 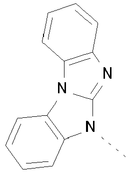 | 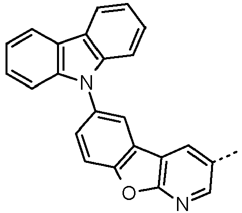 |
| **F-55** | 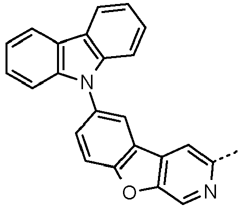 | 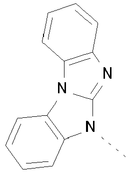 |
| **F-56** | 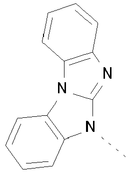 | 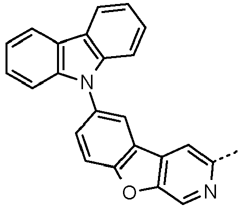 |
| **F-57** | H | 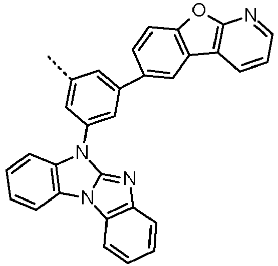 |
| **F-58** | 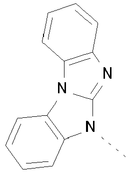 | 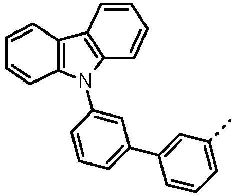 |
| **F-59** | 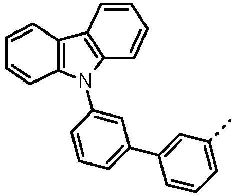 | 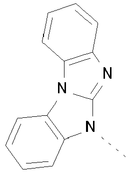 |
| **F-60** | 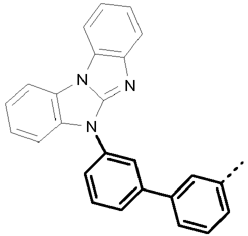 | H |
| **F-61** | H | 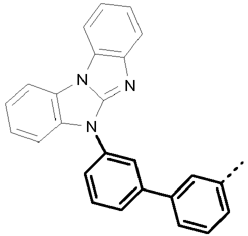 |
| **F-62** | H | 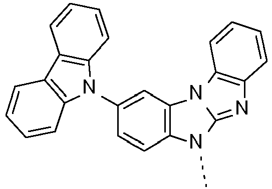 |
| **F-63** | H | 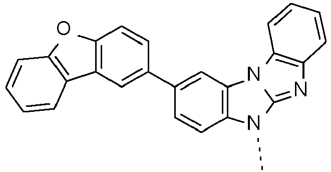 |
| **F-64** | H | 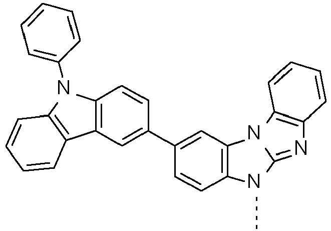 |
| **F-65** | H | 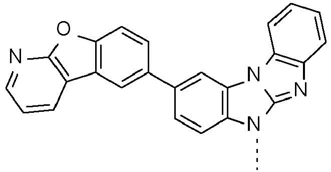 |
| **F-66** | 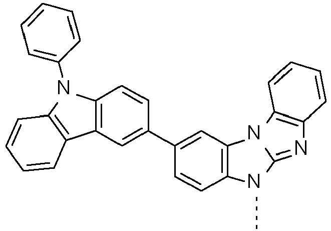 | 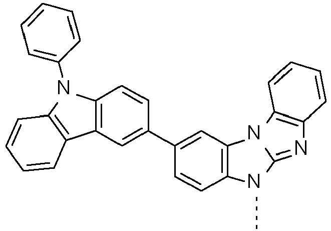 |
| **F-67** | 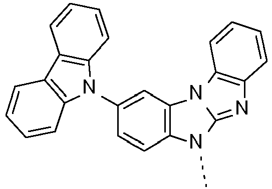 | 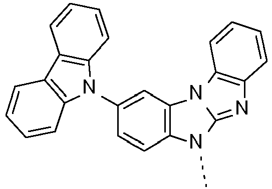 |
| **F-68** | 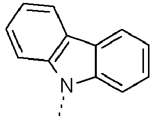 | 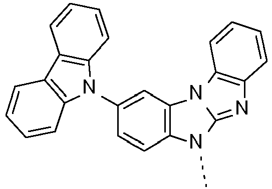 |
| **F-69** | 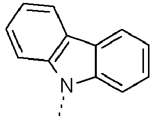 | 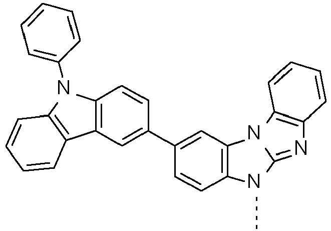 |
| **F-70** | 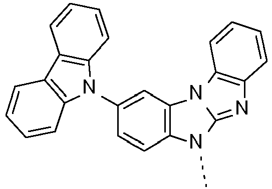 | 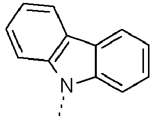 |
| **F-71** | 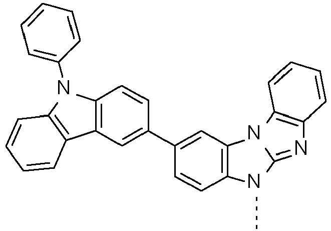 | 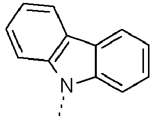 |
| **F-72** | -C₈H₁₇ | 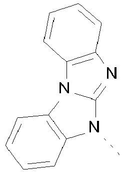 |
| **F-73** | -C₈H₁₇ | 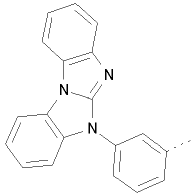 |
| **F-74** | 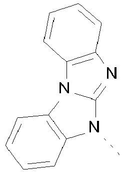 | -C₈H₁₇ |
| **F-75** | 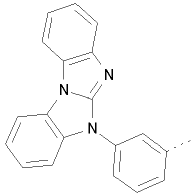 | -C₈H₁₇ |

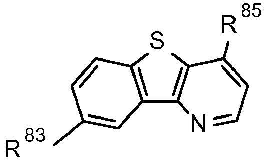

| **Compound** | **R⁸³** | **R⁸⁵** |
|---|---|---|
| **G-1** | 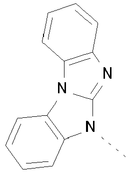 | 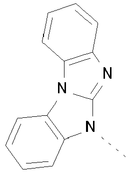 |
| **G-2** | 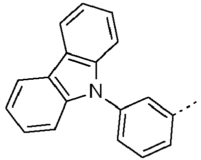 | 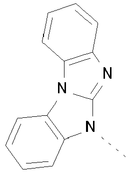 |
| **G-3** | 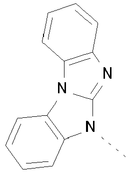 | 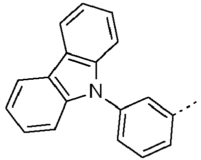 |
| **G-4** | 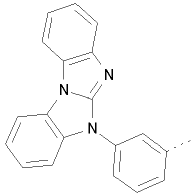 | 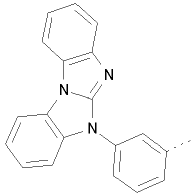 |
| **G-5** | H | 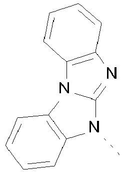 |
| **G-6** | 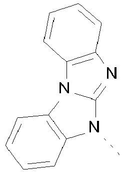 | H |
| **G-7** | H | 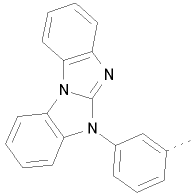 |
| **G-8** | 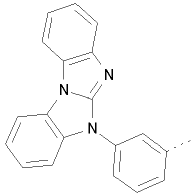 | H |
| **G-9** | H | 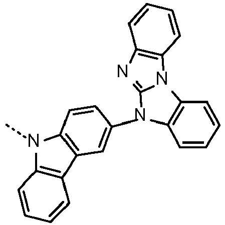 |
| **G-10** | 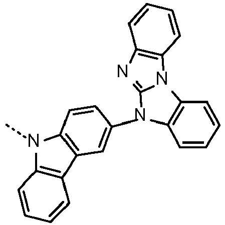 | H |
| **G-11** | 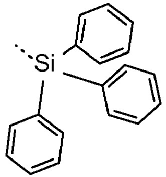 | 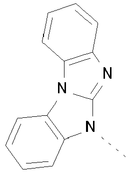 |
| **G-12** | 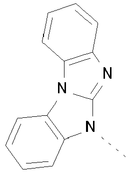 | 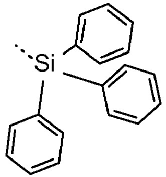 |
| **G-13** | 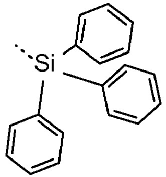 | 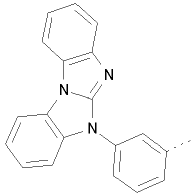 |
| **G-14** | 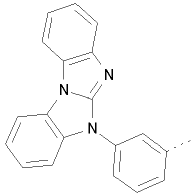 | 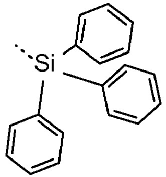 |
| **G-15** | 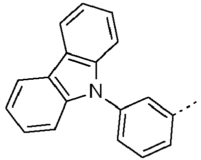 | 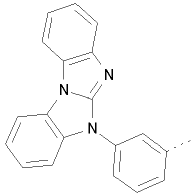 |
| **G-16** | 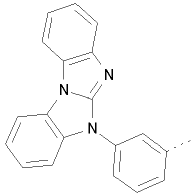 | 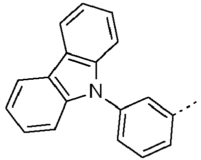 |
| **G-17** | 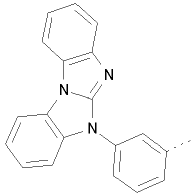 | 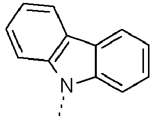 |
| **G-18** | 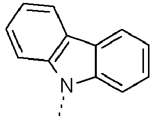 | 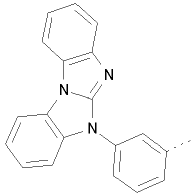 |
| **G-19** | 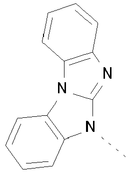 | 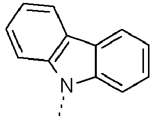 |
| **G-20** | 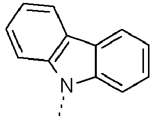 | 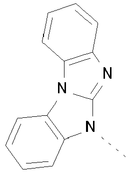 |
| **G-21** | 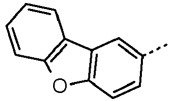 | 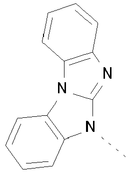 |
| **G-22** | 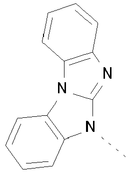 | 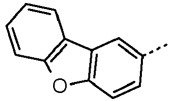 |
| **G-23** | 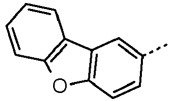 | 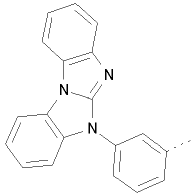 |
| **G-24** | 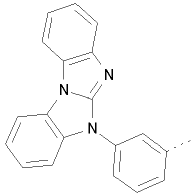 | 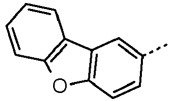 |
| **G-25** | 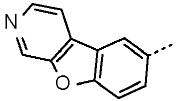 | 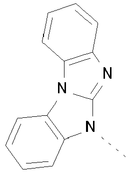 |
| **G-26** | 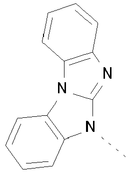 | 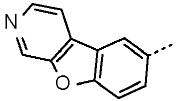 |
| **G-27** | 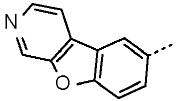 | 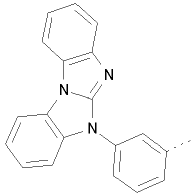 |
| **G-28** | 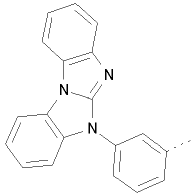 | 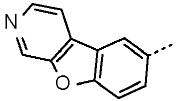 |
| **G-29** | 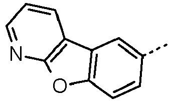 | 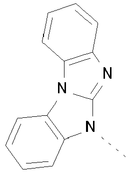 |
| **G-30** | 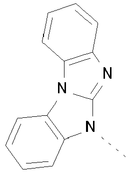 | 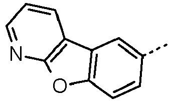 |
| **G-31** | 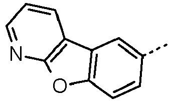 | 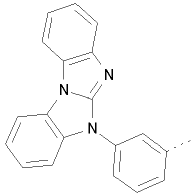 |
| **G-32** | 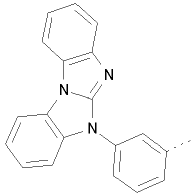 | 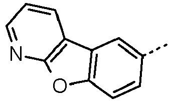 |
| **G-33** | 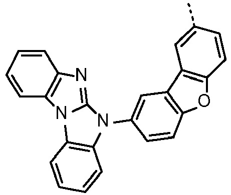 | 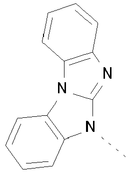 |
| **G-34** | 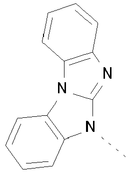 | 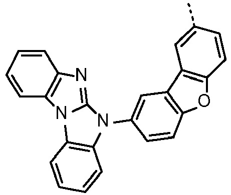 |
| **G-35** | 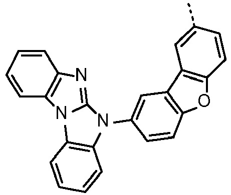 | H |
| **G-36** | H | 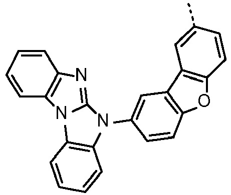 |
| **G-37** | 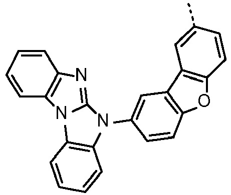 | 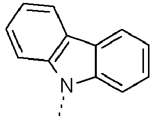 |
| **G-38** | 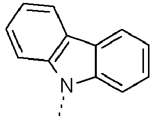 | 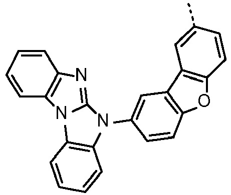 |
| **G-39** | 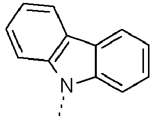 | 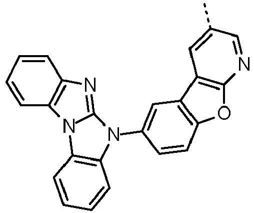 |
| **G-40** | 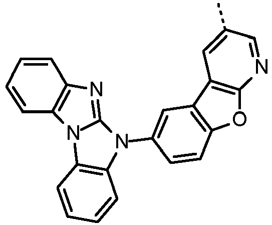 | 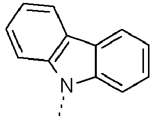 |
| **G-41** | 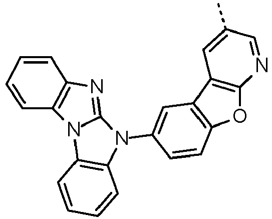 | H |
| **G-42** | H | 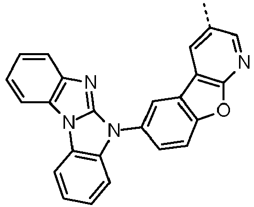 |
| **G-43** | 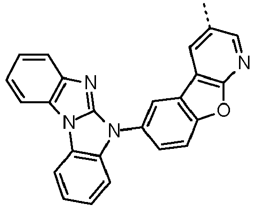 | 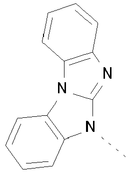 |
| **G-44** | 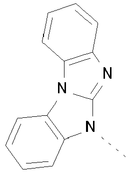 | 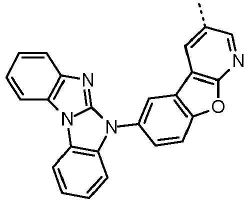 |
| **G-45** | 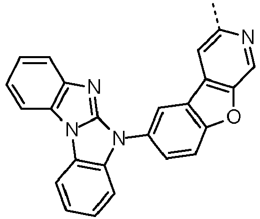 | 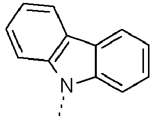 |
| **G-46** | 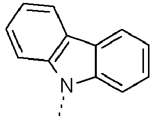 | 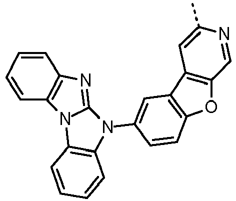 |
| **G-47** | 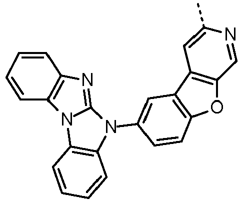 | H |
| **G-48** | H | 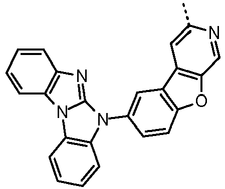 |
| **G-49** | 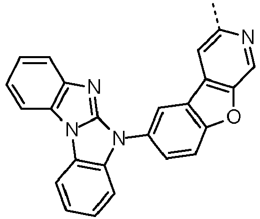 | 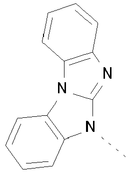 |
| **G-50** | 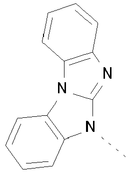 | 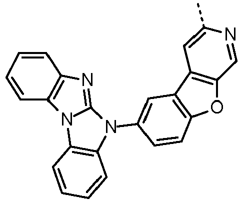 |
| **G-51** | 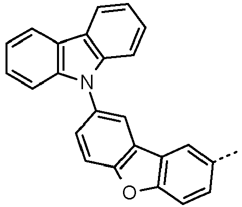 | 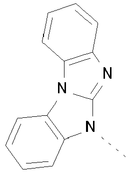 |
| **G-52** | 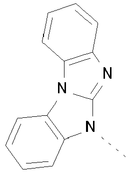 | 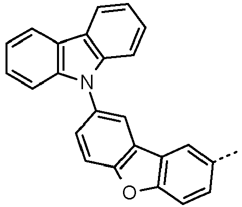 |
| **G-53** | 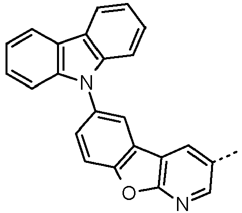 | 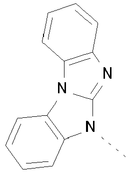 |
| **G-54** | 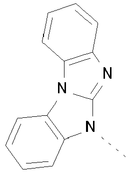 | 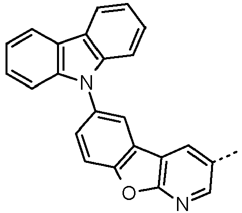 |
| **G-55** | 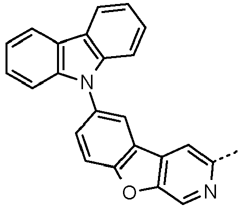 | 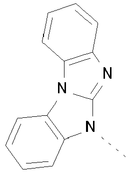 |
| **G-56** | 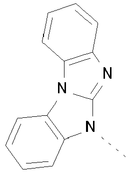 | 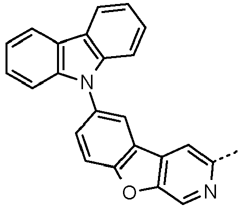 |
| **G-57** | H | 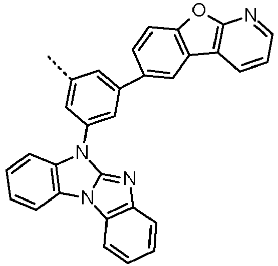 |
| **G-58** | 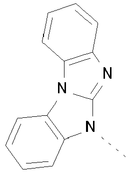 | 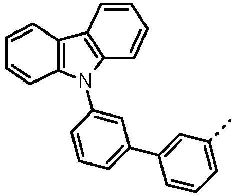 |
| **G-59** | 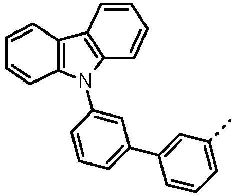 | 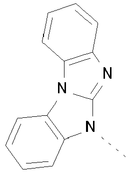 |
| **G-60** | 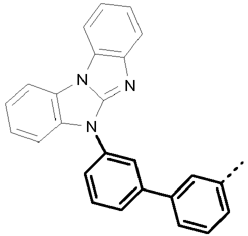 | H |
| **G-61** | H | 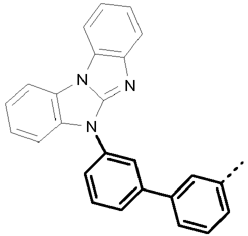 |
| **G-62** | H | 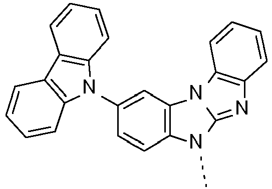 |
| **G-63** | H | 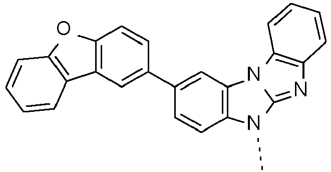 |
| **G-64** | H | 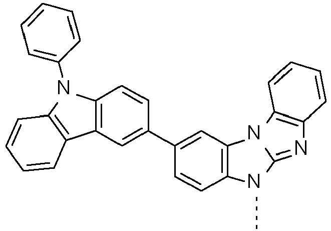 |
| **G-65** | H | 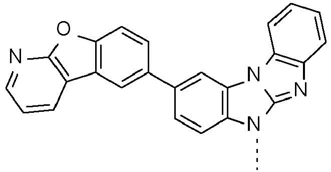 |
| **G-66** | 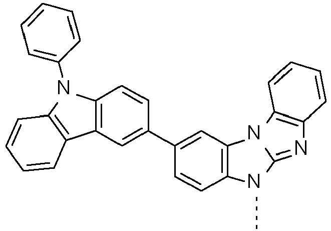 | 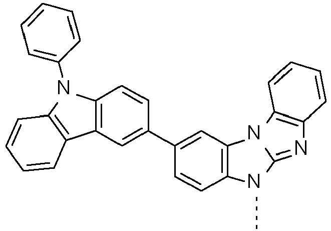 |
| **G-67** | 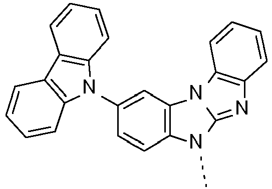 | 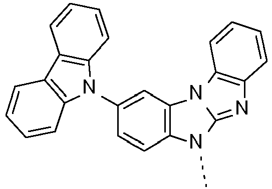 |
| **G-68** | 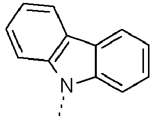 | 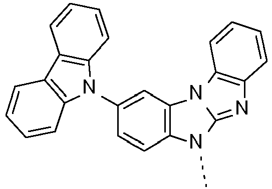 |
| **G-69** | 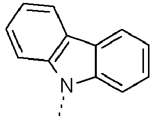 | 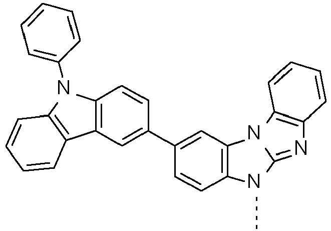 |
| **G-70** | 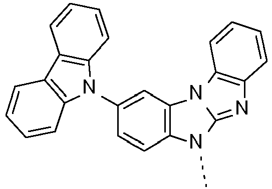 | 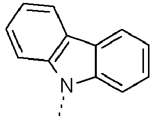 |
| **G-71** | 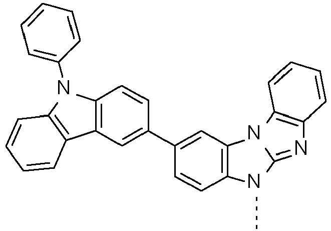 | 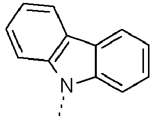 |
| **G-72** | -C₈H₁₇ | 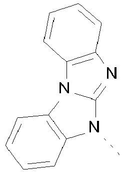 |
| **G-73** | -C₈H₁₇ | 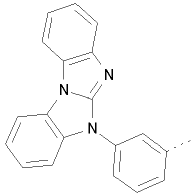 |
| **G-74** | 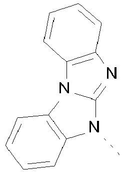 | -C₈H₁₇ |
| **G-75** | 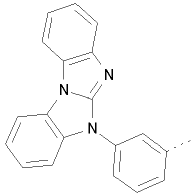 | -C₈H₁₇ |

| | | |
|---|---|---|
| ---- indicates the bonding to the azadibenzothiophene. | | |

In a particularly preferred embodiment the present invention is directed to compounds of formula (**Ia**), wherein R⁸³ is a group of formula (**XIIa**), (**XIIb**), (**XIIc**), (**XIId**), (**XIIe**), (**XIIf**), (**XIIg**), (**XIIj**), (**XIIk**), (**XIIn**), (**XIIo**), (**XIIq**), (**XIIs**), (**XIIt**), (**XIIu**), (**XIIv**), (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined above, and
R⁸⁷ is H, a C₁-C₂₅alkyl group, a group of formula (**XIIa**), (**XIIb**), (**XIIc**), (**XIId**), (**XIIe**), (**XIIf**), (**XIIg**), (**XIIj**), (**XIIk**), (**XIIn**), (**XIIo**), (**XIIq**), (**XIIs**), (**XIIt**), (**XIIu**), (**XIIv**) (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined above, or a group of formula (**XIVb**), (**XIVc**), (**XIVd**), (**XIVe**), (**XIVf**), (**XIVg**), (**XIVo**), (**XIVp**), (**XIVq**), (**XIVr**), (**XIVt**), (**XIVu**), (**XIVv**), (**XIVw**), (**XIVx**), (**XIVz**), (**XVa**), (**XVb**), (**XVc**), (**XVk**), (**XVl**), (**XVo**), (**XVp**), (**XVs**), (**XVw**), (**XVx**), (**XVy**), (**XVIa**), (**XVIb**), (**XVIc**) or (**XVId**) as defined above; or to compounds of formula (**Ia**), wherein R⁸⁷ is a group of formula (**XIIa**), (**XIIb**), (**XIIc**), (**XIId**), **(XIIe),** (**XIIf**), (**XIIg**), (**XIIj**), (**XIIk**), (**XIIn**), (**XIIo**), (**XIIq**), (**XIIs**), (**XIIt**), (**XIIu**), (**XIIv**), (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined above, and
R⁸³ is H, a C₁-C₂₅alkyl group, a group of formula (**XIIa**), (**XIIb**), (**XIIc**), (**XIId**), **(XIIe),** (**XIIf**), (**XIIg**), (**XIIj**), (**XIIk**), (**XIIn**), (**XIIo**), (**XIIq**), (**XIIs**), (**XIIt**), (**XIIu**), (**XIIv**) (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined above, or a group of formula (**XIVb**), (**XIVc**), (**XIVd**), (**XIVe**), (**XIVf**), (**XIVg**), (**XIVo**), (**XIVp**), (**XIVq**), (**XIVr**), (**XIVt**), (**XIVu**), (**XIVv**), (**XIVw**), (**XIVx**), (**XIVz**), (**XVa**), (**XVb**), (**XVc**), (**XVk**), (**XVl**), (**XVo**), (**XVp**), (**XVs**), (**XVw**), (**XVx**), (**XVy**), (**XVIa**), (**XVIb**), (**XVIc**) or (**XVId**) as defined above; such as, for example, compound 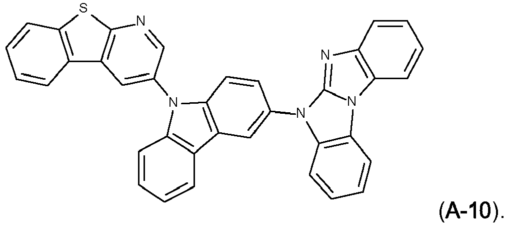 Compound **A-10** shows electron and hole transporting properties and, hence, can be used as bipolar host.

In another particularly preferred embodiment the present invention is directed to compounds of formula (**Im**), wherein R⁸³ is a group of formula (**XIIa**), (**XIIb**), (**XIIc**), (**XIId**), (**XIIe**), (**XIIf**), (**XIIg**), (**XIIj**), (**XIIk**), (**XIIn**), (**XIIo**), (**XIIq**), (**XIIs**), (**XIIt**), (**XIIu**), (**XIIv**), (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined above, and
R⁸⁵ is H, a C₁-C₂₅alkyl group, a group of formula (**XIIa**), (**XIIb**), (**XIIc**), (**XIId**), **(XIIe),** (**XIIf**), (**XIIg**), (**XIIj**), (**XIIk**), (**XIIn**), (**XIIo**), (**XIIq**), (**XIIs**), (**XIIt**), (**XIIu**), (**XIIv**) (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined above, or a group of formula (**XIVb**), (**XIVc**), (**XIVd**), (**XIVe**), (**XIVf**), (**XIVg**), (**XIVo**), (**XIVp**), (**XIVq**), (**XIVr**), (**XIVt**), (**XIVu**), (**XIVv**), (**XIVw**), (**XIVx**), (**XIVz**), (**XVa**), (**XVb**), (**XVc**), (**XVk**), (**XVl**), (**XVo**), (**XVp**), (**XVs**), (**XVw**), (**XVx**), (**XVy**), (**XVIa**), (**XVIb**), (**XVIc**) or (**XVId**) as defined above; or compounds of formula (**Im**), wherein R⁸⁵ is a group of formula (**XIIa**), (**XIIb**), (**XIIc**), (**XIId**), (**XIIe**), (**XIIf**), (**XIIg**), (**XIIj**), (**XIIk**), (**XIIn**), (**XIIo**), (**XIIq**), (**XIIs**), (**XIIt**), (**XIIu**), (**XIIv**), (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined above, and
R⁸³ is H, a C₁-C₂₅alkyl group, a group of formula (**XIIa**), (**XIIb**), (**XIIc**), (**XIId**), **(XIIe),** (**XIIf**), (**XIIg**), (**XIIj**), (**XIIk**), (**XIIn**), (**XIIo**), (**XIIq**), (**XIIs**), (**XIIt**), (**XIIu**), (**XIIv**) (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined above, or a group of formula (**XIVb**), (**XIVc**), (**XIVd**), (**XIVe**), (**XIVf**), (**XIVg**), (**XIVo**), (**XIVp**), (**XIVq**), (**XIVr**), (**XIVt**), (**XIVu**), (**XIVv**), (**XIVw**), (**XIVx**), (**XIVz**), (**XVa**), (**XVb**), (**XVc**), (**XVk**), (**XVl**), (**XVo**), (**XVp**), (**XVs**), (**XVw**), (**XVx**), (**XVy**), (**XVIa**), (**XVIb**), (**XVIc**) or (**XVId**) as defined above, such as, for example, a compound of formula 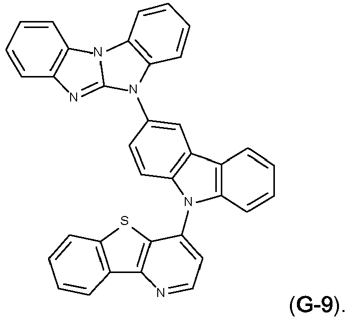 Compounds, such as, for example, (**G-9**) can advantageously be used as host and/or hole and electron transport material. Compounds, such as, for example, 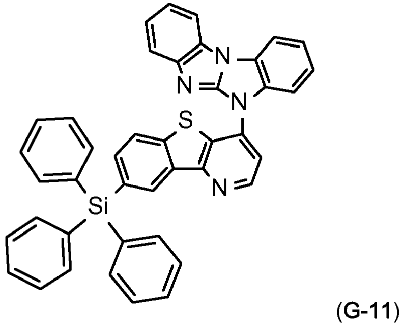 are particularly suitable as electron and hole transport material.

Halogen is fluorine, chlorine, bromine and iodine.

C₁-C₂₅alkyl (C₁-C₁₈alkyl) is typically linear or branched, where possible. Examples are methyl, ethyl, n-propyl, isopropyl, n-butyl, sec.-butyl, isobutyl, tert.-butyl, n-pentyl, 2-pentyl, 3-pentyl, 2,2-dimethylpropyl, 1,1,3,3-tetramethylpentyl, n-hexyl, 1-methylhexyl, 1,1,3,3,5,5-hexamethylhexyl, n-heptyl, isoheptyl, 1,1,3,3-tetramethylbutyl, 1-methylheptyl, 3-methylheptyl, n-octyl, 1,1,3,3-tetramethylbutyl and 2-ethylhexyl, n-nonyl, decyl, undecyl, dodecyl, tridecyl, tetradecyl, pentadecyl, hexadecyl, heptadecyl, or octadecyl. C₁-C₈alkyl is typically methyl, ethyl, n-propyl, isopropyl, n-butyl, sec.-butyl, isobutyl, tert.-butyl, n-pentyl, 2-pentyl, 3-pentyl, 2,2-dimethyl-propyl, n-hexyl, n-heptyl, n-octyl, 1,1,3,3-tetramethylbutyl and 2-ethylhexyl. C₁-C₄alkyl is typically methyl, ethyl, n-propyl, isopropyl, n-butyl, sec.-butyl, isobutyl, tert.-butyl.

C₁-C₂₅alkoxy groups (C₁-C₁₈alkoxy groups) are straight-chain or branched alkoxy groups, e.g. methoxy, ethoxy, n-propoxy, isopropoxy, n-butoxy, sec-butoxy, tert-butoxy, amyloxy, isoamyloxy or tert-amyloxy, heptyloxy, octyloxy, isooctyloxy, nonyloxy, decyloxy, un-decyloxy, dodecyloxy, tetradecyloxy, pentadecyloxy, hexadecyloxy, heptadecyloxy and octadecyloxy. Examples of C₁-C₈alkoxy are methoxy, ethoxy, n-propoxy, isopropoxy, n-butoxy, sec.-butoxy, isobutoxy, tert.-butoxy, n-pentyloxy, 2-pentyloxy, 3-pentyloxy, 2,2-dimethylpropoxy, n-hexyloxy, n-heptyloxy, n-octyloxy, 1,1,3,3-tetramethylbutoxy and 2-ethylhexyloxy, preferably C₁-C₄alkoxy such as typically methoxy, ethoxy, n-propoxy, isopropoxy, n-butoxy, sec.-butoxy, isobutoxy, tert.-butoxy.

C₁-C₁₈perfluoroalkyl, especially C₁-C₄perfluoroalkyl, is a branched or unbranched radical such as for example -CF₃, -CF₂CF₃, -CF₂CF₂CF₃, -CF(CF₃)₂, -(CF₂)₃CF₃, and -C(CF₃)₃.

The term "cycloalkyl group" is typically C₃-C₁₈cycloalkyl, especially C₅-C₁₂cycloalkyl, such as cyclopentyl, cyclohexyl, cycloheptyl, cyclooctyl, cyclononyl, cyclodecyl, cycloundecyl, cyclododecyl, preferably cyclopentyl, cyclohexyl, cycloheptyl, or cyclooctyl, which may be unsubstituted or substituted.

C₆-C₂₄aryl (C₆-C₁₈aryl), which optionally can be substituted, is typically phenyl, 4-methylphenyl, 4-methoxyphenyl, naphthyl, especially 1-naphthyl, or 2-naphthyl, biphenylyl, terphenylyl, pyrenyl, 2- or 9-fluorenyl, phenanthryl, or anthryl, which may be unsubstituted or substituted. Phenyl, 1-naphthyl and 2-naphthyl are examples of a C₆-C₁₀aryl group.

C₂-C₃₀heteroaryl represents a ring with five to seven ring atoms or a condensed ring system, wherein nitrogen, oxygen or sulfur are the possible hetero atoms, and is typically a heterocyclic group with five to 30 atoms having at least six conjugated π-electrons such as thienyl, benzothiophenyl, dibenzothiophenyl, thianthrenyl, furyl, furfuryl, 2H-pyranyl, benzofuranyl, isobenzofuranyl, dibenzofuranyl, phenoxythienyl, pyrrolyl, imidazolyl, pyrazolyl, pyridyl, bipyridyl, triazinyl, pyrimidinyl, pyrazinyl, pyridazinyl, indolizinyl, isoindolyl, indolyl, indazolyl, purinyl, quinolizinyl, chinolyl, isochinolyl, phthalazinyl, naphthyridinyl, chinoxalinyl, chinazolinyl, cinnolinyl, pteridinyl, carbazolyl, carbolinyl, benzotriazolyl, benzoxazolyl, phenanthridinyl, acridinyl, pyrimidinyl, phenanthrolinyl, phenazinyl, isothiazolyl, phenothiazinyl, isoxazolyl, furazanyl, 4-imidazo[1,2-a]benzimidazoyl, 5-benzimidazo[1,2-a]benzimidazoyl, carbazolyl, or phenoxazinyl, which can be unsubstituted or substituted. Benzimidazo[1,2-a]benzimidazo-5-yl, benzimidazo[1,2-a]benzimidazo-2-yl, carbazolyl and dibenzofuranyl are examples of a C₂-C₁₄heteroaryl group.

C₆-C₂₄arylen groups, which optionally can be substituted by G, are typically phenylene, 4-methylphenylene, 4-methoxyphenylene, naphthylene, especially 1-naphthylene, or 2-naphthylene, biphenylylene, terphenylylene, pyrenylene, 2- or 9-fluorenylene, phenanthrylene, or anthrylene, which may be unsubstituted or substituted. Preferred C₆-C₂₄arylen groups are 1,3-phenylene, 3,3'-biphenylylene, 3,3'-m-terphenylene, 2- or 9-fluorenylene, phenanthrylene, which may be unsubstituted or substituted.

C₂-C₃₀heteroarylen groups, which optionally can be substituted by G, represent a ring with five to seven ring atoms or a condensed ring system, wherein nitrogen, oxygen or sulfur are the possible hetero atoms, and is typically a heterocyclic group with five to 30 atoms having at least six conjugated -electrons such as thienylene, benzothiophenylene, dibenzothiophenylene, thianthrenylene, furylene, furfurylene, 2H-pyranylene, benzofuranylene, isobenzofuranylene, dibenzofuranylene, phenoxythienylene, pyrrolylene, imidazolylene, pyrazolylene, pyridylene, bipyridylene, triazinylene, pyrimidinylene, pyrazinylene, pyridazinylene, indolizinylene, isoindolylene, indolylene, indazolylene, purinylene, quinolizinylene, chinolylene, isochinolylene, phthalazinylene, naphthyridinylene, chinoxalinylene, chinazolinylene, cinnolinylene, pteridinylene, carbolinylene, benzotriazolylene, benzoxazolylene, phenanthridinylene, acridinylene, pyrimidinylene, phenanthrolinylene, phenazinylene, isothiazolylene, phenothiazinylene, isoxazolylene, furazanylene, carbazolylene, benzimidazo[1,2-a]benzimidazo-2,5-ylene, or phenoxazinylene, which can be unsubstituted or substituted. Preferred C₂-C₃₀heteroarylen groups are pyridylene, triazinylene, pyrimidinylene, carbazolylene, dibenzofuranylene and benzimidazo[1,2-a]benzimidazo-2,5-ylene ( 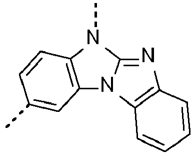 ), which can be unsubstituted or substituted, especially by C₆-C₁₀aryl, C₆-C₁₀aryl which is substituted by C₁-C₄alkyl; or C₂-C₁₄heteroaryl.

Possible substituents of the above-mentioned groups are C₁-C₈alkyl, a hydroxyl group, a mercapto group, C₁-C₈alkoxy, C₁-C₈alkylthio, halogen, halo-C₁-C₈alkyl, or a cyano group. The C₆-C₂₄aryl (C₆-C₁₈aryl) and C₂-C₃₀heteroaryl groups are preferably substituted by one, or more C₁-C₈alkyl groups.
If a substituent occurs more than one time in a group, it can be different in each occurrence.
The wording "substituted by G" means that one, or more, especially one to three substituents G might be present.

As described above, the aforementioned groups may be substituted by E and/or, if desired, interrupted by D. Interruptions are of course possible only in the case of groups containing at least 2 carbon atoms connected to one another by single bonds; C₆-C₁₈aryl is not interrupted; interrupted arylalkyl contains the unit D in the alkyl moiety. C₁-C₁₈alkyl substituted by one or more E and/or interrupted by one or more units D is, for example, (CH₂CH₂O)₁₋₉-R^{x}, where R^{x} is H or C₁-C₁₀alkyl or C₂-C₁₀alkanoyl (e.g. CO-CH(C₂H₅)C₄H₉), CH₂-CH(OR^{y}')-CH₂-O-R^{y}, where R^{y} is C₁-C₁₈alkyl, C₅-C₁₂cycloalkyl, phenyl, C₇-C₁₅phenylalkyl, and R^{y}' embraces the same definitions as R^{y} or is H;
C₁-C₈alkylene-COO-R^{z}, e.g. CH2COORz, CH(CH₃)COOR^{z}, C(CH₃)₂COOR^{z}, where R^{z} is H, C₁-C₁₈alkyl, (CH₂CH₂O)₁₋₉-R^{x}, and R^{x} embraces the definitions indicated above; CH₂CH₂-O-CO-CH=CH₂; CH₂CH(OH)CH₂-O-CO-C(CH₃)=CH₂. The synthesis of 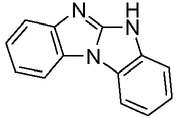 is described, for example, in Achour, Reddouane; Zniber, Rachid, Bulletin des Societes Chimiques Belges 96 (1987) 787-92. 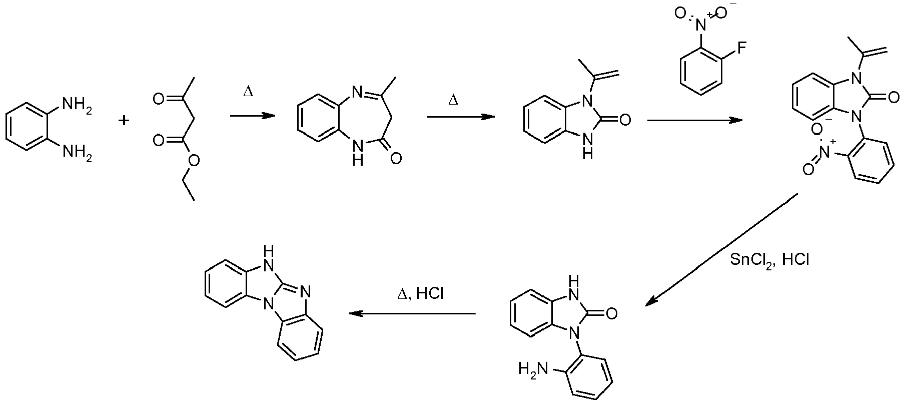 Suitable base skeletons of the formula 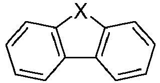 are either commercially available (especially in the cases when X is S, O, NH), or can be obtained by processes known to those skilled in the art. Reference is made to WO2010079051 and EP1885818.

The halogenation can be performed by methods known to those skilled in the art. Preference is given to brominating or iodinating in the 3 and 6 positions (dibromination) or in the 3 or 6 positions (monobromination) of the base skeleton of the formula (II) 2,8 positions (dibenzofuran and dibenzothiophene) or 3,6 positions (carbazole).

Optionally substituted dibenzofurans, dibenzothiophenes and carbazoles can be dibrominated in the 2,8 positions (dibenzofuran and dibenzothiophene) or 3,6 positions (carbazole) with bromine or NBS in glacial acetic acid or in chloroform. For example, the bromination with Br₂ can be effected in glacial acetic acid or chloroform at low temperatures, e.g. 0°C. Suitable processes are described, for example, in M. Park, J.R. Buck, C.J. Rizzo, Tetrahedron, 54 (1998) 12707-12714 for X= NPh, and in W. Yang et al., J. Mater. Chem. 13 (2003) 1351 for X= S. In addition, 3,6-dibromocarbazole, 3,6-dibromo-9-phenylcarbazole, 2,8-dibromodibenzothiophene, 2,8-dibromodibenzofuran, 2-bromocarbazole, 3-bromodibenzothiophene, 3-bromodibenzofuran, 3-bromocarbazole, 2-bromodibenzothiophene and 2-bromodibenzofuran are commercially available.

Monobromination in the 4 position of dibenzofuran (and analogously for dibenzothiophene) is described, for example, in J. Am. Chem. Soc. 1984, 106, 7150. Dibenzofuran (dibenzothiophene) can be monobrominated in the 3 position by a sequence known to those skilled in the art, comprising a nitration, reduction and subsequent Sandmeyer reaction.

Monobromination in the 2 position of dibenzofuran or dibenzothiophene and monobromination in the 3 position of carbazole are effected analogously to the dibromination, with the exception that only one equivalent of bromine or NBS is added.

Alternatively, it is also possible to utilize iodinated dibenzofurans, dibenzothiophenes and carbazoles. The preparation is described, inter alia, in Tetrahedron. Lett. 47 (2006) 6957-6960, Eur. J. Inorg. Chem. 24 (2005) 4976-4984, J. Heterocyclic Chem. 39 (2002) 933-941, J. Am. Chem. Soc. 124 (2002) 11900-11907, J. Heterocyclic Chem, 38 (2001) 77-87.

For the nucleophilic substitution, Cl- or F-substituted dibenzofurans, dibenzothiophenes and carbazoles are required. The chlorination is described, inter alia, in J. Heterocyclic Chemistry, 34 (1997) 891-900, Org. Lett., 6 (2004) 3501-3504; J. Chem. Soc. [Section] C: Organic, 16 (1971) 2775-7, Tetrahedron Lett. 25 (1984) 5363-6, J. Org. Chem. 69 (2004) 8177-8182. The fluorination is described in J. Org. Chem. 63 (1998) 878-880 and J. Chem. Soc., Perkin Trans. 2, 5 (2002) 953-957.

The introduction of the group 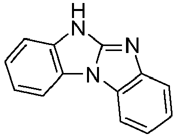 is performed in the presence of a base. Suitable bases are known to those skilled in the art and are preferably selected from the group consisting of alkali metal and alkaline earth metal hydroxides such as NaOH, KOH, Ca(OH)₂, alkali metal hydrides such as NaH, KH, alkali metal amides such as NaNH₂, alkali metal or alkaline earth metal carbonates such as K₂CO₃ or Cs₂CO₃, and alkali metal alkoxides such as NaOMe, NaOEt. In addition, mixtures of the aforementioned bases are suitable. Particular preference is given to NaOH, KOH, NaH or K₂CO₃.
Heteroarylation can be effected, for example, by copper-catalyzed coupling of 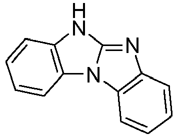 to a halogenated compound of the formula 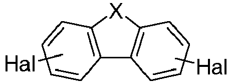 (Ullmann reaction).
The N-arylation was, for example, disclosed in H. Gilman and D. A. Shirley, J. Am. Chem. Soc. 66 (1944) 888; D. Li et al., Dyes and Pigments 49 (2001) 181 - 186 and Eur. J. Org. Chem. (2007) 2147-2151. The reaction can be performed in solvent or in a melt. Suitable solvents are, for example, (polar) aprotic solvents such as dimethyl sulfoxide, dimethylformamide, NMP, tridecane or alcohols.

The synthesis of 9-(8-bromodibenzofuran-2-yl)carbazole, 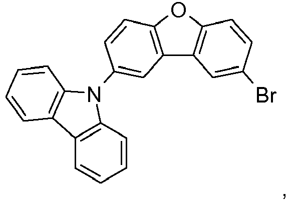 is described in WO2010079051. The synthesis of 2-bromo-8-iodo-dibenzofurane, 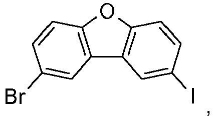 is described in EP1885818.

A possible synthesis route for the compound of formula 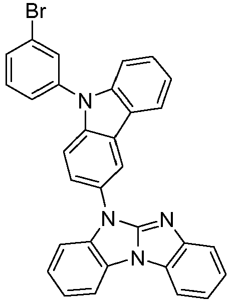 is shown in the following scheme: 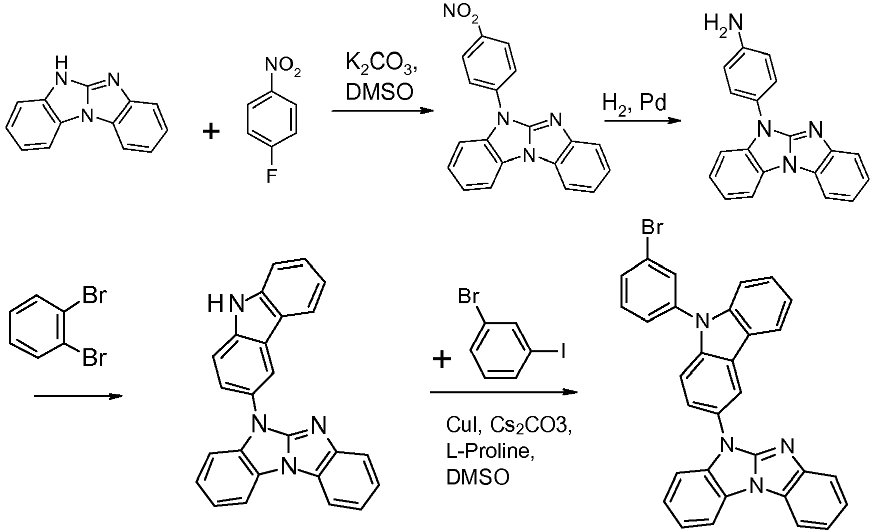 Reference is made to Angew. Chem. Int. Ed. 46 (2007)1627-1629 and Synthesis 20 (2009) 3493.

Diboronic acid or diboronate group containing dibenzofurans, dibenzothiophenes and carbazoles can be readily prepared by an increasing number of routes. An overview of the synthetic routes is, for example, given in Angew. Chem. Int. Ed. 48 (2009) 9240 - 9261.

By one common route diboronic acid or diboronate group containing dibenzofurans, dibenzothiophenes, and carbazoles can be obtained by reacting halogenated dibenzofurans, dibenzothiophenes and carbazoles with (Y¹O)₂B-B(OY¹)₂, 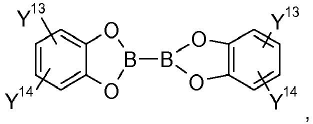 or 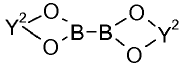 in the presence of a catalyst, such as, for example, [1,1'-bis(diphenylphosphino)ferrocene]dichloropalladium(II), complex (Pd(Cl)₂(dppf)), and a base, such as, for example, potassium acetate, in a solvent, such as, for example, dimethyl formamide, dimethyl sulfoxide, dioxane and/or toluene (cf. Prasad Appukkuttan et al., Synlett 8 (2003) 1204), wherein Y¹ is independently in each occurrence a C₁-C₁₈alkylgroup and Y² is independently in each occurrence a C₂-C₁₀alkylene group, such as -CY³Y⁴-CY⁵Y⁶-, or -CY⁷Y⁸-CY⁹Y¹⁰- CY¹¹Y¹²-, wherein Y³, Y⁴, Y⁵, Y⁶, Y⁷, Y⁸, Y⁹, Y¹⁰, Y¹¹ and Y¹² are independently of each other hydrogen, or a C₁-C₁₈alkylgroup, especially -C(CH₃)₂C(CH₃)₂-, - C(CH₃)₂CH₂C(CH₃)₂-, or -CH₂C(CH₃)₂CH₂-, and Y¹³ and Y¹⁴ are independently of each other hydrogen, or a C₁-C₁₈alkylgroup.

Diboronic acid or diboronate group containing dibenzofurans, dibenzothiophenes and carbazoles can also be prepared by reacting halogenated dibenzofurans, dibenzothiophenes and carbazoles with alkyl lithium reagents, such as, for example, n-butyl lithium, or t-buthyl lithium, followed by reaction with boronic esters, such as, for example, B(isopropoxy)₃, B(methoxy)₃, or 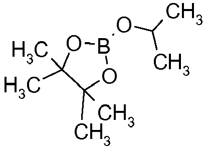 (cf. Synthesis (2000) 442-446). Diboronic acid or diboronate group containing dibenzofurans, dibenzothiophenes and carbazoles can also be prepared by reacting dibenzofurans, dibenzothiophenes and carbazoles with lithium amides, such as, for example, lithium diisopropylamide (LDA) followed by reaction with boronic esters such as, for example, B(isopropoxy)₃, B(methoxy)₃, or 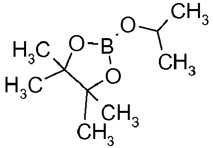 (J. Org. Chem. 73 (2008) 2176-2181).

Diboronic acid or diboronate group containing dibenzofurans, dibenzothiophenes and carbazoles, such as, for example, 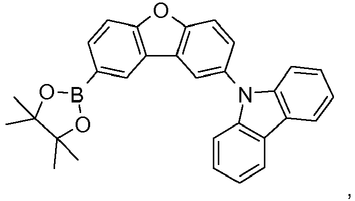 can be reacted with equimolar amounts of halogenated dibenzofurans, dibenzothiophenes, carbazoles and 4H-imidazo[1,2-a]imidazoles, such as, for example, 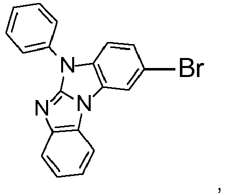 in a solvent and in the presence of a catalyst. The catalyst may be one of the µ-halo(triisopropylphosphine)(η³-allyl)palladium(II) type (see for example WO99/47474).

Preferably, the Suzuki reaction is carried out in the presence of an organic solvent, such as an aromatic hydrocarbon or a usual polar organic solvent, such as benzene, toluene, xylene, tetrahydrofurane, or dioxane, or mixtures thereof, most preferred toluene. Usually, the amount of the solvent is chosen in the range of from 1 to 10 l per mol of boronic acid derivative. Also preferred, the reaction is carried out under an inert atmosphere such as nitrogen, or argon. Further, it is preferred to carry out the reaction in the presence of an aqueous base, such as an alkali metal hydroxide or carbonate such as NaOH, KOH, Na₂CO₃, K₂CO₃, Cs₂CO₃ and the like, preferably an aqueous K₂CO₃ solution is chosen. Usually, the molar ratio of the base to boronic acid or boronic ester derivative is chosen in the range of from 0.5:1 to 50:1, very especially 1:1. Generally, the reaction temperature is chosen in the range of from 40 to 180°C, preferably under reflux conditions. Preferred, the reaction time is chosen in the range of from 1 to 80 hours, more preferably from 20 to 72 hours. In a preferred embodiment a usual catalyst for coupling reactions or for polycondensation reactions is used, preferably Pd-based, which is described in WO2007/101820. The palladium compound is added in a ratio of from 1:10000 to 1:50, preferably from 1:5000 to 1:200, based on the number of bonds to be closed. Preference is given, for example, to the use of palladium(II) salts such as PdAc₂ or Pd₂dba₃ and to the addition of ligands selected from the group consisting of 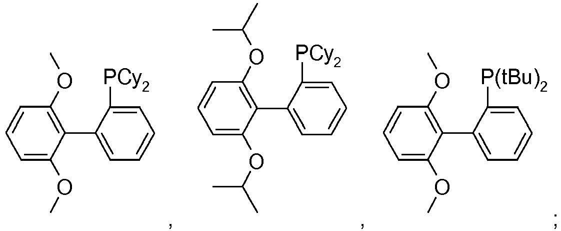 wherein 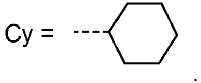 The ligand is added in a ratio of from 1:1 to 1:10, based on Pd. Also preferred, the catalyst is added as in solution or suspension. Preferably, an appropriate organic solvent such as the ones described above, preferably benzene, toluene, xylene, THF, dioxane, more preferably toluene, or mixtures thereof, is used. The amount of solvent usually is chosen in the range of from 1 to 10 l per mol of boronic acid derivative. Organic bases, such as, for example, tetraalkylammonium hydroxide, and phase transfer catalysts, such as, for example TBAB, can promote the activity of the boron (see, for example, Lead-beater & Marco; Angew. Chem. Int. Ed. Eng. 42 (2003) 1407 and references cited therein). Other variations of reaction conditions are given by T. I. Wallow and B. M. Novak in J. Org. Chem. 59 (1994) 5034-5037; and M. Remmers, M. Schulze, G. Wegner in Macromol. Rapid Commun. 17 (1996) 239-252 and G. A. Molander und B. Canturk, Angew. Chem. , 121 (2009) 9404 - 9425.

The synthesis of aza- and diaza-dibenzofuran starting materials is known in the literature, or can be done in analogy to known procedures.

JP2011084531 describes, for example, the synthesis of benzofuro[3,2-b]pyridine in two steps starting from 2-bromopyridin-3-ol using a base catalyzed cyclisation. The brominated compound is received by bromination with bromine in the presence of silver sulfate. 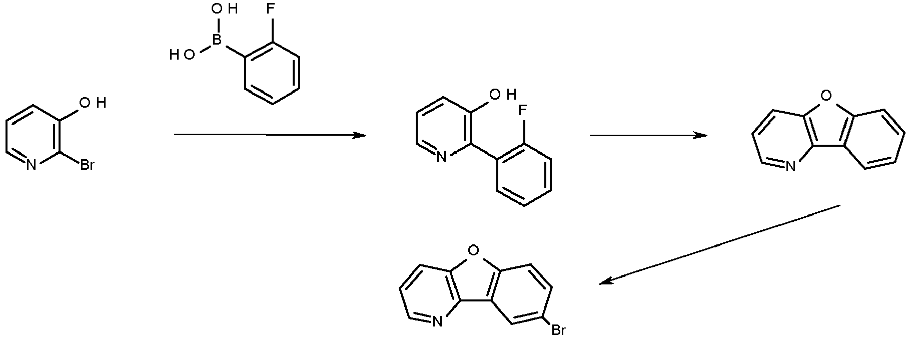 US2010/0187984 describes, for example, the synthesis of 3,6-dichloro-benzofuro[2,3-b]pyridine in three steps starting from 2-amino-5-chloropyridine using a cyclisation of a diazoniumion salt. 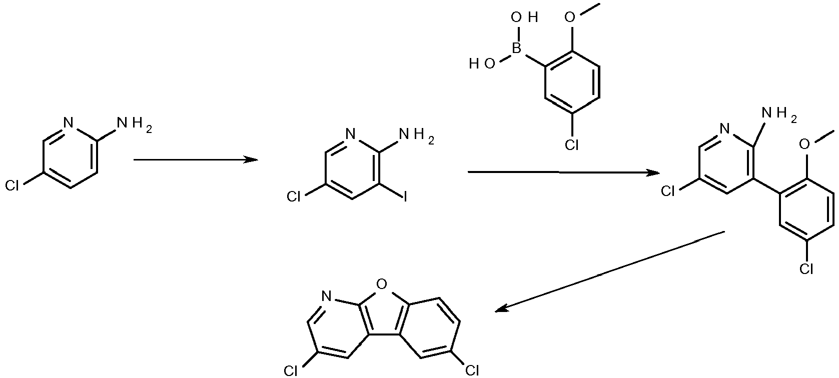 L. Kaczmarek, Polish Journal of Chemistry 59 (1985) 1141 describes the synthesis of furo[3,2-b:4,5-b]dipyridine starting from 2-(3-amino-2-pyridyl)pyridin-3-amine using an acid catalyzed cyclisation of a diazoniumion salt. 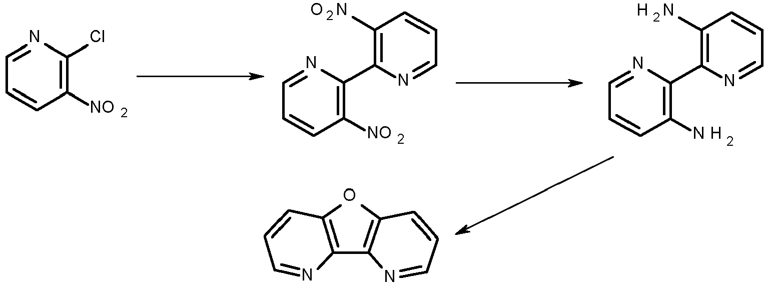 JP2002284862 describes the syntheses of 2,7-dibromo-furo[3,2-b:4,5-b]dipyridine starting from 2-(3-amino-5-bromo-2-pyridyl)-5-bromo-pyridin-3-amine using an acid catalyzed cyclisation of a diazoniumion salt. The synthesis of the starting material is described by Y. Fort, Tetrahedron 50 (41),11893 (1994). 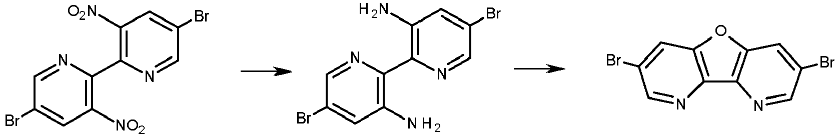 J. Liu, J. Org. Chem. 73, 2951 (2008 ) describes e.g. the synthesis of benzofuro[2,3-c]pyridine using a copper catalyzed cyclisation step.

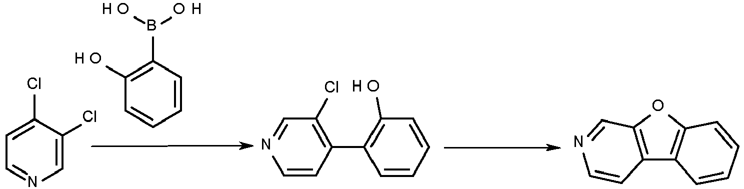

The synthesis of aza- and diaza-dibenzothiophene starting materials is known in the literature, or can be done in analogy to known procedures.
In WO2009086028 the synthesis of benzothiopheno[3,2-b]pyridine is described: 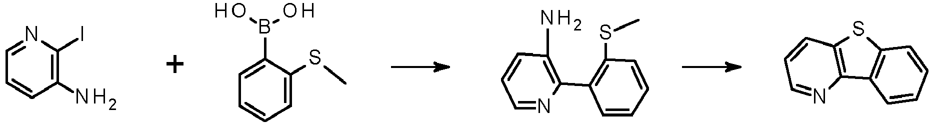 A possible synthetic route for compound **E-57** is shown in the reaction scheme below: 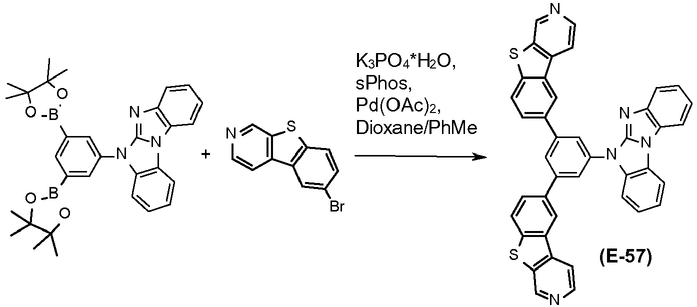 A possible synthetic route for compound **E-12** is shown in the reaction scheme below: 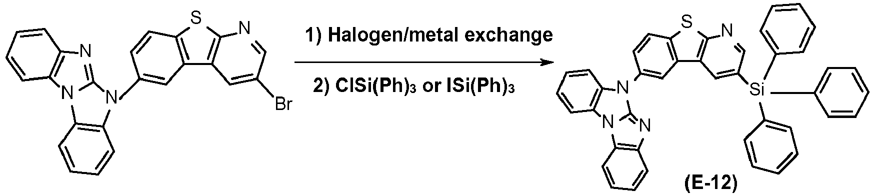 The halogen/metal exchange is done with nBuLi/THF at -78°C, or tBuLi/THF at -78°C. Reference is made to WO2010/079051, where the synthesis of such compounds is described.

Compounds of formula 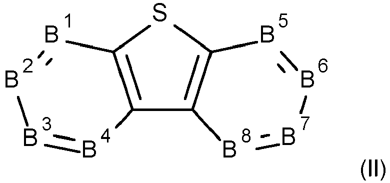 are intermediates in the production of compounds of formula (I) and form a further subject of the present invention.

B¹ is N, or CR⁸¹,
B² is N, or CR⁸²,
B³ is N, or CR⁸³,
B⁴ is N, or CR⁸⁴,
B⁵ is N, or CR⁸⁵,
B⁶ is N, or CR⁸⁶,
B⁷ is N, or CR⁸⁷,
B⁸ is N, or CR⁸⁸, wherein R⁸¹, R⁸², R⁸³, R⁸⁴, R⁸⁵, R⁸⁶, R⁸⁷ and R⁸⁸ are independently of each other H, a C₁-C₂₅alkyl group, which can optionally be substituted by E and or interupted by D; a C₆-C₂₄aryl group, which can optionally be substituted by G, a C₂-C₃₀heteroaryl group, which can optionally be substituted by G; a group of formula --(A¹)ₒ-(A²)ₚ-(A³)_{q}-(A⁴)ᵣ-R¹⁶, or -(A¹)ₒ-(A²)ₚ-(A³)_{q}-(A⁴)ᵣ-X¹,
o is 0, or 1, p is 0, or 1, q is 0, or 1, r is 0, or 1,
at least one of the substituents R⁸¹, R⁸², R⁸³, R⁸⁴, R⁸⁵, R⁸⁶, R⁸⁷ and R⁸⁸ represents a group of formula -(A¹)ₒ-(A²)ₚ-(A³)_{q}-(A⁴)ᵣX¹; wherein
X¹ is CI, Br, or I, ZnX¹², X¹² is a halogen atom; -SnR²⁰⁷R²⁰⁸R²⁰⁹, wherein R²⁰⁷, R²⁰⁸ and R²⁰⁹ are identical or different and are H or C₁-C₈alkyl, wherein two radicals optionally form a common ring and these radicals are optionally branched or unbranched; -B(OH)₂, -B(OY¹)₂, 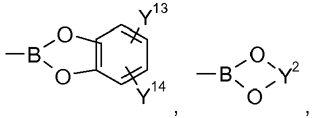 -BF₄Na, or -BF₄K, wherein Y¹ is independently in each occurrence a C₁-C₁₈alkyl group and Y² is independently in each occurrence a C₂-C₁₀alkylene group, and Y¹³ and Y¹⁴ are independently of each other hydrogen, or a C₁-C₁₈alkyl group, and o, p, q, r, E, D, G, A¹, A², A³, A⁴ and R¹⁶ are as defined above. The following compounds are examples of compounds known from the prior art: 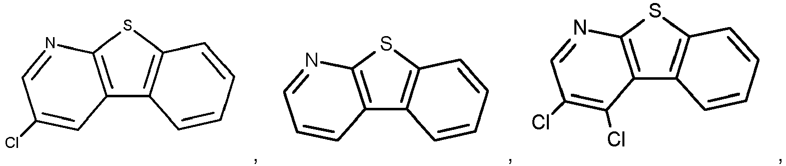 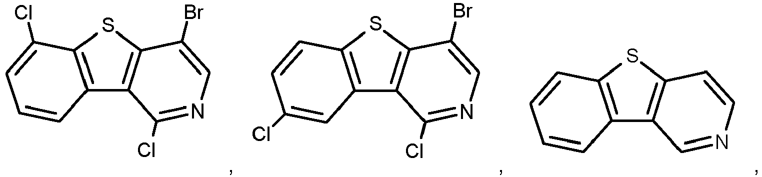 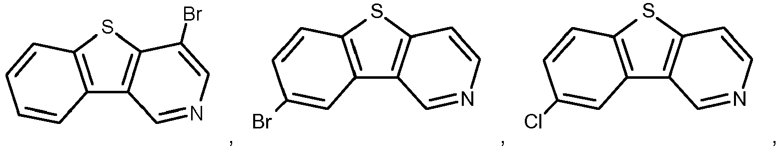 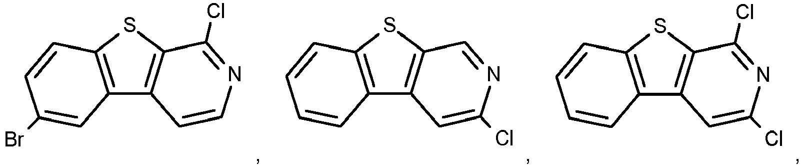 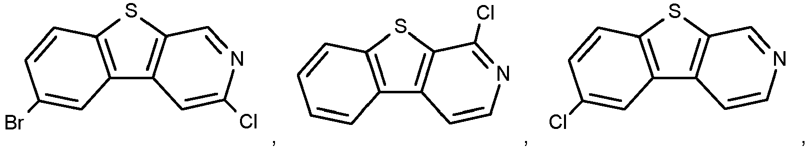 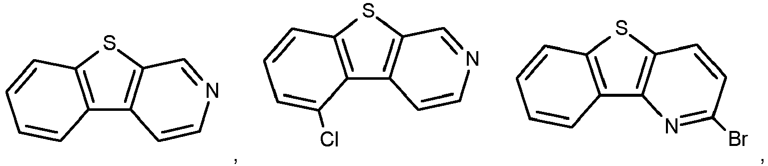 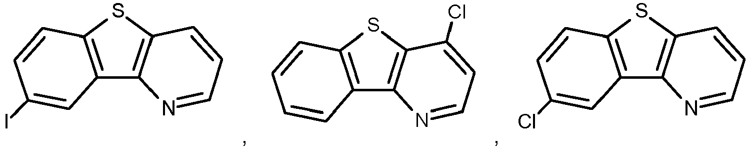 and 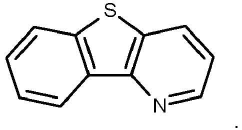 The preferences for R⁸¹, R⁸², R⁸³, R⁸⁴, R⁸⁵, R⁸⁶, R⁸⁷, R⁸⁸, A¹, A², A³, A⁴ and R¹⁶ are in principal the same as in case of the compounds of formula (**I**).

If X¹ is CI, Br, or I, p is 0, q is 0 and r is 0; o in the at least one group of formula -(A¹)ₒ-(A²)ₚ-(A³)_{q}-(A⁴)ᵣ-X¹ is preferably 1. Compounds of formula (**II**) are preferred, wherein X¹ is -B(OH)₂, -B(OY¹)₂,

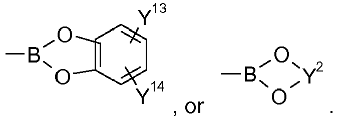

Among the compounds of formula (**II**) compounds of formula 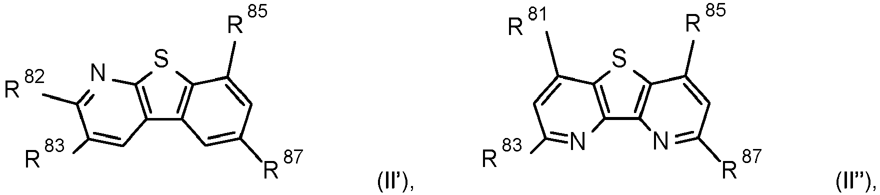 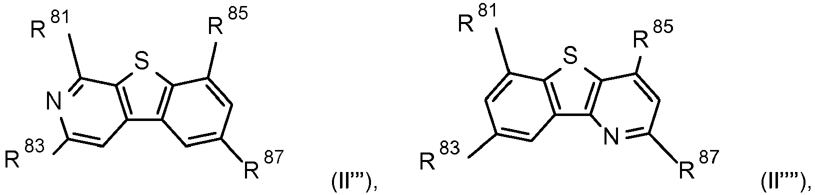 and 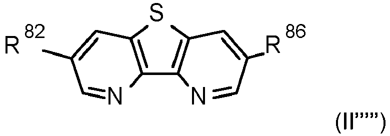 are preferred, where R⁸¹, R⁸², R⁸³, R⁸⁵, R⁸⁶ and R⁸⁷ have the meanings given above.

In a preferred embodiment of the present invention one of the substituents R⁸¹, R⁸², R⁸³, R⁸⁵, R⁸⁶ and R⁸⁷ in the compounds of formula (**II'**), (**II"**), (**II"'**), (**II""**) and (**II""'**) is a group of formula -(A¹)ₒ-(A²)ₚ-(A³)_{q}-(A⁴)ᵣ-X¹.

In another preferred embodiment of the present invention two of the substituents R⁸¹, R⁸², R⁸³, R⁸⁵, R⁸⁶ and R⁸⁷ in the compounds of formula (**II'**), (**II"**), (**II"'**), (**II""**) and (**II""'**) are a group of formula -(A¹)ₒ-(A²)ₚ-(A³)_{q}-(A⁴)ᵣ-X¹.

In a preferred embodiment the present invention is directed to compounds of formula 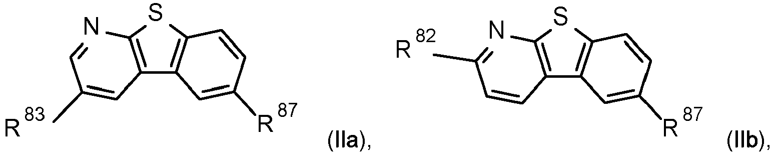 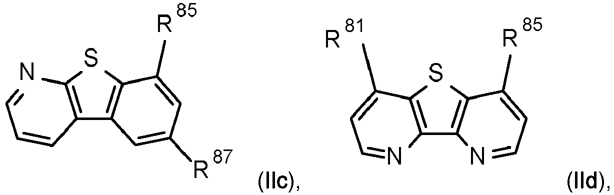 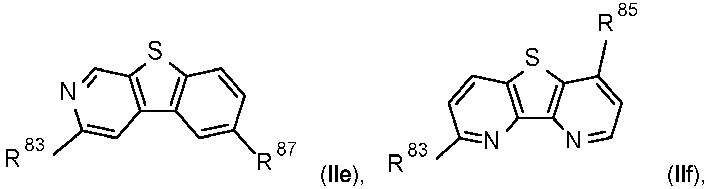 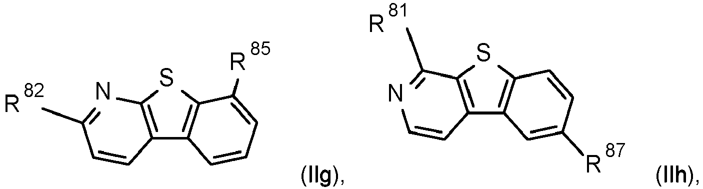 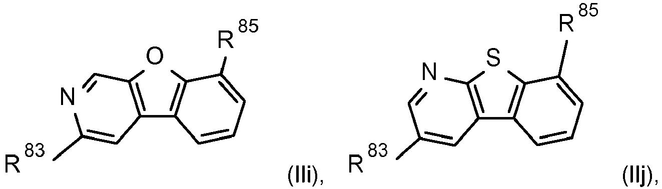 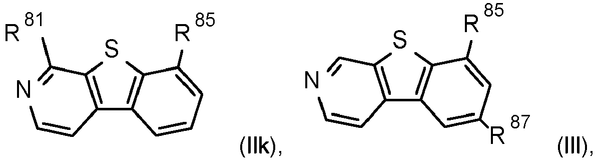 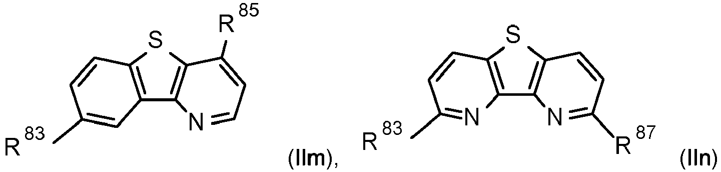 and 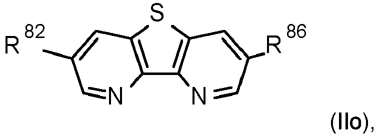 wherein R⁸¹, R⁸², R⁸³, R⁸⁵, R⁸⁶ and R⁸⁷ are as defined above.

In a preferred embodiment of the present invention one of the substituents R⁸¹, R⁸², R⁸³, R⁸⁵, R⁸⁶ and R⁸⁷ in the compounds of formula (**IIa**), (**IIb**), (**IIc**), (**IId**), (**IIe**), (**IIf**), **(IIg), (IIh), (IIi), (IIj),** (**IIk**), (**IIl**), **(IIm), (IIn)** and **(IIo)** is a group of formula -(A¹)ₒ-(A²)ₚ-(A³)_{q}-(A⁴)ᵣ-X¹.

In another preferred embodiment of the present invention two of the substituents R⁸¹, R⁸², R⁸³, R⁸⁵, R⁸⁶ and R⁸⁷ in the compounds of formula (**IIa**), (**IIb**), (**IIc**), (**IId**), (**IIe**), (**IIf**), (**IIg**), **(IIh), (IIi), (IIj),** (**IIk**), (**IIl**), (**IIm**), (**IIn**) and (**IIo**) are a group of formula -(A¹)ₒ-(A²)ₚ-(A3)_{q}-(A⁴)ᵣ-X¹.

Compounds of formula (**IIa**), (**IIb**), (**IIc**), (**IId**), (**IIe**), (**IIf**), **(IIi), (IIj),** (**IIl**), (**IIm**) and (**IIn**) are preferred. Compounds of formula (**IIa**), (**IIb**), (**IIc**), (**IId**), (**IIe**), (**IIf**), (**IIj**), (**IIm**) and (**IIn**) are especially preferred.

Examples of the intermediates are shown below:

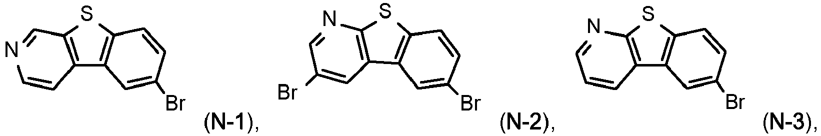

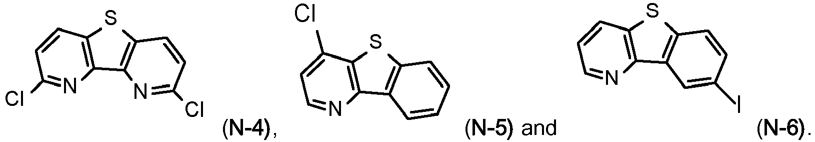

The synthesis of intermediate (**N-5**) is, for example, described in J. of the Kenya Chem. Soc., 1(1) (1999) 5-9. 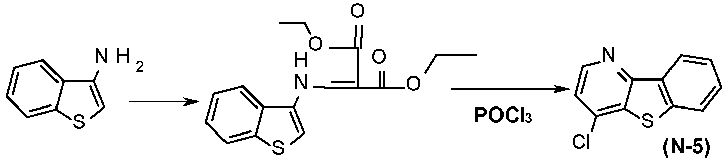 Iodation of furopyridine with iodine via lithiation is described in WO2009/086028 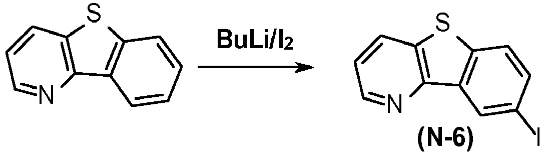 The bromination of the azadibenzofurans can be carried out in analogy to the bromination of carbazole, which is, for example, described in J. Mater. Chem. 18 (2008) 1296-1301.

Other bromination methods are, for example, described in Helvetica Chimica Acta 89 (2006) 1123 and SYNLETT 17 (2006) 2841-2845. 10.206

Selective halogenation of (III) with a halogenation agent results in the compounds of formula (II). Halogenation agents are, for example, N-chlorosuccinimide (NCS) (Synlett 18 (2005) 2837-2842); Br₂ (Synthesis 10 (2005) 1619-1624), N-bromosuccinimide (NBS)(Organic Letters 12 (2010) 2194-2197; Synlett (2006) 2841-2845), 1,3-dibromo-5,5-dimethylhydantoin (DBH) (Organic Process Research & Development 10 (2006) 822-828, US2002/0151456), CuBr₂ (Synthetic Communications 37 (2007) 1381-1388); R₄NBr₃ (Can. J. Chem. 67 (1989) 2062), N-iodosuccinimide (NIS) (Synthesis 12 (2001) 1794-1799, J. Heterocyclic Chem. 39 (2002) 933), KI/KIO₃( Org. Lett. 9 (2007) 797, Macromolecules 44 (2011) 1405-1413), NaIO₄/I₂/H₂SO₄ or NaIO₄/KI/H₂SO₄ (J. Heterocyclic Chem. 38 (2001) 77; J. Org. Chem. 75 (2010) 2578-2588); iodine monochloride (ICI; Synthesis (2008) 221-224). Additional methods are described in J. Org. Chem. 74 (2009) 3341-3349; J. Org. Chem. 71 (2006) 7422-7432, Eur. J. Org. Chem. (2008) 1065-1071, Chem. Asian J. 5 (2010) 2162 - 2167, Synthetic. Commun. 28 (1998) 3225.

Examples of solvents, which can be used in the halogenation, are dimethylformamide (DMF), CH₂Cl₂, CHCl₃, CCl₄, ethanol (EtOH), acetic acid (AcOH), H₂SO₄, C₆H₅Cl and mixtures thereof. The halogenation can be done in the presence of acids and lewis acids, respectively, such as, for example, H₂SO₄, ZrCl₄, TiCl₄, AlCl₃, HfCl₄ and AlCl₃ (Synlett 18 (2005) 2837-2842).

The halogenated intermediates (**II**), wherein X³ is Cl, Br, or I, can be transformed to the boronic ester intermediates (**II**) by reacting halogenated intermediates (**II**) with (Y¹O)₂B-B(OY¹)₂, 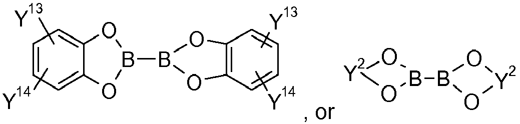 in the presence of a catalyst, such as, for example, [1,1'-bis(diphenylphosphino)ferrocene]dichloro-palladium(II), complex (Pd(Cl)₂(dppf)), and a base, such as, for example, potassium acetate, in a solvent, such as, for example, dimethyl formamide, dimethyl sulfoxide, dioxane and/or toluene (cf. Prasad Appukkuttan et al., Synlett 8 (2003) 1204).

An overview of the preparation of boronic reagents is given in Angew. Chem. 121 (2009) 9404 - 9425, Chem. Rev. 95 (1995) 2457-2483, Angew. Chem. Int. Ed. 41 (2002) 4176-4211, Tetrahedron 66 (2010) 8121- 8136.

Diboronic acid or diboronate intermediates (**II**) can also be prepared by reacting halogenated intermediates (**II**) with alkyl lithium reagents, such as, for example, n-butyl lithium, or t-buthyl lithium, followed by reaction with boronic esters, such as, for example, B(isopropoxy)₃, B(methoxy)₃, or 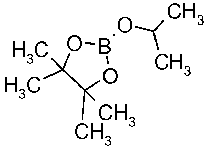 (cf. Synthesis (2000) 442-446).

The compounds of formula (**I**) can be obtained starting from the intermediates and suitable co-reactants, for example, by Suzuki-, Stille-, or Negishi-coupling reactions.
The halogenated intermediates, wherein X¹ is Cl, Br, or I, such as for example, 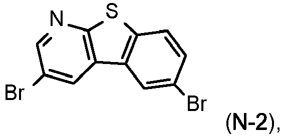 can, for example, be transformed to a compound of formula (**I**) by reacting intermediate (**N-2**) with X²-(A¹)ₒ-(A²)ₚ-(A³)_{q}-(A⁴)ᵣ-R¹⁶, wherein X² is (Y¹O)₂B-, 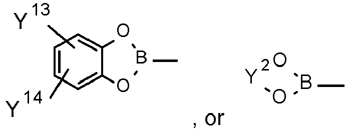 in the presence of a catalyst, such as, for example, [1,1'-bis(diphenylphosphino)ferrocene]dichloropalladium(II), complex (Pd(Cl)₂(dppf)), and a base, such as, for example, potassium acetate, in a solvent, such as, for example, dimethyl formamide, dimethyl sulfoxide, dioxane and/or toluene (cf. Prasad Appukkuttan et al., Synlett 8 (2003) 1204).

It has been found that the compounds of the formula **I** are particularly suitable for use in applications in which charge carrier conductivity is required, especially for use in organic electronics applications, for example selected from switching elements such as organic transistors, e.g. organic FETs and organic TFTs, organic solar cells and organic light-emitting diodes (OLEDs), the compounds of the formula **I** being particularly suitable in OLEDs for use as matrix material in a light-emitting layer and/or as hole and/or exciton blocker material and/or as electron and/or exciton blocker material, especially in combination with a phosphorescence emitter. In the case of use of the inventive compounds of the formula **I** in OLEDs, OLEDs which have good efficiencies and a long lifetime and which can be operated especially at a low use and operating voltage are obtained. The inventive compounds of the formula **I** are suitable especially for use as matrix and/or hole/exciton blocker materials for blue and green emitters, for example light blue or deep blue emitters, these being especially phosphorescence emitters. Furthermore, the compounds of the formula I can be used as transport/complementary materials in organic electronics applications selected from switching elements and organic solar cells.

The compounds of the formula **I,** especially of formula (**Ia**), (**Ib**), (**Ic**), (**Id**), (**Ie**), (**If**), (**Ii**), (**Ij**), (**Il**), (**Im**) and (**In**), can be used as matrix material and/or hole/exciton blocker material and/or electron/exciton blocker material and/or hole injection material and/or electron injection material and/or hole conductor material (hole transport material) and/or electron conductor material (electron transport material) in organic electronics applications, especially in OLEDs. The inventive compounds of the formula **I** are more preferably used as matrix materials in organic electronics applications, especially in OLEDs.

In the emission layer or one of the emission layers of an OLED, it is also possible to combine an emitter material with a matrix material of the compound of the formula **I** and a further matrix material which has, for example, a good hole transport property, or a good electron transporting property. This achieves a high quantum efficiency of this emission layer.

Certain compounds of formula **I,** such as, for example, **G-11,** have a ionisation potential of greater than 6 eV [calculated] and, hence, suited as electron transport material.

When a compound of the formula **I** is used as matrix material in an emission layer and additionally as hole/exciton blocker material and/or electron/exciton blocker material, owing to the chemical identity or similarity of the materials, an improved interface between the emission layer and the adjacent hole/exciton blocker material and/or electron/exciton blocker material is obtained, which can lead to a decrease in the voltage with equal luminance and to an extension of the lifetime of the OLED. Moreover, the use of the same material for hole/exciton blocker material and/or electron/exciton blocker material and for the matrix of an emission layer allows the production process of an OLED to be simplified, since the same source can be used for the vapor deposition process of the material of one of the compounds of the formula **I.**

Suitable structures of organic electronic devices are known to those skilled in the art and are specified below.

The organic transistor generally includes a semiconductor layer formed from an organic layer with hole transport capacity and/or electron transport capacity; a gate electrode formed from a conductive layer; and an insulating layer introduced between the semiconductor layer and the conductive layer. A source electrode and a drain electrode are mounted on this arrangement in order thus to produce the transistor element. In addition, further layers known to those skilled in the art may be present in the organic transistor.

The organic solar cell (photoelectric conversion element) generally comprises an organic layer present between two plate-type electrodes arranged in parallel. The organic layer may be configured on a comb-type electrode. There is no particular restriction regarding the site of the organic layer and there is no particular restriction regarding the material of the electrodes. When, however, plate-type electrodes arranged in parallel are used, at least one electrode is preferably formed from a transparent electrode, for example an ITO electrode or a fluorine-doped tin oxide electrode. The organic layer is formed from two sublayers, i.e. a layer with p-type semiconductor properties or hole transport capacity, and a layer formed with n-type semiconductor properties or electron transport capacity. In addition, it is possible for further layers known to those skilled in the art to be present in the organic solar cell. The layer with hole transport capacity may comprise the compounds of formula **I.**

It is likewise possible that the compounds of the formula **I** are present in the light-emitting layer (preferably as matrix material), in the blocking layer for electrons (as electron/exciton blockers), in the blocking layer for holes, in the hole transport layer and/or the electron transport layer.

The present invention further provides an organic light-emitting diode comprising an anode An and a cathode Ka and a light-emitting layer E arranged between the anode An and the cathode Ka, and if appropriate at least one further layer selected from the group consisting of at least one blocking layer for holes/excitons, at least one blocking layer for electrons/excitons, at least one hole injection layer, at least one hole transport layer, at least one electron injection layer and at least one electron transport layer, wherein the at least one compound of the formula I is present in the light-emitting layer E and/or in at least one of the further layers. The at least one compound of the formula I is preferably present in the light-emitting layer and/or the blocking layer for holes.

The present application further relates to a light-emitting layer comprising at least one compound of the formula **I,** especially a compound of formula (**Ia**), (**Ib**), (**Ic**), (**Id**), (**Ie**), (**If**), (**Ii**), (**Ij**), (**Il**), (**Im**) or (**In**), very especially a compound **A-1** to **A-75, B-1** to **B-8**, **C-1** to **C-75, D-1** to **D-8, E-1** to **E-75, F-1** to **F-75, or G-1** to **G-75.**

### Structure of the inventive OLED

The inventive organic light-emitting diode (OLED) thus generally has the following structure:
an anode (An) and a cathode (Ka) and a light-emitting layer E arranged between the anode (An) and the cathode (Ka).

The inventive OLED may, for example - in a preferred embodiment - be formed from the following layers:
1. Anode
2. Hole transport layer
3. Light-emitting layer
4. Blocking layer for holes/excitons
5. Electron transport layer
6. Cathode

Layer sequences different than the aforementioned structure are also possible, and are known to those skilled in the art. For example, it is possible that the OLED does not have all of the layers mentioned; for example, an OLED with layers (1) (anode), (3) (light-emitting layer) and (6) (cathode) is likewise suitable, in which case the functions of the layers (2) (hole transport layer) and (4) (blocking layer for holes/excitons) and (5) (electron transport layer) are assumed by the adjacent layers. OLEDs which have layers (1), (2), (3) and (6), or layers (1), (3), (4), (5) and (6), are likewise suitable. In addition, the OLEDs may have a blocking layer for electrons/excitons between the hole transport layer (2) and the Light-emitting layer (3).

It is additionally possible that a plurality of the aforementioned functions (electron/exciton blocker, hole/exciton blocker, hole injection, hole conduction, electron injection, electron conduction) are combined in one layer and are assumed, for example, by a single material present in this layer. For example, a material used in the hole transport layer, in one embodiment, may simultaneously block excitons and/or electrons.

Furthermore, the individual layers of the OLED among those specified above may in turn be formed from two or more layers. For example, the hole transport layer may be formed from a layer into which holes are injected from the electrode, and a layer which transports the holes away from the hole-injecting layer into the light-emitting layer. The electron conduction layer may likewise consist of a plurality of layers, for example a layer in which electrons are injected by the electrode, and a layer which receives electrons from the electron injection layer and transports them into the light-emitting layer. These layers mentioned are each selected according to factors such as energy level, thermal resistance and charge carrier mobility, and also energy difference of the layers specified with the organic layers or the metal electrodes. The person skilled in the art is capable of selecting the structure of the OLEDs such that it is matched optimally to the organic compounds used as emitter substances in accordance with the invention.

In order to obtain particularly efficient OLEDs, for example, the HOMO (highest occupied molecular orbital) of the hole transport layer should be matched to the work function of the anode, and the LUMO (lowest unoccupied molecular orbital) of the electron transport layer should be matched to the work function of the cathode, provided that the aforementioned layers are present in the inventive OLEDs.

The anode (1) is an electrode which provides positive charge carriers. It may be formed, for example, from materials which comprise a metal, a mixture of various metals, a metal alloy, a metal oxide or a mixture of various metal oxides. Alternatively, the anode may be a conductive polymer. Suitable metals comprise metals and alloys of the metals of the main groups, transition metals and of the lanthanoids, especially the metals of groups Ib, IVa, Va and VIa of the periodic table of the elements, and the transition metals of group VIIIa. When the anode is to be transparent, generally mixed metal oxides of groups IIb, IIIb and IVb of the periodic table of the elements (IUPAC version) are used, for example indium tin oxide (ITO). It is likewise possible that the anode (1) comprises an organic material, for example polyaniline, as described, for example, in Nature, Vol. 357, pages 477 to 479 (June 11, 1992). At least either the anode or the cathode should be at least partly transparent in order to be able to emit the light formed. The material used for the anode (1) is preferably ITO.

Suitable hole transport materials for layer (2) of the inventive OLEDs are disclosed, for example, in Kirk-Othmer Encyclopedia of Chemical Technology, 4th edition, Vol. 18, pages 837 to 860, 1996. Both hole-transporting molecules and polymers can be used as the hole transport material. Hole-transporting molecules typically used are selected from the group consisting of tris[N-(1-naphthyl)-N-(phenylamino)]triphenylamine (1-NaphDATA), 4,4'-bis[N-(1-naphthyl)-N-phenylamino]biphenyl (α-NPD), N,N'-diphenyl-N,N'-bis(3-methylphenyl)-[1,1'-biphenyl]-4,4'-diamine (TPD), 1,1-bis[(di-4-tolylamino)phenyl]cyclohexane (TAPC), N,N'-bis(4-methylphenyl)-N,N'-bis(4-ethylphenyl)-[1,1'-(3,3'-dimethyl)biphenyl]-4,4'-diamine (ETPD), tetrakis(3-methylphenyl)-N,N,N',N'-2,5-phenylenediamine (PDA), α-phenyl-4-N,N-diphenylaminostyrene (TPS), p-(diethylamino)benzaldehyde diphenylhydrazone (DEH), triphenylamine (TPA), bis[4-(N,N-diethylamino)-2-methylphenyl)(4-methylphenyl)methane (MPMP), 1-phenyl-3-[p-(diethylamino)styryl]-5-[p-(diethylamino)phenyl]pyrazoline (PPR or DEASP), 1,2-trans-bis(9H-carbazol-9-yl)cyclobutane (DCZB), N,N,N',N'-tetrakis(4-methylphenyl)-(1,1'-biphenyl)-4,4'-diamine (TTB), 4,4',4"-tris(N,N-diphenylamino)triphenylamine (TDTA), 4,4',4"-tris(N-carbazolyl)triphenylamine (TCTA), N,N'-bis(naphthalen-2-yl)-N,N'-bis(phenyl)benzidine (β-NPB), N,N'-bis(3-methylphenyl)-N,N'-bis(phenyl)-9,9-spirobifluorene (Spiro-TPD), N,N'-bis(naphthalen-1-yl)-N,N'-bis(phenyl)-9,9-spirobifluorene (Spiro-NPB), N,N'-bis(3-methylphenyl)-N,N'-bis(phenyl)-9,9-dimethylfluorene (DMFL-TPD), di[4-(N,N-ditolylamino)phenyl]cyclohexane, N,N'-bis(naphthalen-1-yl)-N,N'-bis(phenyl)-9,9-dimethylfluorene, N,N'-bis(naphthalen-1-yl)-N,N'-bis(phenyl)-2,2-dimethylbenzidine, N,N'-bis(naphthalen-1-yl)-N,N'-bis(phenyl)benzidine, N,N'-bis(3-methylphenyl)-N,N'-bis(phenyl)benzidine, 2,3,5,6-tetrafluoro-7,7,8,8-tetracyanoquinodimethane (F4-TCNQ), 4,4',4"-tris(N-3-methylphenyl-N-phenylamino)triphenylamine, 4,4',4"-tris(N-(2-naphthyl)-N-phenyl-amino)triphenylamine, pyrazino[2,3-f][1,10]phenanthroline-2,3-dicarbonitrile (PPDN), N,N,N',N'-tetrakis(4-methoxyphenyl)benzidine (MeO-TPD), 2,7-bis[N,N-bis(4-methoxyphenyl)amino]-9,9-spirobifluorene (MeO-Spiro-TPD), 2,2'-bis[N,N-bis(4-methoxyphenyl)amino]-9,9-spirobifluorene (2,2'-MeO-Spiro-TPD), N,N'-diphenyl-N,N'-di[4-(N,N-ditolylamino)phenyl]benzidine (NTNPB), N,N'-diphenyl-N,N'-di[4-(N,N-diphenylamino)phenyl]benzidine (NPNPB), N,N'-di(naphthalen-2-yl)-N,N'-diphenylbenzene-1,4-diamine (β-NPP), N,N'-bis(3-methylphenyl)-N,N'-bis(phenyl)-9,9-diphenylfluorene (DPFL-TPD), N,N'-bis(naphthalen-1-yl)-N,N'-bis(phenyl)-9,9-diphenylfluorene (DPFL-NPB), 2,2',7,7'-tetrakis(N,N-diphenylamino)-9,9'-spirobifluorene (Spiro-TAD), 9,9-bis[4-(N,N-bis(biphenyl-4-yl)amino)phenyl]-9H-fluorene (BPAPF), 9,9-bis[4-(N,N-bis(naphthalen-2-yl)amino)phenyl]-9H-fluorene (NPAPF), 9,9-bis[4-(N,N-bis(naphthalen-2-yl)-N,N'-bisphenylamino)phenyl]-9H-fluorene (NPBAPF), 2,2',7,7'-tetrakis[N-naphthalenyl(phenyl)amino]-9,9'-spirobifluorene (Spiro-2NPB), N,N'-bis(phenanthren-9-yl)-N,N'-bis(phenyl)benzidine (PAPB), 2,7-bis[N,N-bis(9,9-spirobifluoren-2-yl)amino]-9,9-spirobifluorene (Spiro-5), 2,2'-bis[N,N-bis(biphenyl-4-yl)amino]-9,9-spirobifluorene (2,2'-Spiro-DBP), 2,2'-bis(N,N-diphenylamino)-9.9-spirobifluorene (Spiro-BPA), 2,2',7,7'-tetra(N,N-ditolyl)aminospirobifluorene (Spiro-TTB), N,N,N',N'-tetranaphthalen-2-ylbenzidine (TNB), porphyrin compounds and phthalocyanines such as copper phthalocyanines and titanium oxide phthalocyanines. Hole-transporting polymers typically used are selected from the group consisting of polyvinylcarbazoles, (phenylmethyl)polysilanes and polyanilines. It is likewise possible to obtain hole-transporting polymers by doping hole-transporting molecules into polymers such as polystyrene and polycarbonate. Suitable hole-transporting molecules are the molecules already mentioned above.
In addition - in one embodiment - it is possible to use carbene complexes as hole transport materials, the band gap of the at least one hole transport material generally being greater than the band gap of the emitter material used. In the context of the present application, "band gap" is understood to mean the triplet energy. Suitable carbene complexes are, for example, carbene complexes as described in WO 2005/019373 A2, WO 2006/056418 A2, WO 2005/113704, WO 2007/115970, WO 2007/115981, WO2008/000727 and US2012/319050. One example of a suitable carbene complex is Ir(dpbic)₃ with the formula: 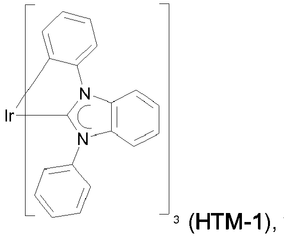 which is disclosed, for example, in WO2005/019373. Another example of a suitable carbene complex is Ir(ABIC)₃ with the formu-la: 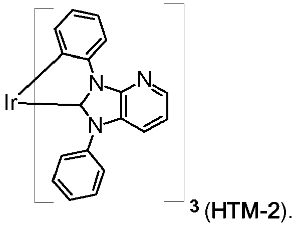 In principle, it is possible that the hole transport layer comprises at least one compound of the formula **I** as hole transport material, especially a compound of formula (**Ia**), (**Ib**), (**Ic**), (**Id**), (**Ie**), (**If**), (**Ii**), (**Ij**), (**Il**), (**Im**) or (**In**), very especially a compound **A-1** to **A-75, B-1** to **B-8**, **C-1** to **C-75, D-1** to **D-8, E-1** to **E-75, F-1** to **F-75, or G-1** to **G-75.**
The hole-transporting layer may also be electronically doped in order to improve the transport properties of the materials used, in order firstly to make the layer thicknesses more generous (avoidance of pinholes/short circuits) and in order secondly to minimize the operating voltage of the device. Electronic doping is known to those skilled in the art and is disclosed, for example, in W. Gao, A. Kahn, J. Appl. Phys., Vol. 94, 2003, 359 (p-doped organic layers); A. G. Werner, F. Li, K. Harada, M. Pfeiffer, T. Fritz, K. Leo, Appl. Phys. Lett., Vol. 82, No. 25, 2003, 4495 and Pfeiffer et al., Organic Electronics 2003, 4, 89 - 103 and K. Walzer, B. Maennig, M. Pfeiffer, K. Leo, Chem. Soc. Rev. 2007, 107, 1233. For example it is possible to use mixtures in the hole-transporting layer, in particular mixtures which lead to electrical p-doping of the hole-transporting layer. p-Doping is achieved by the addition of oxidizing materials. These mixtures may, for example, be the following mixtures: mixtures of the abovementioned hole transport materials with at least one metal oxide, for example MoO₂, MoO₃, WOₓ, ReO₃ and/or V₂O₅, preferably MoO₃ and/or ReO₃, more preferably MoO₃, or mixtures comprising the aforementioned hole transport materials and one or more compounds selected from 7,7,8,8-tetracyanoquinodimethane (TCNQ), 2,3,5,6-tetrafluoro-7,7,8,8-tetracyanoquinodimethane (F₄-TCNQ), 2,5-bis(2-hydroxyethoxy)-7,7,8,8-tetracyanoquinodimethane, bis(tetra-n-butylammonium)tetracyanodiphenoquinodimethane, 2,5-dimethyl-7,7,8,8-tetracyanoquinodimethane, tetracyanoethylene, 11,11,12,12-tetracyanonaphtho2,6-quinodimethane, 2-fluoro-7,7,8,8-tetracyanoquino-dimethane, 2,5-difluoro-7,7,8,8etracyanoquinodimethane, dicyanomethylene-1,3,4,5,7,8-hexafluoro-6H-naphthalen-2-ylidene)malononitrile (F₆-TNAP), Mo(tfd)₃ (from Kahn et al., J. Am. Chem. Soc. 2009, 131 (35), 12530-12531), compounds as described in EP1988587, US2008265216, EP2180029, US20100102709, WO2010132236, EP2180029 and quinone compounds as mentioned in EP2401254. Preferred mixtures comprise the aforementioned carbene complexes, such as, for example, the carbene complex **HTM-1,** or **HTM-2,** and MoO₃ and/or ReO₃, especially MoO₃. In a particularly preferred embodiment the hole transport layer comprises from 0.1 to 10 wt % of MoO₃ and 90 to 99.9 wt % carbene complex, especially of the carbene complex **HTM-1,** or **HTM-2,** wherein the total amount of the MoO₃ and the carbene complex is 100 wt %.

The light-emitting layer (3) comprises at least one emitter material. In principle, it may be a fluorescence or phosphorescence emitter, suitable emitter materials being known to those skilled in the art. The at least one emitter material is preferably a phosphorescence emitter. The phosphorescence emitter compounds used with preference are based on metal complexes, and especially the complexes of the metals Ru, Rh, Ir, Pd and Pt, in particular the complexes of Ir, have gained significance. The compounds of the formula I can be used as the matrix in the light-emitting layer.

Suitable metal complexes for use in the inventive OLEDs are described, for example, in documents WO02/60910A1, US2001/0015432A1, US2001/0019782A1, US2002/0055014A1, US2002/0024293A1, US2002/0048689A1, EP1191612A2, EP1191613A2, EP1211257A2, US2002/0094453A1, WO02/02714A2, WO00/70655A2, WO01/41512A1, WO02/15645A1, WO2005/019373A2, WO2005/113704A2, WO2006/115301A1, WO2006/067074A1, WO2006/056418, WO2006121811A1, WO2007095118A2, WO2007/115970, WO2007/115981, WO2008/000727, WO2010129323, WO2010056669, WO10086089, , US2011/0057559, WO2011/106344, US2011/0233528, WO2012/048266 and WO2012/172482.

Further suitable metal complexes are the commercially available metal complexes tris(2-phenylpyridine)iridium(III), iridium(III) tris(2-(4-tolyl)pyridinato-N,C^{2'}), bis(2-phenylpyridine)(acetylacetonato)iridium(III), iridium(III) tris(1-phenylisoquinoline), iridium(III) bis(2,2'-benzothienyl)pyridinato-N,C^{3'})(acetylacetonate), tris(2-phenylquinoline)iridium(III), iridium(III) bis(2-(4,6-difluorophenyl)pyridinato-N,C²)picolinate, iridium(III) bis(1-phenylisoquinoline)(acetylacetonate), bis(2-phenylquinoline)(acetylacetonato)iridium(III), iridium(III) bis(di-benzo[f,h]quinoxaline)(acetylacetonate), iridium(III) bis(2-methyldi-benzo[f,h]quinoxaline)(acetylacetonate) and tris(3-methyl-1-phenyl-4-trimethylacetyl-5-pyrazolino)terbium(III), bis[1-(9,9-dimethyl-9H-fluoren-2-yl)isoquinoline](acetyl-acetonato)iridium(III), bis(2-phenylbenzothiazolato)(acetylacetonato)iridium(III), bis(2-(9,9-dihexylfluorenyl)-1-pyridine)(acetylacetonato)iridium(III), bis(2-benzo[b]thiophen-2-yl-pyridine)(acetylacetonato)iridium(III).

In addition, the following commercially available materials are suitable: tris(dibenzoylacetonato)mono(phenanthroline)europium(III), tris(dibenzoylmethane)-mono(phenanthroline)europium(III), tris(dibenzoylmethane)mono(5-aminophenanthroline)-europium(III), tris(di-2-naphthoylmethane)mono(phenanthroline)europium(III), tris(4-bromobenzoylmethane)mono(phenanthroline)europium(III), tris(di(biphenyl)methane)-mono(phenanthroline)europium(III), tris(dibenzoylmethane)mono(4,7-diphenyl-phenanthroline)europium(III), tris(dibenzoylmethane)mono(4,7-di-methyl-phenanthroline)europium(III), tris(dibenzoylmethane)mono(4,7-dimethylphenan-throlinedisulfonic acid)europium(III) disodium salt, tris[di(4-(2-(2-ethoxyethoxy)ethoxy)-benzoylmethane)]mono(phenanthroline)europium(III) and tris[di[4-(2-(2-ethoxy-ethoxy)ethoxy)benzoylmethane)]mono(5-aminophenanthroline)europium(III), osmium(II) bis(3-(trifluoromethyl)-5-(4-tert-butylpyridyl)-1,2,4-triazolato)diphenylmethylphosphine, osmium(II) bis(3-(trifluoromethyl)-5-(2-pyridyl)-1,2,4-triazole)dimethylphenylphosphine, osmium(II) bis(3-(trifluoromethyl)-5-(4-tert-butylpyridyl)-1,2,4-triazolato)dimethylphenylphosphine, osmium(II) bis(3-(trifluoromethyl)-5-(2-pyridyl)-pyrazolato)dimethylphenylphosphine, tris[4,4'-di-tert-butyl(2,2')-bipyridine]ruthenium(III), osmium(II) bis(2-(9,9-dibutylfluorenyl)-1-isoquinoline(acetylacetonate).

Preferred phosphorescence emitters are carbene complexes. Suitable phosphorescent blue emitters are specified in the following publications: WO2006/056418A2, WO2005/113704, WO2007/115970, WO2007/115981, WO2008/000727, WO2009050281, WO2009050290, WO2011051404, US2011/057559 WO2011/073149, WO2012/121936A2, US2012/0305894A1, WO2012/170571, WO2012/170461, WO2012/170463, WO2006/121811, WO2007/095118, WO2008/156879, WO2008/156879, WO2010/068876, US2011/0057559, WO2011/106344, US2011/0233528, WO2012/048266 and WO2012/172482.

The light emitting layer comprises preferably a compound of the formula [L]_{m1[}K]ₒ₁M[carbene]ₙ₁ (**IX**), which are described in WO 2005/019373A2, wherein the symbols have the following meanings:
M is a metal atom selected from the group consisting of Co, Rh, Ir, Nb, Pd, Pt, Fe, Ru, Os, Cr, Mo, W, Mn, Tc, Re, Cu, Ag and Au in any oxidation state possible for the respective metal atom;
Carbene is a carbene ligand which may be uncharged or monoanionic and monodentate, bidentate or tridentate, with the carbene ligand also being able to be a biscarbene or triscarbene ligand;
L is a monoanionic or dianionic ligand, which may be monodentate or bidentate;
K is an uncharged monodentate or bidentate ligand selected from the group consisting of phosphines; phosphonates and derivatives thereof, arsenates and derivatives thereof; phosphites; CO; pyridines; nitriles and conjugated dienes which form a π complex with M¹;
n1 is the number of carbene ligands, where n1 is at least 1 and when n1 > 1 the carbene ligands in the complex of the formula (**IX**) can be identical or different;
m1 is the number of ligands L, where m1 can be 0 or ≥ 1 and when m1 > 1 the ligands L can be identical or different;
o1 is the number of ligands K, where o1 can be 0 or ≥ 1 and when o1 > 1 the ligands K can be identical or different;
where the sum n1 + m1 + o1 is dependent on the oxidation state and coordination number of the metal atom and on the denticity of the ligands carbene, L and K and also on the charge on the ligands, carbene and L, with the proviso that n1 is at least 1.

Carbene complexes which are suitable triplet emitters are described, for example, in WO 2006/056418 A2, WO 2005/113704, WO 2007/115970, WO 2007/115981 and WO 2008/000727, WO2009050281, WO2009050290, WO2011051404 and WO2011073149.

More preferred are metal-carbene complexes of the general formula 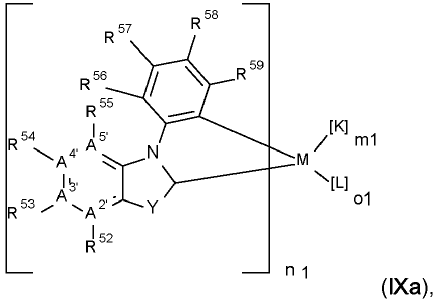 which are described in WO2011/73149, where M, n1, Y, A^{2'}, A^{3'}, A^{4'}, A^{5'}, R⁵¹, R⁵², R⁵³, R⁵⁴, R⁵⁵, R⁵⁶, R⁵⁷, R⁵⁸, R⁵⁹, K, L, m1 and o1 are each defined as follows:
M is Ir, or Pt,
n1 is an integer selected from 1, 2 and 3,
Y is NR⁵¹, O, S or C(R²⁵)₂,
A^{2'}, A^{3'}, A^{4'}, and A^{5'} are each independently N or C, where 2 A = nitrogen atoms and at least one carbon atom is present between two nitrogen atoms in the ring,
R⁵¹ is a linear or branched alkyl radical optionally interrupted by at least one heteroatom, optionally bearing at least one functional group and having 1 to 20 carbon atoms, cycloalkyl radical optionally interrupted by at least one heteroatom, optionally bearing at least one functional group and having 3 to 20 carbon atoms, substituted or unsubstituted aryl radical optionally interrupted by at least one heteroatom, optionally bearing at least one functional group and having 6 to 30 carbon atoms, substituted or unsubstituted heteroaryl radical optionally interrupted by at least one heteroatom, optionally bearing at least one functional group and having a total of 5 to 18 carbon atoms and/or heteroatoms,
R⁵², R⁵³, R⁵⁴ and R⁵⁵ are each, if A^{2'}, A^{3'}, A^{4'} and/or A^{5'} is N, a free electron pair, or, if A^{2'}, A^{3'}, A^{4'} and/or A^{5'} is C, each independently hydrogen, linear or branched alkyl radical optionally interrupted by at least one heteroatom, optionally bearing at least one functional group and having 1 to 20 carbon atoms, cycloalkyl radical optionally interrupted by at least one heteroatom, optionally bearing at least one functional group and having 3 to 20 carbon atoms, substituted or unsubstituted aryl radical optionally interrupted by at least one heteroatom, optionally bearing at least one functional group and having 6 to 30 carbon atoms, substituted or unsubstituted heteroaryl radical optionally interrupted by at least one heteroatom, optionally bearing at least one functional group and having a total of 5 to 18 carbon atoms and/or heteroatoms, group with donor or acceptor action, or R⁵³ and R⁵⁴ together with A^{3'} and A^{4'} form an optionally substituted, unsaturated ring optionally interrupted by at least one further heteroatom and having a total of 5 to 18 carbon atoms and/or heteroatoms,
R⁵⁶, R⁵⁷, R⁵⁸ and R⁵⁹ are each independently hydrogen, linear or branched alkyl radical optionally interrupted by at least one heteroatom, optionally bearing at least one functional group and having 1 to 20 carbon atoms, cycloalkyl radical optionally interrupted by at least one heteroatom, optionally bearing at least one functional group and having 3 to 20 carbon atoms, cycloheteroalkyl radical optionally interrupted by at least one heteroatom, optionally bearing at least one functional group and having 3 to 20 carbon atoms, substituted or unsubstituted aryl radical optionally interrupted by at least one heteroatom, optionally bearing at least one functional group and having 6 to 30 carbon atoms, substituted or unsubstituted heteroaryl radical optionally interrupted by at least one heteroatom, optionally bearing at least one functional group and having a total of 5 to 18 carbon atoms and/or heteroatoms, group with donor or acceptor action, or
R⁵⁶ and R⁵⁷, R⁵⁷ and R⁵⁸ or R⁵⁸ and R⁵⁹, together with the carbon atoms to which they are bonded, form a saturated, unsaturated or aromatic, optionally substituted ring optionally interrupted by at least one heteroatom and having a total of 5 to 18 carbon atoms and/or heteroatoms, and/or
if A^{5'} is C, R⁵⁵ and R⁵⁶ together form a saturated or unsaturated, linear or branched bridge optionally comprising heteroatoms, an aromatic unit, heteroaromatic unit and/or functional groups and having a total of 1 to 30 carbon atoms and/or heteroatoms, to which is optionally fused a substituted or unsubstituted, five- to eight-membered ring comprising carbon atoms and/or heteroatoms,
R²⁵ is independently a linear or branched alkyl radical optionally interrupted by at least one heteroatom, optionally bearing at least one functional group and having 1 to 20 carbon atoms, cycloalkyl radical optionally interrupted by at least one heteroatom, optionally bearing at least one functional group and having 3 to 20 carbon atoms, substituted or unsubstituted aryl radical optionally interrupted by at least one heteroatom, optionally bearing at least one functional group and having 6 to 30 carbon atoms, substituted or unsubstituted heteroaryl radical optionally interrupted by at least one heteroatom, optionally bearing at least one functional group and having a total of 5 to 18 carbon atoms and/or heteroatoms,
K is an uncharged mono- or bidentate ligand,
L is a mono- or dianionic ligand, preferably monoanionic ligand, which may be mono- or bidentate,
m1 is 0, 1 or 2, where, when m1 is 2, the K ligands may be the same or different,
o1 is 0, 1 or 2, where, when o1 is 2, the L ligands may be the same or different.

The compound of formula **IX** is preferably a compound of the formula:

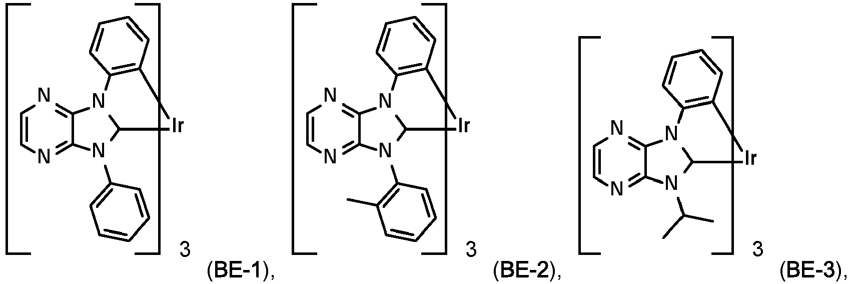

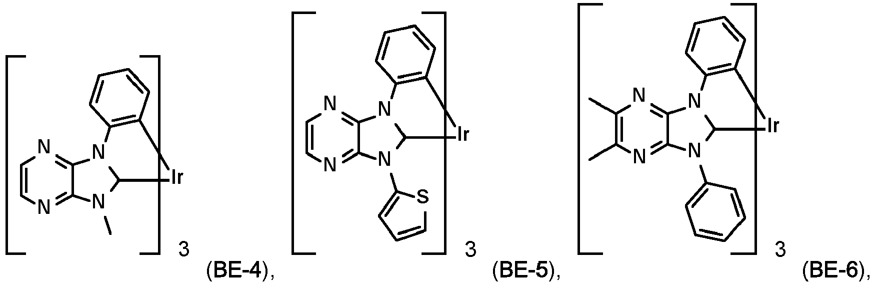

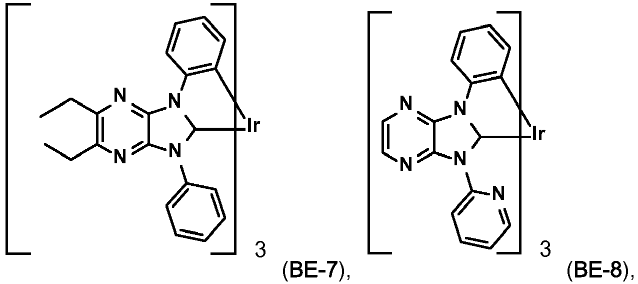

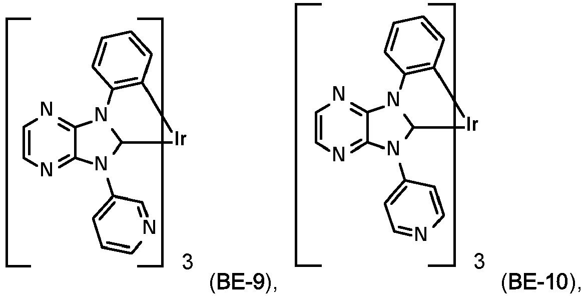

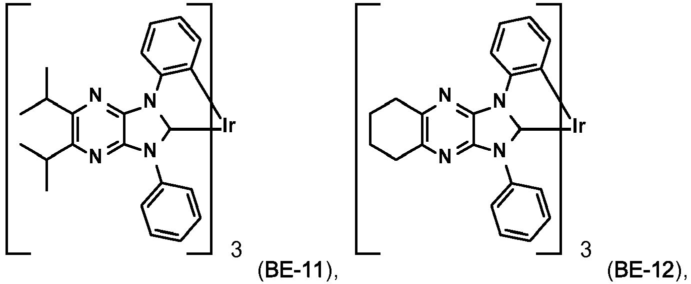

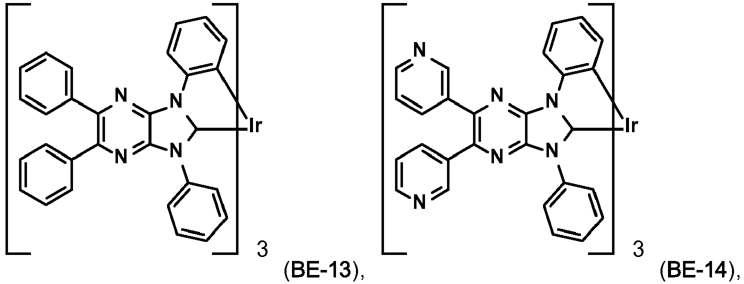

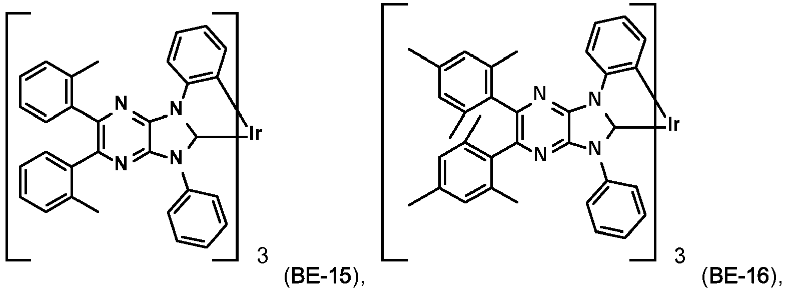

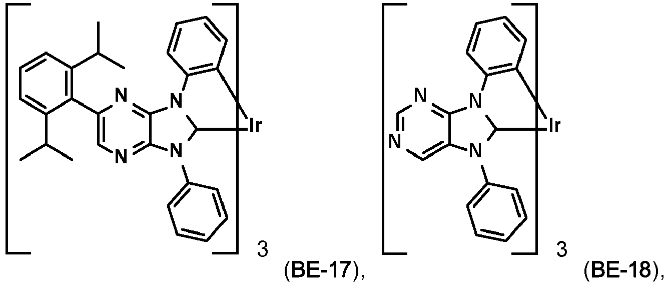

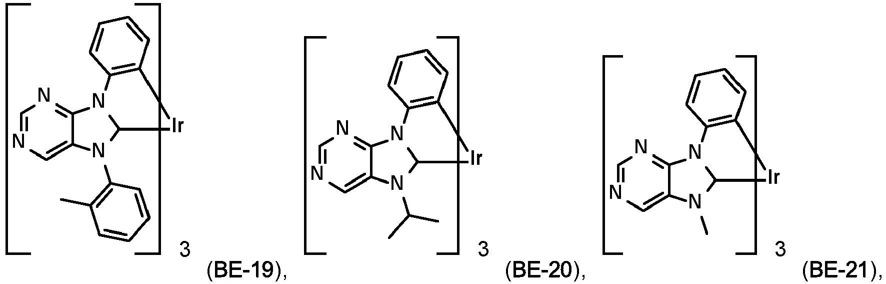

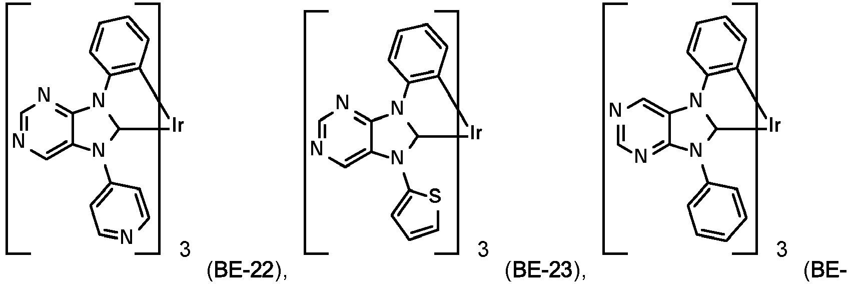

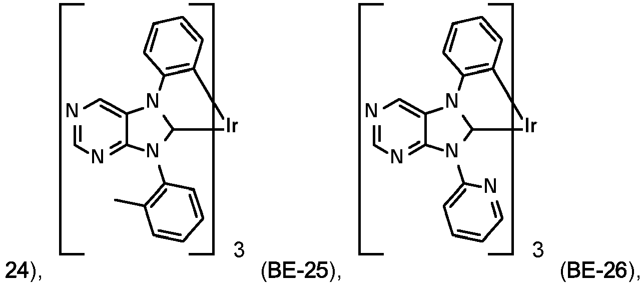

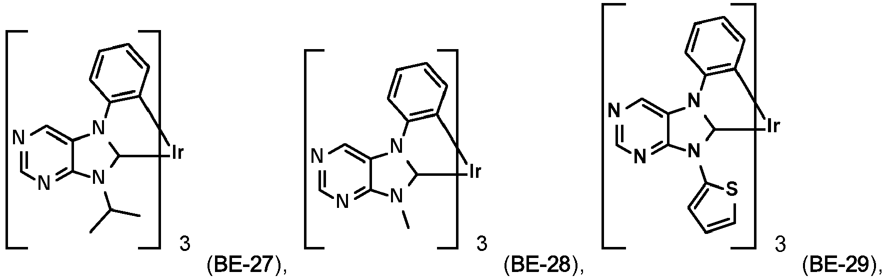

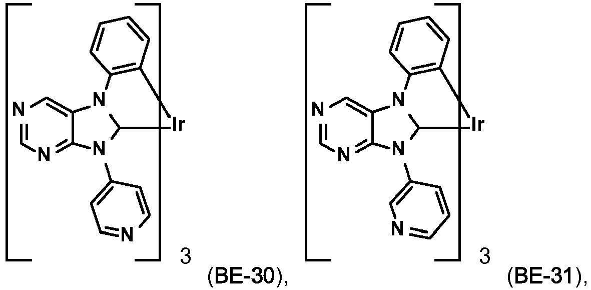

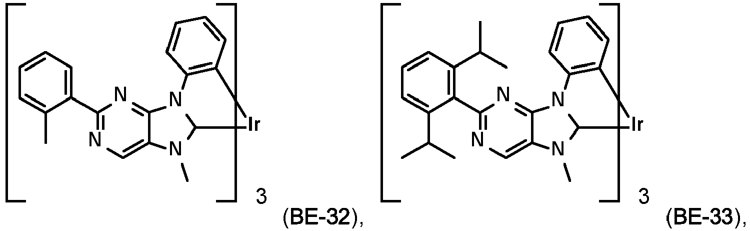

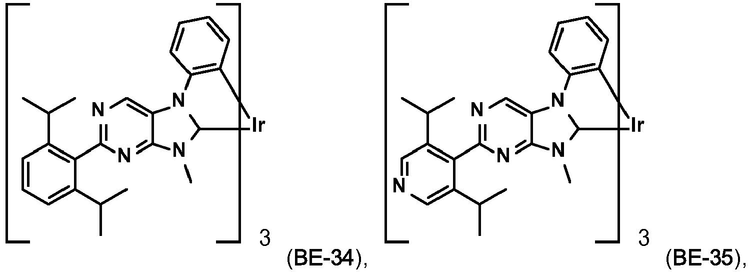

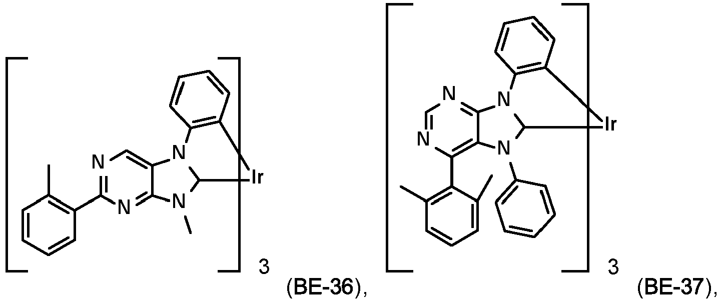

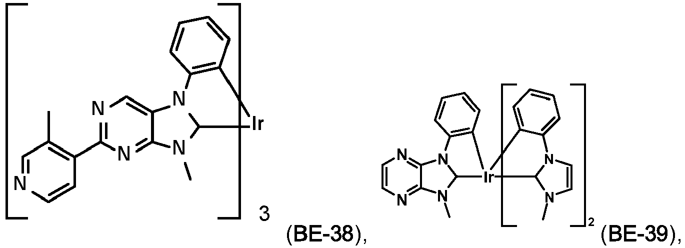

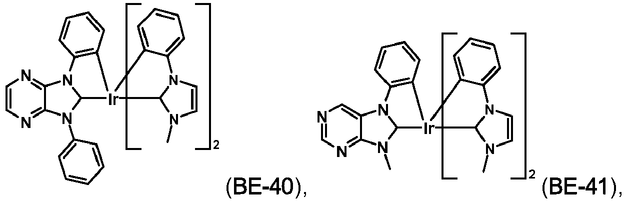

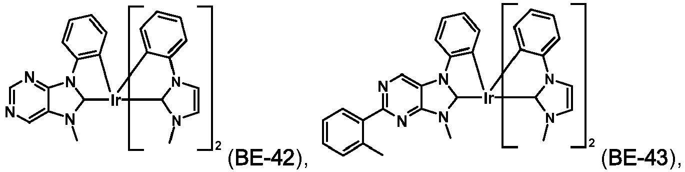

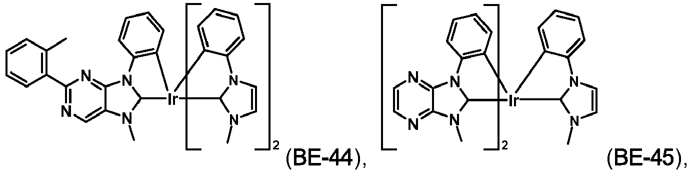

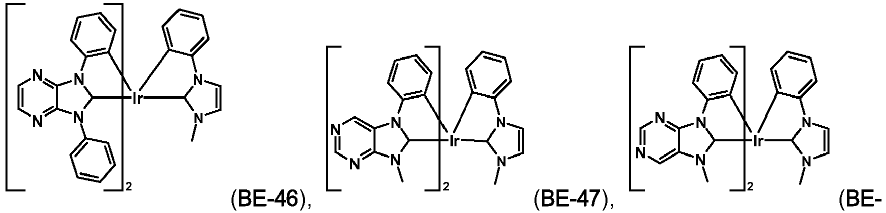

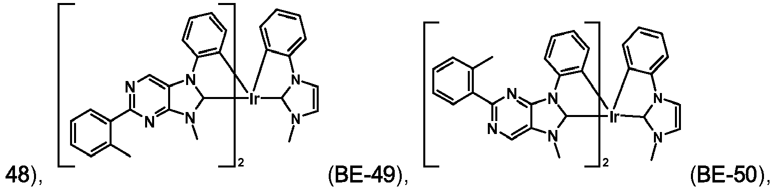

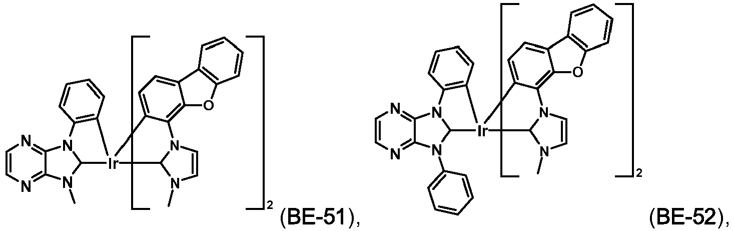

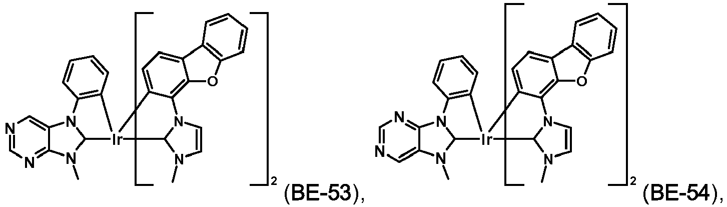

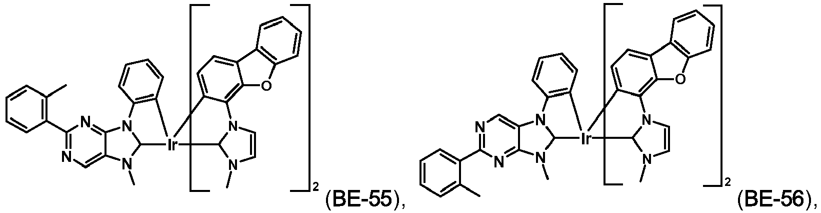

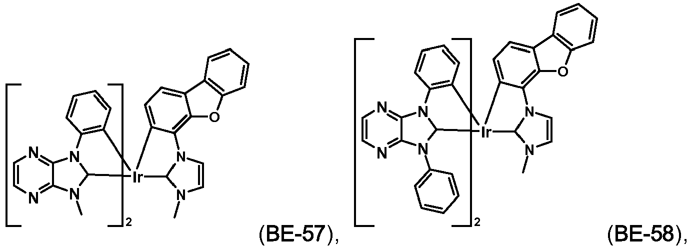

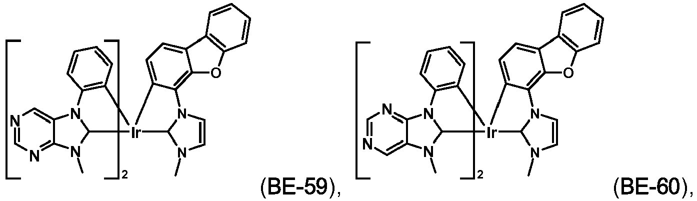

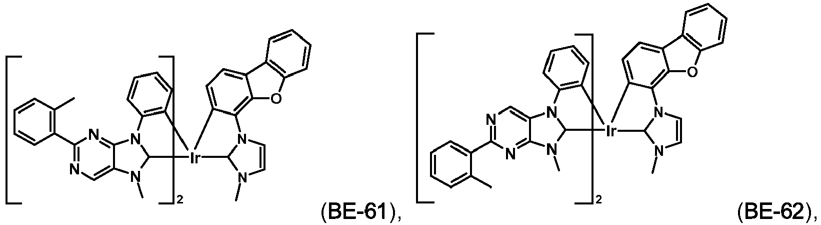

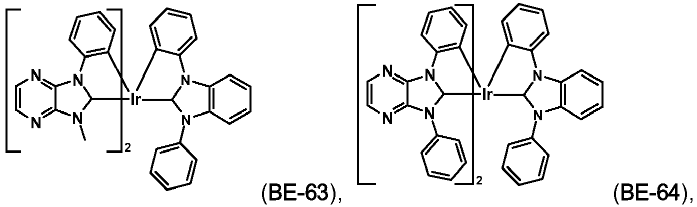

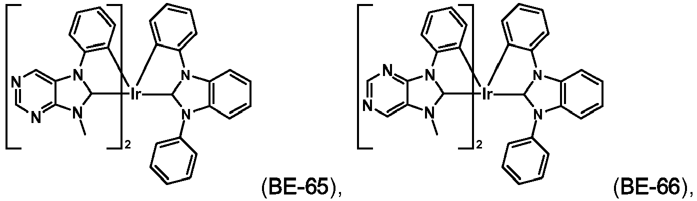

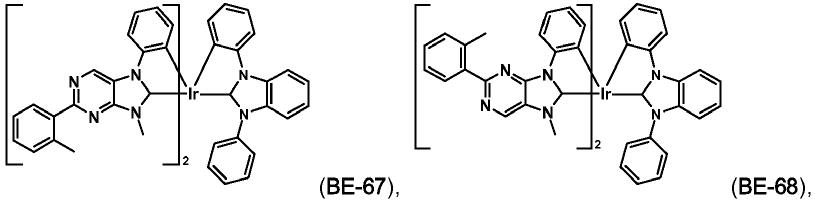

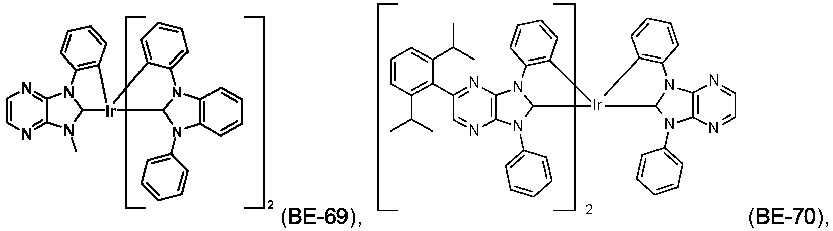

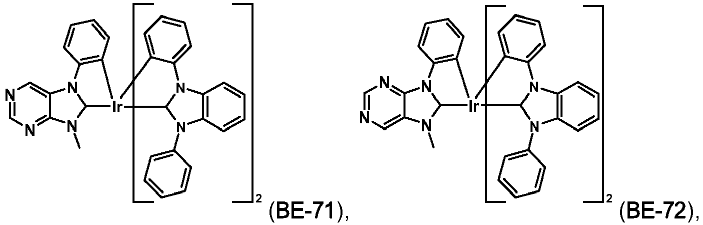

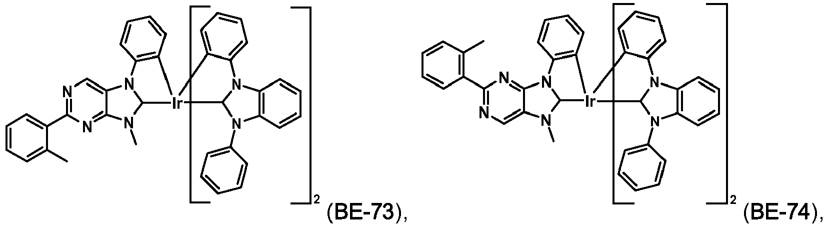

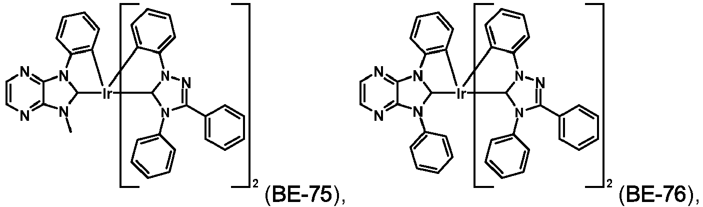

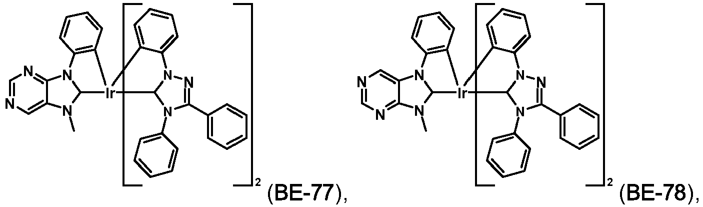

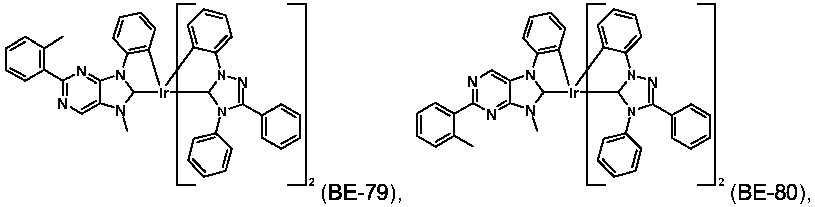

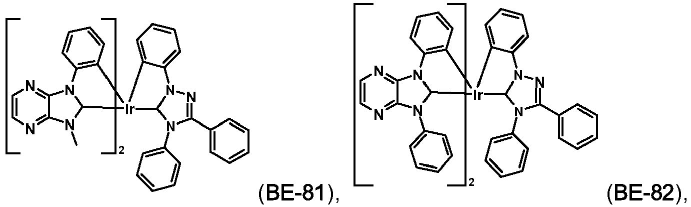

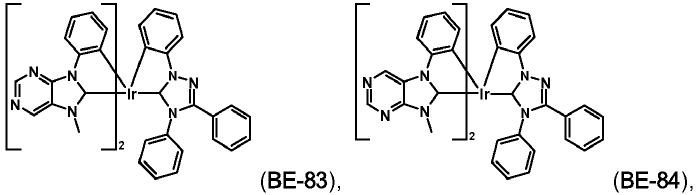

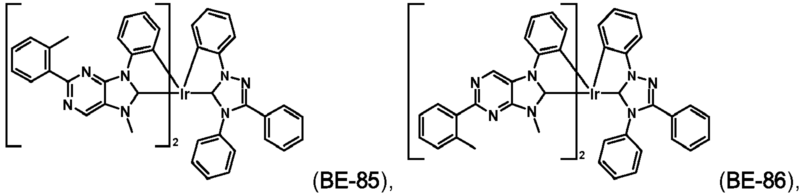

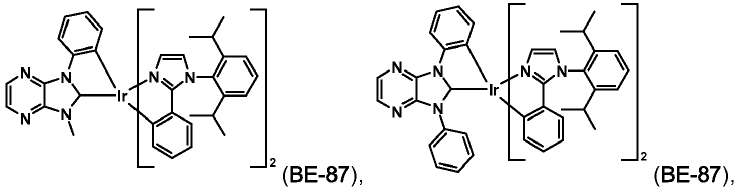

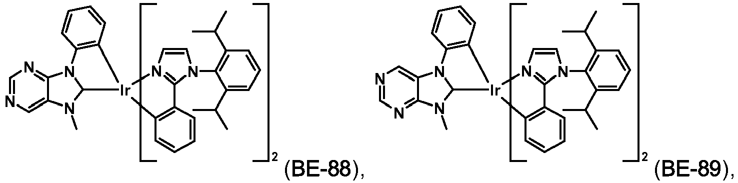

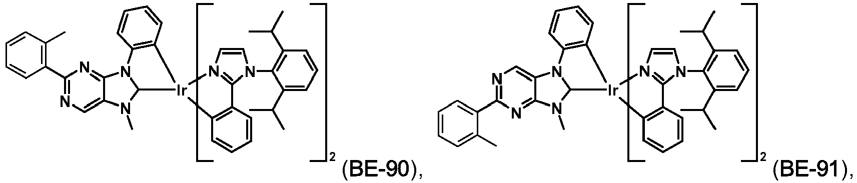

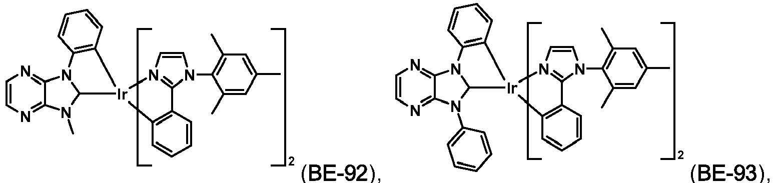

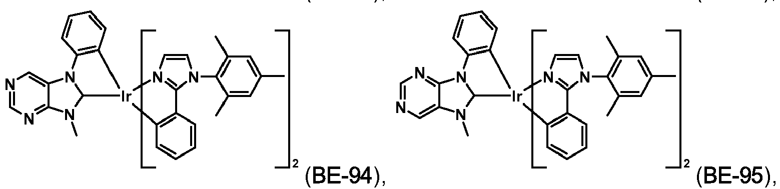

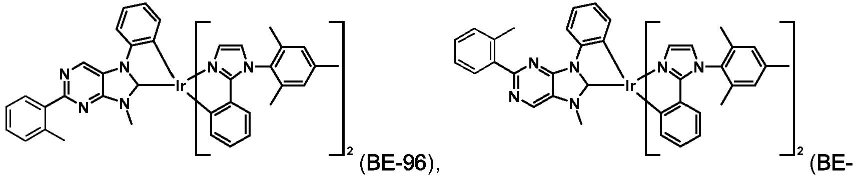

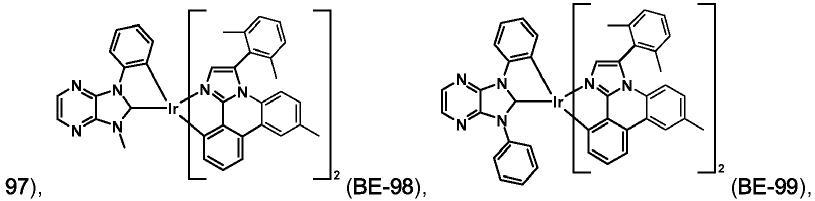

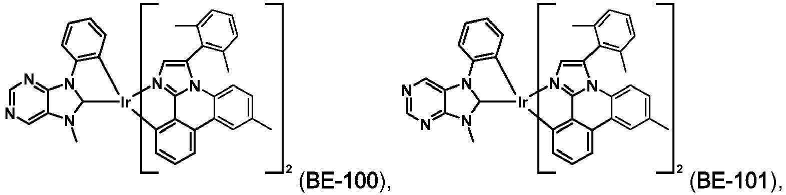

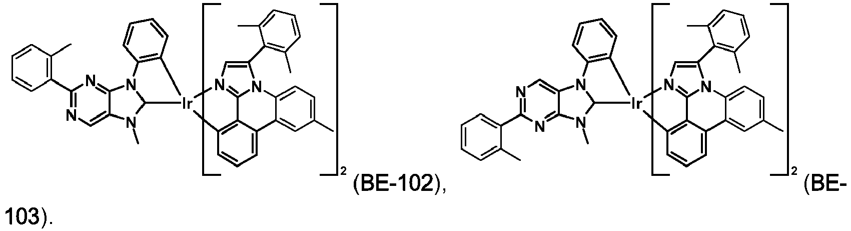

Further suitable non-carbene emitter materials are mentioned below:

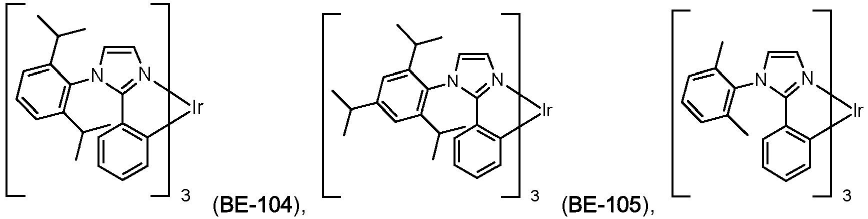

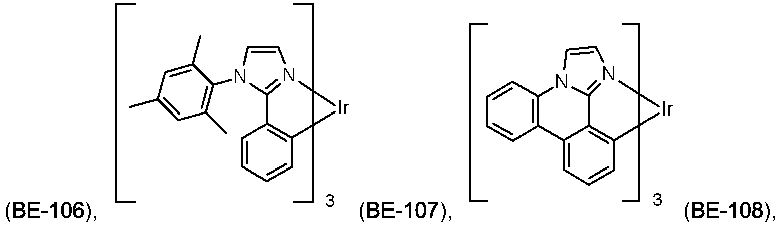

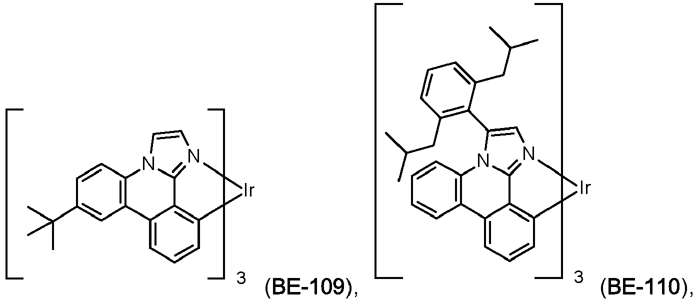

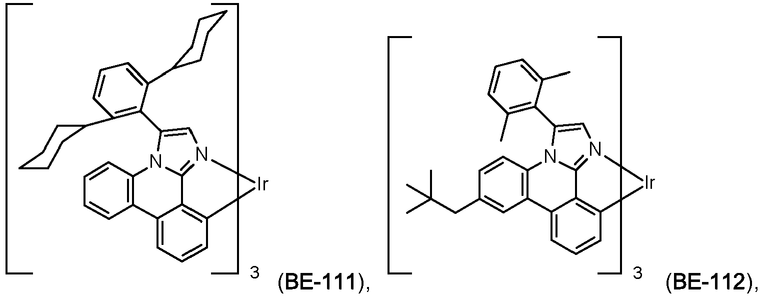

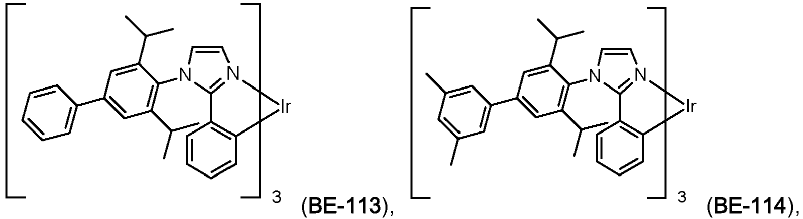

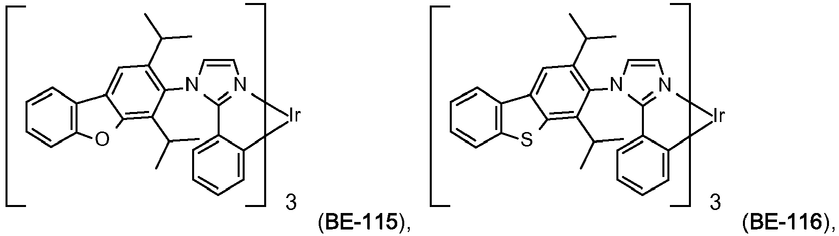

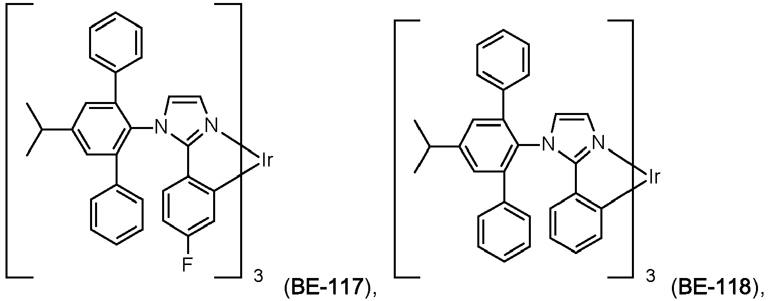

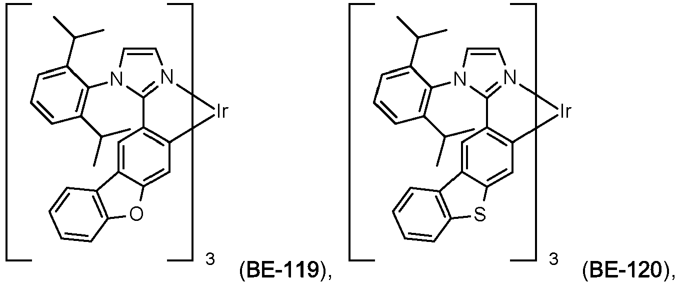

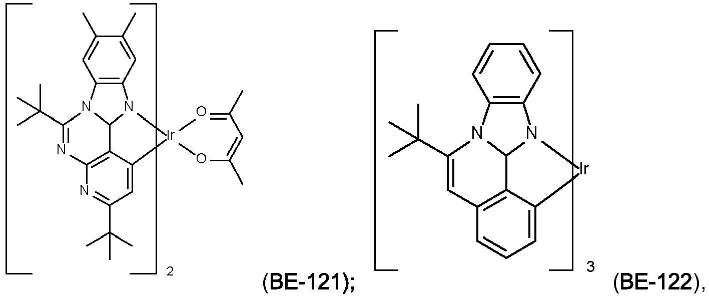

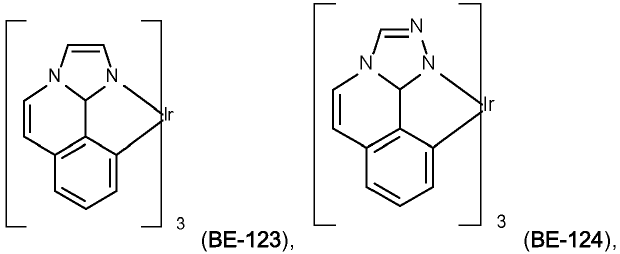

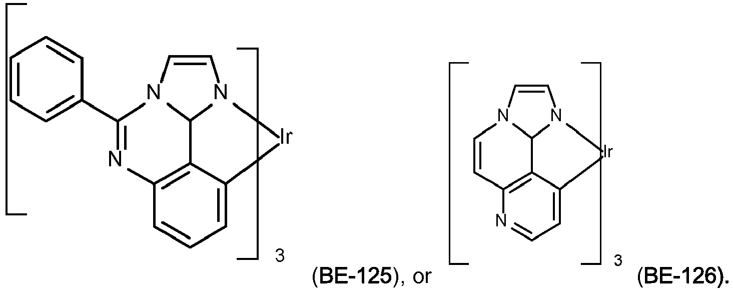

The compound of formula **IX** is more preferably a compound (**BE-1**), (**BE-2**), (**BE-7**), (**BE-12**), (**BE-16**), (**BE-64**), or (**BE-70**). The most preferred phosphorescent blue emitters are compounds (**BE-1**) and (**BE-12**).

The homoleptic metal-carbene complexes may be present in the form of facial or meridional isomers, preference being given to the facial isomers.

In the case of the heteroleptic metal-carbene complexes, four different isomers may be present, preference being given to the pseudo-facial isomers.

The light-emitting layer may comprise further components in addition to the emitter material. For example, a fluroescent dye may be present in the light-emitting layer in order to alter the emission color of the emitter material. In addition - in a preferred embodiment - a matrix material can be used. This matrix material may be a polymer, for example poly(N-vinylcarbazole) or polysilane. The matrix material may, however, be a small molecule, for example 4,4'-N,N'-dicarbazolebiphenyl (CDP=CBP) or tertiary aromatic amines, for example TCTA. In a preferred embodiment of the present invention, at least one compound of the formula **I** is used as matrix material.

In a preferred embodiment, the light-emitting layer is formed from 2 to 40% by weight, preferably 5 to 35% by weight, of at least one of the above mentioned emitter materials and 60 to 98% by weight, preferably 75 to 95% by weight, of at least one of the above, or below mentioned matrix materials - in one embodiment at least one compound of the formula I - where the sum total of the emitter material and of the matrix material adds up to 100% by weight.

The compound of the formula I is especially a compound of formula (**Ia**), (**Ib**), (**Ic**), (**Id**), (**Ie**), (**If**), (**Ii**), (**Ij**), (**Il**), (**Im**) or (**In**), very especially a compound **A-1** to **A-75, B-1** to **B-8**, **C-1** to **C-75, D-1** to **D-8, E-1** to **E-75, F-1** to **F-75, or G-1** to **G-75.**

In particularly preferred embodiment, the light-emitting layer comprises a compound of formula **I,** such as, for example, 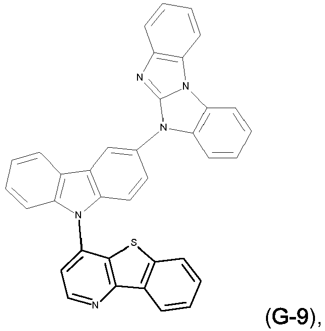 and two carbene complexes, preferably of formula 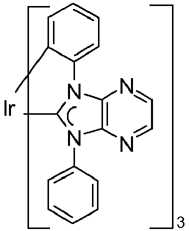 and **HTM-1,** or **HTM-2.** In said embodiment, the light-emitting layer is formed from 2 to 40% by weight, preferably 5 to 35% by weight, of 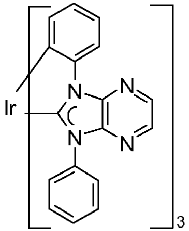 and 60 to 98% by weight, preferably 65 to 95% by weight, of a compound of the formula **I** and **HTM-1,** or **HTM-2,** where the sum total of the carben complexes and of the compound of formula **I** adds up to 100% by weight.

Suitable metal complexes for use together with the compounds of the formula **I** as matrix material and/or hole/exciton blocker material and/or electron/exciton blocker material and/or hole injection material and/or electron injection material and/or hole transport material and/or electron transport material, preferably as matrix material and/or hole/exciton blocker material, in OLEDs are thus, for example, also carbene complexes as described in WO 2005/019373 A2, WO 2006/056418 A2, WO 2005/113704, WO 2007/115970, WO 2007/115981, WO 2008/000727 and US2012/319050. Explicit reference is made here to the disclosure of the WO applications cited. The compounds of the present invention can also be used as host for phosphorescent green emitters. Suitable phosphorescent green emitters are, for example, specified in the following publications: WO2006014599, WO20080220265, WO2009073245, WO2010027583, WO2010028151, US20110227049, WO2011090535, WO2012/08881, WO20100056669, WO20100118029, WO20100244004, WO2011109042, WO2012166608, US20120292600, EP2551933A1; US6687266, US20070190359, US20070190359, US20060008670; WO2006098460, US20110210316, WO 2012053627; US6921915, US20090039776; and JP2007123392.

Examples of suitable phosphorescent green emitters are shown below:

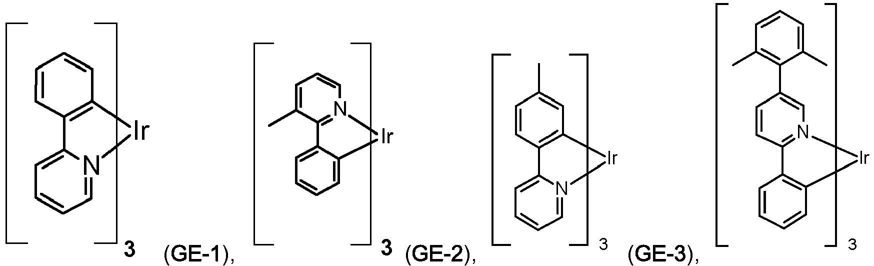

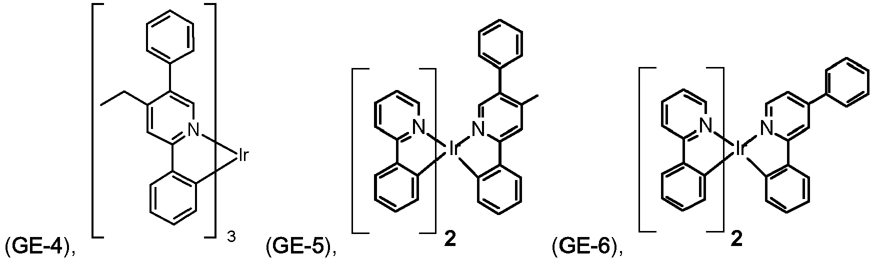

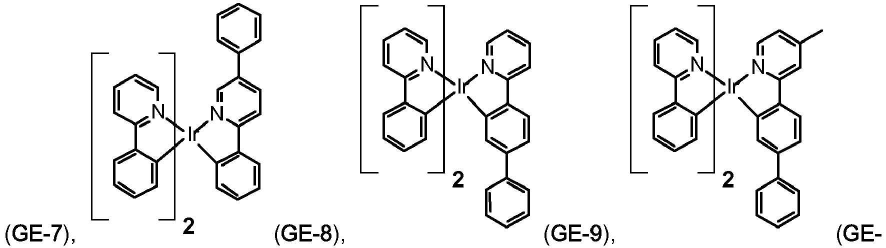

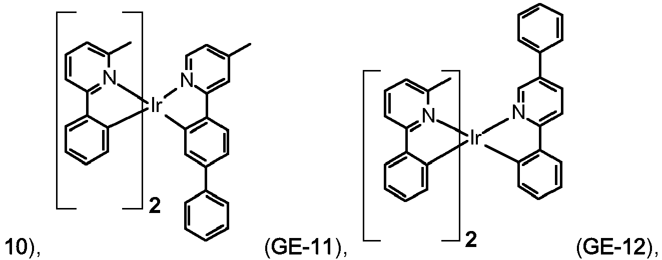

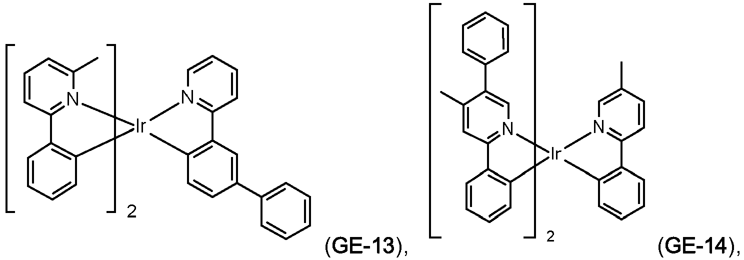

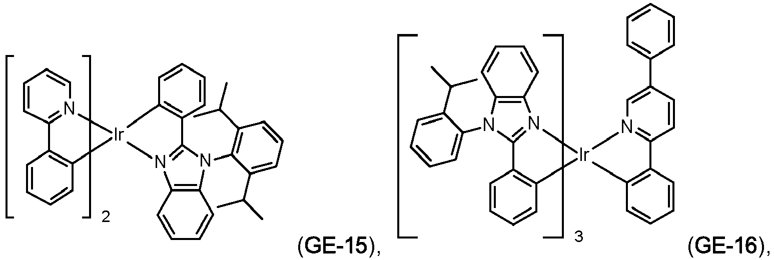

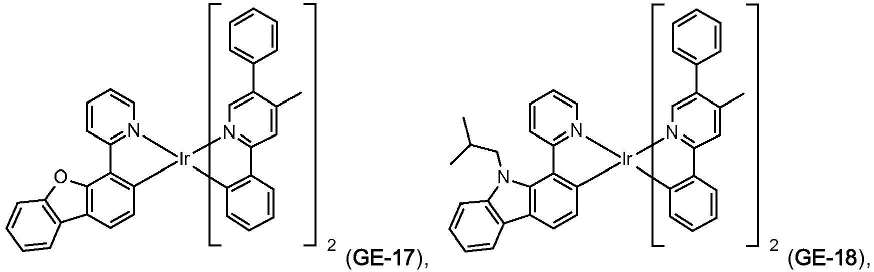

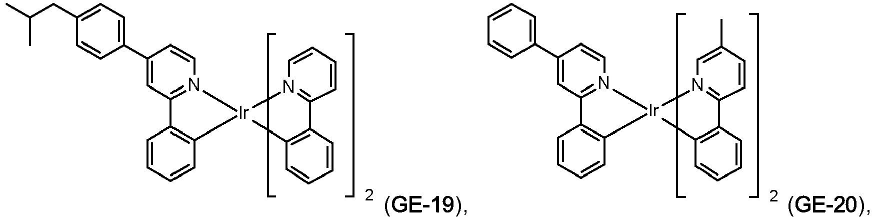

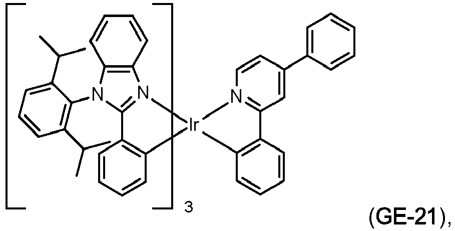

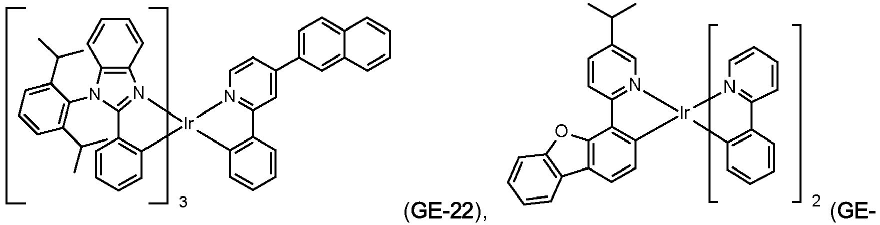

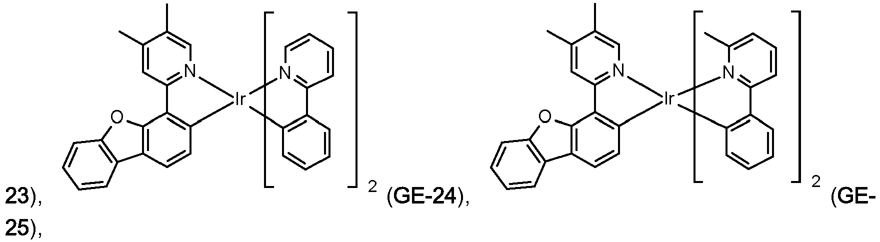

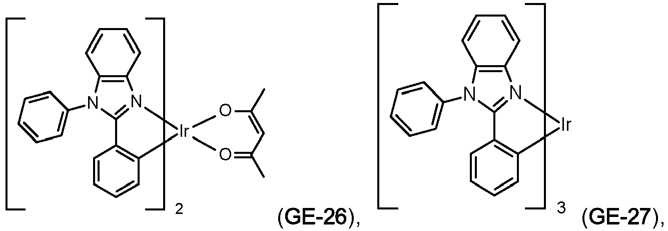

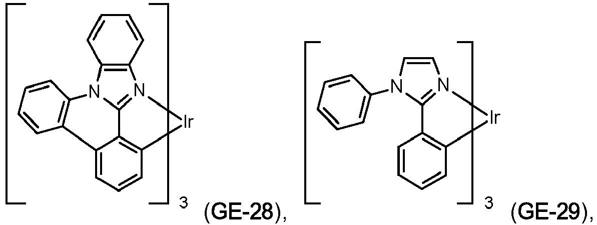

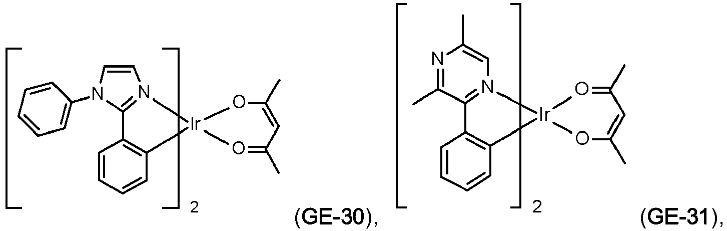

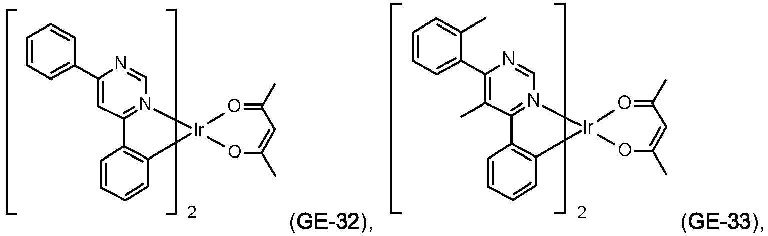

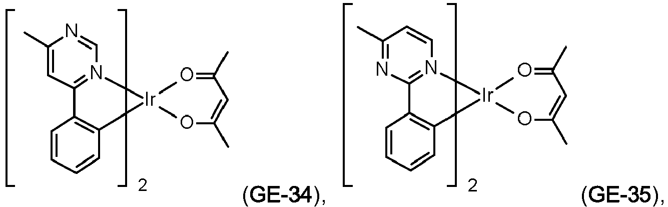

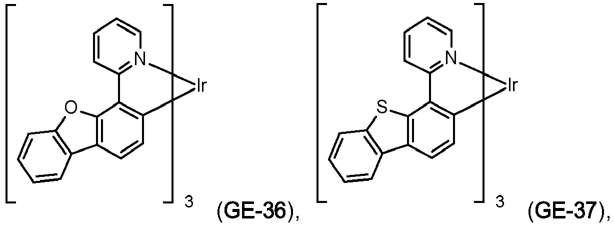

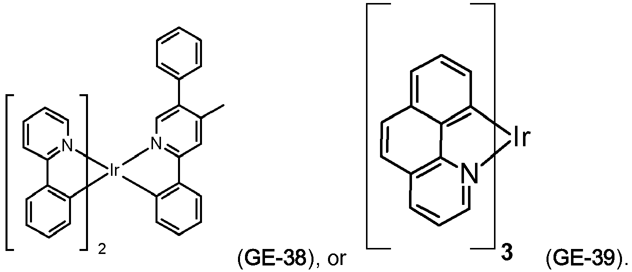

If a blocking layer for holes is present - hole blocker materials typically used in OLEDs, are, for example, 2,6-bis(N-carbazolyl)pyridine (mCPy), 2,9-dimethyl-4,7-diphenyl-1,10-phenanthroline (bathocuproin, (BCP)), bis(2-methyl-8-quinolinato)-4-phenylphenylato)aluminum(III) (BAlq), phenothiazine S,S-dioxide derivates and 1,3,5-tris(N-phenyl-2-benzylimidazolyl)benzene) (TPBI), TPBI also being suitable as electron-conducting material. Further suitable hole blockers and/or electron transport materials are 2,2',2"-(1,3,5-benzenetriyl)tris(1-phenyl-1-H-benzimidazole), 2-(4-biphenylyl)-5-(4-tert-butylphenyl)-1,3,4-oxadiazole, 8-hydroxyquinolinolatolithium, 4-(naphthalen-1-yl)-3,5-diphenyl-4H-1,2,4-triazole, 1,3-bis[2-(2,2'-bipyridin-6-yl)-1,3,4-oxadiazo-5-yl]benzene, 4,7-diphenyl-1,10-phenanthroline, 3-(4-biphenylyl)-4-phenyl-5-tert-butylphenyl-1,2,4-triazole, 6,6'-bis[5-(biphenyl-4-yl)-1,3,4-oxadiazo-2-yl]-2,2'-bipyridyl, 2-phenyl-9,10-di(naphthalene-2-yl)anthracene, 2,7-bis[2-(2,2'-bipyridin-6-yl)-1,3,4-oxadiazo-5-yl]-9,9-dimethylfluorene, 1,3-bis[2-(4-tert-butylphenyl)-1,3,4-oxadiazo-5-yl]benzene, 2-(naphthalene-2-yl)-4,7-diphenyl-1,10-phenanthroline, tris(2,4,6-trimethyl-3-(pyridin-3-yl)phenyl)borane, 2,9-bis(naphthalene-2-yl)-4,7-diphenyl-1,10-phenanthroline, 1-methyl-2-(4-(naphthalene-2-yl)phenyl)-1H-imidazo[4,5-f][1,10]phenanthroline. In a further embodiment, it is possible to use compounds which comprise aromatic or heteroaromatic rings joined via groups comprising carbonyl groups, as disclosed in WO2006/100298, disilyl compounds selected from the group consisting of disilylcarbazoles, disilylbenzofurans, disilylbenzothiophenes, disilylbenzophospholes, disilylbenzothiophene S-oxides and disilylbenzothiophene S,S-dioxides, as specified, for example, in WO2009/003919 and WO2009003898 and disilyl compounds as disclosed in WO2008/034758, as a blocking layer for holes/excitons (4) or as matrix materials in the light-emitting layer (3). In principle, it is possible that the hole blocking layer comprises at least one compound of the formula **I,** especially a compound of formula (**Ia**), (**Ib**), (**Ic**), (**Id**), (**Ie**), (**If**), (**Ii**), (**Ij**), (**Il**), (**Im**) or (**In**), very especially a compound **A-1** to **A-75, B-1** to **B-8**, **C-1** to **C-75, D-1** to **D-8, E-1** to **E-75, F-1** to **F-75, or G-1** to **G-75.**

Suitable electron transport materials for the layer (5) of the inventive OLEDs comprise metals chelated to oxinoid compounds, such as 2,2',2"-(1,3,5-phenylene)tris[1-phenyl-1H-benzimidazole] (TPBI), tris(8-quinolinolato)aluminum (Alq₃), compounds based on phenanthroline, such as 2,9-dimethyl-4,7-diphenyl-1,10-phenanthroline (DDPA = BCP) or 4,7-diphenyl-1,10-phenanthroline (DPA), and azole compounds such as 2-(4-biphenylyl)-5-(4-t-butylphenyl)-1,3,4-oxadiazole (PBD) and 3-(4-biphenylyl)-4-phenyl-5-(4-t-butylphenyl)-1,2,4-triazole (TAZ), 8-hydroxyquinolinolatolithium (Liq), 4,7-diphenyl-1,10-phenanthroline (BPhen), bis(2-methyl-8-quinolinolato)-4-(phenylphenolato)aluminum (BAlq), 1,3-bis[2-(2,2'-bipyridin-6-yl)-1,3,4-oxadiazo-5-yl]benzene (Bpy-OXD), 6,6'-bis[5-(biphenyl-4-yl)-1,3,4-oxadiazo-2-yl]-2,2'-bipyridyl (BP-OXD-Bpy), 4-(naphthalen-1-yl)-3,5-diphenyl-4H-1,2,4-triazole (NTAZ), 2,9-bis(naphthalen-2-yl)-4,7-diphenyl-1,10-phenanthroline (NBphen), 2,7-bis[2-(2,2'-bipyridin-6-yl)-1,3,4-oxadiazo-5-yl]-9,9-dimethylfluorene (Bby-FOXD), 1,3-bis[2-(4-tert-butylphenyl)-1,3,4-oxadiazo-5-yl]benzene (OXD-7), tris(2,4,6-trimethyl-3-(pyridin-3-yl)phenyl)borane (3TPYMB), 1-methyl-2-(4-(naphthalen-2-yl)phenyl)-1H-imidazo[4,5-f][1,10]phenanthroline (2-NPIP), 2-phenyl-9,10-di(naphthalen-2-yl)anthracene (PADN), 2-(naphthalen-2-yl)-4,7-diphenyl-1,10-phenanthroline (HNBphen). The layer (5) may serve both to facilitate electron transport and as a buffer layer or barrier layer in order to prevent quenching of the exciton at the interfaces of the layers of the OLED. The layer (5) preferably improves the mobility of the electrons and reduces quenching of the exciton. In a preferred embodiment, TPBI is used as the electron transport material. In another preferred embodiment, BCP is used as the electron transport material. In principle, it is possible that the electron transport layer comprises at least one compound of the formula **I** as electron transport material. In principle, it is possible that the electron transport layer comprises at least one compound of the formula **I** as hole transport material, especially a compound of formula (**Ia**), (**Ib**), (**Ic**), (**Id**), (**Ie**), (**If**), (**Ii**), (**Ij**), (**Il**), (**Im**) or (**In**).

The electron-transport layer may also be electronically doped in order to improve the transport properties of the materials used, in order firstly to make the layer thicknesses more generous (avoidance of pinholes/short circuits) and in order secondly to minimize the operating voltage of the device. Electronic doping is known to those skilled in the art and is disclosed, for example, in W. Gao, A. Kahn, J. Appl. Phys., Vol. 94, No. 1, 1 July 2003 (p-doped organic layers); A. G. Werner, F. Li, K. Harada, M. Pfeiffer, T. Fritz, K. Leo, Appl. Phys. Lett., Vol. 82, No. 25, 23 June 2003 and Pfeiffer et al., Organic Electronics 2003, 4, 89 - 103 and K. Walzer, B. Maennig, M. Pfeiffer, K. Leo, Chem. Soc. Rev. 2007, 107, 1233. For example, it is possible to use mixtures which lead to electrical n-doping of the electron-transport layer. n-Doping is achieved by the addition of reducing materials. These mixtures may, for example, be mixtures of the abovementioned electron transport materials with alkali/alkaline earth metals or alkali/alkaline earth metal salts, for example Li, Cs, Ca, Sr, Cs₂CO₃, with alkali metal complexes, for example 8-hydroxyquinolatolithium (Liq), and with Y, Ce, Sm, Gd, Tb, Er, Tm, Yb, Li₃N, Rb₂CO₃, dipotassium phthalate, W(hpp)₄ from EP1786050, or with compounds described in EP1837926B1, EP1837927, EP2246862 and WO2010132236.

It is likewise possible to use mixtures of alkali metal hydroxyquinolate complexes, preferably Liq, and dibenzofuran compounds in the electron-transport layer. Reference is made to WO2011/157790. Dibenzofuran compounds **A-1** to **A-36** and **B-1** to **B-22** described in WO2011/157790 are preferred, wherein dibenzofuran compound 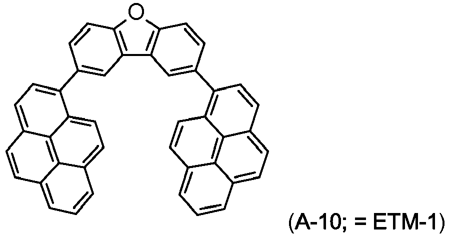 is most preferred.

In a preferred embodiment, the electron-transport layer comprises **Liq** in an amount of 99 to 1% by weight, preferably 75 to 25% by weight, more preferably about 50% by weight, where the amount of **Liq** and the amount of the dibenzofuran compound(s), especially **ETM-1,** adds up to a total of 100% by weight.

It is likewise possible to use mixtures of alkali metal hydroxyquinolate complexes, preferably Liq, and pyridine compounds in the electron-transport layer. Reference is made to WO2011/157779. Particular preference is given to a pyridine compound of the formula 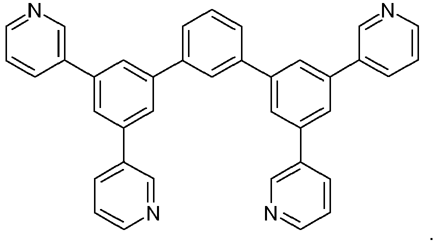 In a further preferred embodiment, the electron-transport layer comprises a compound described in WO2012/111462, WO2012/147397, WO2012014621, such as, for example, a compound of formula 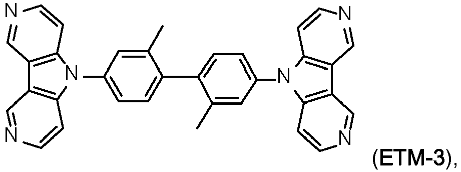 US2012/0261654 , such as, for example, a compound of formula 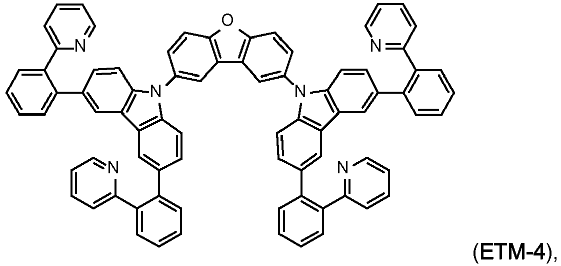 and WO2012/115034, such as for example, such as, for example, a compound of formula 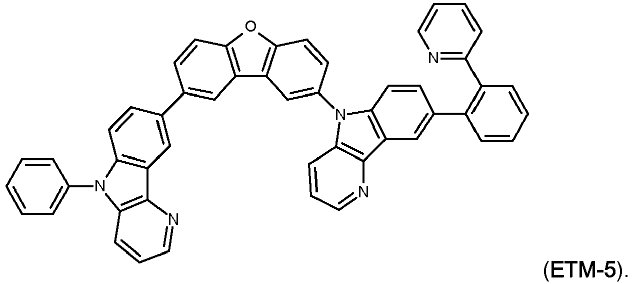 Among the materials mentioned above as hole transport materials and electron transport materials, some may fulfil several functions. For example, some of the electron-conducting materials are simultaneously hole-blocking materials when they have a low-lying HOMO. These can be used, for example, in the blocking layer for holes/excitons (4). However, it is likewise possible that the function as a hole/exciton blocker is also adopted by the layer (5), such that the layer (4) can be dispensed with.

The charge transport layers can also be electronically doped in order to improve the transport properties of the materials used, in order firstly to make the layer thicknesses more generous (avoidance of pinholes/short circuits) and in order secondly to minimize the operating voltage of the device. For example, the hole transport materials can be doped with electron acceptors; for example, phthalocyanines or arylamines such as TPD or TDTA can be doped with tetrafluorotetracyanquinodimethane (F4-TCNQ) or with MoO₃ or WO₃. The electron transport materials can be doped, for example, with alkali metals, for example Alq₃ with lithium. In addition, electron transport materials can be doped with salts such as Cs₂CO₃, or 8-hydroxyquinolatolithium (Liq). Electronic doping is known to those skilled in the art and is disclosed, for example, in W. Gao, A. Kahn, J. Appl. Phys., Vol. 94, No. 1, 1 July 2003 (p-doped organic layers); A. G. Werner, F. Li, K. Harada, M. Pfeiffer, T. Fritz, K. Leo. Appl. Phys. Lett., Vol. 82, No. 25, 23 June 2003 and Pfeiffer et al., Organic Electronics 2003, 4, 89 - 103. For example, the hole transport layer may, in addition to a carbene complex, e.g. Ir(dpbic)₃, be doped with MoO₃ or WO₃. For example, the electron transport layer may comprise BCP doped with Cs₂CO₃.

The cathode (6) is an electrode which serves to introduce electrons or negative charge carriers. Suitable materials for the cathode are selected from the group consisting of alkali metals of group Ia, for example Li, Cs, alkaline earth metals of group IIa, for example calcium, barium or magnesium, metals of group IIb of the periodic table of the elements (old IUPAC version), comprising the lanthanides and actinides, for example samarium. In addition, it is also possible to use metals such as aluminum or indium, and combinations of all metals mentioned. In addition, alkali metal, especially lithium-comprising organometallic compounds, or alkali metal fluorides, such as, for example, LiF, CsF, or KF can be applied between the organic layer and the cathode in order to reduce the operating voltage.

The OLED according to the present invention may additionally comprise further layers which are known to those skilled in the art. For example, a layer which facilitates the transport of the positive charge and/or matches the band gaps of the layers to one another may be applied between the layer (2) and the light-emitting layer (3). Alternatively, this further layer may serve as a protective layer. In an analogous manner, additional layers may be present between the light-emitting layer (3) and the layer (4) in order to facilitate the transport of negative charge and/or to match the band gaps between the layers to one another. Alternatively, this layer may serve as a protective layer.

In a preferred embodiment, the inventive OLED, in addition to layers (1) to (6), comprises at least one of the following layers mentioned below:
- a hole injection layer between the anode (1) and the hole-transporting layer (2) having a thickness of 2 to 100 nm, prefreably 5 to 50 nm;
- a blocking layer for electrons between the hole-transporting layer (2) and the light-emitting layer (3);
- an electron injection layer between the electron-transporting layer (5) and the cathode (6).

Materials for a hole injection layer may be selected from copper phthalocyanine, 4,4',4"-tris(N-3-methylphenyl-N-phenylamino)triphenylamine (m-MTDATA), 4,4',4"-tris(N-(2-naphthyl)-N-phenylamino)triphenylamine (2T-NATA), 4,4',4"-tris(N-(1-naphthyl)-N-phenylamino)triphenylamine (1T-NATA), 4,4',4"-tris(N,N-diphenylamino)triphenylamine (NATA), titanium oxide phthalocyanine, 2,3,5,6-tetrafluoro-7,7,8,8-tetracyanoquinodimethane (F4-TCNQ), pyrazino[2,3-f][1,10]phenanthroline-2,3-dicarbonitrile (PPDN), N,N,N',N'-tetrakis(4-methoxyphenyl)benzidine (MeO-TPD), 2,7-bis[N,N-bis(4-methoxyphenyl)amino]-9,9-spirobifluorene (MeO-Spiro-TPD), 2,2'-bis[N,N-bis(4-methoxyphenyl)amino]-9,9-spirobifluorene (2,2'-MeO-Spiro-TPD), N,N'-diphenyl-N,N'-di-[4-(N,N-ditolylamino)phenyl]benzidine (NTNPB), N,N'-diphenyl-N,N'-di-[4-(N,N-diphenyl-amino)phenyl]benzidine (NPNPB), N,N'-di(naphthalen-2-yl)-N,N'-diphenylbenzene-1,4-diamine (α-NPP), or dipyrazino[2,3-f:2',3'-h]quinoxaline-2,3,6,7,10,11-hexacarbonitrile (HAT-CN). In principle, it is possible that the hole injection layer comprises at least one compound of the formula I as hole injection material. In addition, polymeric hole-injection materials can be used such as poly(N-vinylcarbazole) (PVK), polythiophenes, polypyrrole, polyaniline, self-doping polymers, such as, for example, sulfonated poly(thiophene-3-[2[(2-methoxyethoxy)ethoxy]-2,5-diyl) (Plexcore® OC Conducting Inks commercially available from Plextronics), and copolymers such as poly(3,4-ethylenedioxythiophene)/poly(4-styrenesulfonate) also called PEDOT/PSS.

The compound of formula **I,** especially the compound of formula (**Ia**), (**Ib**), (**Ic**), (**Id**), (**Ie**), (**If**), (**Ii**), (**Ij**), (**Il**), (**Im**), or (**In**), very especially a compound **A-1** to **A-75, B-1** to **B-8**, **C-1** to **C-75, D-1** to **D-8, E-1** to **E-75, F-1** to **F-75, G-1** to **G-75** can be used as electron/exciton blocker material.

Suitable metal complexes for use as electron/exciton blocker material are, for example, carbene complexes as described in WO2005/019373A2, WO2006/056418A2, WO2005/113704, WO2007/115970, WO2007/115981, WO2008/000727 and US2012/319050. One example of a suitable carbene complex is compound **HTM-1.** Another example of a suitable carbene complex is compound **HTM-2.**

As a material for the electron injection layer, LiF, for example, can be selected. In principle, it is possible that the electron injection layer comprises at least one compound of the formula I as electron injection material.

The person skilled in the art is aware (for example on the basis of electrochemical studies) of how suitable materials have to be selected. Suitable materials for the individual layers are known to those skilled in the art and are disclosed, for example, in WO 00/70655.

In addition, it is possible that some of the layers used in the inventive OLED have been surface-treated in order to increase the efficiency of charge carrier transport. The selection of the materials for each of the layers mentioned is preferably determined by obtaining an OLED with a high efficiency and lifetime.

The inventive OLED can be produced by methods known to those skilled in the art. In general, the inventive OLED is produced by successive vapor deposition of the individual layers onto a suitable substrate. Suitable substrates are, for example, glass, inorganic semiconductors or polymer films. For vapor deposition, it is possible to use customary techniques, such as thermal evaporation, chemical vapor deposition (CVD), physical vapor deposition (PVD) and others. In an alternative process, the organic layers of the OLED can be applied from solutions or dispersions in suitable solvents, employing coating techniques known to those skilled in the art.

In general, the different layers have the following thicknesses: anode (1) 50 to 500 nm, preferably 100 to 200 nm; hole-conducting layer (2) 5 to 100 nm, preferably 20 to 80 nm, light-emitting layer (3) 1 to 100 nm, preferably 10 to 80 nm, blocking layer for holes/excitons (4) 2 to 100 nm, preferably 5 to 50 nm, electron-conducting layer (5) 5 to 100 nm, preferably 20 to 80 nm, cathode (6) 20 to 1000 nm, preferably 30 to 500 nm. The relative position of the recombination zone of holes and electrons in the inventive OLED in relation to the cathode and hence the emission spectrum of the OLED can be influenced, among other factors, by the relative thickness of each layer. This means that the thickness of the electron transport layer should preferably be selected such that the position of the recombination zone is matched to the optical resonator property of the diode and hence to the emission wavelength of the emitter. The ratio of the layer thicknesses of the individual layers in the OLED depends on the materials used. The layer thicknesses of any additional layers used are known to those skilled in the art. It is possible that the electron-conducting layer and/or the hole-conducting layer have greater thicknesses than the layer thicknesses specified when they are electrically doped.

Use of the compounds of the formula I in at least one layer of the OLED, preferably in the light-emitting layer (preferably as a matrix material) and/or in the blocking layer for holes/excitons makes it possible to obtain OLEDs with high efficiency and with low use and operating voltage. Frequently, the OLEDs obtained by the use of the compounds of the formula I additionally have high lifetimes. The efficiency of the OLEDs can additionally be improved by optimizing the other layers of the OLEDs. For example, high-efficiency cathodes such as Ca or Ba, if appropriate in combination with an intermediate layer of LiF, can be used. Shaped substrates and novel hole-transporting materials which bring about a reduction in the operating voltage or an increase in the quantum efficiency are likewise usable in the inventive OLEDs. Moreover, additional layers may be present in the OLEDs in order to adjust the energy level of the different layers and to facilitate electroluminescence.

The OLEDs may further comprise at least one second light-emitting layer. The overall emission of the OLEDs may be composed of the emission of the at least two light-emitting layers and may also comprise white light.

The OLEDs can be used in all apparatus in which electroluminescence is useful. Suitable devices are preferably selected from stationary and mobile visual display units and illumination units. Stationary visual display units are, for example, visual display units of computers, televisions, visual display units in printers, kitchen appliances and advertising panels, illuminations and information panels. Mobile visual display units are, for example, visual display units in cellphones, tablet PCs, laptops, digital cameras, MP3 players, vehicles and destination displays on buses and trains. Further devices in which the inventive OLEDs can be used are, for example, keyboards; items of clothing; furniture; wallpaper. In addition, the present invention relates to a device selected from the group consisting of stationary visual display units such as visual display units of computers, televisions, visual display units in printers, kitchen appliances and advertising panels, illuminations, information panels, and mobile visual display units such as visual display units in cellphones, tablet PCs, laptops, digital cameras, MP3 players, vehicles and destination displays on buses and trains; illumination units; keyboards; items of clothing; furniture; wallpaper, comprising at least one inventive organic light-emitting diode or at least one inventive light-emitting layer.

The following examples are included for illustrative purposes only and do not limit the scope of the claims. Unless otherwise stated, all parts and percentages are by weight.

### Examples

### Synthesis Example 1

### 5-(9H-carbazol-3-yl)benzimidazolo[1,2-a]benzimidazole

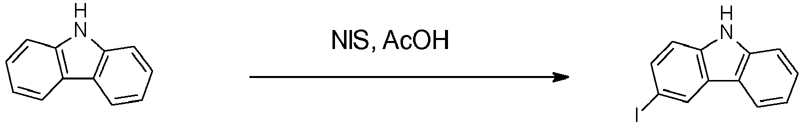

a) 76.9 g (0.460 mol) of carbazole and 104 g (0.460 mol) of 1-iodopyrrolidine-2,5-dione (NIS) in 100m ml of acetic acid are stirred under nitrogen at 20 °C. After 5 h the product is filtered off. The product is dissolved in 900 ml of ethanol at reflux, then 2 g of charcoal are added. The ethanol solution is filtered hot and cooled to 20 °C. The product is filtered off to yield 59.5 g (44 %) of 3-iodo-9H-carbazole. 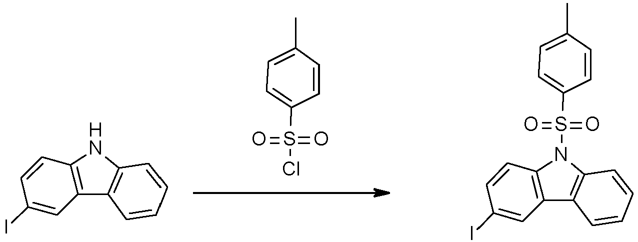
b) 19.7 g (67.0 mmol) of 3-iodo-9H-carbazole and 2.95 g (73.7 mmol) of sodium hydride 60 % dispersion in mineral oil are stirred in 500 ml of tetrahydrofuran (THF) at 50 °C under nitrogen for 1h. 12.8 g (67.0 mmol) of 4-methylbenzenesulfonyl chloride in 100 ml of THF are added at 20 °C. The reaction mixture is stirred for 1 h at 20 °C and then for 1 h at 50 °C. The solution is filtered and the solvent distilled off. 200 ml of ethyl acetate are added and the organic phase is washed with a solution of citric acid, sodium hydrogen carbonate and water. The solvent is partly removed until the product starts to crystalize. Then it is filtered. The product is washed with methanol to yield 23 g (79 %) of 3-iodo-9-(p-tolylsulfonyl)carbazole. 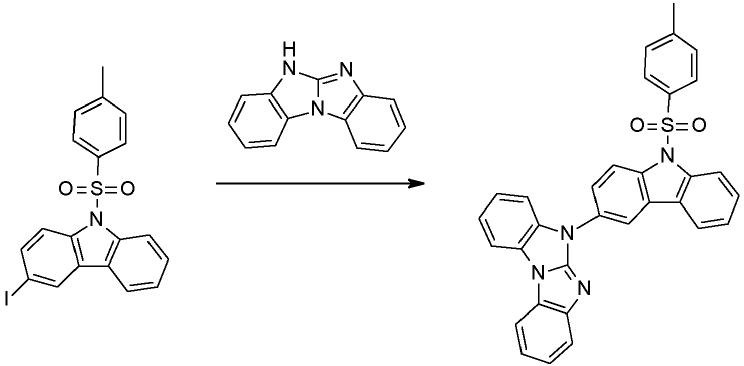
c) 36.0 g (174 mmol) of 6H-benzimidazolo[1,2-a]benzimidazole, 77.8 g (174 mmol) of 3-iodo-9-(p-tolylsulfonyl) carbazole, 106 g (500 mmol) of potassium phosphate, 5.5 g (28.9 mmol) of copper iodide and 111 g (0.972 mol) of trans-cyclohexane-1.2-diamine in 900 ml of dioxane are stirred at 100 °C for 48 h under nitrogen. The product is filtered and the residue washed with dioxane and ethanol. The product is used without purification for the next reaction step. 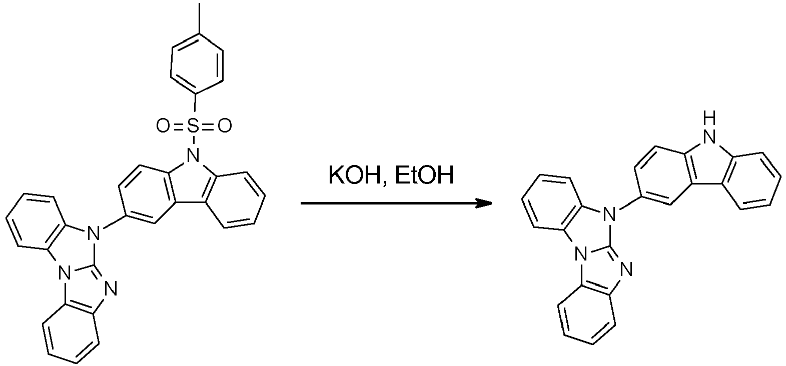
d) A solution of 11.3 g (202 mmol) of potassium hydroxide in 500 ml of ethanol is added to 53 g (101 mmol) of 5-[9-(p-tolylsulfonyl)carbazol-3-yl]benzimidazolo[1,2-a]benzimidazole in 500 ml of hot ethanol within 5 minutes under nitrogen. After 5 h the product is filtered off and washed with ethanol, water and methanol to yield 32g (85.4 %) of 5-(9H-carbazol-3-yl)benzimidazolo[1,2-a]benzimidazole.
   ¹H NMR (400 MHz, DMSO-d6): δ 11.57 (s, 1H), 8.56 (s, 1H), 8.21-8.27 (m, 3H), 7.73-7-82 (m, 2H), 7.57-7.62 (m, 2H), 7.38-7.54 (m, 4H), 7.27-7.34 (m, 2H), 7.20-7.24 (m, 1H).

### Example 1

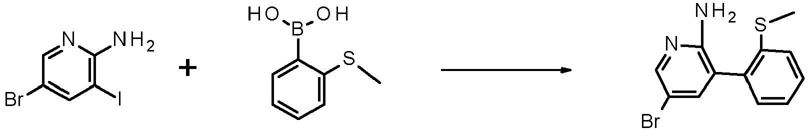

a) 4.95 g (16.60 mmol) of 5-bromo-3-iodo-pyridin-2-amine, 2.86 g (17.04 mmol) of (2-methylsulfanylphenyl)boronic acid, 6.86 g (50 mmol) of potassium carbonate, 45 ml of toluene, 25 ml of ethanol and 6 ml of water are mixed and evacuated and flushed with argon four times. Then 574 mg (0.03 mmol) of tetrakis triphenyl phosphine palladium are added. The mixture is evacuated and flushed with argon four times and heated to reflux over night while stirring, then cooled to room temperature. The phases are separated and the aqueous phase extracted twice with toluene (50 ml each). The combined organic phases are washed three times with water (30 ml each), dried with magnesium sulfate, filtered and the solvent is evaporated on the rotavap. The crude product (5.1 g) is purified by flash chromatography using heptane/ethyl acetate as eluent yielding 3.6 g (74%) of 5-bromo-3-(2-methylsulfanylphenyl)pyridin-2-amine. 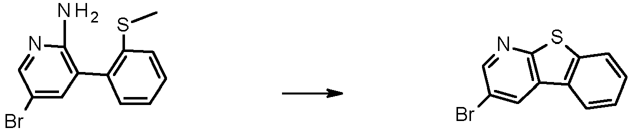
b) A solution of 3.6 g (12.16 mmol) of 5-bromo-3-(2-methylsulfanylphenyl)pyridin-2-amine in 50 ml of THF and 125 ml of glacial acetic acid is cooled to 0°C and 2.79 g (24.32 mmol) of tert-butyl nitrite are added trop by trop. The reaction mixture is stirred over night at 0°C, warmed to room temperature, poured on 300 ml of ice water and stirred for one hour. The yellow suspension is filtered, washed with ice water and dried (70°C / 150 mbar) to yield 2.1 g (65.6 %) of pure 3-bromobenzothiopheno[2,3-b]pyridine. 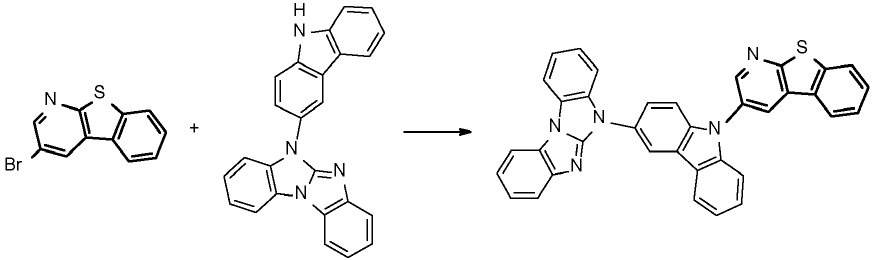
c) 1.86 g (7.0 mmol) of 3-bromobenzothiopheno[2,3-b]pyridine, 2.62 g (7.0 mmol) of 5-(9H-carbazol-3-yl)benzimidazolo[1,2-a]benzimidazole, 3.0 g (14.3 mmol) of potassium carbonate, 9.7 g (85 mmol) of trans-cyclohexane-1,2-diamine, 41 mg (0.21 mmol) of copper iodide and 100 ml of 1,4-dioxane are mixed together under argon at room temperature. The suspension is heated to a reaction temperature of 105°C and stirred at this temperature for 20 hours. 15 g (131 mmol) of trans-cyclohexane-1,2-diamine are added and stirring at 105°C is continued for 8 hours, then 9.6 g (84 mmol) of trans-cyclohexane-1,2-diamine are added again and stirring and heating are continued for another 17 hours. As the starting material is not totally consumed (HPLC analysis) 9.7 g (85 mmol) of trans-cyclohexane-1,2-diamine are added again. After a reaction time of totally 50 hours 100 ml of 1,4-dioxane are added and stirring and heating were continued for another 17 hours. According to HPLC analysis, the reaction is finished, the mixture is filtered hot and cooled to room temperature. The solvent is evaporated and the residue stirred in 200 ml of dichloromethane. A 5% solution of hydrochloric acid is added to get a pH value of 1. The phases are separated, the organic phase is extracted twice with a HCl/H₂O solution and twice with a 1% solution of NH₃ in H₂O. The organic layer is dried over MgSO₄, filtered and evaporated.
The crude product is purified by flash chromatography using chloroform as eluent to yield 1.8g of 3-[3-(benzimidazolo[1,2-a]benzimidazol-5-yl)carbazol-9-yl]benzothiopheno[2,3-b]pyridine with a HPLC purity of 99.6 %.
¹H NMR (400 MHz, CDCl₃): δ 8.92 (s, 1H), 8.59 (s, 1H), 8.53 (s, 1H), 8.19 (t, J = 10 Hz, 2H), 7.97 (d, J = 10 Hz, 1H), 7.89-7.77 (m, 4H), 7.64-7.49 (m, 5H), 7.45-7.28 (m, 6H)

### Example 2

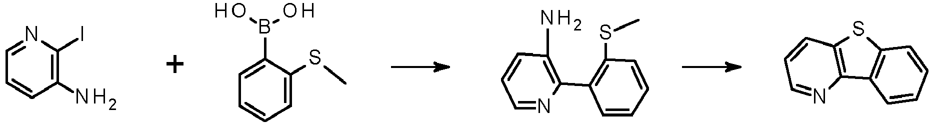

a) Benzothiopheno[3,2-b]pyridine is synthesized according to WO2009086028, compound 50, step 1 and 2. The structure is confirmed by ¹H-NMR. 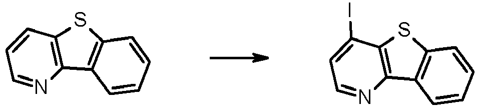
b) 2.914 g (28.8 mmol) of diisopropylamine are dissolved in 25 ml of THF under nitrogen at room temperature and cooled to -78°C. 10.8 ml (27 mmol) of butyllithium (2.5 M) are added drop by drop within 10 minutes at -78°C. The reaction mixture is warmed to room temperature (within 20 minutes). 3.33 g (18 mmol) of benzothiopheno[3,2-b]pyridine is dissolved in 325 ml of THF under nitrogen at room temperature and heated in a water bath to get the starting material into solution. The clear LDA solution is cooled to -78°C. The LDA solution prepared as described above is now added drop by drop within 15 minutes and the reaction mixture is stirred at -78°C for 20 minutes. 9.132 g (32.4 mmol) of diiodoethan are added, the reaction mixture is stirred for 5 minutes at -78°C and then warmed to room temperature. After stirring 90 minutes at room temperature, the solution is poured on 200 ml of a 5% solution of Na₂SO₃, the phases are separated and the water phase is extracted with 200 ml of ethylacetate. The organic phases are washed twice with 200 ml of brine each, dried over Na₂SO₃, filtered and evaporated at the rotavap to get 5.75 g of brown crystals as crude product.The crude product is purified by flash chromatography using toluene as eluent yielding 2.99 g (53%) of 4-iodobenzothiopheno[3,2-b]pyridine.
   ¹H NMR (400 MHz, CDCl₃): δ 8.49 (d, J = 7.2 Hz, 1H), 8.37 (d, J = 4.8 Hz, 1H), 7.88 (d, J = 7.2 Hz, 1H), 7.77 (d, J = 4.8 Hz, 1H), 7.62-7.55 (m, 2H) 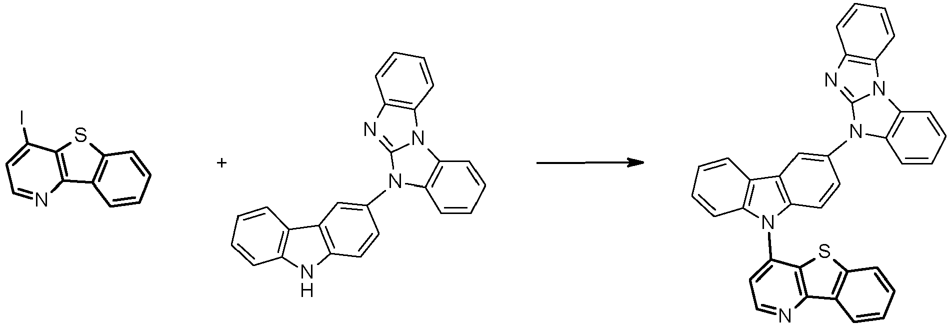
c) 2.8 g (9.0 mmol) of 4-iodobenzothiopheno[3,2-b]pyridine, 3.4 g (9.0 mmol) of 5-(9H-carbazol-3-yl)benzimidazolo[1,2-a]benzimidazole, 5.7 g (26.8 mmol) of potassium carbonate, 5.1 g (44.7 mmol) of trans-cyclohexane-1,2-diamine, 342.8 mg (1.8 mmol) of copper iodide and 58 ml of 1,4-dioxane are mixed together under argon at room temperature. The suspension is heated to 100°C and stirred at this temperature for 17 hours, then cooled to room temperature. To the brown suspension are added 500 ml of dichloromethane and 400 ml of water, the suspension is warmed slightly, stirred for 5 minutes and filtered through a sintered glass funnel into the separation funnel. The residue is washed with dichloromethane, the phases are separated and the organic phase is evaporated at the rotavap to get 4.42g of crude product. The crude product is purified twice by flash chromatography using toluene / ethylacetate as eluent yielding 1.33 g of 4-[3-(benzimidazolo[1,2-a]benzimidazol-5-yl)carbazol-9-yl]benzothiopheno[3,2-b]pyridine (99.9 % pure according to HPLC analysis; compound **G-9**).
   ¹H NMR (400 MHz, CDCl₃): δ 9.01 (d, J = 5.2 Hz, 1H), 8.65-8.62 (m, 1H), 8.55 (s, 1H), 8.22 (d, J = 7.6 Hz, 1H), 7.87-7.78 (m, 5H), 7.65-7.60 (m, 3H), 7.54-7.46 (m, 3H), 7.41-7.29 (m, 6H)

### Application Example 1

The ITO substrate used as the anode is first cleaned with an acetone/isopropanol mixture in an ultrasound bath. To eliminate any possible organic residues, the substrate is exposed to a continuous ozone flow in an ozone oven for further 25 minutes. This treatment also improves the hole injection properties of the ITO. Then Plexcore® OC AJ20-1000 (commercially available from Plextronics Inc.) is spin-coated and dried to form a hole injection layer (-40 nm).

As a hole transport and exciton blocker, 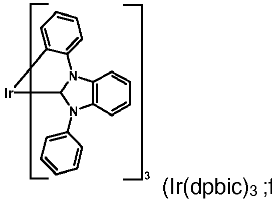 for preparation, see Ir complex (7) in the application WO2005/019373), is applied to the substrate with a thickness of 20 nm, wherein the first 10 nm are doped with MoOₓ (∼10%) to improve the conductivity.

Subsequently, a mixture of 10% by weight of emitter compound, 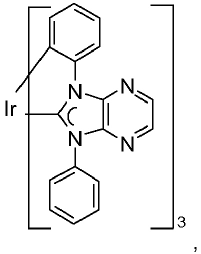 85% by weight of compound 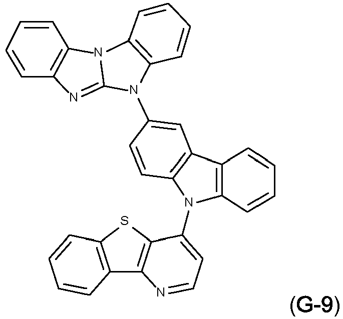 and 5% of Ir(dpbic)₃ is applied by vapor deposition in a thickness of 40 nm.
Material **(G-9)** is deposited then with 5 nm thickness as the blocker. Thereafter, a 20 nm thick electron transport layer is deposited consisting of 50% by weight of 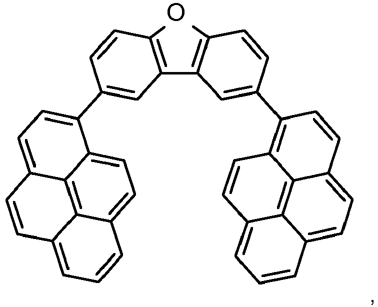 and of 50% of LiQ 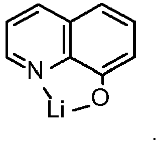 Finally a 2 nm KF layer serves as an electron injection layer and a 100 nm-thick Al electrode completes the device.
All fabricated parts are sealed with a glass lid and a getter in an inert nitrogen atmosphere. To characterize the OLED, electroluminescence spectra are recorded at various currents and voltages. In addition, the current-voltage characteristic is measured in combination with the light output emitted. The light output can be converted to photometric parameters by calibration with a photometer. To determine the lifetime, the OLED is operated at a constant current density and the decrease in the light output is recorded. The lifetime is defined as that time which lapses until the luminance decreases to 70 % of the initial luminance. The results are shown in Table 1. V at 300 cd/m² (nits) and EQE (%) at 300 cd/m² of Application Example 1 are set to 100 and the data of Application Example 2 and Comparative Appplication Example 1 are specified in relation to those of Application Example 1.

### Application Example 2

The device structure is the same as in Application Example 1 except that compound **(G-9)** in the emissive layer and the hole blocker layer is replaced by compound

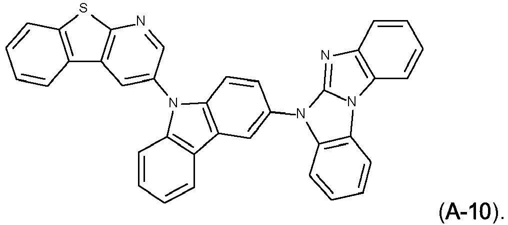

### Comparative Application Example 1

The device structure is the same as in Application Example 1 except that compound (G-9) in the emissive layer and the hole blocker layer is replaced by compound

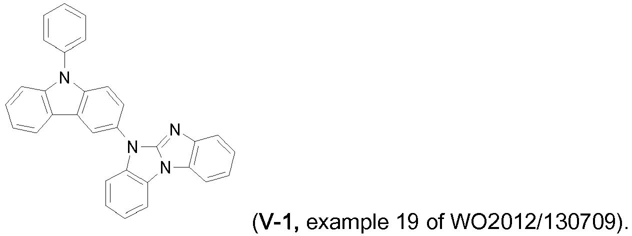

**Table 1**

| | **Voltage @ 300nits [V]** | **EQE¹⁾ @ 300nits [%]** |
|---|---|---|
| **Appl. Ex. 1** | 4.05 | 11.39 |
| **Appl. Ex. 2** | 4.58 | 8.29 |
| **Comp. Appl. Ex. 1** | 7.54 | 1.59 |

| | | |
|---|---|---|
| ¹⁾ External quantum efficiency (EQE) is # of generated photons escaped from a substance or a device / # of electrons flowing through it. | | |

It is evident that the voltage is significantly reduced and the EQE increased in case of Application Examples 1 and 2, where the inventive compounds **(G-9)** and **(A-10)** are used as host and hole blocker material, in comparison to Comparative Application Example 1, where compound **(V-1)** is used as host and hole blocker material.

## Claims

1. A compound of the formula 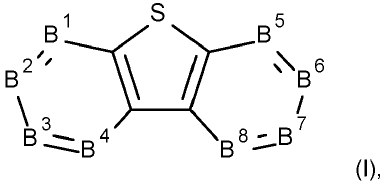 wherein
B¹ is N, or CR⁸¹,
B² is N, or CR⁸²,
B³ is N, or CR⁸³,
B⁴ is N, or CR⁸⁴,
B⁵ is N, or CR⁸⁵,
B⁶ is N, or CR⁸⁶,
B⁷ is N, or CR⁸⁷,
B⁸ is N, or CR⁸⁸,
R⁸¹, R⁸², R⁸³, R⁸⁴, R⁸⁵, R⁸⁶, R⁸⁷ and R⁸⁸ are independently of each other H, F, a cyano group, a C₁-C₂₅alkyl group, which can optionally be substituted by E and or interupted by D; a C₁-C₂₅alkoxy group; a C₃-C₁₈cycloalkyl group, which can optionally be substituted by E; a C₆-C₂₄aryl group, which can optionally be substituted by G, a C₂-C₃₀heteroaryl group, which can optionally be substituted by G; or a group of formula --(A¹)ₒ-(A²)ₚ-(A³)_{q}-(A⁴)ᵣ-R¹⁶,
o is 0, or 1, p is 0, or 1, q is 0, or 1, r is 0, or 1,
A¹, A², A³ and A⁴ are independently of each other a C₆-C₂₄arylen group, which can optionally be substituted by G, or a C₂-C₃₀heteroarylen group, which can optionally be substituted by G;
R¹⁶ is -NR¹⁰R¹¹, or -Si(R¹²)(R¹³)(R¹⁴), a C₆-C₂₄aryl group, which can optionally be substituted by G; or a C₂-C₃₀heteroaryl group, which can optionally be substituted by G;
R¹⁰ and R¹¹ are independently of each other a C₆-C₂₄aryl group, which can optionally be substituted by G; or a C₂-C₃₀heteroaryl group, which can optionally be substituted by G;
R¹², R¹³ and R¹⁴ are independently of each other a C₁-C₂₅alkyl group, which can optionally be substituted by E and or interupted by D; C₆-C₂₄aryl group, which can optionally be substituted by G; or a C₂-C₃₀heteroaryl group, which can optionally be substituted by G;
D is -CO-, -COO-, -S-, -SO-, -SO₂-, -O-, -NR⁶⁵-, -SiR⁷⁰R⁷¹-, -POR⁷²-, -CR⁶³=CR⁶⁴-, or -C≡C-,
E is -OR69, -SR69, -NR⁶⁵R⁶⁶, -COR68, -COOR⁶⁷, -CONR⁶⁵R⁶⁶, -CN, or F,
G is E, or a C₁-C₁₈alkyl group, a C₃-C₁₈cycloalkyl group, a C₆-C₂₄aryl group, a C₆-C₂₄aryl group, which is substituted by F, C₁-C₁₈alkyl, or C₁-C₁₈alkyl which is interrupted by O; a C₂-C₃₀heteroaryl group, or a C₂-C₃₀heteroaryl group, which is substituted by F, C₁-C₁₈alkyl, or C₁-C₁₈alkyl which is interrupted by O;
R⁶³ and R⁶⁴ are independently of each other H, C₆-C₁₈aryl; C₆-C₁₈aryl which is substituted by C₁-C₁₈alkyl, or C₁-C₁₈alkoxy; C₁-C₁₈alkyl; or C₁-C₁₈alkyl which is interrupted by -O-;
R⁶⁵ and R⁶⁶ are independently of each other a C₆-C₁₈aryl group; a C₆-C₁₈aryl which is substituted by C₁-C₁₈alkyl, or C₁-C₁₈alkoxy; a C₁-C₁₈alkyl group; or a C₁-C₁₈alkyl group, which is interrupted by-O-; or
R⁶⁵ and R⁶⁶ together form a five or six membered ring,
R⁶⁷ is a C₆-C₁₈aryl group; a C₆-C₁₈aryl group, which is substituted by C₁-C₁₈alkyl, or C₁-C₁₈alkoxy; a C₁-C₁₈alkyl group; or a C₁-C₁₈alkyl group, which is interrupted by -O-, R⁶⁸ is H; a C₆-C₁₈aryl group; a C₆-C₁₈aryl group, which is substituted by C₁-C₁₈alkyl, or C₁-C₁₈alkoxy; a C₁-C₁₈alkyl group; or a C₁-C₁₈alkyl group, which is interrupted by - O-,
R⁶⁹ is a C₆-C₁₈aryl; a C₆-C₁₈aryl, which is substituted by C₁-C₁₈alkyl, or C₁-C₁₈alkoxy; a C₁-C₁₈alkyl group; or a C₁-C₁₈alkyl group, which is interrupted by-O-,
R⁷⁰ and R⁷¹ are independently of each other a C₁-C₁₈alkyl group, a C₆-C₁₈aryl group, or a C₆-C₁₈aryl group, which is substituted by C₁-C₁₈alkyl, and
R⁷² is a C₁-C₁₈alkyl group, a C₆-C₁₈aryl group, or a C₆-C₁₈aryl group, which is substituted by C₁-C₁₈alkyl, with the proviso that
at least one of the substituents B¹, B², B³, B⁴, B⁵, B⁶, B⁷ and B⁸ represents N;
not more than two of the groups B¹, B², B³ and B⁴ represent N; and
not more than two of the groups B⁵, B⁶, B⁷ and B⁸ represent N; and
with the further proviso that at least one of the substituents R⁸¹, R⁸², R⁸³, R⁸⁴, R⁸⁵, R⁸⁶, R⁸⁷ and R⁸⁸ represent a group of formula -(A¹)ₒ-(A²)ₚ-(A³)_{q}-(A⁴)ᵣ-R¹⁶, wherein R¹⁶ represents a benzimidazo[1,2-a]benzimidazo-5-yl group, which can optionally be substituted by G; and/or at least one of the groups A¹, A², A³ and A⁴ respresents a benzimidazo[1,2-a]benzimidazo-2,5-ylene group, which can optionally be substituted by G.

2. The compound according to claim 1, which is a compound of formula 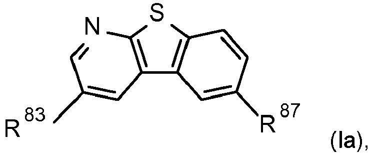 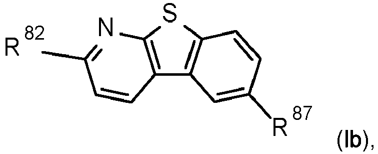 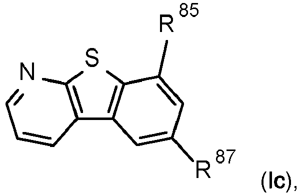 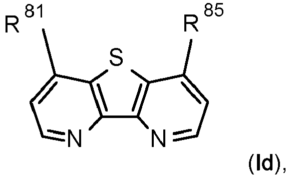 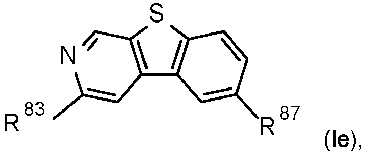 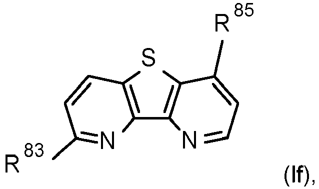 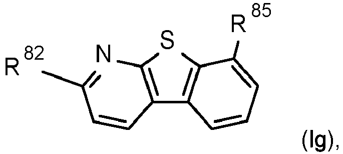 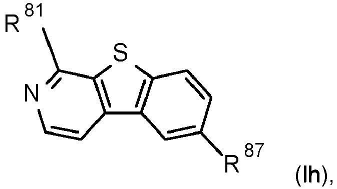 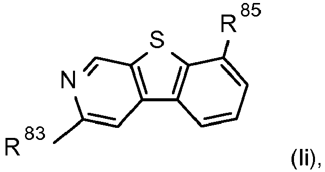 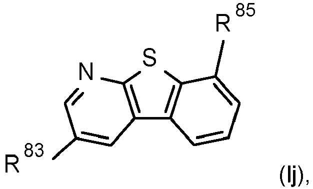 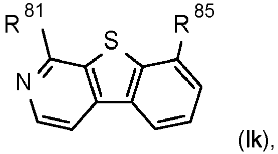 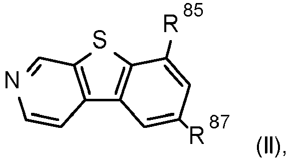 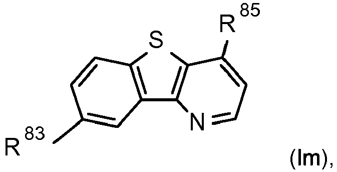 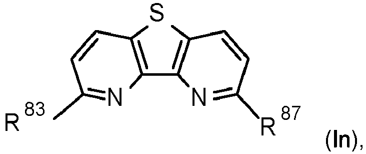 or 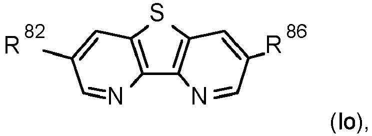 wherein R⁸¹, R⁸², R⁸³, R⁸⁵, R⁸⁶ and R⁸⁷ are as defined in claim 1.

3. The compound according to claim 2, wherein
in the compounds of formula **(Ia)**
R⁸³ is a group of the formula -(A¹)ₒ-(A²)ₚ-(A³)_{q}-(A⁴)ᵣ-R¹⁶; and
R⁸⁷ is H, a C₁-C₂₅alkyl group, or a group of the formula -(A¹)ₒ-(A²)ₚ-(A³)_{q}-(A⁴)ᵣ-R^{16'}; or
R⁸³ is H, a C₁-C₂₅alkyl group, or a group of the formula -(A¹)ₒ-(A²)ₚ-(A³)_{q}-(A⁴)ᵣ-R^{16'};
and
R⁸⁷ is a group of the formula -(A¹)ₒ-(A²)ₚ-(A³)_{q}-(A⁴)ᵣ-R¹⁶;
in the compounds of formula (**Ib**)
R⁸² is a group of the formula -(A¹)ₒ-(A²)ₚ-(A³)_{q}-(A⁴)ᵣ-R¹⁶; and
R⁸⁷ is H, a C₁-C₂₅alkyl group, or a group of the formula -(A¹)ₒ-(A²)ₚ-(A³)_{q}-(A⁴)ᵣ-R^{16'}; or
R⁸² is H, a C₁-C₂₅alkyl group, or a group of the formula -(A¹)ₒ-(A²)ₚ-(A³)_{q}-(A⁴)ᵣ-R^{16'};
and
R⁸⁷ is a group of the formula -(A¹)ₒ-(A²)ₚ-(A³)_{q}-(A⁴)ᵣ-R¹⁶;
in the compounds of formula **(Ic)**
R⁸⁵ is a group of the formula -(A¹)ₒ-(A²)ₚ-(A³)_{q}-(A⁴)ᵣ-R¹⁶; and
R⁸⁷ is H, a C₁-C₂₅alkyl group, or a group of the formula -(A¹)ₒ-(A²)ₚ-(A³)_{q}-(A⁴)ᵣ-R^{16'}; or
R⁸⁵ is H, a C₁-C₂₅alkyl group, or a group of the formula -(A¹)ₒ-(A²)ₚ-(A³)_{q}-(A⁴)ᵣ-R^{16'};
and
R⁸⁷ is a group of the formula -(A¹)ₒ-(A²)ₚ-(A³)_{q}-(A⁴)ᵣ-R¹⁶;
in the compounds of formula **(Id)**
R⁸¹ is a group of the formula -(A¹)ₒ-(A²)ₚ-(A³)_{q}-(A⁴)ᵣ-R¹⁶; and
R⁸⁵ is H, a C₁-C₂₅alkyl group, or a group of the formula -(A¹)ₒ-(A²)ₚ-(A³)_{q}-(A⁴)ᵣ-R^{16'}; or
R⁸¹ is H, a C₁-C₂₅alkyl group, or a group of the formula -(A¹)ₒ-(A²)ₚ-(A³)_{q}-(A⁴)ᵣ-R^{16'};
and
R⁸⁵ is a group of the formula -(A¹)ₒ-(A²)ₚ-(A³)_{q}-(A⁴)ᵣ-R¹⁶;
in the compounds of formula (**Ie**)
R⁸³ is a group of the formula -(A¹)ₒ-(A²)ₚ-(A³)_{q}-(A⁴)ᵣ-R¹⁶; and
R⁸⁷ is H, a C₁-C₂₅alkyl group, or a group of the formula -(A¹)ₒ-(A²)ₚ-(A³)_{q}-(A⁴)ᵣ-R^{16'}; or
R⁸³ is H, a C₁-C₂₅alkyl group, or a group of the formula -(A¹)ₒ-(A²)ₚ-(A³)_{q}-(A⁴)ᵣ-R^{16'};
and
R⁸⁷ is a group of the formula -(A¹)ₒ-(A²)ₚ-(A³)_{q}-(A⁴)ᵣ-R¹⁶;
in the compounds of formula (**If**)
R⁸³ is a group of the formula -(A¹)ₒ-(A²)ₚ-(A³)_{q}-(A⁴)ᵣ-R¹⁶; and
R⁸⁵ is H, a C₁-C₂₅alkyl group, or a group of the formula -(A¹)ₒ-(A²)ₚ-(A³)_{q}-(A⁴)ᵣ-R^{16'}; or
R⁸³ is H, a C₁-C₂₅alkyl group, or a group of the formula -(A¹)ₒ-(A²)ₚ-(A³)_{q}-(A⁴)ᵣ-R^{16'};
and
R⁸⁵ is a group of the formula -(A¹)ₒ-(A²)ₚ-(A³)_{q}-(A⁴)ᵣ-R¹⁶;
in the compounds of formula (**Ig**)
R⁸² is a group of the formula -(A¹)ₒ-(A²)ₚ-(A³)_{q}-(A⁴)ᵣ-R¹⁶; and
R⁸⁵ is H, a C₁-C₂₅alkyl group, or a group of the formula -(A¹)ₒ-(A²)ₚ-(A³)_{q}-(A⁴)ᵣ-R^{16'}; or
R⁸² is H, a C₁-C₂₅alkyl group, or a group of the formula -(A¹)ₒ-(A²)ₚ-(A³)_{q}-(A⁴)ᵣ-R^{16'};
and
R⁸⁵ is a group of the formula -(A¹)ₒ-(A²)ₚ-(A³)_{q}-(A⁴)ᵣ-R¹⁶;
in the compounds of formula (**Ih**)
R⁸¹ is a group of the formula -(A¹)ₒ-(A²)ₚ-(A³)_{q}-(A⁴)ᵣ-R¹⁶; and
R⁸⁷ is H, a C₁-C₂₅alkyl group, or a group of the formula -(A¹)ₒ-(A²)ₚ-(A³)_{q}-(A⁴)ᵣ-R^{16'}; or
R⁸¹ is H, a C₁-C₂₅alkyl group, or a group of the formula -(A¹)ₒ-(A²)ₚ-(A³)_{q}-(A⁴)ᵣ-R^{16'};
and
R⁸⁷ is a group of the formula -(A¹)ₒ-(A²)ₚ-(A³)_{q}-(A⁴)ᵣ-R¹⁶;
in the compounds of formula (**Ii**)
R⁸³ is a group of the formula -(A¹)ₒ-(A²)ₚ-(A³)_{q}-(A⁴)ᵣ-R¹⁶; and
R⁸⁵ is H, a C₁-C₂₅alkyl group, or a group of the formula -(A¹)ₒ-(A²)ₚ-(A³)_{q}-(A⁴)ᵣ-R^{16'}; or
R⁸³ is H, a C₁-C₂₅alkyl group, or a group of the formula -(A¹)ₒ-(A²)ₚ-(A³)_{q}-(A⁴)ᵣ-R^{16'};
and
R⁸⁵ is a group of the formula -(A¹)ₒ-(A²)ₚ-(A³)_{q}-(A⁴)ᵣ-R¹⁶;
in the compounds of formula (**Ij**)
R⁸³ is a group of the formula -(A¹)ₒ-(A²)ₚ-(A³)_{q}-(A⁴)ᵣ-R¹⁶; and
R⁸⁵ is H, a C₁-C₂₅alkyl group, or a group of the formula -(A¹)ₒ-(A²)ₚ-(A³)_{q}-(A⁴)ᵣ-R^{16'}; or
R⁸³ is H, a C₁-C₂₅alkyl group, or a group of the formula -(A¹)ₒ-(A²)ₚ-(A³)_{q}-(A⁴)ᵣ-R^{16'};
and
R⁸⁵ is a group of the formula -(A¹)ₒ-(A²)ₚ-(A³)_{q}-(A⁴)ᵣ-R¹⁶;
in the compounds of formula (**Ik**)
R⁸¹ is a group of the formula -(A¹)ₒ-(A²)ₚ-(A³)_{q}-(A⁴)ᵣ-R¹⁶; and
R⁸⁵ is H, a C₁-C₂₅alkyl group, or a group of the formula -(A¹)ₒ-(A²)ₚ-(A³)_{q}-(A⁴)ᵣ-R^{16'}; or
R⁸¹ is H, a C₁-C₂₅alkyl group, or a group of the formula -(A¹)ₒ-(A²)ₚ-(A³)_{q}-(A⁴)ᵣ-R^{16'};
and
R⁸⁵ is a group of the formula -(A¹)ₒ-(A²)ₚ-(A³)_{q}-(A⁴)ᵣ-R¹⁶;
in the compounds of formula **(Il)**
R⁸⁵ is a group of the formula -(A¹)ₒ-(A²)ₚ-(A³)_{q}-(A⁴)ᵣ-R¹⁶; and
R⁸⁷ is H, a C₁-C₂₅alkyl group, or a group of the formula -(A¹)ₒ-(A²)ₚ-(A³)_{q}-(A⁴)ᵣ-R^{16'}; or
R⁸⁵ is H, a C₁-C₂₅alkyl group, or a group of the formula -(A¹)ₒ-(A²)ₚ-(A³)_{q}-(A⁴)ᵣ-R^{16'};
and
R⁸⁷ is a group of the formula -(A¹)ₒ-(A²)ₚ-(A³)_{q}-(A⁴)ᵣ-R¹⁶;
in the compounds of formula (**Im**)
R⁸³ is a group of the formula -(A¹)ₒ-(A²)ₚ-(A³)_{q}-(A⁴)ᵣ-R¹⁶; and
R⁸⁵ is H, a C₁-C₂₅alkyl group, or a group of the formula -(A¹)ₒ-(A²)ₚ-(A³)_{q}-(A⁴)ᵣ-R^{16'}; or
R⁸³ is H, a C₁-C₂₅alkyl group, or a group of the formula -(A¹)ₒ-(A²)ₚ-(A³)_{q}-(A⁴)ᵣ-R^{16'};
and
R⁸⁵ is a group of the formula -(A¹)ₒ-(A²)ₚ-(A³)_{q}-(A⁴)ᵣ-R¹⁶;
in the compounds of formula (**In**)
R⁸³ is a group of the formula -(A¹)ₒ-(A²)ₚ-(A³)_{q}-(A⁴)ᵣ-R¹⁶; and
R⁸⁷ is H, a C₁-C₂₅alkyl group, or a group of the formula -(A¹)ₒ-(A²)ₚ-(A³)_{q}-(A⁴)ᵣ-R^{16'}; or
R⁸³ is H, a C₁-C₂₅alkyl group, or a group of the formula -(A¹)ₒ-(A²)ₚ-(A³)_{q}-(A⁴)ᵣ-R^{16'};
and
R⁸⁷ is a group of the formula -(A¹)ₒ-(A²)ₚ-(A³)_{q}-(A⁴)ᵣ-R¹⁶; and
in the compounds of formula (**Io**)
R⁸² is a group of the formula -(A¹)ₒ-(A²)ₚ-(A³)_{q}-(A⁴)ᵣ-R¹⁶; and
R⁸⁶ is H, a C₁-C₂₅alkyl group, or a group of the formula -(A¹)ₒ-(A²)ₚ-(A³)_{q}-(A⁴)ᵣ-R^{16'}; or
R⁸² is H, a C₁-C₂₅alkyl group, or a group of the formula -(A¹)ₒ-(A²)ₚ-(A³)_{q}-(A⁴)ᵣ-R^{16'};
and
R⁸⁶ is a group of the formula -(A¹)ₒ-(A²)ₚ-(A³)_{q}-(A⁴)ᵣ-R¹⁶; wherein
o is 0, or 1, p is 0, or 1, q is 0, or 1, r is 0, or 1;
A¹, A², A³ and A⁴ are independently of each other a group of the formula 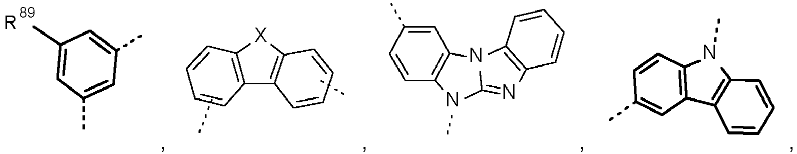 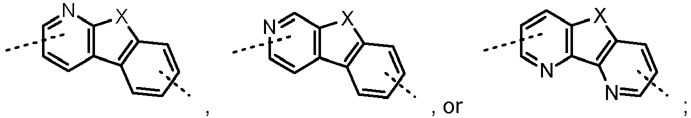 R¹⁶ is a group of the formula 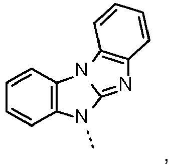 or R¹⁶ has the meaning of R^{16'}, if at least one of the groups A¹, A², A³ and A⁴ respresent a group of formu-la 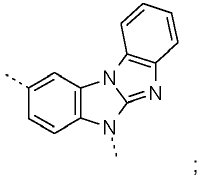 R^{16'} is H, or a group of the formula -Si(R¹²)(R¹³)(R¹⁴), 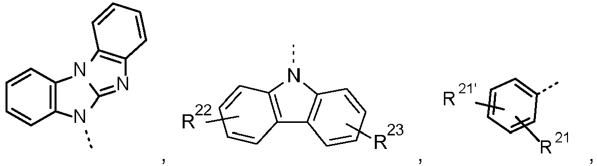 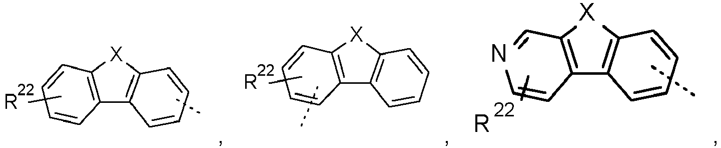 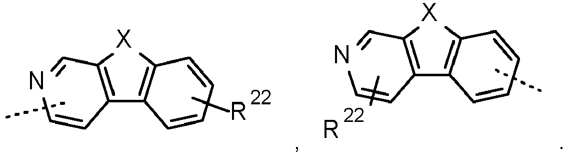 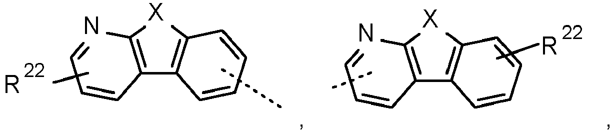 or 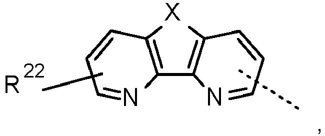 wherein
R¹², R¹³ and R¹⁴ are independently of each other a phenyl group, which can optionally be substituted by one, or more alkyl groups;
R²¹ and R^{21'} are independently of each other H, a phenyl group, or a C₁-C₁₈alkyl group;
R²² and R²³ are independently of each other H, or a group of the formula 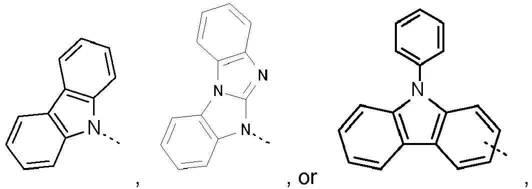
X is O, S, or NR²⁴,
R²⁴ is a C₆-C₂₄aryl group, or a C₂-C₃₀heteroaryl group, which can optionally be substituted by G, wherein G is as defined in claim 1; and
R⁸⁹ is H, a group of formula

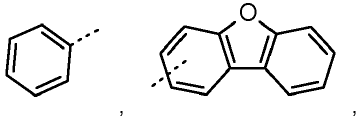

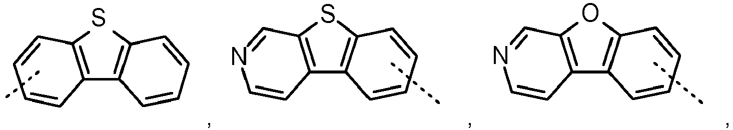

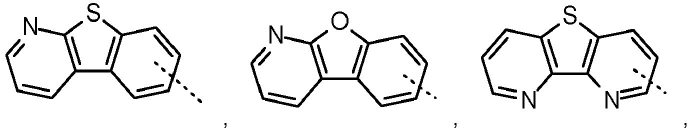

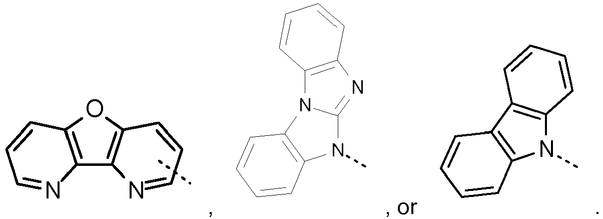

4. The compound according to claims 2, or 3, wherein
o is 0, or 1, p is 0, or 1, q is 0, or 1, r is 0, or 1,
A¹, A², A³ and A⁴ are independently of each other a group of the formula 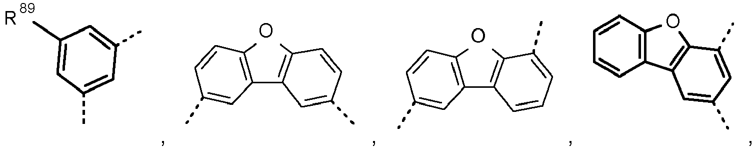 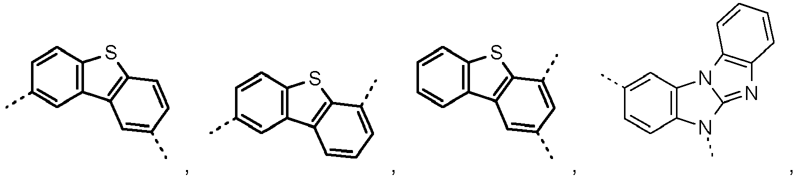 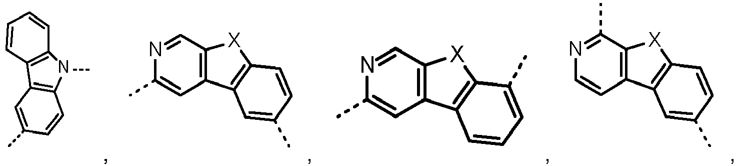 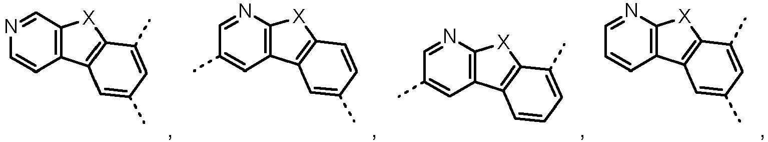 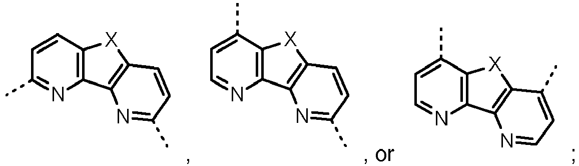 R¹⁶ is a group of the formula 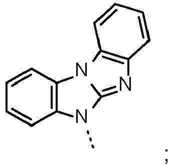 or R¹⁶ has the meaning of R^{16'}, if at least one of the groups A¹, A², A³ and A⁴ respresent a group of formula 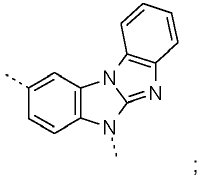 R^{16'} is H, or a group of the formula 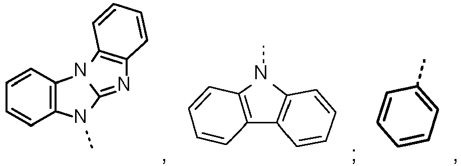 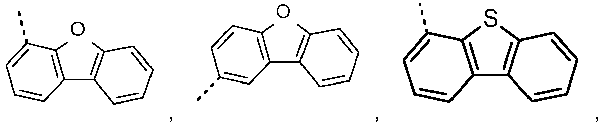 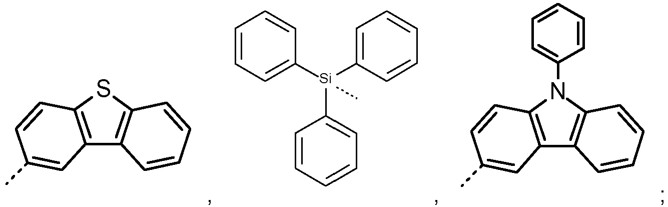 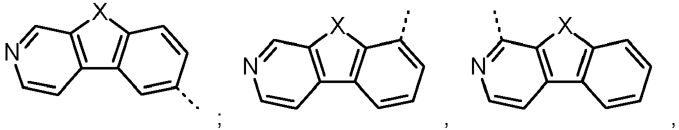 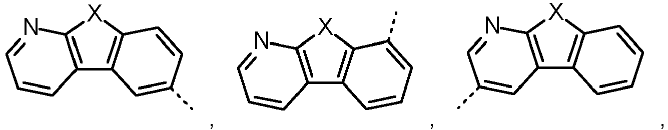 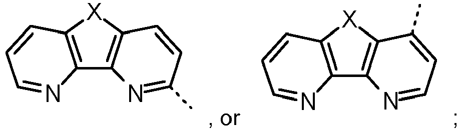 and R⁸⁹ is H, a group of formula 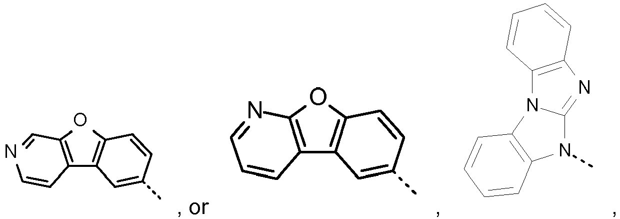 or 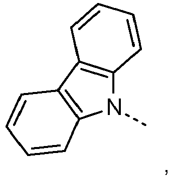 wherein X is O, S, or NR²⁴, wherein
R²⁴ is

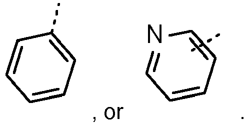

5. The compound according to any of claims 2 to 4, wherein the group of the formula - (A¹)ₒ-(A²)ₚ-(A³)_{q}-(A⁴)ᵣ-R¹⁶ is a group of formula 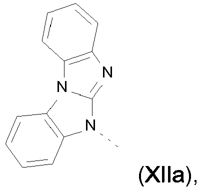 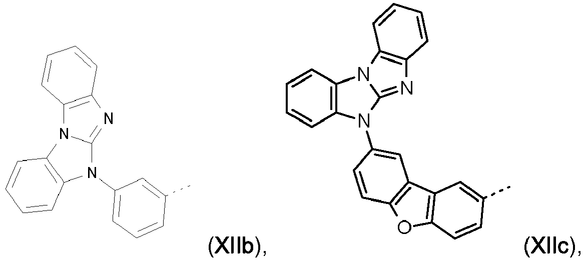 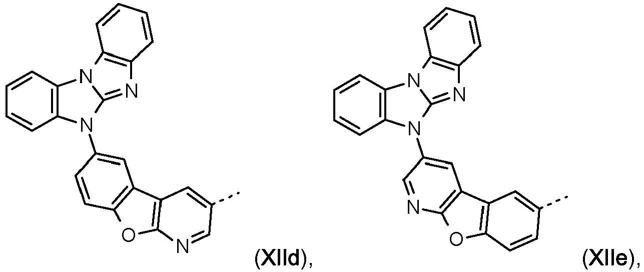 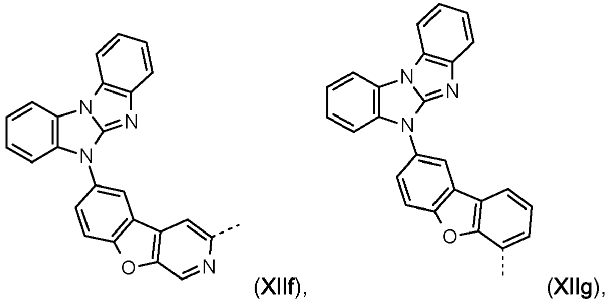 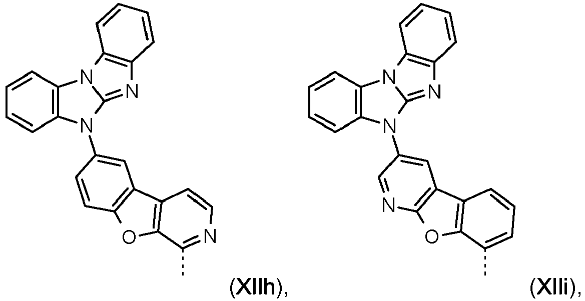 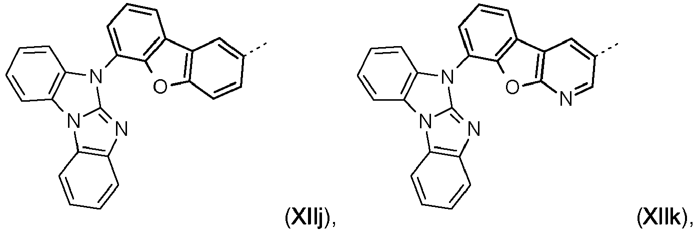 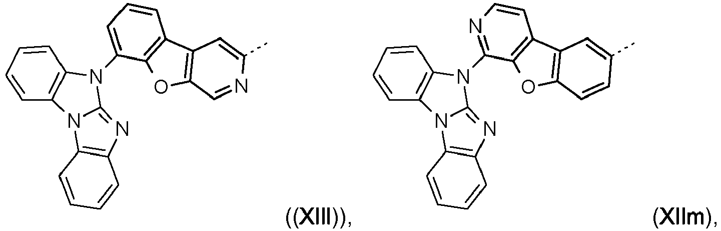 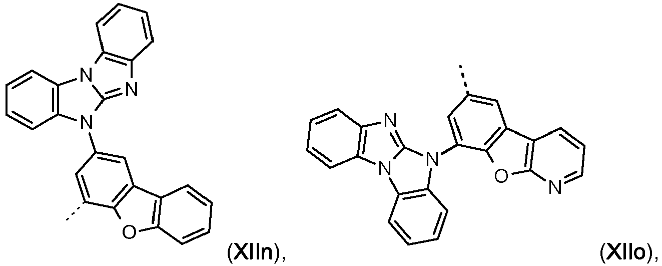 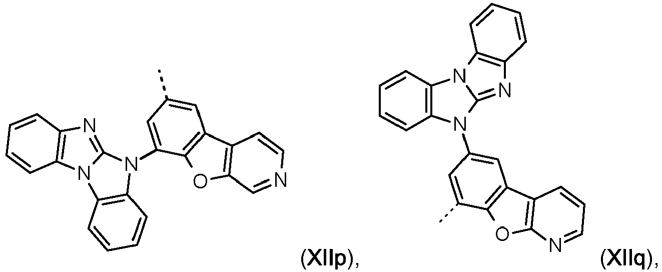 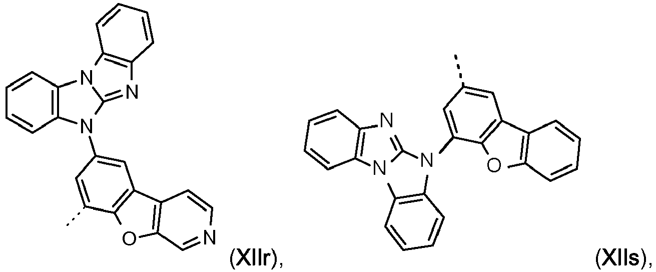 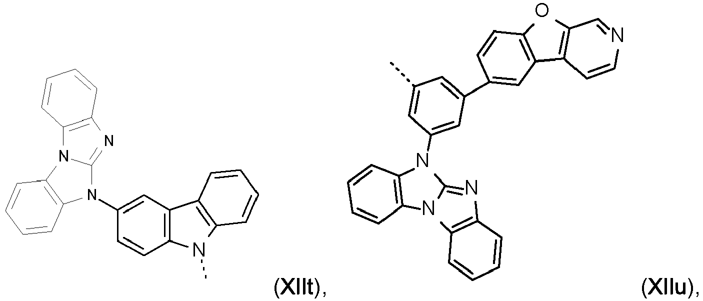 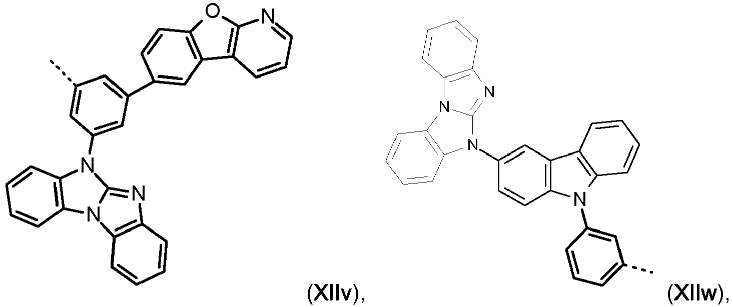 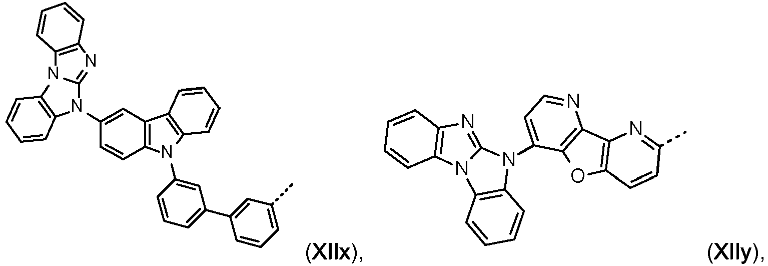 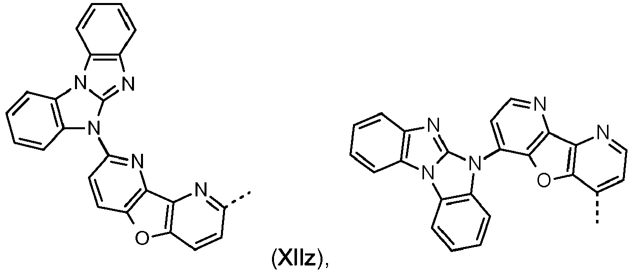 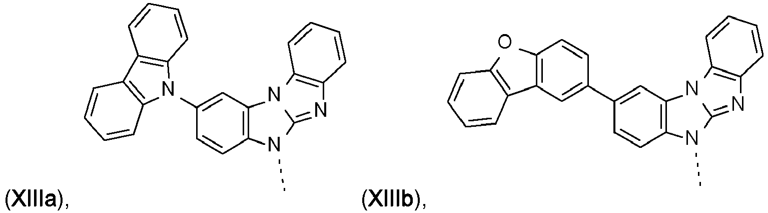 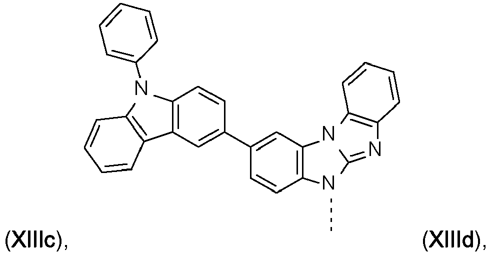 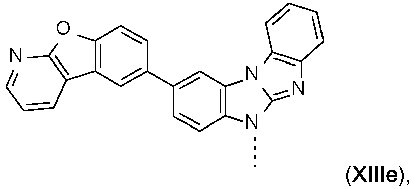 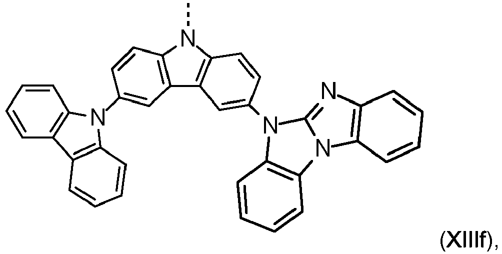 or

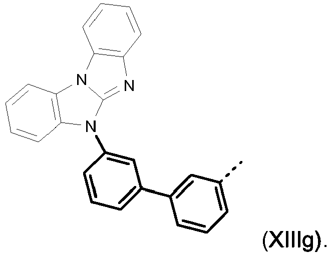

6. The compound according to any of claims 2 to 5, wherein the group of the formula - (A¹)ₒ-(A²)ₚ-(A³)_{q}-(A⁴)ᵣ-R^{16'} is H, a C₁-C₂₅alkyl group, or a group of formula (**XIIa**), (**XIIb**), (**XIIc**), (**XIId**), (**XIIe**), (**XIIf**), (**XIIg**), (**XIIh**), (**XIIi**), (**XIIj**), (**XIIk**), ((**XIII**)), (**XIIm**), (**XI-In**), (**XIIo**), (**XIIp**), (**XIIq**), (**XIIr**), (**XIIs**), (**XIIt**), (**XIIu**), (**XIIv**), (**XIIw**), (**XIIx**), (**XIIy**), (**XIIz**), (**XIIIa**), (**XIIIb**), (**XIIIc**), (**XIIId**), (**XIIIe**), (**XIIIf**), or (**XIIIg**), as defined in claim 5; or a group of formula 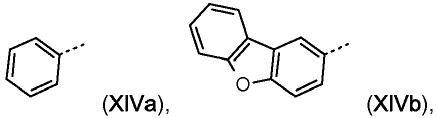 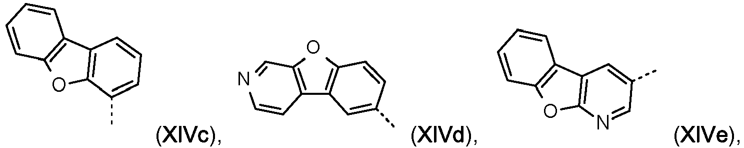 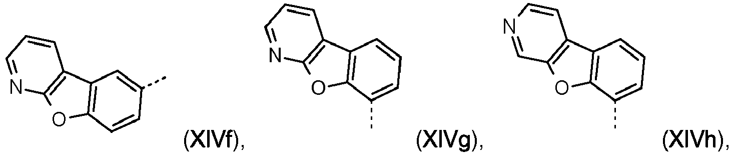 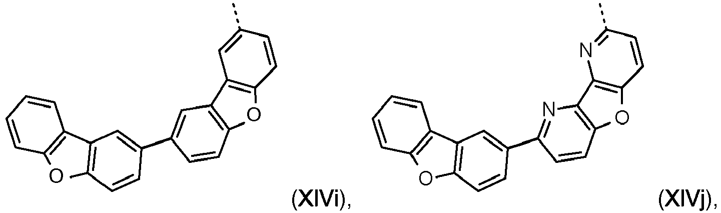 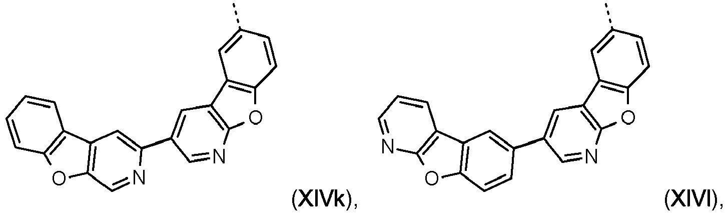 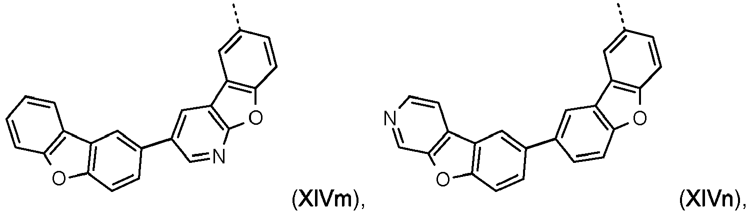 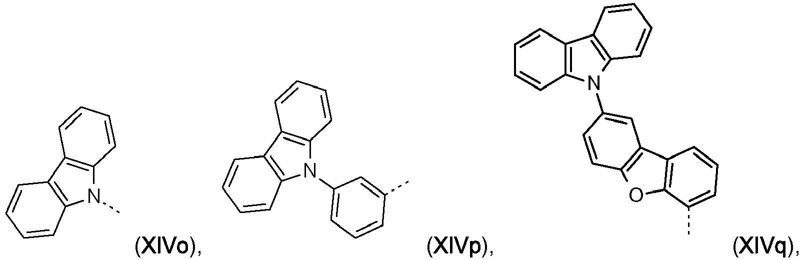 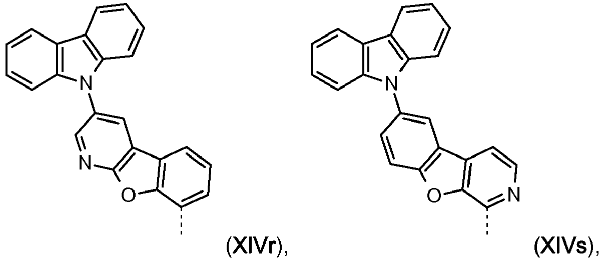 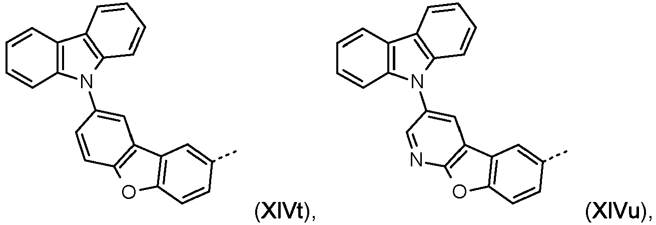 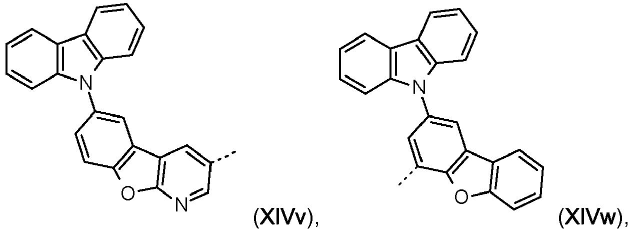 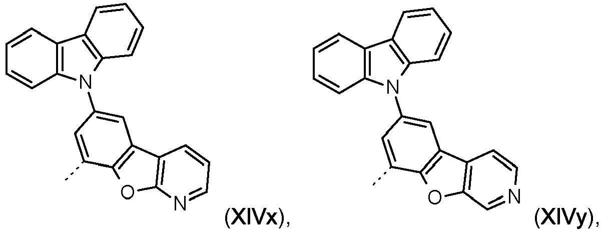 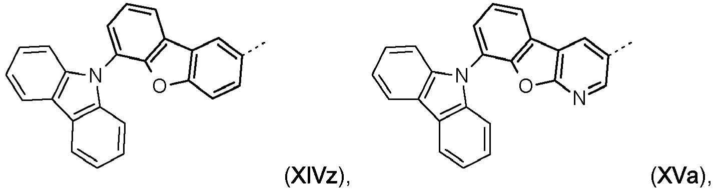 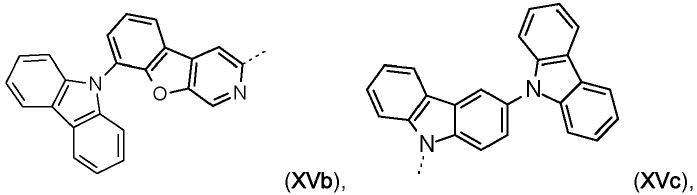 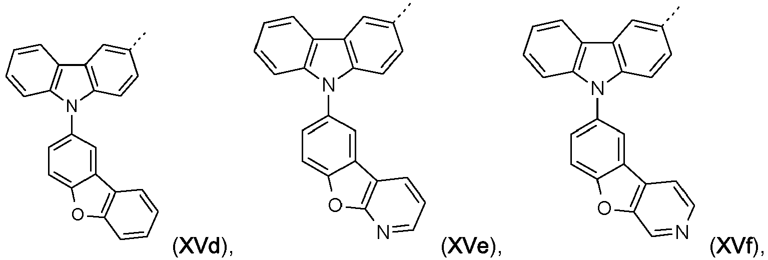 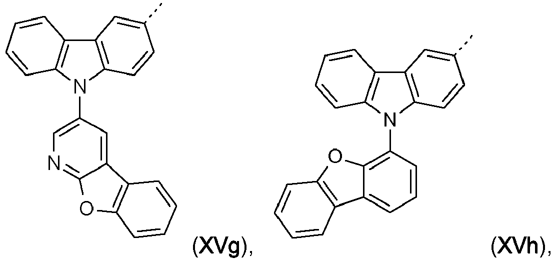 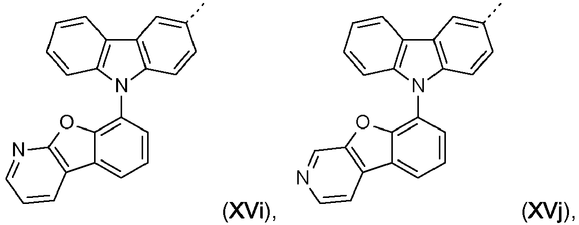 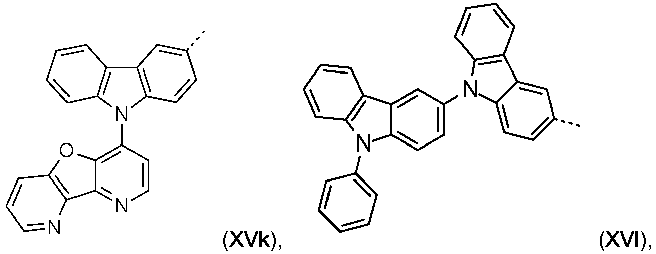 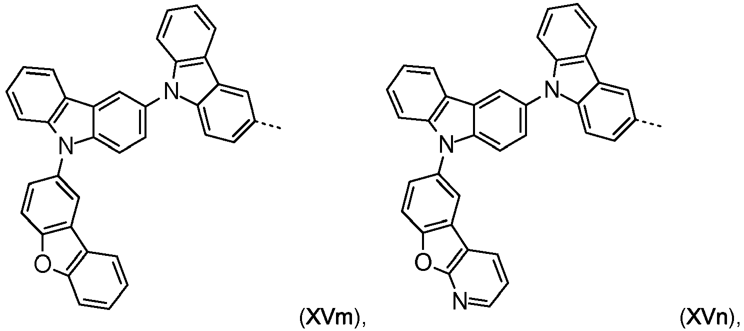 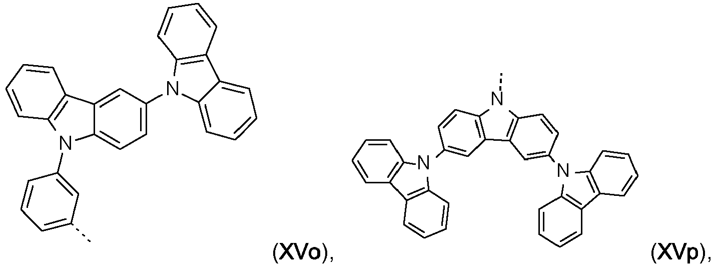 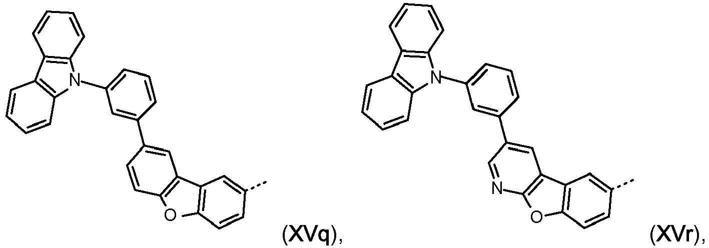 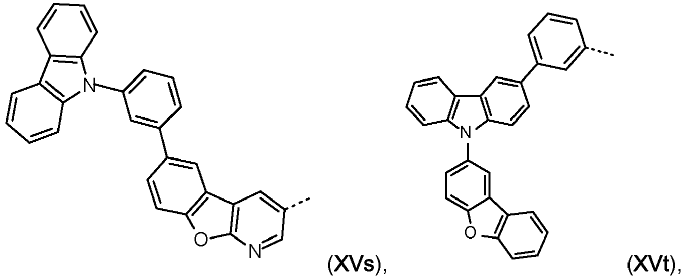 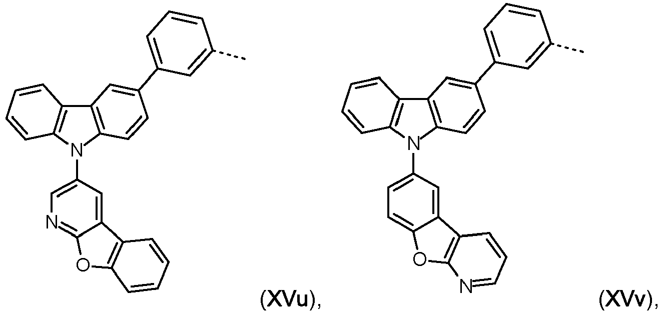 or 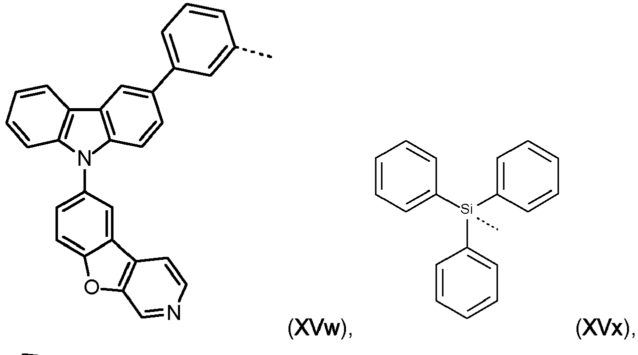 or 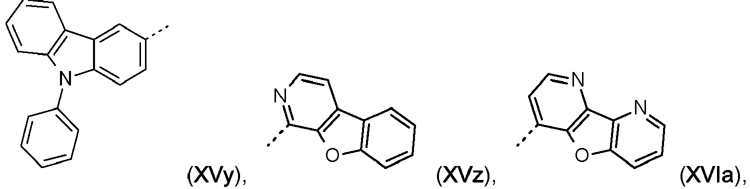 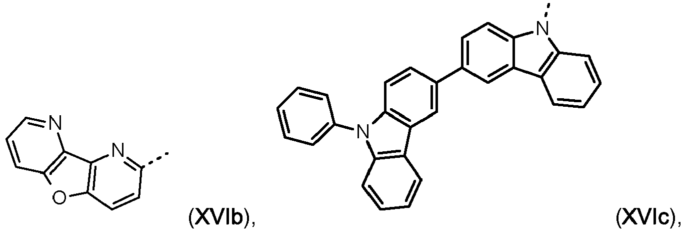 or

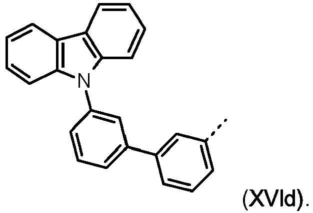

7. The compound according to any of claims 2 to 6, which is
a compound of formula (Ia), wherein R⁸³ is a group of formula (**XIIa**), (**XIIb**), (**XIIc**), (**XIId**), (**XIIe**), (**XIIf**), (**XIIg**), (**XIIh**), (**XIIi**), (**XIIj**), (**XIIk**), ((**XIIl**)), (**XIIm**), (**XIIn**), (**XIIo**), (**XIIp**), (**XIIq**), (**XIIr**), (**XIIs**), (**XIIt**), (**XIIu**), (**XIIv**), (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined in claim 5, and
R⁸⁷ is H, a C₁-C₂₅alkyl group, a group of formula (**XIIa**), (**XIIb**), (**XIIc**), (**XIId**), (**XIIe**), (**XIIf**), (**XIIg**), (**XIIh**), (**XIIi**), (**XIIj**), (**XIIk**), ((**XIIl**)), (**XIIm**), (**XIIn**), (**XIIo**), (**XIIp**), (**XIIq**), (**XIIr**), (**XIIs**), (**XIIt**), (**XIIu**), (**XIIv**) (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined in claim 5, or a group of formula (**XIVb**), (**XIVc**), (**XIVd**), (**XIVe**), (**XIVf**), (**XIVg**), (**XIVh**), (**XIVo**), (**XIVp**), (**XIVq**), (**XIVr**), (**XIVs**), (**XIVt**), (**XIVu**), (**XIVv**), (**XIVw**), (**XIVx**),(**XIVy**), (**XIVz**), (**XVa**), (**XVb**), (**XVc**), (**XVd**), (**XVe**), (**XVf**), (**XVg**), (**XVh**), (**XVi**), (**XVj**), (**XVk**), (**XVl**), (**XVm**), (**XVn**), (**XVo**), (**XVp**), (**XVq**), (**XVr**), (**XVs**), (**XVt**), (**XVu**), (**XVv**), (**XVw**), (**XVx**), (**XVy**), (**XVz**), (**XVIa**), (**XVIb**), (**XVIc**) or (**XVId**) as defined in claim 6; or
a compound of formula **(Ia),** wherein R⁸⁷ is a group of formula (**XIIa**), (**XIIb**), (**XIIc**), (**XIId**), (**XIIe**), (**XIIf**), (**XIIg**), (**XIIh**), (**XIIi**), (**XIIj**), (**XIIk**), ((**XIIl**)), (**XIIm**), (**XIIn**), (**XIIo**), (**XIIp**), (**XIIq**), (**XIIr**), (**XIIs**), (**XIIt**), (**XIIu**), (**XIIv**), (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined in claim 5, and
R⁸³ is H, a C₁-C₂₅alkyl group, a group of formula (**XIIa**), (**XIIb**), (**XIIc**), (**XIId**), (**XIIe**), (**XIIf**), (**XIIg**), (**XIIh**), (**XIIi**), (**XIIj**), (**XIIk**), ((**XIIl**)), (**XIIm**), (**XIIn**), (**XIIo**), (**XIIp**), (**XIIq**), (**XIIr**), (**XIIs**), (**XIIt**), (**XIIu**), (**XIIv**) (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined in claim 5, or a group of formula (**XIVb**), (**XIVc**), (**XIVd**), (**XIVe**), (**XIVf**), (**XIVg**), (**XIVh**), (**XIVo**), (**XIVp**), (**XIVq**), (**XIVr**), (**XIVs**), (**XIVt**), (**XIVu**), (**XIVv**), (**XIVw**), (**XIVx**),(**XIVy**), (**XIVz**), (**XVa**), (**XVb**), (**XVc**), (**XVd**), (**XVe**), (**XVf**), (**XVg**), (**XVh**), (**XVi**), (**XVj**), (**XVk**), (**XVl**), (**XVm**), (**XVn**), (**XVo**), (**XVp**), (**XVq**), (**XVr**), (**XVs**), (**XVt**), (**XVu**), (**XVv**), (**XVw**), (**XVx**), (**XVy**), (**XVz**), (**XVIa**), (**XVIb**), (**XVIc**) or (**XVId**) as defined in claim 6; or
a compound of formula **(Ib),** wherein R⁸² is a group of formula (**XIIa**), (**XIIb**), (**XIIc**), (**XIId**), (**XIIe**), (**XIIf**), (**XIIg**), (**XIIh**), (**XIIi**), (**XIIj**), (**XIIk**), ((**XIIl**)), (**XIIm**), (**XIIn**), (**XIIo**), (**XIIp**), (**XIIq**), (**XIIr**), (**XIIs**), (**XIIt**), (**XIIu**), (**XIIv**), (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined in claim 5, and
R⁸⁷ is H, a group of formula (**XIIa**), (**XIIb**), (**XIIc**), (**XIId**), (**XIIe**), (**XIIf**), (**XIIg**), (**XIIh**), (**XIIi**), (**XIIj**), (**XIIk**), ((**XIIl**)), (**XIIm**), (**XIIn**), (**XIIo**), (**XIIp**), (**XIIq**), (**XIIr**), (**XIIs**), (**XIIt**), (**XIIu**), (**XIIv**) (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined in claim 5, or a group of formula (**XIVb**), (**XIVc**), (**XIVd**), (**XIVe**), (**XIVf**), (**XIVg**), (**XIVh**), (**XIVo**), (**XIVp**), (**XIVq**), (**XIVr**), (**XIVs**), (**XIVt**), (**XIVu**), (**XIVv**), (**XIVw**), (**XIVx**),(**XIVy**), (**XIVz**), (**XVa**), (**XVb**), (**XVc**), (**XVd**), (**XVe**), (**XVf**), (**XVg**), (**XVh**), (**XVi**), (**XVj**), (**XVk**), (**XVl**), (**XVm**), (**XVn**), (**XVo**), (**XVp**), (**XVq**), (**XVr**), (**XVs**), (**XVt**), (**XVu**), (**XVv**), (**XVw**), (**XVx**), (**XVy**), (**XVz**), (**XVIa**), (**XVIb**), (**XVIc**) or (**XVId**) as defined in claim 6; or
a compound of formula **(Ib),** wherein R⁸⁷ is a group of formula (**XIIa**), (**XIIb**), (**XIIc**), (**XIId**), (**XIIe**), (**XIIf**), (**XIIg**), (**XIIh**), (**XIIi**), (**XIIj**), (**XIIk**), ((**XIIl**)), (**XIIm**), (**XIIn**), (**XIIo**), (**XIIp**), (**XIIq**), (**XIIr**), (**XIIs**), (**XIIt**), (**XIIu**), (**XIIv**), (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined in claim 5, and
R⁸² is H, a group of formula (**XIIa**), (**XIIb**), (**XIIc**), (**XIId**), (**XIIe**), (**XIIf**), (**XIIg**), (**XIIh**), (**XIIi**), (**XIIj**), (**XIIk**), ((**XIIl**)), (**XIIm**), (**XIIn**), (**XIIo**), (**XIIp**), (**XIIq**), (**XIIr**), (**XIIs**), (**XIIt**), (**XIIu**), (**XIIv**) (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined in claim 5, or a group of formula (**XIVb**), (**XIVc**), (**XIVd**), (**XIVe**), (**XIVf**), (**XIVg**), (**XIVh**), (**XIVo**), (**XIVp**), (**XIVq**), (**XIVr**), (**XIVs**), (**XIVt**), (**XIVu**), (**XIVv**), (**XIVw**), (**XIVx**),(**XIVy**), (**XIVz**), (**XVa**), (**XVb**), (**XVc**), (**XVd**), (**XVe**), (**XVf**), (**XVg**), (**XVh**), (**XVi**), (**XVj**), (**XVk**), (**XVl**), (**XVm**), (**XVn**), (**XVo**), (**XVp**), (**XVq**), (**XVr**), (**XVs**), (**XVt**), (**XVu**), (**XVv**), (**XVw**), (**XVx**), (**XVy**), (**XVz**), (**XVIa**), (**XVIb**), (**XVIc**) or (**XVId**) as defined in claim 6; or
a compound of formula **(Ic),** wherein R⁸⁵ is a group of formula (**XIIa**), (**XIIb**), (**XIIc**), (**XIId**), (**XIIe**), (**XIIf**), (**XIIg**), (**XIIh**), (**XIIi**), (**XIIj**), (**XIIk**), ((**XIIl**)), (**XIIm**), (**XIIn**), (**XIIo**), (**XIIp**), (**XIIq**), (**XIIr**), (**XIIs**), (**XIIt**), (**XIIu**), (**XIIv**), (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined in claim 5, and
R⁸⁷ is H, a C₁-C₂₅alkyl group, a group of formula (**XIIa**), (**XIIb**), (**XIIc**), (**XIId**), (**XIIe**), (**XIIf**), (**XIIg**), (**XIIh**), (**XIIi**), (**XIIj**), (**XIIk**), ((**XIIl**)), (**XIIm**), (**XIIn**), (**XIIo**), (**XIIp**), (**XIIq**), (**XIIr**), (**XIIs**), (**XIIt**), (**XIIu**), (**XIIv**) (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined in claim 5, or a group of formula (**XIVb**), (**XIVc**), (**XIVd**), (**XIVe**), (**XIVf**), (**XIVg**), (**XIVh**), (**XIVo**), (**XIVp**), (**XIVq**), (**XIVr**), (**XIVs**), (**XIVt**), (**XIVu**), (**XIVv**), (**XIVw**), (**XIVx**),(**XIVy**), (**XIVz**), (**XVa**), (**XVb**), (**XVc**), (**XVd**), (**XVe**), (**XVf**), (**XVg**), (**XVh**), (**XVi**), (**XVj**), (**XVk**), (**XVI**), (**XVm**), (**XVn**), (**XVo**), (**XVp**), (**XVq**), (**XVr**), (**XVs**), (**XVt**), (**XVu**), (**XVv**), (**XVw**), (**XVx**), (**XVy**), (**XVz**), (**XVIa**), (**XVIb**), (**XVIc**) or (**XVId**) as defined in claim 6; or
a compound of formula **(Ic),** wherein R⁸⁷ is a group of formula (**XIIa**), (**XIIb**), (**XIIc**), (**XIId**), (**XIIe**), (**XIIf**), (**XIIg**), (**XIIh**), (**XIIi**), (**XIIj**), (**XIIk**), ((**XIII**)), (**XIIm**), (**XIIn**), (**XIIo**), (**XIIp**), (**XIIq**), (**XIIr**), (**XIIs**), (**XIIt**), (**XIIu**), (**XIIv**), (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined in claim 5; and
R⁸⁵ is H, a C₁-C₂₅alkyl group, a group of formula (**XIIa**), (**XIIb**), (**XIIc**), (**XIId**), (**XIIe**), (**XIIf**), (**XIIg**), (**XIIh**), (**XIIi**), (**XIIj**), (**XIIk**), ((**XIIl**)), (**XIIm**), (**XIIn**), (**XIIo**), (**XIIp**), (**XIIq**), (**XIIr**), (**XIIs**), (**XIIt**), (**XIIu**), (**XIIv**) (**XIIw**), (**XIIx**), (**XIIy**), (**XIIz**), (**XIIIa**), (**XIIIb**), (**XIIIc**),
(**XIIId**), (**XIIIe**), (**XIIIf**) or (**XIIIg**) as defined in claim 5, or a group of formula (**XIVb**), (**XIVc**), (**XIVd**), (**XIVe**), (**XIVf**), (**XIVg**), (**XIVh**), (**XIVo**), (**XIVp**), (**XIVq**), (**XIVr**), (**XIVs**), (**XIVt**), (**XIVu**), (**XIVv**), (**XIVw**), (**XIVx**), (**XIVy**), (**XIVz**), (**XVa**), (**XVb**), (**XIVc**), (**XVd**), (**XVe**), (**XVf**), (**XVg**), (**XIVh**), (**XVi**), (**XVj**), (**XIVk**), (**XVl**), (**XVm**), (**XVn**), (**XVo**), (**XVp**), (**XVq**), (**XVr**), (**XVs**), (**XVt**), (**XVu**), (**XVv**), (**XVw**), (**XVx**), (**XVy**), (**XVz**), (**XVIa**), (**XVIb**), (**XVIc**) or (**XVId**) as defined in claim 6; or
a compound of formula (**Id**), wherein R⁸¹ is a group of formula (**XIIa**), (**XIIb**), (**XIIc**), (**XIId**), (**XIIe**), (**XIIf**), (**XIIg**), (**XIIh**), (**XIIi**), (**XIIj**), (**XIIk**), ((**XIIl**)), (**XIIm**), (**XIIn**), (**XIIo**), (**XIIp**), (**XIIq**), (**XIIr**), (**XIIs**), (**XIIt**), (**XIIu**), (**XIIv**), (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined in claim 5, and
R⁸⁵ is H, a group of formula (**XIIa**), (**XIIb**), (**XIIc**), (**XIId**), (**XIIe**), (**XIIf**), (**XIIg**), (**XIIh**), (**XIIi**), (**XIIj**), (**XIIk**), ((**XIIl**)), (**XIIm**), (**XIIn**), (**XIIo**), (**XIIp**), (**XIIq**), (**XIIr**), (**XIIs**), (**XIIt**), (**XIIu**), (**XIIv**) (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined in claim 5; or a group of formula (**XIVb**), (**XIVc**), (**XIVd**), (**XIVe**), (**XIVf**), (**XIVg**), (**XIVh**), (**XIVo**), (**XIVp**), (**XIVq**), (**XIVr**), (**XIVs**), (**XIVt**), (**XIVu**), (**XIVv**), (**XIVw**), (**XIVx**),(**XIVy**), (**XIVz**), (**XVa**), (**XVb**), (**XVc**), (**XVd**), (**XVe**), (**XVf**), (**XVg**), (**XVh**), (**XVi**), (**XVj**), (**XVk**), (**XVl**), (**XVm**), (**XVn**), (**XVo**), (**XVp**), (**XVq**), (**XVr**), (**XVs**), (**XVt**), (**XVu**), (**XVv**), (**XVw**), (**XVx**), (**XVy**), (**XVz**), (**XVIa**), (**XVIb**), (**XVIc**) or (**XVId**) as defined in claim 6; or
a compound of formula (**Id**), wherein R⁸⁵ is a group of formula (**XIIa**), (**XIIb**), (**XIIc**), (**XIId**), (**XIIe**), (**XIIf**), (**XIIg**), (**XIIh**), (**XIIi**), (**XIIj**), (**XIIk**), ((**XIIl**)), (**XIIm**), (**XIIn**), (**XIIo**), (**XIIp**), (**XIIq**), (**XIIr**), (**XIIs**), (**XIIt**), (**XIIu**), (**XIIv**), (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined in claim 5; and
R⁸¹ is H, a group of formula (**XIIa**), (**XIIb**), (**XIIc**), (**XIId**), (**XIIe**), (**XIIf**), (**XIIg**), (**XIIh**), (**XIIi**), (**XIIj**), (**XIIk**), ((**XIIl**)), (**XIIm**), (**XIIn**), (**XIIo**), (**XIIp**), (**XIIq**), (**XIIr**), (**XIIs**), (**XIIt**), (**XIIu**), (**XIIv**) (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined in claim 5, or a group of formula (**XIVb**), (**XIVc**), (**XIVd**), (**XIVe**), (**XIVf**), (**XIVg**), (**XIVh**), (**XIVo**), (**XIVp**), (**XIVq**), (**XIVr**), (**XIVs**), (**XIVt**), (**XIVu**), (**XIVv**), (**XIVw**), (**XIVx**),(**XIVy**), (**XIVz**), (**XVa**), (**XVb**), (**XVc**), (**XVd**), (**XVe**), (**XVf**), (**XVg**), (**XVh**), (**XVi**), (**XVj**), (**XVk**), (**XVl**), (**XVm**), (**XVn**), (**XVo**), (**XVp**), (**XVq**), (**XVr**), (**XVs**), (**XVt**), (**XVu**), (**XVv**), (**XVw**), (**XVx**), (**XVy**), (**XVz**), (**XVIa**), (**XVIb**), (**XVIc**) or (**XVId**) as defined in claim 6; or
a compound of formula (**Ie**), wherein R⁸³ is a group of formula (**XIIa**), (**XIIb**), (**XIIc**), (**XIId**), (**XIIe**), (**XIIf**), (**XIIg**), (**XIIh**), (**XIIi**), (**XIIj**), (**XIIk**), ((**XIIl**)), (**XIIm**), (**XIIn**), (**XIIo**), (**XIIp**), (**XIIq**), (**XIIr**), (**XIIs**), (**XIIt**), (**XIIu**), (**XIIv**), (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined in claim 5, and
R⁸⁷ is H, a C₁-C₂₅alkyl group, a group of formula (**XIIa**), (**XIIb**), (**XIIc**), (**XIId**), (**XIIe**), (**XIIf**), (**XIIg**), (**XIIh**), (**XIIi**), (**XIIj**), (**XIIk**), ((**XIIl**)), (**XIIm**), (**XIIn**), (**XIIo**), (**XIIp**), (**XIIq**), (**XIIr**), (**XIIs**), (**XIIt**), (**XIIu**), (**XIIv**) (**XIIw**), (**XIIx**), (**XIIy**), (**XIIz**), (**XIIIa**), (**XIIIb**), (**XIIIe**), (**XIIId**), (**XIIIe**), (**XIIIf**) or (**XIIIg**) as defined in claim 5, or a group of formula (**XIVb**), (**XIVc**), (**XIVd**), (**XIVe**), (**XIVf**), (**XIVg**), (**XIVh**), (**XIVo**), (**XIVp**), (**XIVq**), (**XIVr**), (**XIVs**), (**XIVt**), (**XIVu**), (**XIVv**), (**XIVw**), (**XIVx**),(**XIVy**), (**XIVz**), (**XVa**), (**XVb**), (**XVc**), (**XVd**), (**XVe**), (**XVf**), (**XVg**), (**XVh**), (**XVi**), (**XVj**), (**XVk**), (**XVl**), (**XVm**), (**XVn**), (**XVo**), (**XVp**), (**XVq**), (**XVr**), (**XVs**), (**XVt**), (**XVu**), (**XVv**), (**XVw**), (**XVx**), (**XVy**), (**XVz**), (**XVIa**), (**XVIb**), (**XVIc**) or (**XVId**) as defined in claim 6; or
a compound of formula (**Ie**), wherein R⁸⁷ is a group of formula (**XIIa**), (**XIIb**), (**XIIc**), (**XIId**), (**XIIe**), (**XIIf**), (**XIIg**), (**XIIh**), (**XIIi**), (**XIIj**), (**XIIk**), ((**XIIl**)), (**XIIm**), (**XIIn**), (**XIIo**), (**XIIp**), (**XIIq**), (**XIIr**), (**XIIs**), (**XIIt**), (**XIIu**), (**XIIv**), (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined in claim 5, and
R⁸³ is H, a C₁-C₂₅alkyl group, a group of formula (**XIIa**), (**XIIb**), (**XIIc**), (**XIId**), (**XIIe**), (**XIIf**), (**XIIg**), (**XIIh**), (**XIIi**), (**XIIj**), (**XIIk**), ((**XIIl**)), (**XIIm**), (**XIIn**), (**XIIo**), (**XIIp**), (**XIIq**), (**XIIr**), (**XIIs**), (**XIIt**), (**XIIu**), (**XIIv**) (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined in claim 5, or a group of formula (**XIVb**), (**XIVc**), (**XIVd**), (**XIVe**), (**XIVf**), (**XIVg**), (**XIVh**), (**XIVo**), (**XIVp**), (**XIVq**), (**XIVr**), (**XIVs**), (**XIVt**), (**XIVu**), (**XIVv**), (**XIVw**), (**XIVx**),(**XIVy**), (**XIVz**), (**XVa**), (**XVb**), (**XVc**), (**XVd**), (**XVe**), (**XVf**), (**XVg**), (**XVh**), (**XVi**), (**XVj**), (**XVk**), (**XVl**), (**XVm**), (**XVn**), (**XVo**), (**XVp**), (**XVq**), (**XVr**), (**XVs**), (**XVt**), (**XVu**), (**XVv**), (**XVw**), (**XVx**), (**XVy**), (**XVz**), (**XVIa**), (**XVIb**), (**XVIc**) or (**XVId**) as defined in claim 6; or
a compound of formula (**If**), wherein R⁸³ is a group of formula (**XIIa**), (**XIIb**), (**XIIc**), (**XIId**), (**XIIe**), (**XIIf**), (**XIIg**), (**XIIh**), (**XIIi**), (**XIIj**), (**XIIk**), ((**XIIl**)), (**XIIm**), (**XIIn**), (**XIIo**), (**XIIp**), (**XIIq**), (**XIIr**), (**XIIs**), (**XIIt**), (**XIIu**), (**XIIv**), (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined in claim 5, and
R⁸⁵ is H, a group of formula (**XIIa**), (**XIIb**), (**XIIc**), (**XIId**), (**XIIe**), (**XIIf**), (**XIIg**), (**XIIh**), (**XIIi**), (**XIIj**), (**XIIk**), ((**XIIl**)), (**XIIm**), (**XIIn**), (**XIIo**), (**XIIp**), (**XIIq**), (**XIIr**), (**XIIs**), (**XIIt**), (**XIIu**), (**XIIv**) (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined in claim 5, or a group of formula (**XIVb**), (**XIVc**), (**XIVd**), (**XIVe**), (**XIVf**), (**XIVg**), (**XIVh**), (**XIVo**), (**XIVp**), (**XIVq**), (**XIVr**), (**XIVs**), (**XIVt**), (**XIVu**), (**XIVv**), (**XIVw**), (**XIVx**),(**XIVy**), (**XIVz**), (**XVa**), (**XVb**), (**XVc**), (**XVd**), (**XVe**), (**XVf**), (**XVg**), (**XVh**), (**XVi**), (**XVj**), (**XVk**), (**XVl**), (**XVm**), (**XVn**), (**XVo**), (**XVp**), (**XVq**), (**XVr**), (**XVs**), (**XVt**), (**XVu**), (**XVv**), (**XVw**), (**XVx**), (**XVy**), (**XVz**), (**XVIa**), (**XVIb**), (**XVIc**) or (**XVId**) as defined in claim 6; or
a compound of formula (**If**), wherein R⁸⁵ is a group of formula (**XIIa**), (**XIIb**), (**XIIc**), (**XIId**), (**XIIe**), (**XIIf**), (**XIIg**), (**XIIh**), (**XIIi**), (**XIIj**), (**XIIk**), ((**XIIl**)), (**XIIm**), (**XIIn**), (**XIIo**), (**XIIp**), (**XIIq**), (**XIIr**), (**XIIs**), (**XIIt**), (**XIIu**), (**XIIv**), (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined in claim 5, and
R⁸³ is H, a group of formula (**XIIa**), (**XIIb**), (**XIIc**), (**XIId**), (**XIIe**), (**XIIf**), (**XIIg**), (**XIIh**), (**XIIi**), (**XIIj**), (**XIIk**), ((**XIIl**)), (**XIIm**), (**XIIn**), (**XIIo**), (**XIIp**), (**XIIq**), (**XIIr**), (**XIIs**), (**XIIt**), (**XIIu**), (**XIIv**) (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined in claim 5, or a group of formula (**XIVb**), (**XIVc**), (**XIVd**), (**XIVe**), (**XIVf**), (**XIVg**), (**XIVh**), (**XIVo**), (**XIVp**), (**XIVq**), (**XIVr**), (**XIVs**), (**XIVt**), (**XIVu**), (**XIVv**), (**XIVw**), (**XIVx**),(**XIVy**), (**XIVz**), (**XVa**), (**XVb**), (**XVc**), (**XVd**), (**XVe**), (**XVf**), (**XVg**), (**XVh**), (**XVi**), (**XVj**), (**XVk**), (**XVl**), (**XVm**), (**XVn**), (**XVo**), (**XVp**), (**XVq**), (**XVr**), (**XVs**), (**XVt**), (**XVu**), (**XVv**), (**XVw**), (**XVx**), (**XVy**), (**XVz**), (**XVIa**), (**XVIb**), (**XVIc**) or (**XVId**) as defined in claim 6; or
a compound of formula (**Ii**), wherein R⁸³ is a group of formula (**XIIa**), (**XIIb**), (**XIIc**), (**XIId**), (**XIIe**), (**XIIf**), (**XIIg**), (**XIIh**), (**XIIi**), (**XIIj**), (**XIIk**), ((**XIIl**)), (**XIIm**), (**XIIn**), (**XIIo**), (**XIIp**), (**XIIq**), (**XIIr**), (**XIIs**), (**XIIt**), (**XIIu**), (**XIIv**), (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined in claim 5, and
R⁸⁵ is H, a group of formula (**XIIa**), (**XIIb**), (**XIIc**), (**XIId**), (**XIIe**), (**XIIf**), (**XIIg**), (**XIIh**), (**XIIi**), (**XIIj**), (**XIIk**), ((**XIIl**)), (**XIIm**), (**XIIn**), (**XIIo**), (**XIIp**), (**XIIq**), (**XIIr**), (**XIIs**), (**XIIt**), (**XIIu**), (**XIIv**) (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined in claim 5, or a group of formula (**XIVb**), (**XIVc**), (**XIVd**), (**XIVe**), (**XIVf**), (**XIVg**), (**XIVh**), (**XIVo**), (**XIVp**), (**XIVq**), (**XIVr**), (**XIVs**), (**XIVt**), (**XIVu**), (**XIVv**), (**XIVw**), (**XIVx**), (**XIVy**), (**XIVz**), (**XVa**), (**XVb**), (**XVc**), (**XVd**), (**XVe**), (**XVf**), (**XVg**), (**XVh**), (**XVi**), (**XVj**), (**XVk**), (**XVl**), (**XVm**), (**XVn**), (**XVo**), (**XVp**), (**XVq**), (**XVr**), (**XVs**), (**XVt**), (**XVu**), (**XVv**), (**XVw**), (**XVx**), (**XVy**), (**XVz**), (**XVIa**), (**XVIb**), (**XVIc**) or (**XVId**) as defined in claim 6; or
a compound of formula (**Ii**), wherein R⁸⁵ is a group of formula (**XIIa**), (**XIIb**), (**XIIc**), (**XIId**), (**XIIe**), (**XIIf**), (**XIIg**), (**XIIh**), (**XIIi**), (**XIIj**), (**XIIk**), ((**XIIl**)), (**XIIm**), (**XIIn**), (**XIIo**), (**XIIp**), (**XIIq**), (**XIIr**), (**XIIs**), (**XIIt**), (**XIIu**), (**XIIv**), (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined in claim 5, and
R⁸³ is H, a group of formula (**XIIa**), (**XIIb**), (**XIIc**), (**XIId**), (**XIIe**), (**XIIf**), (**XIIg**), (**XIIh**), (**XIIi**), (**XIIj**), (**XIIk**), ((**XIIl**)), (**XIIm**), (**XIIn**), (**XIIo**), (**XIIp**), (**XIIq**), (**XIIr**), (**XIIs**), (**XIIt**), (**XIIu**), (**XIIv**) (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined in claim 5, or a group of formula (**XIVb**), (**XIVc**), (**XIVd**), (**XIVe**), (**XIVf**), (**XIVg**), (**XIVh**), (**XIVo**), (**XIVp**), (**XIVq**), (**XIVr**), (**XIVs**), (**XIVt**), (**XIVu**), (**XIVv**), (**XIVw**), (**XIVx**),(**XIVy**), (**XIVz**), (**XVa**), (**XVb**), (**XVc**), (**XVd**), (**XVe**), (**XVf**), (**XVg**), (**XVh**), (**XVi**), (**XVj**), (**XVk**), (**XVl**), (**XVm**), (**XVn**), (**XVo**), (**XVp**), (**XVq**), (**XVr**), (**XVs**), (**XVt**), (**XVu**), (**XVv**), (**XVw**), (**XVx**), (**XVy**), (**XVz**), (**XVIa**), (**XVIb**), (**XVIc**) or (**XVId**) as defined in claim 6; or
a compound of formula (**Ij**), wherein R⁸³ is a group of formula (**XIIa**), (**XIIb**), (**XIIc**), (**XIId**), (**XIIe**), (**XIIf**), (**XIIg**), (**XIIh**), (**XIIi**), (**XIIj**), (**XIIk**), ((**XIIl**)), (**XIIm**), (**XIIn**), (**XIIo**), (**XIIp**), (**XIIIq**), (**XIIr**), (**XIIs**), (**XIIt**), (**XIIu**), (**XIIv**), (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined in claim 5, and
R⁸⁵ is H, a C₁-C₂₅alkyl group, a group of formula (**XIIa**), (**XIIb**), (**XIIc**), (**XIId**), (**XIIe**), (**XIIf**), (**XIIg**), (**XIIh**), (**XIIi**), (**XIIj**), (**XIIk**), ((**XIIl**)), (**XIIm**), (**XIIn**), (**XIIo**), (**XIIp**), (**XIIq**), (**XIIr**), (**XIIs**), (**XIIt**), (**XIIu**), (**XIIv**) (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined in claim 5, or a group of formula (**XIVb**), (**XIVc**), (**XIVd**), (**XIVe**), (**XIVf**), (**XIVg**), (**XIVh**), (**XIVo**), (**XIVp**), (**XIVq**), (**XIVr**), (**XIVs**), (**XIVt**), (**XIVu**), (**XIVv**), (**XIVw**), (**XIVx**),(**XIVy**), (**XIVz**), (**XVa**), (**XVb**), (**XVc**), (**XVd**), (**XVe**), (**XVf**), (**XVg**), (**XVh**), (**XVi**), (**XVj**), (**XVk**), (**XVl**), (**XVm**), (**XVn**), (**XVo**), (**XVp**), (**XVq**), (**XVr**), (**XVs**), (**XVt**), (**XVu**), (**XVv**), (**XVw**), (**XVx**), (**XVy**), (**XVz**), (**XVIa**), (**XVIb**), (**XVIc**) or (**XVId**) as defined in claim 6; or
a compound of formula (**Ij**), wherein R⁸⁵ is a group of formula (**XIIa**), (**XIIb**), (**XIIc**), (**XIId**), (**XIIe**), (**XIIf**), (**XIIg**), (**XIIh**), (**XIIi**), (**XIIj**), (**XIIk**), ((**XIIl**)), (**XIIm**), (**XIIn**), (**XIIo**), (**XIIp**), (**XIIq**), (**XIIr**), (**XIIs**), (**XIIt**), (**XIIu**), (**XIIv**), (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined in claim 5, and
R⁸³ is H, a C₁-C₂₅alkyl group, a group of formula (**XIIa**), (**XIIb**), (**XIIc**), (**XIId**), (**XIIe**), (**XIIf**), (**XIIg**), (**XIIh**), (**XIIi**), (**XIIj**), (**XIIk**), ((**XIIl**)), (**XIIm**), (**XIIn**), (**XIIo**), (**XIIp**), (**XIIq**), (**XIIr**), (**XIIs**), (**XIIt**), (**XIIu**), (**XIIv**) (**XIIw**), (**XIIx**), (**XIIy**), (**XIIz**), (**XIIIa**), (**XIIIb**), (**XIIIc**),
(**XIIId**), (**XIIIe**), (**XIIIf**) or (**XIIIg**) as defined in claim 5, or a group of formula (**XIVb**), (**XIVc**), (**XIVd**), (**XIVe**), (**XIVf**), (**XIVg**), (**XIVh**), (**XIVo**), (**XIVp**), (**XIVq**), (**XIVr**), (**XIVs**), (**XIVt**), (**XIVu**), (**XIVv**), (**XIVw**), (**XIVx**),(**XIVy**), (**XIVz**), (**XVa**), (**XVb**), (**XVc**), (**XVd**), (**XVe**), (**XVf**), (**XVg**), (**XVh**), (**XVi**), (**XVj**), (**XVk**), (**XVl**), (**XVm**), (**XVn**), (**XVo**), (**XVp**), (**XVq**), (**XVr**), (**XVs**), (**XVt**), (**XVu**), (**XVv**), (**XVw**), (**XVx**), (**XVy**), (**XVz**), (**XVIa**), (**XVIb**), (**XVIc**) or (**XVId**) as defined in claim 6;
a compound of formula (**Il**), wherein R⁸⁵ is a group of formula (**XIIa**), (**XIIb**), (**XIIc**), (**XIId**), (**XIIe**), (**XIIf**), (**XIIg**), (**XIIh**), (**XIIi**), (**XIIj**), (**XIIk**), ((**XIIl**)), (**XIIm**), (**XIIn**), (**XIIo**), (**XIIp**), (**XIIq**), (**XIIr**), (**XIIs**), (**XIIt**), (**XIIu**), (**XIIv**), (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined in claim 5, and
R⁸⁷ is H, a group of formula (**XIIa**), (**XIIb**), (**XIIc**), (**XIId**), (**XIIe**), (**XIIf**), (**XIIg**), (**XIIh**), (**XIIi**), (**XIIj**), (**XIIk**), ((**XIIl**)), (**XIIm**), (**XIIn**), (**XIIo**),(**XIIp**), (**XIIq**), (**XIIr**), (**XIIs**), (**XIIt**), (**XIIu**), (**XIIv**) (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined in claim 5, or a group of formula (**XIVb**), (**XIVc**), (**XIVd**), (**XIVe**), (**XIVf**), (**XIVg**), (**XIVh**), (**XIVo**), (**XIVp**), (**XIVq**), (**XIVr**), (**XIVs**), (**XIVt**), (**XIVu**), (**XIVv**), (**XIVw**), (**XIVx**),(**XIVy**), (**XIVz**), (**XVa**), (**XVb**), (**XVc**), (**XVd**), (**XVe**), (**XVf**), (**XVg**), (**XVh**), (**XVi**), (**XVj**), (**XVk**), (**XVl**), (**XVm**), (**XVn**), (**XVo**), (**XVp**), (**XVq**), (**XVr**), (**XVs**), (**XVt**), (**XVu**), (**XVv**), (**XVw**), (**XVx**), (**XVy**), (**XVz**), (**XVIa**), (**XVIb**), (**XVIc**) or (**XVId**) as defined in claim 6; or
a compound of formula (**Il**), wherein R⁸⁷ is a group of formula (**XIIa**), (**XIIb**), (**XIIc**), (**XIId**), (**XIIe**), (**XIIf**), (**XIIg**), (**XIIh**), (**XIIi**), (**XIIj**), (**XIIk**), ((**XIIl**)), (**XIIm**), (**XIIn**), (**XIIo**), (**XIIp**), (**XIIq**), (**XIIr**), (**XIIs**), (**XIIt**), (**XIIu**), (**XIIv**), (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined in claim 5, and
R⁸⁵ is H, a group of formula (**XIIa**), (**XIIb**), (**XIIc**), (**XIId**), (**XIIe**), (**XIIf**), (**XIIg**), (**XIIh**), (**XIIi**), (**XIIj**), (**XIIk**), ((**XIIl**)), (**XIIm**), (**XIIn**), (**XIIo**),(**XIIp**), (**XIIq**), (**XIIr**), (**XIIs**), (**XIIt**), (**XIIu**), (**XIIv**) (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined in claim 5, or a group of formula (**XIVb**), (**XIVc**), (**XIVd**), (**XIVe**), (**XIVf**), (**XIVg**), (**XIVh**), (**XIVo**), (**XIVp**), (**XIVq**), (**XIVr**), (**XIVs**), (**XIVt**), (**XIVu**), (**XIVv**), (**XIVw**), (**XIVx**),(**XIVy**), (**XIVz**), (**XVa**), (**XVb**), (**XVc**), (**XVd**), (**XVe**), (**XVf**), (**XVg**), (**XVh**), (**XVi**), (**XVj**), (**XVk**), (**XVl**), (**XVm**), (**XVn**), (**XVo**), (**XVp**), (**XVq**), (**XVr**), (**XVs**), (**XVt**), (**XVu**), (**XVv**), (**XVw**), (**XVx**), (**XVy**), (**XVz**), (**XVIa**), (**XVIb**), (**XVIc**) or (**XVId**) as defined in claim 6; or
a compound of formula (**Im**), wherein R⁸³ is a group of formula (**XIIa**), (**XIIb**), (**XIIc**), (**XIId**), (**XIIe**), (**XIIf**), (**XIIg**), (**XIIh**), (**XIIi**), (**XIIj**), (**XIIk**), ((**XIIl**)), (**XIIm**), (**XIIn**), (**XIIo**), (**XIIp**), (**XIIq**), (**XIIr**), (**XIIs**), (**XIIt**), (**XIIu**), (**XIIv**), (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined in claim 5, and
R⁸⁵ is H, a C₁-C₂₅alkyl group, a group of formula (**XIIa**), (**XIIb**), (**XIIc**), (**XIId**), (**XIIe**), (**XIIf**), (**XIIg**), (**XIIh**), (**XIIi**), (**XIIj**), (**XIIk**), ((**XIIl**)), (**XIIm**), (**XIIn**), (**XIIo**),(**XIIp**), (**XIIq**), (**XIIr**), (**XIIs**), (**XIIt**), (**XIIu**), (**XIIv**) (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined in claim 5, or a group of formula (**XIVb**), (**XIVc**), (**XIVd**), (**XIVe**), (**XIVf**), (**XIVg**), (**XIVh**), (**XIVo**), (**XIVp**), (**XIVq**), (**XIVr**), (**XIVs**), (**XIVt**), (**XIVu**), (**XIVv**), (**XIVw**), (**XIVx**), (**XIVy**), (**XIVz**), (**XVa**), (**XVb**), (**XVc**), (**XVd**), (**XVe**), (**XVf**), (**XVg**), (**XVh**), (**XVi**), (**XVj**), (**XVk**), (**XVl**), (**XVm**), (**XVn**), (**XVo**), (**XVp**), (**XVq**), (**XVr**), (**XVs**), (**XVt**), (**XVu**), (**XVv**), (**XVw**), (**XVx**), (**XVy**), (**XVz**), (**XVIa**), (**XVIb**), (**XVIc**) or (**XVId**) as defined in claim 6; or
a compound of formula (**Im**), wherein R⁸⁵ is a group of formula (**XIIa**), (**XIIb**), (**XIIc**), (**XIId**), (**XIIe**), (**XIIf**), (**XIIg**), (**XIIh**), (**XIIi**), (**XIIj**), (**XIIk**), ((**XIIl**)), (**XIIm**), (**XIIn**), (**XIIo**), (**XIIp**), (**XIIq**), (**XIIr**), (**XIIs**), (**XIIt**), (**XIIu**), (**XIIv**), (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined in claim 5, and
R⁸³ is H, a C₁-C₂₅alkyl group, a group of formula (**XIIa**), (**XIIb**), (**XIIc**), (**XIId**), (**XIIe**), (**XIIf**), (**XIIg**), (**XIIh**), (**XIIi**), (**XIIj**), (**XIIk**), ((**XIIl**)), (**XIIm**), (**XIIn**), (**XIIo**),(**XIIp**), (**XIIq**), (**XIIr**), (**XIIs**), (**XIIt**), (**XIIu**), (**XIIv**) (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined in claim 5, or a group of formula (**XIVb**), (**XIVc**), (**XIVd**), (**XIVe**), (**XIVf**), (**XIVg**), (**XIVh**), (**XIVo**), (**XIVp**), (**XIVq**), (**XIVr**), (**XIVs**), (**XIVt**), (**XIVu**), (**XIVv**), (**XIVw**), (**XIVx**),(**XIVy**), (**XIVz**), (**XVa**), (**XVb**), (**XVc**), (**XVd**), (**XVe**), (**XVf**), (**XVg**), (**XVh**), (**XVi**), (**XVj**), (**XVk**), (**XVl**), (**XVm**), (**XVn**), (**XVo**), (**XVp**), (**XVq**), (**XVr**), (**XVs**), (**XVt**), (**XVu**), (**XVv**), (**XVw**), (**XVx**), (**XVy**), (**XVz**), (**XVIa**), (**XVIb**), (**XVIc**) or (**XVId**) as defined in claim 6, or
a compound of formula (**In**), wherein R⁸³ is a group of formula (**XIIa**), (**XIIb**), (**XIIc**), (**XIId**), (**XIIe**), (**XIIf**), (**XIIg**), (**XIIh**), (**XIIi**), (**XIIj**), (**XIIk**), ((**XIIl**)), (**XIIm**), (**XIIn**), (**XIIo**), (**XIIp**), (**XIIq**), (**XIIr**), (**XIIs**), (**XIIt**), (**XIIu**), (**XIIv**), (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined in claim 5, and
R⁸⁷ is H, a group of formula (**XIIa**), (**XIIb**), (**XIIc**), (**XIId**), (**XIIe**), (**XIIf**), (**XIIg**), (**XIIh**), (**XIIi**), (**XIIj**), (**XIIk**), ((**XIIl**)), (**XIIm**), (**XIIn**), (**XIIo**),(**XIIp**), (**XIIq**), (**XIIr**), (**XIIs**), (**XIIt**), (**XIIu**), (**XIIv**) (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined in claim 5, or a group of formula (**XIVb**), (**XIVc**), (**XIVd**), (**XIVe**), (**XIVf**), (**XIVg**), (**XIVh**), (**XIVo**), (**XIVp**), (**XIVq**), (**XIVr**), (**XIVs**), (**XIVt**), (**XIVu**), (**XIVv**), (**XIVw**), (**XIVx**),(**XIVy**), (**XIVz**), (**XVa**), (**XVb**), (**XVc**), (**XVd**), (**XVe**), (**XVf**), (**XVg**), (**XVh**), (**XVi**), (**XVj**), (**XVk**), (**XVl**), (**XVm**), (**XVn**), (**XVo**), (**XVp**), (**XVq**), (**XVr**), (**XVs**), (**XVt**), (**XVu**), (**XVv**), (**XVw**), (**XVx**), (**XVy**), (**XVz**), (**XVIa**), (**XVIb**), (**XVIc**) or (**XVId**) as defined in claim 6.

8. The compound according to any of claims 2 to 7, which is
a compound of formula (**Ia**), wherein R⁸³ is a group of formula (**XIIa**), (**XIIb**), (**XIIc**), (**XIId**), (**XIIe**), (**XIIf**), (**XIIg**), (**XIIj**), (**XIIk**), (**XIIn**), (**XIIo**), (**XIIq**), (**XIIs**), (**XIIt**), (**XIIu**), (**XIIv**), (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined in claim 5, and
R⁸⁷ is H, a C₁-C₂₅alkyl group, a group of formula (**XIIa**), (**XIIb**), (**XIIc**), (**XIId**), (**XIIe**), (**XIIf**), (**XIIg**), (**XIIj**), (**XIIk**), (**XIIn**), (**XIIo**), (**XIIq**), (**XIIs**), (**XIIt**), (**XIIu**), (**XIIv**) (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined in claim 5, or a group of formula (**XIVb**), (**XIVc**), (**XIVd**), (**XIVe**), (**XIVf**), (**XIVg**), (**XIVo**), (**XIVp**), (**XIVq**), (**XIVr**), (**XIVt**), (**XIVu**), (**XIVv**), (**XIVw**), (**XIVx**),(**XIVz**), (**XVa**), (**XVb**), (**XVc**), (**XVk**), (**XVl**), (**XVo**), (**XVp**), (**XVs**), (**XVw**), (**XVx**), (**XVy**), (**XVIa**), (**XVIb**), (**XVIc**) or (**XVId**) as defined in claim 6; or
a compound of formula (**Ia**), wherein R⁸⁷ is a group of formula (**XIIa**), (**XIIb**), (**XIIc**), (**XIId**), (**XIIe**), (**XIIf**), (**XIIg**), (**XIIj**), (**XIIk**), (**XIIn**), (**XIIo**), (**XIIq**), (**XIIs**), (**XIIt**), (**XIIu**), (**XIIv**), (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined in claim 5, and
R⁸³ is H, a C₁-C₂₅alkyl group, a group of formula (**XIIa**), (**XIIb**), (**XIIc**), (**XIId**), (**XIIe**), (**XIIf**), (**XIIg**), (**XIIj**), (**XIIk**), (**XIIn**), (**XIIo**), (**XIIq**), (**XIIs**), (**XIIt**), (**XIIu**), (**XIIv**) (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined in claim 5, or a group of formula (**XIVb**), (**XIVc**), (**XIVd**), (**XIVe**), (**XIVf**), (**XIVg**), (**XIVo**), (**XIVp**), (**XIVq**), (**XIVr**), (**XIVt**), (**XIVu**), (**XIVv**), (**XIVw**), (**XIVx**),(**XIVz**), (**XVa**), (**XVb**), (**XVc**), (**XVk**), (**XVl**), (**XVo**), (**XVp**), (**XVs**), (**XVw**), (**XVx**), (**XVy**), (**XVIa**), (**XVIb**), (**XVIc**) or (**XVId**) as defined in claim 6;
a compound of formula (**Ib**), wherein R⁸² is a group of formula (**XIIa**), (**XIIb**), (**XIIc**), (**XIId**), (**XIIe**), (**XIIf**), (**XIIg**), (**XIIj**), (**XIIk**), (**XIIn**), (**XIIo**), (**XIIq**), (**XIIs**), (**XIIt**), (**XIIu**), (**XIIv**), (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined in claim 5, and
R⁸⁷ is H, a group of formula (**XIIa**), (**XIIb**), (**XIIc**), (**XIId**), (**XIIe**), (**XIIf**), (**XIIg**), (**XIIj**), (**XIIk**), (**XIIn**), (**XIIo**), (**XIIq**), (**XIIs**), (**XIIt**), (**XIIu**), (**XIIv**) (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined in claim 5, or a group of formula (**XIVb**), (**XIVc**), (**XIVd**), (**XIVe**), (**XIVf**), (**XIVg**), (**XIVo**), (**XIVp**), (**XIVq**), (**XIVr**), (**XIVt**), (**XIVu**), (**XIVv**), (**XIVw**), (**XIVx**),(**XIVz**), (**XVa**), (**XVb**), (**XVc**), (**XVk**), (**XVl**), (**XVo**), (**XVp**), (**XVs**), (**XVw**), (**XVx**), (**XVy**), (**XVIa**), (**XVIb**), (**XVIc**) or (**XVId**) as defined in claim 6; or
a compound of formula (**Ib**), wherein R⁸⁷ is a group of formula (**XIIa**), (**XIIb**), (**XIIc**), (**XIId**), (**XIIe**), (**XIIf**), (**XIIg**), (**XIIj**), (**XIIk**), (**XIIn**), (**XIIo**), (**XIIq**), (**XIIs**), (**XIIt**), (**XIIu**), (**XIIv**), (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined in claim 5, and
R⁸² is H, a group of formula (**XIIa**), (**XIIb**), (**XIIc**), (**XIId**), (**XIIe**), (**XIIf**), (**XIIg**), (**XIIj**), (**XIIk**), (**XIIn**), (**XIIo**), (**XIIq**), (**XIIs**), (**XIIt**), (**XIIu**), (**XIIv**) (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined in claim 5, or a group of formula (**XIVb**), (**XIVc**), (**XIVd**), (**XIVe**), (**XIVf**), (**XIVg**), (**XIVo**), (**XIVp**), (**XIVq**), (**XIVr**), (**XIVt**), (**XIVu**), (**XIVv**), (**XIVw**), (**XIVx**),(**XIVz**), (**XVa**), (**XVb**), (**XVc**), (**XVk**), (**XVl**), (**XVo**), (**XVp**), (**XVs**), (**XVw**), (**XVx**), (**XVy**), (**XVIa**), (**XVIb**), (**XVIc**) or (**XVId**) as defined in claim 6;
a compound of formula (**Ic**), wherein R⁸⁵ is a group of formula (**XIIa**), (**XIIb**), (**XIIc**), (**XIId**), (**XIIe**), (**XIIf**), (**XIIg**), (**XIIj**), (**XIIk**), (**XIIn**), (**XIIo**), (**XIIq**), (**XIIs**), (**XIIt**), (**XIIu**), (**XIIv**), (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined in claim 5, and
R⁸⁷ is H, a C₁-C₂₅alkyl group, a group of formula (**XIIa**), (**XIIb**), (**XIIc**), (**XIId**), (**XIIe**), (**XIIf**), (**XIIg**), (**XIIj**), (**XIIk**), (**XIIn**), (**XIIo**), (**XIIq**), (**XIIs**), (**XIIt**), (**XIIu**), (**XIIv**) (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined in claim 5, or a group of formula (**XIVb**), (**XIVc**), (**XIVd**), (**XIVe**), (**XIVf**), (**XIVg**), (**XIVo**), (**XIVp**), (**XIVq**), (**XIVr**), (**XIVt**), (**XIVu**), (**XIVv**), (**XIVw**), (**XIVx**),(**XIVz**), (**XVa**), (**XVb**), (**XVc**), (**XVk**), (**XVl**), (**XVo**), (**XVp**), (**XVs**), (**XVw**), (**XVx**), (**XVy**), (**XVIa**), (**XVIb**), (**XVIc**) or (**XVId**) as defined in claim 6; or
a compound of formula (**Ic**), wherein R⁸⁷ is a group of formula (**XIIa**), (**XIIb**), (**XIIc**), (**XIId**), (**XIIe**), (**XIIf**), (**XIIg**), (**XIIj**), (**XIIk**), (**XIIn**), (**XIIo**), (**XIIq**), (**XIIs**), (**XIIt**), (**XIIu**), (**XIIv**), (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined in claim 5, and
R⁸⁵ is H, a C₁-C₂₅alkyl group, a group of formula (**XIIa**), (**XIIb**), (**XIIc**), (**XIId**), (**XIIe**), (**XIIf**), (**XIIg**), (**XIIj**), (**XIIk**), (**XIIn**), (**XIIo**), (**XIIq**), (**XIIs**), (**XIIt**), (**XIIu**), (**XIIv**) (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined in claim 5, or a group of formula (**XIVb**), (**XIVc**), (**XIVd**), (**XIVe**), (**XIVf**), (**XIVg**), (**XIVo**), (**XIVp**), (**XIVq**), (**XIVr**), (**XIVt**), (**XIVu**), (**XIVv**), (**XIVw**), (**XIVx**),(**XIVz**), (**XVa**), (**XVb**), (**XVc**), (**XVk**), (**XVl**), (**XVo**), (**XVp**), (**XVs**), (**XVw**), (**XVx**), (**XVy**), (**XVIa**), (**XVIb**), (**XVIc**) or (**XVId**) as defined in claim 6; or
a compound of formula (**Id**), wherein R⁸¹ is a group of formula (**XIIa**), (**XIIb**), (**XIIc**), (**XIId**), (**XIIe**), (**XIIf**), (**XIIg**), (**XIIj**), (**XIIk**), (**XIIn**), (**XIIo**), (**XIIq**), (**XIIs**), (**XIIt**), (**XIIu**), (**XIIv**), (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined in claim 5, and
R⁸⁵ is H, a group of formula (**XIIa**), (**XIIb**), (**XIIc**), (**XIId**), (**XIIe**), (**XIIf**), (**XIIg**), (**XIIj**), (**XIIk**), (**XIIn**), (**XIIo**), (**XIIq**), (**XIIs**), (**XIIt**), (**XIIu**), (**XIIv**) (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined in claim 5, or a group of formula (**XIVb**), (**XIVc**), (**XIVd**), (**XIVe**), (**XIVf**), (**XIVg**), (**XIVo**), (**XIVp**), (**XIVq**), (**XIVr**), (**XIVt**), (**XIVu**), (**XIVv**), (**XIVw**), (**XIVx**),(**XIVz**), (**XVa**), (**XVb**), (**XVc**), (**XVk**), (**XVl**), (**XVo**), (**XVp**), (**XVs**), (**XVw**), (**XVx**), (**XVy**), (**XVIa**), (**XVIb**), (**XVIc**) or (**XVId**) as defined in claim 6;
a compound of formula (**Ie**), wherein R⁸³ is a group of formula (**XIIa**), (**XIIb**), (**XIIc**), (**XIId**), (**XIIe**), (**XIIf**), (**XIIg**), (**XIIj**), (**XIIk**), (**XIIn**), (**XIIo**), (**XIIq**), (**XIIs**), (**XIIt**), (**XIIu**), (**XIIv**), (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined in claim 5, and
R⁸⁷ is H, a C₁-C₂₅alkyl group, a group of formula (**XIIa**), (**XIIb**), (**XIIc**), (**XIId**), (**XIIe**), (**XIIf**), (**XIIg**), (**XIIj**), (**XIIk**), (**XIIn**), (**XIIo**), (**XIIq**), (**XIIs**), (**XIIt**), (**XIIu**), (**XIIv**) (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined in claim 5, or a group of formula (**XIVb**), (**XIVc**), (**XIVd**), (**XIVe**), (**XIVf**), (**XIVg**), (**XIVo**), (**XIVp**), (**XIVq**), (**XIVr**), (**XIVt**), (**XIVu**), (**XIVv**), (**XIVw**), (**XIVx**),(**XIVz**), (**XVa**), (**XVb**), (**XVc**), (**XVk**), (**XVl**), (**XVo**), (**XVp**), (**XVs**), (**XVw**), (**XVx**), (**XVy**), (**XVIa**), (**XVIb**), (**XVIc**) or (**XVId**) as defined in claim 6; or
a compound of formula (**Ie**), wherein R⁸⁷ is a group of formula (**XIIa**), (**XIIb**), (**XIIc**), (**XIId**), (**XIIe**), (**XIIf**), (**XIIg**), (**XIIj**), (**XIIk**), (**XIIn**), (**XIIo**), (**XIIq**), (**XIIs**), (**XIIt**), (**XIIu**), (**XIIv**), (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined in claim 5, and
R⁸³ is H, a C₁-C₂₅alkyl group, a group of formula (**XIIa**), (**XIIb**), (**XIIc**), (**XIId**), (**XIIe**), (**XIIf**), (**XIIg**), (**XIIj**), (**XIIk**), (**XIIn**), (**XIIo**), (**XIIq**), (**XIIs**), (**XIIt**), (**XIIu**), (**XIIv**) (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined in claim 5, or a group of formula (**XIVb**), (**XIVc**), (**XIVd**), (**XIVe**), (**XIVf**), (**XIVg**), (**XIVo**), (**XIVp**), (**XIVq**), (**XIVr**), (**XIVt**), (**XIVu**), (**XIVv**), (**XIVw**), (**XIVx**),(**XIVz**), (**XVa**), (**XVb**), (**XVc**), (**XVk**), (**XVl**), (**XVo**), (**XVp**), (**XVs**), (**XVw**), (**XVx**), (**XVy**), (**XVIa**), (**XVIb**), (**XVIc**) or (**XVId**) as defined in claim 6;
a compound of formula (**If**), wherein R⁸³ is a group of formula (**XIIa**), (**XIIb**), (**XIIc**), (**XIId**), (**XIIe**), (**XIIf**), (**XIIg**), (**XIIj**), (**XIIk**), (**XIIn**), (**XIIo**), (**XIIq**), (**XIIs**), (**XIIt**), (**XIIu**), (**XIIv**), (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined in claim 5, and
R⁸⁵ is H, a group of formula (**XIIa**), (**XIIb**), (**XIIc**), (**XIId**), (**XIIe**), (**XIIf**), (**XIIg**), (**XIIj**), (**XIIk**), (**XIIn**), (**XIIo**), (**XIIq**), (**XIIs**), (**XIIt**), (**XIIu**), (**XIIv**) (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined in claim 5, or a group of formula (**XIVb**), (**XIVc**), (**XIVd**), (**XIVe**), (**XIVf**), (**XIVg**), (**XIVo**), (**XIVp**), (**XIVq**), (**XIVr**), (**XIVt**), (**XIVu**), (**XIVv**), (**XIVw**), (**XIVx**),(**XIVz**), (**XVa**), (**XVb**), (**XVc**), (**XVk**), (**XVl**), (**XVo**), (**XVp**), (**XVs**), (**XVw**), (**XVx**), (**XVy**), (**XVIa**), (**XVIb**), (**XVIc**) or (**XVId**) as defined in claim 6; or
a compound of formula (**If**), wherein R⁸⁵ is a group of formula (**XIIa**), (**XIIb**), (**XIIc**), (**XIId**), (**XIIe**), (**XIIf**), (**XIIg**), (**XIIj**), (**XIIk**), (**XIIn**), (**XIIo**), (**XIIq**), (**XIIs**), (**XIIt**), (**XIIu**), (**XIIv**), (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined in claim 5, and
R⁸³ is H, a group of formula (**XIIa**), (**XIIb**), (**XIIc**), (**XIId**), (**XIIe**), (**XIIf**), (**XIIg**), (**XIIj**), (**XIIk**), (**XIIn**), (**XIIo**), (**XIIq**), (**XIIs**), (**XIIt**), (**XIIu**), (**XIIv**) (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined in claim 5, or a group of formula (**XIVb**), (**XIVc**), (**XIVd**), (**XIVe**), (**XIVf**), (**XIVg**), (**XIVo**), (**XIVp**), (**XIVq**), (**XIVr**), (**XIVt**), (**XIVu**), (**XIVv**), (**XIVw**), (**XIVx**),(**XIVz**), (**XVa**), (**XVb**), (**XVc**), (**XVk**), (**XVl**), (**XVo**), (**XVp**), (**XVs**), (**XVw**), (**XVx**), (**XVy**), (**XVIa**), (**XVIb**), (**XVIc**) or (**XVId**) as defined in claim 6; or
a compound of formula (**Ij**), wherein R⁸³ is a group of formula (**XIIa**), (**XIIb**), (**XIIc**), (**XIId**), (**XIIe**), (**XIIf**), (**XIIg**), (**XIIj**), (**XIIk**), (**XIIn**), (**XIIo**), (**XIIq**), (**XIIs**), (**XIIt**), (**XIIu**), (**XIIv**), (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined in claim 5, and
R⁸⁵ is H, a C₁-C₂₅alkyl group, a group of formula (**XIIa**), (**XIIb**), (**XIIc**), (**XIId**), (**XIIe**), (**XIIf**), (**XIIg**), (**XIIj**), (**XIIk**), (**XIIn**), (**XIIo**), (**XIIq**), (**XIIs**), (**XIIt**), (**XIIu**), (**XIIv**) (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined in claim 5, or a group of formula (**XIVb**), (**XIVc**), (**XIVd**), (**XIVe**), (**XIVf**), (**XIVg**), (**XIVo**), (**XIVp**), (**XIVq**), (**XIVr**), (**XIVt**), (**XIVu**), (**XIVv**), (**XIVw**), (**XIVx**),(**XIVz**), (**XVa**), (**XVb**), (**XVc**), (**XVk**), (**XVl**), (**XVo**), (**XVp**), (**XVs**), (**XVw**), (**XVx**), (**XVy**), (**XVIa**), (**XVIb**), (**XVIc**) or (**XVId**) as defined in claim 6; or
a compound of formula (**Ij**), wherein R⁸⁵ is a group of formula (**XIIa**), (**XIIb**), (**XIIc**), (**XIId**), (**XIIe**), (**XIIf**), (**XIIg**), (**XIIj**), (**XIIk**), (**XIIn**), (**XIIo**), (**XIIq**), (**XIIs**), (**XIIt**), (**XIIu**), (**XIIv**), (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined in claim 5, and
R⁸³ is H, a C₁-C₂₅alkyl group, a group of formula (**XIIa**), (**XIIb**), (**XIIc**), (**XIId**), (**XIIe**), (**XIIf**), (**XIIg**), (**XIIj**), (**XIIk**), (**XIIn**), (**XIIo**), (**XIIq**), (**XIIs**), (**XIIt**), (**XIIu**), (**XIIv**) (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined in claim 5, or a group of formula (**XIVb**), (**XIVc**), (**XIVd**), (**XIVe**), (**XIVf**), (**XIVg**), (**XIVo**), (**XIVp**), (**XIVq**), (**XIVr**), (**XIVt**), (**XIVu**), (**XIVv**), (**XIVw**), (**XIVx**),(**XIVz**), (**XVa**), (**XVb**), (**XVc**), (**XVk**), (**XVl**), (**XVo**), (**XVp**), (**XVs**), (**XVw**), (**XVx**), (**XVy**), (**XVIa**), (**XVIb**), (**XVIc**) or (**XVId**) as defined in claim 6; or
a compound of formula (**Im**), wherein R⁸³ is a group of formula (**XIIa**), (**XIIb**), (**XIIc**), (**XIId**), (**XIIe**), (**XIIf**), (**XIIg**), (**XIIj**), (**XIIk**), (**XIIn**), (**XIIo**), (**XIIq**), (**XIIs**), (**XIIt**), (**XIIu**), (**XIIv**), (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined in claim 5, and
R⁸⁵ is H, a C₁-C₂₅alkyl group, a group of formula (**XIIa**), (**XIIb**), (**XIIc**), (**XIId**), (**XIIe**), (**XIIf**), (**XIIg**), (**XIIj**), (**XIIk**), (**XIIn**), (**XIIo**), (**XIIq**), (**XIIs**), (**XIIt**), (**XIIu**), (**XIIv**) (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined in claim 5, or a group of formula (**XIVb**), (**XIVc**), (**XIVd**), (**XIVe**), (**XIVf**), (**XIVg**), (**XIVo**), (**XIVp**), (**XIVq**), (**XIVr**), (**XIVt**), (**XIVu**), (**XIVv**), (**XIVw**), (**XIVx**),(**XIVz**), (**XVa**), (**XVb**), (**XVc**), (**XVk**), (**XVl**), (**XVo**), (**XVp**), (**XVs**), (**XVw**), (**XVx**), (**XVy**), (**XVIa**), (**XVIb**), (**XVIc**) or (**XVId**) as defined in claim 6; or
a compound of formula (**Im**), wherein R⁸⁷ is a group of formula (**XIIa**), (**XIIb**), (**XIIc**), (**XIId**), (**XIIe**), (**XIIf**), (**XIIg**), (**XIIj**), (**XIIk**), (**XIIn**), (**XIIo**), (**XIIq**), (**XIIs**), (**XIIt**), (**XIIu**), (**XIIv**), (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined in claim 5, and
R⁸³ is H, a C₁-C₂₅alkyl group, a group of formula (**XIIa**), (**XIIb**), (**XIIc**), (**XIId**), (**XIIe**), (**XIIf**), (**XIIg**), (**XIIj**), (**XIIk**), (**XIIn**), (**XIIo**), (**XIIq**), (**XIIs**), (**XIIt**), (**XIIu**), (**XIIv**) (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined in claim 5, or a group of formula (**XIVb**), (**XIVc**), (**XIVd**), (**XIVe**), (**XIVf**), (**XIVg**), (**XIVo**), (**XIVp**), (**XIVq**), (**XIVr**), (**XIVt**), (**XIVu**), (**XIVv**), (**XIVw**), (**XIVx**),(**XIVz**), (**XVa**), (**XVb**), (**XVc**), (**XVk**), (**XVl**), (**XVo**), (**XVp**), (**XVs**), (**XVw**), (**XVx**), (**XVy**), (**XVIa**), (**XVIb**), (**XVIc**) or (**XVId**) as defined in claim 6; or
a compound of formula (**In**), wherein R⁸³ is a group of formula (**XIIa**), (**XIIb**), (**XIIc**), (**XIId**), (**XIIe**), (**XIIf**), (**XIIg**), (**XIIj**), (**XIIk**), (**XIIn**), (**XIIo**), (**XIIq**), (**XIIs**), (**XIIt**), (**XIIu**), (**XIIv**), (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined in claim 5, and
R⁸⁷ is H, a group of formula (**XIIa**), (**XIIb**), (**XIIc**), (**XIId**), (**XIIe**), (**XIIf**), (**XIIg**), (**XIIj**), (**XIIk**), (**XIIn**), (**XIIo**), (**XIIq**), (**XIIs**), (**XIIt**), (**XIIu**), (**XIIv**) (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined in claim 5, or a group of formula (**XIVb**), (**XIVc**), (**XIVd**), (**XIVe**), (**XIVf**), (**XIVg**), (**XIVo**), (**XIVp**), (**XIVq**), (**XIVr**), (**XIVt**), (**XIVu**), (**XIVv**), (**XIVw**), (**XIVx**),(**XIVz**), (**XVa**), (**XVb**), (**XVc**), (**XVk**), (**XVl**), (**XVo**), (**XVp**), (**XVs**), (**XVw**), (**XVx**), (**XVy**), (**XVIa**), (**XVIb**), (**XVIc**) or (**XVId**) as defined in claim 6.

9. The compound according to any of claims 1 to 8, which is
a compound of formula (**Ia**), wherein R⁸³ is a group of formula (**XIIa**), (**XIIb**), (**XIIc**), (**XIId**), (**XIIe**), (**XIIf**), (**XIIg**), (**XIIj**), (**XIIk**), (**XIIn**), (**XIIo**), (**XIIq**), (**XIIs**), (**XIIt**), (**XIIu**), (**XIIv**), (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined in claim 5, and R⁸⁷ is H, or a C₁-C₂₅alkyl group; or
a compound of formula (**Im**), wherein R⁸⁷ is a group of formula (**XIIa**), (**XIIb**), (**XIIc**), (**XIId**), (**XIIe**), (**XIIf**), (**XIIg**), (**XIIj**), (**XIIk**), (**XIIn**), (**XIIo**), (**XIIq**), (**XIIs**), (**XIIt**), (**XIIu**), (**XIIv**), (**XIIw**), (**XIIx**), (**XIIy**), (**XIIz**), (**XIIIa**), (**XIIIb**), (**XIIIc**), (**XIIId**), (**XIIIe**), (**XIIIf**) or (**XIIIg**) as defined in claim 5, and R⁸³ is H, a C₁-C₂₅alkyl group.

10. An electronic device, comprising a compound according to any of claims 1 to 9.

11. The electronic device according to claim 10, which is an electroluminescent device.

12. A hole transport layer, electron transport layer, hole blocking layer, electron blocking layer, or an emitting layer comprising a compound according to any of claims 1 to 9.

13. The emitting layer according to claim 12, comprising a compound according to any of claims 1 to 9 as host material in combination with a phosphorescent emitter.

14. An apparatus selected from the group consisting of stationary visual display units; mobile visual display units; illumination units; keyboards; items of clothing; furniture; wallpaper, comprising the organic electronic device according to claim 10, or 11, or the hole transport layer, or the emitting layer according to claim 12.

15. Use of the compounds of formula I according to any of claims 1 to 9 for electrophotographic photoreceptors, photoelectric converters, organic solar cells, switching elements, organic light emitting field effect transistors, image sensors, dye lasers and electroluminescent devices.

## Patentansprüche

1. Verbindung mit der Formel 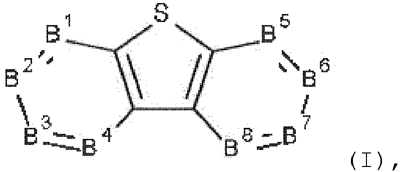 wobei
B¹ N oder CR⁸¹ ist,
B² N oder CR⁸² ist,
B³ N oder CR⁸³ ist,
B⁴ N oder CR⁸⁴ ist,
B⁵ N oder CR⁸⁵ ist,
B⁶ N oder CR⁸⁶ ist,
B⁷ N oder CR⁸⁷ ist,
B⁸ N oder CR⁸⁸ ist,
R⁸¹, R⁸², R⁸³, R⁸⁴, R⁸⁵, R⁸⁶, R⁸⁷ und R⁸⁸ unabhängig voneinander Folgendes sind: H, F, eine Cyanogruppe, eine C₁-C₂₅-Akylgruppe, die optional durch E substituiert oder durch D unterbrochen werden kann; eine C₁-C₂₅-Alkoxygruppe; eine C₃-C₁₈-Cycloalkylgruppe, die optional durch E substituiert werden kann; eine C₆-C₂₄-Arylgruppe, die optional durch G substituiert werden kann, eine C₂-C₃₀-Heteroarylgruppe, die optional durch G substituiert werden kann; oder eine Gruppe mit der Formel -(A¹)ₒ-(A²)ₚ-(A³)_{q}-(A⁴)ᵣ-R¹⁶,
wobei o 0 oder 1 ist, p 0 oder 1 ist, q 0 oder 1 ist, r 0 oder 1 ist,
A¹, A², A³ und A⁴ unabhängig voneinander eine C₆-C₂₄-Arylengruppe, die optional durch G substituiert werden kann, oder eine C₂-C₃₀-Heteroarylengruppe, die optional durch G substituiert werden kann, sind;
R¹⁶ -NR¹⁰R¹¹ oder -Si(R¹²)(R¹³)(R¹⁴), eine C₆-C₂₄-Arylgruppe, die optional durch G substituiert werden kann, oder eine C₂-C₃₀-Heteroarylgruppe, die optional durch G substituiert werden kann, ist;
R¹⁰ und R¹¹ unabhängig voneinander eine C₆-C₂₄-Arylgruppe, die optional durch G substituiert werden kann; oder eine C₂-C₃₀-Heteroarylgruppe, die optional durch G substituiert werden kann, sind;
R¹², R¹³ und R¹⁴ unabhängig voneinander Folgendes sind: eine C₁-C₂₅-Alkylgruppe, die optional durch E substituiert oder durch D unterbrochen werden kann; eine C₆-C₂₄-Arylgruppe, die optional durch G substituiert werden kann; oder eine C₂-C₃₀-Heteroarylgruppe, die optional durch G substituiert werden kann;
D -CO-, -COO-, -S-, -SO-, -SO₂-, -O-, -NR⁶⁵-, -SiR⁷⁰R⁷¹-, -POR⁷²-, -CR⁶³=CR⁶⁴- oder -C≡C- ist,
E -OR⁶⁹, -SR⁶⁹, -NR⁶⁵R⁶⁶, -COR⁶⁸, -COOR⁶⁷, -CONR⁶⁵R⁶⁶, -CN oder F ist,
G Folgendes ist: E oder eine C₁-C₁₈-Alkylgruppe, eine C₃-C₁₈-Cycloalkylgruppe, eine C₆-C₂₄-Arylgruppe, eine C₆-C₂₄-Arylgruppe, die durch F, C₁-C₁₈-Alkyl oder C₁-C₁₈-Alkyl, das durch O unterbrochen wird, substituiert wird; eine C₂-C₃₀-Heteroarylgruppe oder eine C₂-C₃₀-Heteroarylgruppe, die durch F, C₁-C₁₈-Alkyl oder C₁-C₁₈-Alkyl, das durch O unterbrochen wird, substituiert wird;
R⁶³ und R⁶⁴ unabhängig voneinander Folgendes sind: H, C₆-C₁₈-Aryl; C₆-C₁₈-Aryl, das durch C₁-C₁₈-Alkyl oder C₁-C₁₈-Alkoxy substituiert wird; C₁-C₁₈-Alkyl; oder C₁-C₁₈-Alkyl, das durch -O- unterbrochen wird;
R⁶⁵ und R⁶⁶ unabhängig voneinander Folgendes sind: eine C₆-C₁₈-Arylgruppe; ein C₆-C₁₈-Aryl, das durch C₁-C₁₈-Alkyl oder C₁-C₁₈-Alkoxy substituiert wird; eine C₁-C₁₈-Alkylgruppe; oder eine C₁-C₁₈-Alkylgruppe, die durch -O-unterbrochen wird; oder
R⁶⁵ und R⁶⁶ zusammen einen Ring mit fünf oder sechs Gliedern bilden,
R⁶⁷ Folgendes ist: eine C₆-C₁₈-Arylgruppe; eine C₆-C₁₈-Arylgruppe, die durch C₁-C₁₈-Alkyl oder C₁-C₁₈-Alkoxy substituiert wird; eine C₁-C₁₈-Alkylgruppe; oder eine C₁-C₁₈-Alkylgruppe, die durch -O- unterbrochen wird,
R⁶⁸ Folgendes ist: H; eine C₆-C₁₈-Arylgruppe; eine C₆-C₁₈-Arylgruppe, die durch C₁-C₁₈-Alkyl oder C₁-C₁₈-Alkoxy substituiert wird; eine C₁-C₁₈-Alkylgruppe; oder eine C₁-C₁₈-Alkylgruppe, die durch -O- unterbrochen wird;
R⁶⁹ Folgendes ist: ein C₆-C₁₈-Aryl; ein C₆-C₁₈-Aryl, das durch C₁-C₁₈-Alkyl oder C₁-C₁₈-Alkoxy substituiert wird;
eine C₁-C₁₈-Alkylgruppe; oder eine C₁-C₁₈-Alkylgruppe, die durch -O- unterbrochen wird;
R⁷⁰ und R⁷¹ unabhängig voneinander Folgendes sind: eine C₁-C₁₈-Alkylgruppe, eine C₆-C₁₈-Arylgruppe oder eine C₆-C₁₈-Arylgruppe, die durch C₁-C₁₈-Alkyl substituiert wird, und
R⁷² Folgendes ist: eine C₁-C₁₈-Alkylgruppe, eine C₆-C₁₈-Arylgruppe oder eine C₆-C₁₈-Arylgruppe, die durch C₁-C₁₈-Alkyl substituiert wird, mit der Bedingung, dass wenigstens einer der Substituenten B¹, B², B³, B⁴, B⁵, B⁶, B⁷ und B⁸ N repräsentiert;
nicht mehr als zwei der Gruppen B¹, B², B³ und B⁴ N repräsentieren; und
nicht mehr als zwei der Gruppen B⁵, B⁶, B⁷ und B⁸ N repräsentieren; und
mit der weiteren Bedingung, dass wenigstens einer der Substituenten R⁸¹, R⁸², R⁸³, R⁸⁴, R⁸⁵, R⁸⁶, R⁸⁷ und R⁸⁸ eine Gruppe mit der Formel - (A¹)ₒ-(A²)ₚ-(A³)_{q}-(A⁴)ᵣ-R¹⁶ repräsentiert, wobei R¹⁶ eine Benzimidazo[1,2-a]benzimidazo-5-yl-Gruppe repräsentiert, die optional durch G substituiert werden kann; und/oder wenigstens eine der Gruppen A¹, A², A³ und A⁴ eine Benzimidazo[1,2-a]benzimidazo-2,5-ylen-Gruppe repräsentiert, die optional durch G substituiert werden kann.

2. Verbindung nach Anspruch 1, die eine Verbindung mit folgender Formel ist: 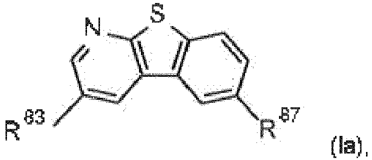 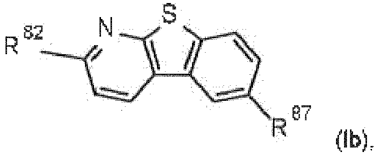 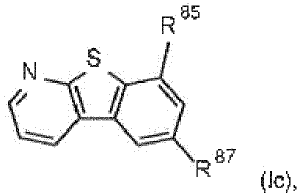 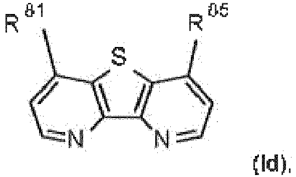 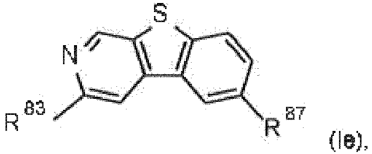 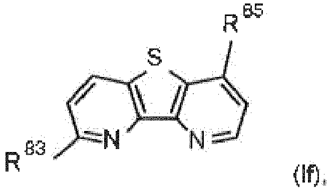 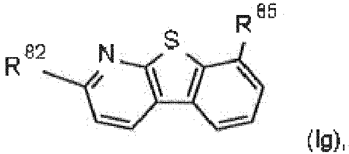 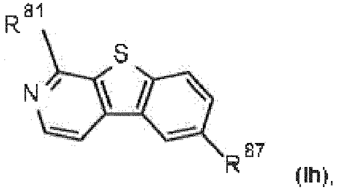 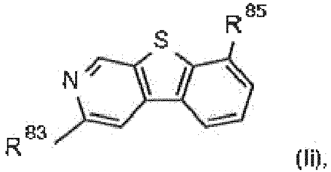 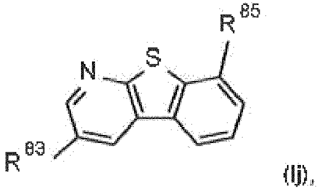 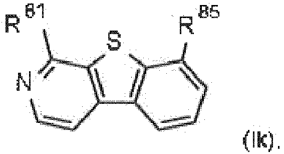 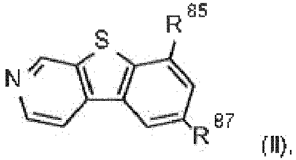 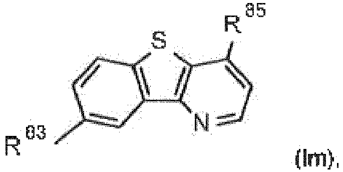 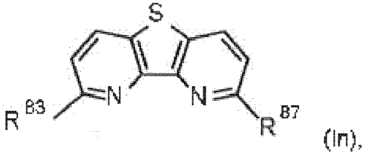 oder 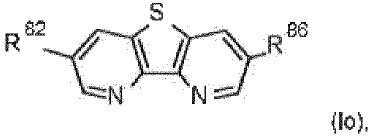 wobei R⁸¹, R⁸², R⁸³, R⁸⁵, R⁸⁶ und R⁸⁷ wie in Anspruch 1 definiert sind.

3. Verbindung nach Anspruch 2, wobei
bei den Verbindungen mit der Formel (Ia)
R⁸³ eine Gruppe mit der Formel - (A¹)ₒ- (A²)ₚ-(A³)_{q}-(A⁴)ᵣ-R¹⁶ ist; und
R⁸⁷ H, eine C₁-C₂₅-Alkylgruppe oder eine Gruppe mit der Formel - (A¹)ₒ-(A²)ₚ-(A³)_{q}-(A⁴)ᵣ-R^{16'} ist; oder
R⁸³ H, eine C₁-C₂₅-Alkylgruppe oder eine Gruppe mit der Formel - (A¹)ₒ-(A²)ₚ-(A³)_{q}-(A⁴)ᵣ-R^{16'} ist; und
R⁸⁷ eine Gruppe mit der Formel - (A¹)ₒ-(A²)ₚ-(A³)_{q}-(A⁴)ᵣ-R¹⁶ ist;
bei den Verbindungen mit der Formel (Ib)
R⁸² eine Gruppe mit der Formel - (A¹)ₒ-(A²)ₚ-(A³)_{q}-(A⁴)ᵣ-R¹⁶ ist; und
R⁸⁷ H, eine C₁-C₂₅-Alkylgruppe oder eine Gruppe mit der Formel - (A¹)ₒ-(A²)ₚ-(A³)_{q}-(A⁴)ᵣ-R^{16'} ist; oder
R⁸² H, eine C₁-C₂₅-Alkylgruppe oder eine Gruppe mit der Formel - (A¹)ₒ-(A²)ₚ-(A³)_{q}-(A⁴)ᵣ-R^{16'} ist; und
R⁸⁷ eine Gruppe mit der Formel - (A¹)ₒ-(A²)ₚ-(A³)_{q}-(A⁴)ᵣ-R¹⁶ ist;
bei den Verbindungen mit der Formel (Ic)
R⁸⁵ eine Gruppe mit der Formel - (A¹)ₒ-(A²)ₚ-(A³)_{q}-(A⁴)ᵣ-R¹⁶ ist; und
R⁸⁷ H, eine C₁-C₂₅-Alkylgruppe oder eine Gruppe mit der Formel - (A¹)ₒ-(A²)ₚ-(A³)_{q}-(A⁴)ᵣ-R^{16'} ist; oder
R⁸⁵ H, eine C₁-C₂₅-Alkylgruppe oder eine Gruppe mit der Formel - (A¹)ₒ-(A²)ₚ-(A³)_{q}-(A⁴)ᵣ-R^{16'} ist; und
R⁸⁷ eine Gruppe mit der Formel - (A¹)ₒ-(A²)ₚ-(A³)_{q}-(A⁴)ᵣ-R¹⁶ ist;
bei den Verbindungen mit der Formel (Id)
R⁸¹ eine Gruppe mit der Formel - (A¹)ₒ-(A²)ₚ-(A³)_{q}-(A⁴)ᵣ-R¹⁶ ist; und
R⁸⁵ H, eine C₁-C₂₅-Alkylgruppe oder eine Gruppe mit der Formel - (A¹)ₒ-(A²)ₚ-(A³)_{q}-(A⁴)ᵣ-R^{16'} ist; oder
R⁸¹ H, eine C₁-C₂₅-Alkylgruppe oder eine Gruppe mit der Formel - (A¹)ₒ-(A²)ₚ-(A³)_{q}-(A⁴)ᵣ-R^{16'} ist; und
R⁸⁵ eine Gruppe mit der Formel - (A¹)ₒ-(A²)ₚ-(A³)_{q}-(A⁴)ᵣ-R¹⁶ ist;
bei den Verbindungen mit der Formel (Ie)
R⁸³ eine Gruppe mit der Formel - (A¹)ₒ-(A²)ₚ-(A³)_{q}-(A⁴)ᵣ-R¹⁶ ist; und
R⁸⁷ H, eine C₁-C₂₅-Alkylgruppe oder eine Gruppe mit der Formel - (A¹)ₒ-(A²)ₚ-(A³)_{q}-(A⁴)ᵣ-R^{16'} ist; oder
R⁸³ H, eine C₁-C₂₅-Alkylgruppe oder eine Gruppe mit der Formel - (A¹)ₒ-(A²)ₚ-(A³)_{q}-(A⁴)ᵣ-R^{16'} ist; und
R⁸⁷ eine Gruppe mit der Formel - (A¹)ₒ-(A²)ₚ-(A³)_{q}-(A⁴)ᵣ-R¹⁶ ist;
bei den Verbindungen mit der Formel (If)
R⁸³ eine Gruppe mit der Formel - (A¹)ₒ-(A²)ₚ-(A³)_{q}-(A⁴)ᵣ-R¹⁶ ist; und
R⁸⁵ H, eine C₁-C₂₅-Alkylgruppe oder eine Gruppe mit der Formel - (A¹)ₒ-(A²)ₚ-(A³)_{q}-(A⁴)ᵣ-R^{16'} ist; oder
R⁸³ H, eine C₁-C₂₅-Alkylgruppe oder eine Gruppe mit der Formel - (A¹)ₒ-(A²)ₚ-(A³)_{q}-(A⁴)ᵣ-R^{16'} ist; und
R⁸⁵ eine Gruppe mit der Formel - (A¹)ₒ-(A²)ₚ-(A³)_{q}-(A⁴)ᵣ-R¹⁶ ist;
bei den Verbindungen mit der Formel (Ig)
R⁸² eine Gruppe mit der Formel - (A¹)ₒ-(A²)ₚ-(A³)_{q}-(A⁴)ᵣ-R¹⁶ ist; und
R⁸⁵ H, eine C₁-C₂₅-Alkylgruppe oder eine Gruppe mit der Formel - (A¹)ₒ-(A²)ₚ-(A³)_{q}-(A⁴)ᵣ-R^{16'} ist; oder
R⁸² H, eine C₁-C₂₅-Alkylgruppe oder eine Gruppe mit der Formel - (A¹)ₒ-(A²)ₚ-(A³)_{q}-(A⁴)ᵣ-R^{16'} ist; und
R⁸⁵ eine Gruppe mit der Formel - (A¹)ₒ-(A²)ₚ-(A³)_{q}-(A⁴)ᵣ-R¹⁶ ist;
bei den Verbindungen mit der Formel (Ih)
R⁸¹ eine Gruppe mit der Formel - (A¹)ₒ-(A²)ₚ-(A³)_{q}-(A⁴)ᵣ-R¹⁶ ist; und
R⁸⁷ H, eine C₁-C₂₅-Alkylgruppe oder eine Gruppe mit der Formel - (A¹)ₒ-(A²)ₚ-(A³)_{q}-(A⁴)ᵣ-R^{16'} ist; oder
R⁸¹ H, eine C₁-C₂₅-Alkylgruppe oder eine Gruppe mit der Formel - (A¹)ₒ-(A²)ₚ-(A³)_{q}-(A⁴)ᵣ-R^{16'} ist; und
R⁸⁷ eine Gruppe mit der Formel - (A¹)ₒ-(A²)ₚ-(A³)_{q}-(A⁴)ᵣ-R¹⁶ ist;
bei den Verbindungen mit der Formel (Ii)
R⁸³ eine Gruppe mit der Formel - (A¹)ₒ-(A²)ₚ-(A³)_{q}-(A⁴)ᵣ-R¹⁶ ist; und
R⁸⁵ H, eine C₁-C₂₅-Alkylgruppe oder eine Gruppe mit der Formel - (A¹)ₒ-(A²)ₚ-(A³)_{q}-(A⁴)ᵣ-R^{16'} ist; oder
R⁸³ H, eine C₁-C₂₅-Alkylgruppe oder eine Gruppe mit der Formel - (A¹)ₒ-(A²)ₚ-(A³)_{q}-(A⁴)ᵣ-R^{16'} ist; und
R⁸⁵ eine Gruppe mit der Formel - (A¹)ₒ-(A²)ₚ-(A³)_{q}-(A⁴)ᵣ-R¹⁶ ist;
bei den Verbindungen mit der Formel (Ij)
R⁸³ eine Gruppe mit der Formel - (A¹)ₒ-(A²)ₚ-(A³)_{q}-(A⁴)ᵣ-R¹⁶ ist; und
R⁸⁵ H, eine C₁-C₂₅-Alkylgruppe oder eine Gruppe mit der Formel - (A¹)ₒ-(A²)ₚ-(A³)_{q}-(A⁴)ᵣ-R^{16'} ist; oder
R⁸³ H, eine C₁-C₂₅-Alkylgruppe oder eine Gruppe mit der Formel - (A¹)ₒ-(A²)ₚ-(A³)_{q}-(A⁴)ᵣ-R^{16'} ist; und
R⁸⁵ eine Gruppe mit der Formel - (A¹)ₒ-(A²)ₚ-(A³)_{q}-(A⁴)ᵣ-R¹⁶ ist;
bei den Verbindungen mit der Formel (Ik)
R⁸¹ eine Gruppe mit der Formel - (A¹)ₒ-(A²)ₚ-(A³)_{q}-(A⁴)ᵣ-R¹⁶ ist; und
R⁸⁵ H, eine C₁-C₂₅-Alkylgruppe oder eine Gruppe mit der Formel - (A¹)ₒ-(A²)ₚ-(A³)_{q}-(A⁴)ᵣ-R^{16'} ist; oder
R⁸¹ H, eine C₁-C₂₅-Alkylgruppe oder eine Gruppe mit der Formel - (A¹)ₒ-(A²)ₚ-(A³)_{q}-(A⁴)ᵣ-R^{16'} ist; und
R⁸⁵ eine Gruppe mit der Formel - (A¹)ₒ-(A²)ₚ-(A³)_{q}-(A⁴)ᵣ-R¹⁶ ist;
bei den Verbindungen mit der Formel (Il)
R⁸⁵ eine Gruppe mit der Formel - (A¹)ₒ-(A²)ₚ-(A³)_{q}-(A⁴)ᵣ-R¹⁶ ist; und
R⁸⁷ H, eine C₁-C₂₅-Alkylgruppe oder eine Gruppe mit der Formel - (A¹)ₒ-(A²)ₚ-(A³)_{q}-(A⁴)ᵣ-R^{16'} ist; oder
R⁸⁵ H, eine C₁-C₂₅-Alkylgruppe oder eine Gruppe mit der Formel - (A¹)ₒ-(A²)ₚ-(A³)_{q}-(A⁴)ᵣ-R^{16'} ist; und
R⁸⁷ eine Gruppe mit der Formel - (A¹)ₒ-(A²)ₚ-(A³)_{q}-(A⁴)ᵣ-R¹⁶ ist;
bei den Verbindungen mit der Formel (Im)
R⁸³ eine Gruppe mit der Formel - (A¹)ₒ-(A²)ₚ-(A³)_{q}-(A⁴)ᵣ-R¹⁶ ist; und
R⁸⁵ H, eine C₁-C₂₅-Alkylgruppe oder eine Gruppe mit der Formel - (A¹)ₒ-(A²)ₚ-(A³)_{q}-(A⁴)ᵣ-R^{16'} ist; oder
R⁸³ H, eine C₁-C₂₅-Alkylgruppe oder eine Gruppe mit der Formel - (A¹)ₒ-(A²)ₚ-(A³)_{q}-(A⁴)ᵣ-R^{16'} ist; und
R⁸⁵ eine Gruppe mit der Formel - (A¹)ₒ-(A²)ₚ-(A³)_{q}-(A⁴)ᵣ-R¹⁶ ist;
bei den Verbindungen mit der Formel (In)
R⁸³ eine Gruppe mit der Formel - (A¹)ₒ-(A²)ₚ-(A³)_{q}-(A⁴)ᵣ-R¹⁶ ist; und
R⁸⁷ H, eine C₁-C₂₅-Alkylgruppe oder eine Gruppe mit der Formel - (A¹)ₒ-(A²)ₚ-(A³)_{q}-(A⁴)ᵣ-R^{16'} ist; oder
R⁸³ H, eine C₁-C₂₅-Alkylgruppe oder eine Gruppe mit der Formel - (A¹)ₒ-(A²)ₚ-(A³)_{q}-(A⁴)ᵣ-R^{16'} ist; und
R⁸⁷ eine Gruppe mit der Formel - (A¹)ₒ-(A²)ₚ-(A³)_{q}-(A⁴)ᵣ-R¹⁶ ist;
bei den Verbindungen mit der Formel (Io)
R⁸² eine Gruppe mit der Formel - (A¹)ₒ-(A²)ₚ-(A³)_{q}-(A⁴)ᵣ-R¹⁶ ist; und
R⁸⁶ H, eine C₁-C₂₅-Alkylgruppe oder eine Gruppe mit der Formel - (A¹)ₒ-(A²)ₚ-(A³)_{q}-(A⁴)ᵣ-R^{16'} ist; oder
R⁸² H, eine C₁-C₂₅-Alkylgruppe oder eine Gruppe mit der Formel - (A¹)ₒ-(A²)ₚ-(A³)_{q}-(A⁴)ᵣ-R^{16'} ist; und
R⁸⁶ eine Gruppe mit der Formel - (A¹)ₒ-(A²)ₚ-(A³)_{q}-(A⁴)ᵣ-R¹⁶ ist; wobei
∘ 0 oder 1 ist, p 0 oder 1 ist, q 0 oder 1 ist, r 0 oder 1 ist;
A¹, A², A³ und A⁴ unabhängig voneinander eine Gruppe mit folgender Formel sind: 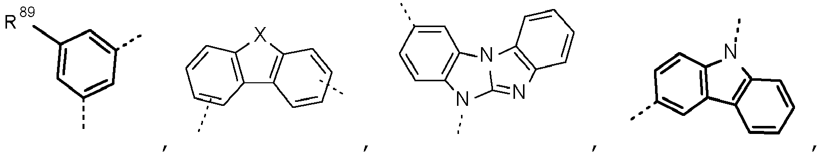 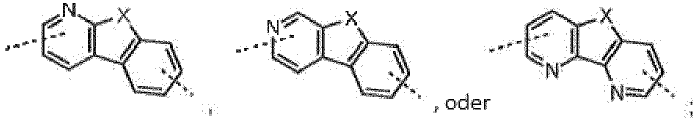 R¹⁶ eine Gruppe mit der Formel 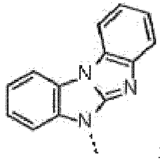 ist oder R¹⁶ die Bedeutung von R^{16'} aufweist, falls wenigstens eine der Gruppen A¹, A², A³ und A⁴ eine Gruppe mit der Formel 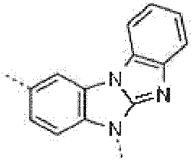 repräsentiert;
R^{16'} H oder eine Gruppe mit der Formel -Si (R¹²) (R¹³) (R¹⁴) ist, 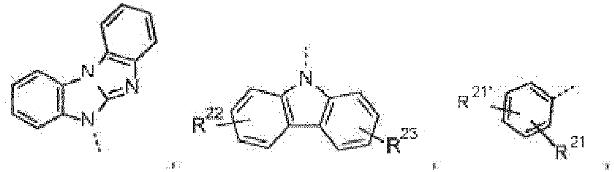 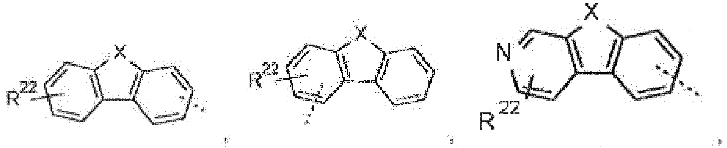 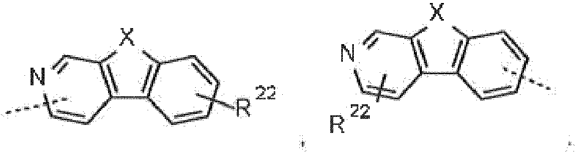 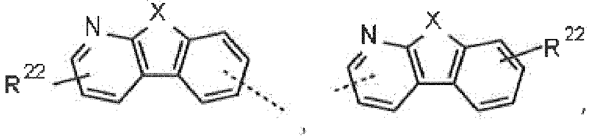 oder 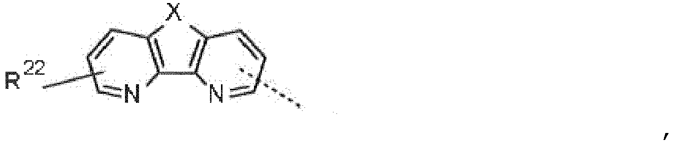 wobei
R¹², R¹³ und R¹⁴ unabhängig voneinander eine Phenylgruppe sind, die optional durch eine oder mehr Alkylgruppen substituiert werden kann;
R²¹ und R^{21'} unabhängig voneinander H, eine Phenylgruppe oder eine C₁-C₁₈-Alkylgruppe sind;
R²² und R²³ unabhängig voneinander H oder eine Gruppe mit der folgenden Formel sind: 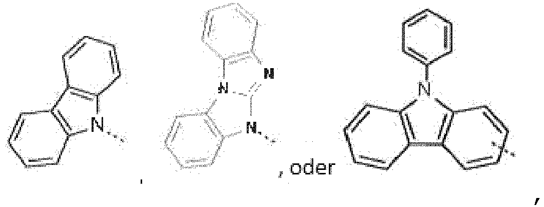
X O, S oder NR²⁴ ist,
R²⁴ eine C₆-C₂₄-Arylgruppe oder eine C₂-C₃₀-Heteroarylgruppe ist, die optional durch G substituiert werden kann, wobei G wie in Anspruch 1 definiert ist; und
R⁸⁹ H, eine Gruppe mit folgender Formel ist:

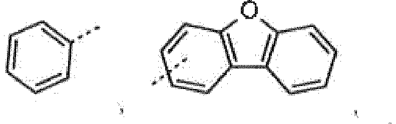

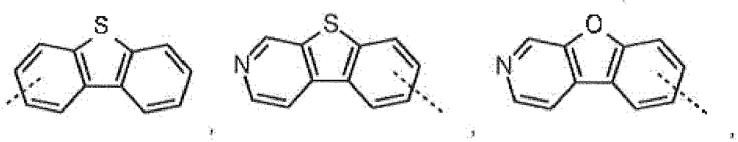

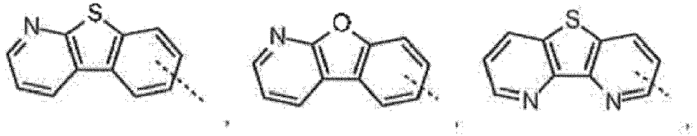

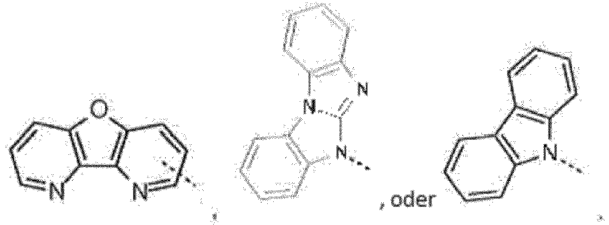

4. Verbindung nach Anspruch 2 oder 3, wobei
∘ 0 oder 1 ist, p 0 oder 1 ist, q 0 oder 1 ist, r 0 oder 1 ist,
A¹, A², A³ und A⁴ unabhängig voneinander eine Gruppe mit der folgenden Formel sind: 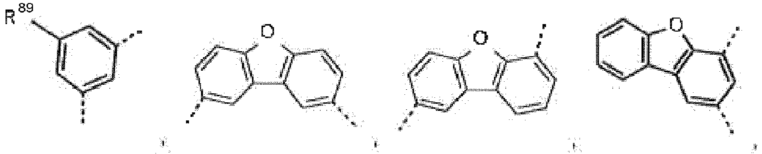 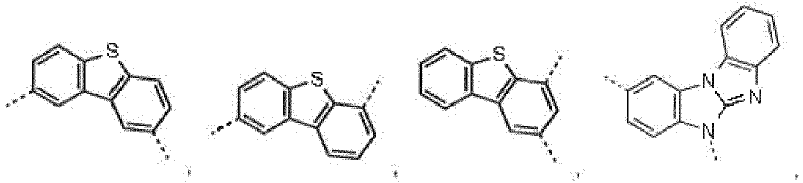 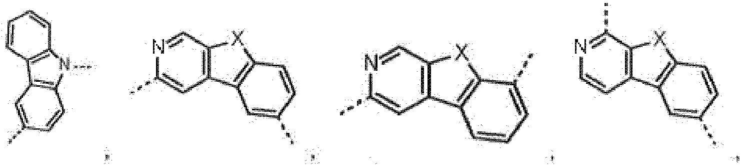 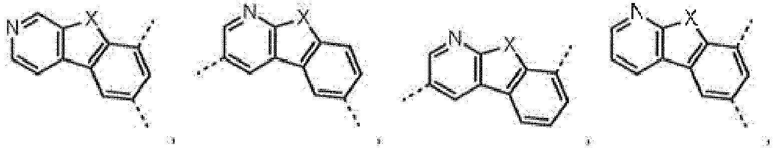 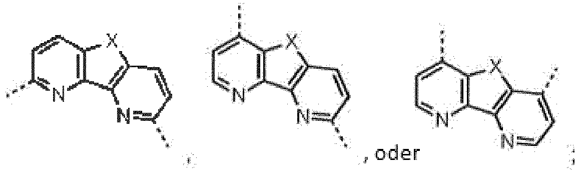 R¹⁶ eine Gruppe mit der Formel 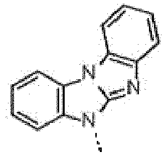 ist; oder R¹⁶ die Bedeutung von R^{16'} aufweist, falls wenigstens eine der Gruppen A¹, A², A³ und A⁴ eine Gruppe mit folgender Formel 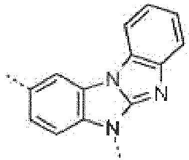 repräsentiert;
R^{16'} H oder eine Gruppe mit der folgenden Formel ist: 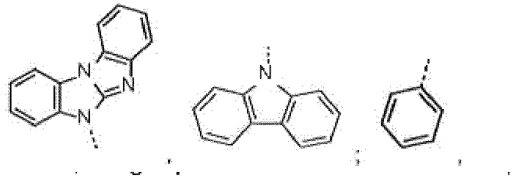 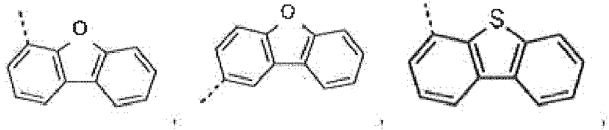 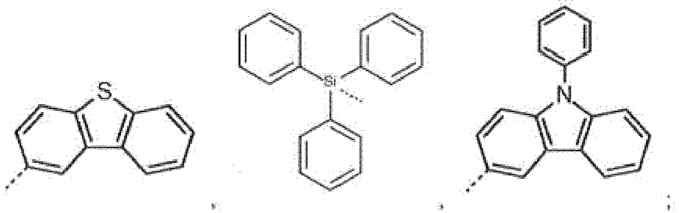 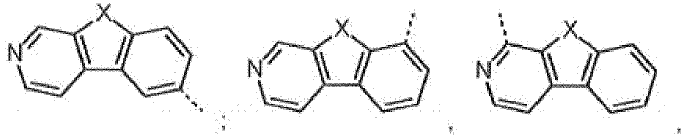 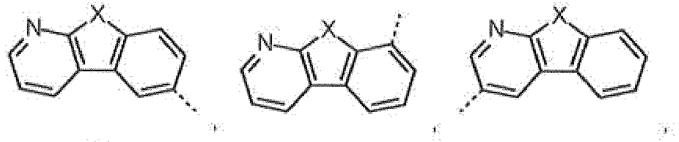 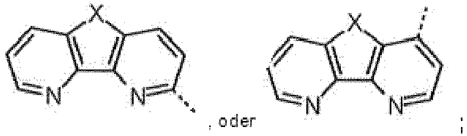 und R⁸⁹ H oder eine Gruppe mit folgender Formel ist: 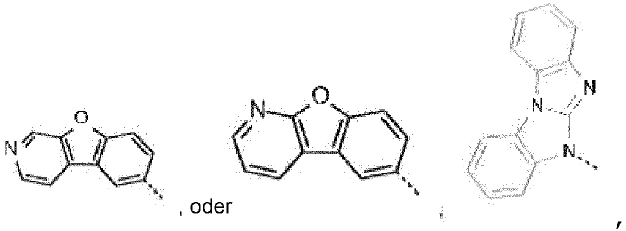 oder 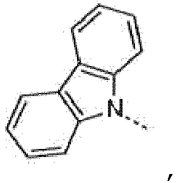 wobei X O, S oder NR²⁴ ist, wobei
R²⁴ 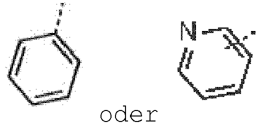 ist.

5. Verbindung nach einem der Ansprüche 2 bis 4, wobei die Gruppe mit der Formel - (A¹)ₒ-(A²)ₚ-(A³)_{q}-(A⁴)ᵣ-R¹⁶ eine Gruppe mit der folgenden Formel ist: 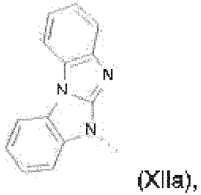 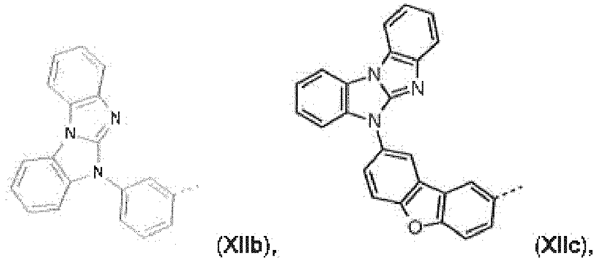 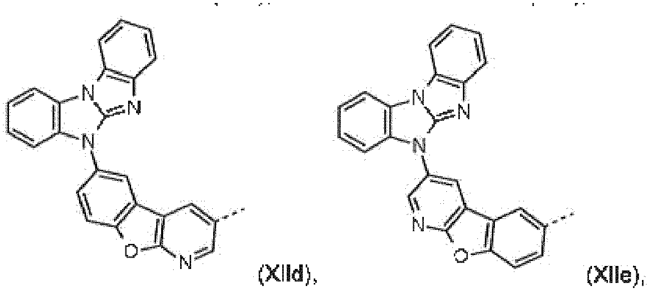 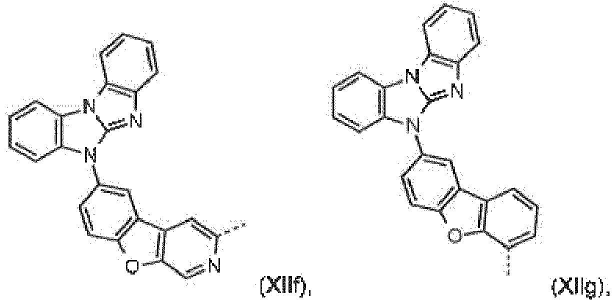 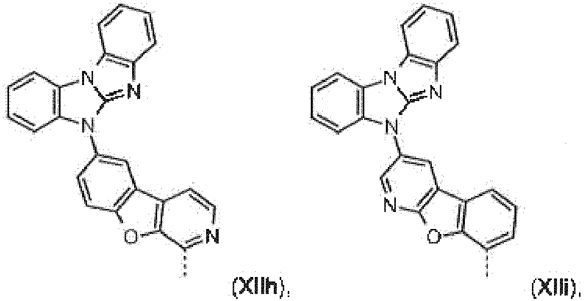 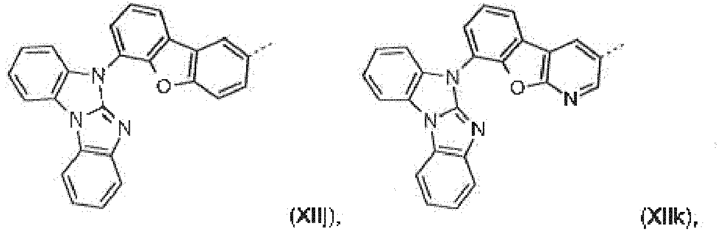 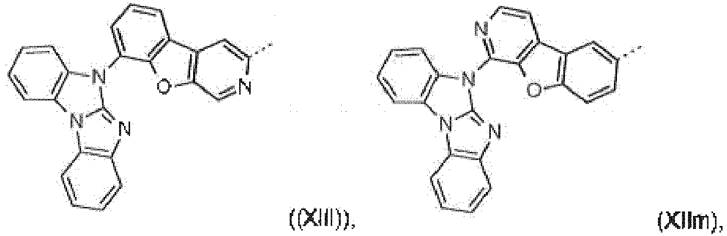 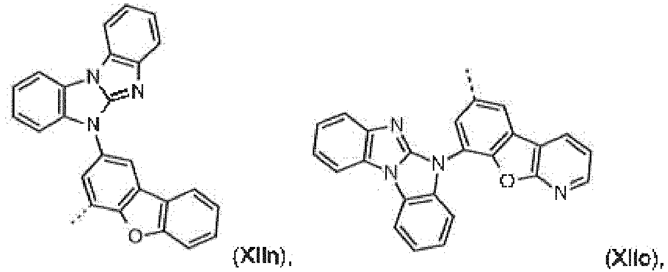 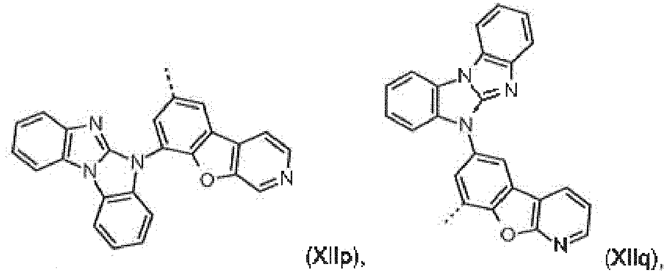 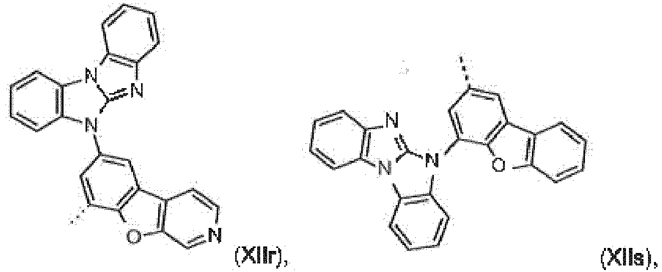 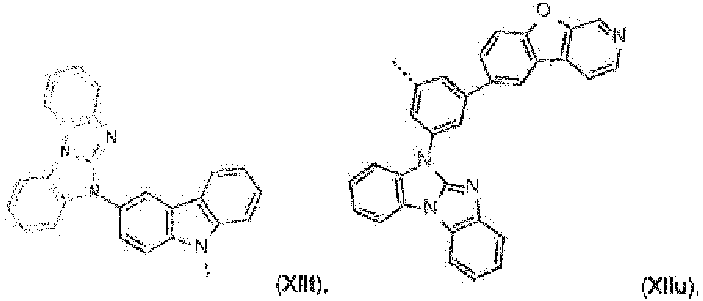 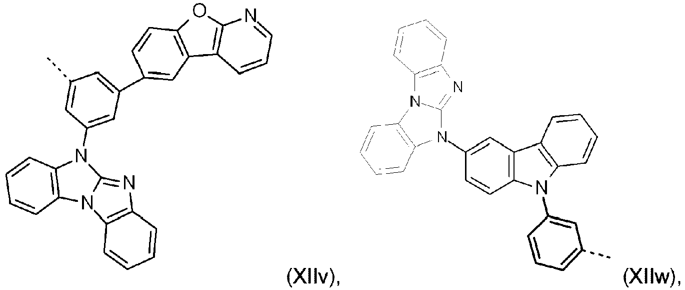 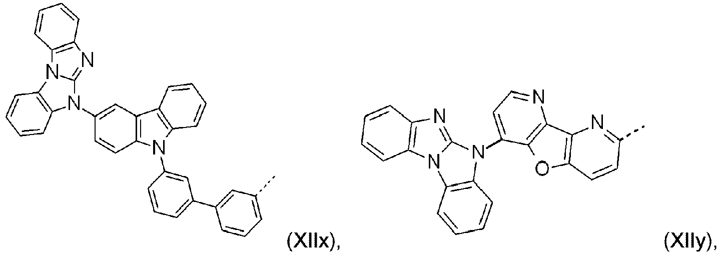 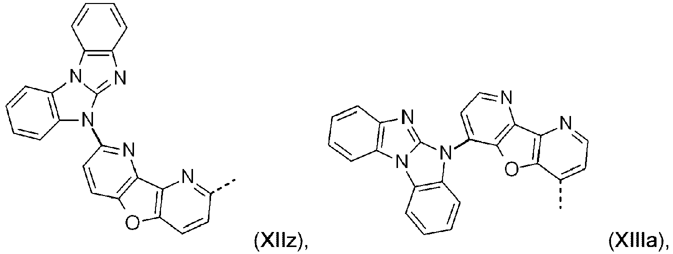 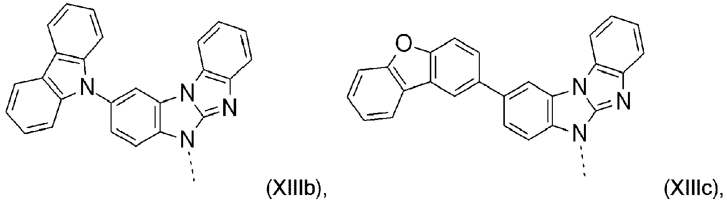 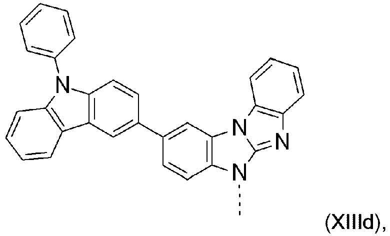 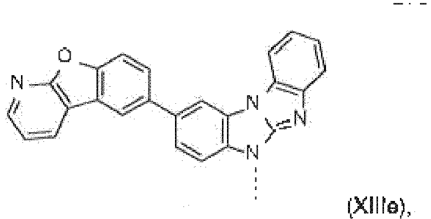 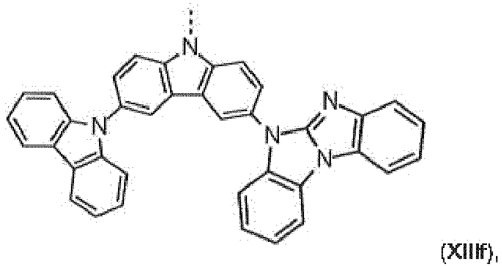 oder

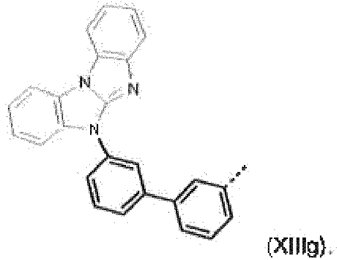

6. Verbindung nach einem der Ansprüche 2 bis 5, wobei die Gruppe mit der Formel -(A¹)ₒ-(A²)ₚ-(A³)_{q}-(A⁴)ᵣ-R^{16'} Folgendes ist: H, eine C₁-C₂₅-Alkylgruppe oder eine Gruppe mit der Formel (XIIa), (XIIb), (XIIc), (XIId), (XIIe), (XIIf), (XIIg), (XIIh), (XIIi), (XIIj), (XIIk), (XIIl), (XIIm), (XIIn), (XIIo), (XIIp), (XIIq), (XIIr), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) oder (XIIIg), wie in Anspruch 5 definiert; oder eine Gruppe mit folgender Formel: 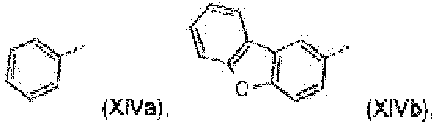 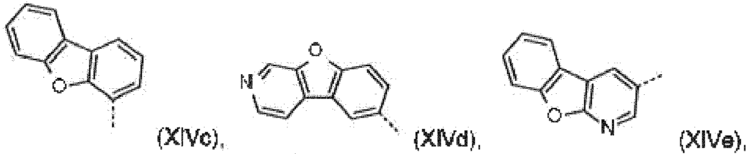 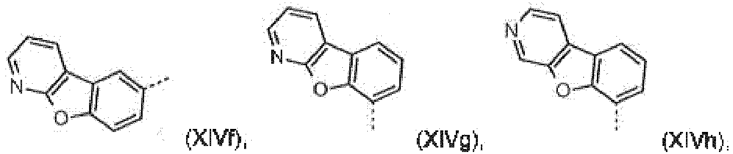 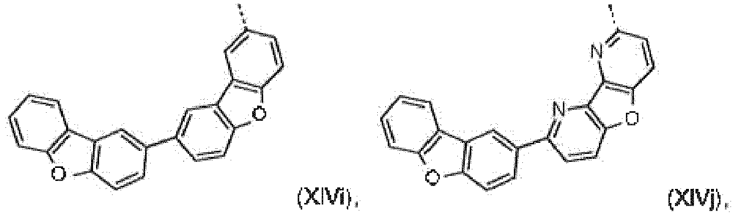 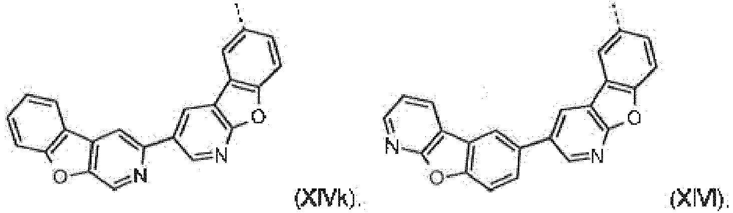 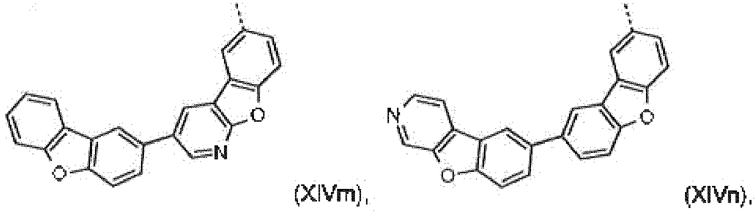 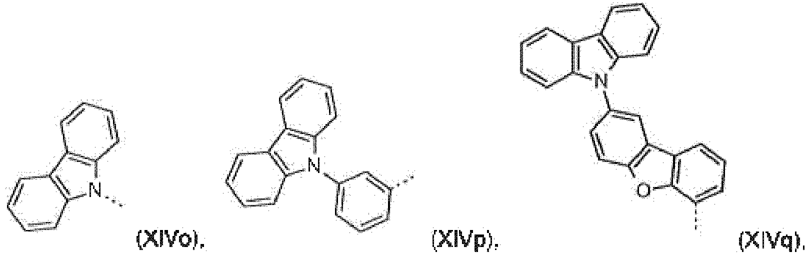 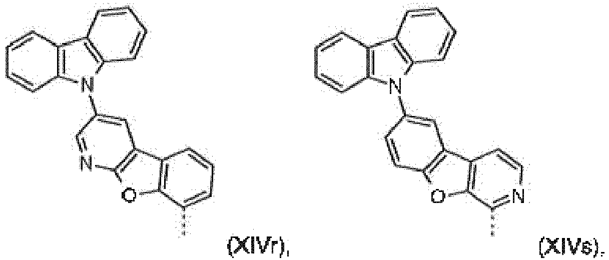 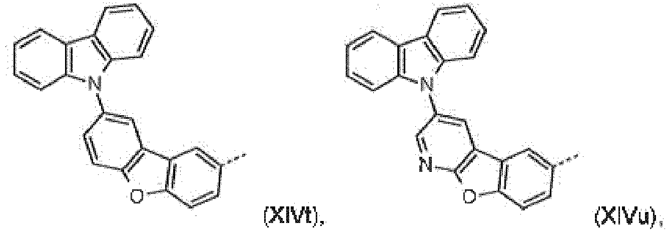 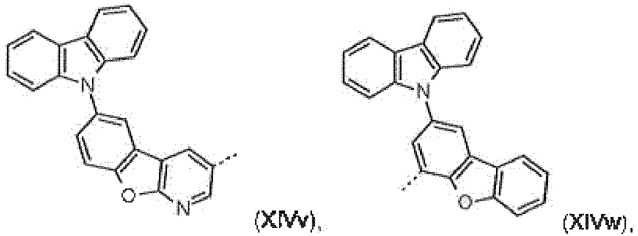 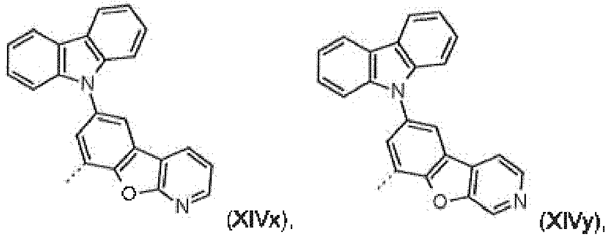 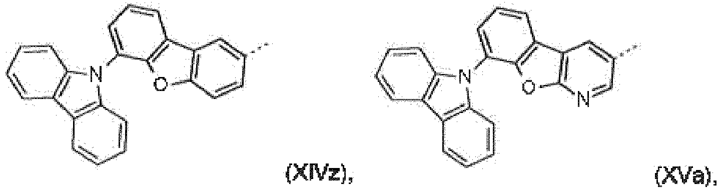 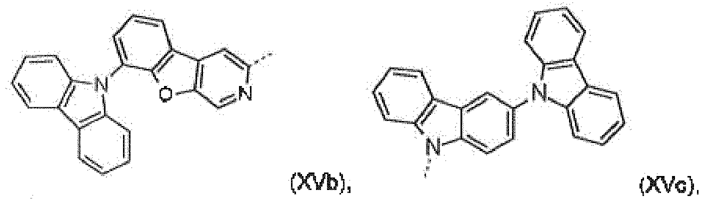 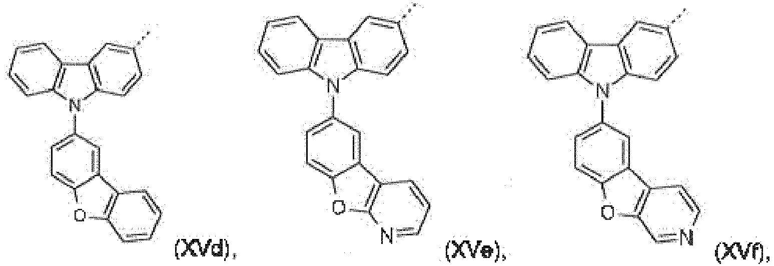 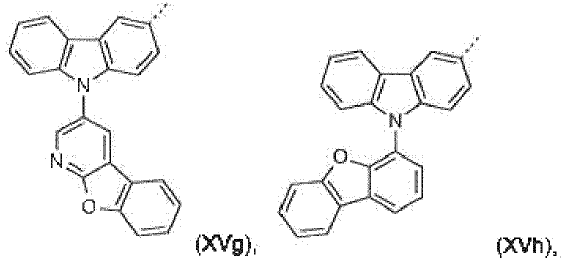 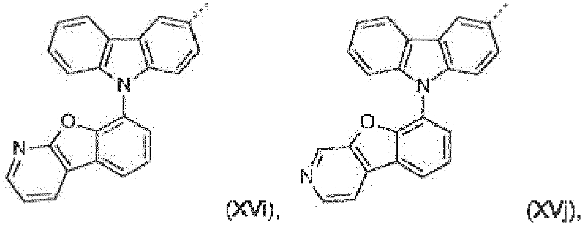 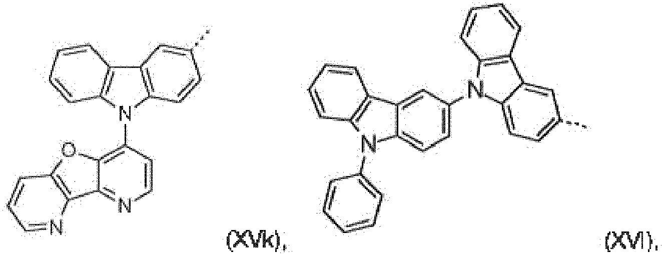 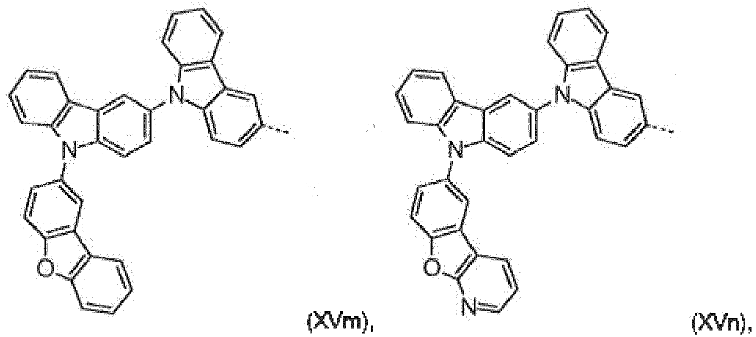 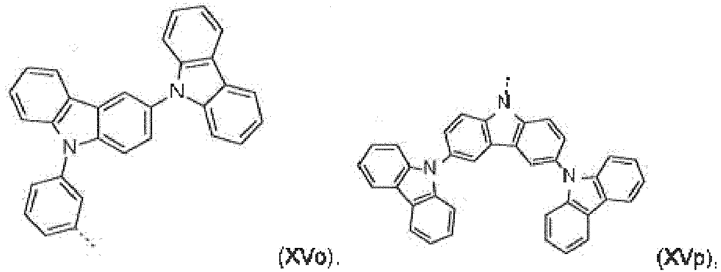 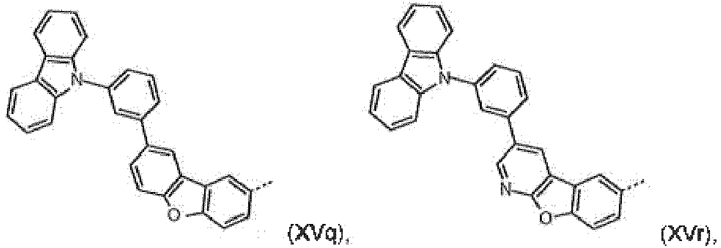 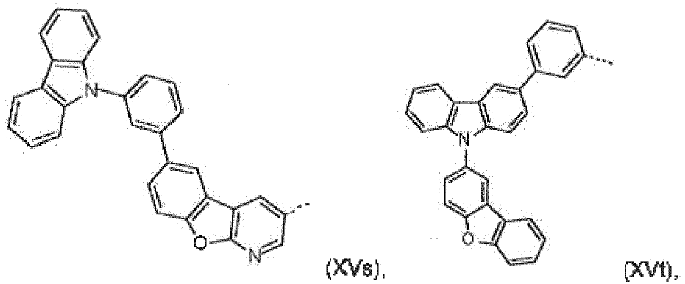 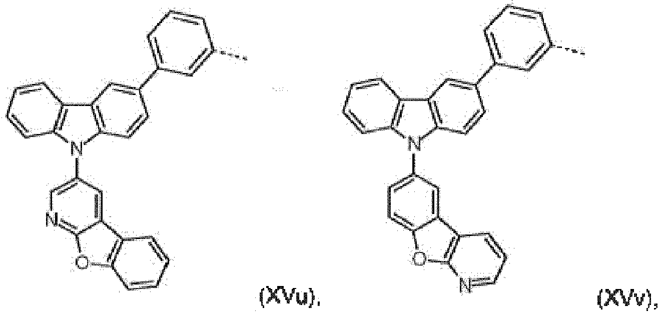 oder 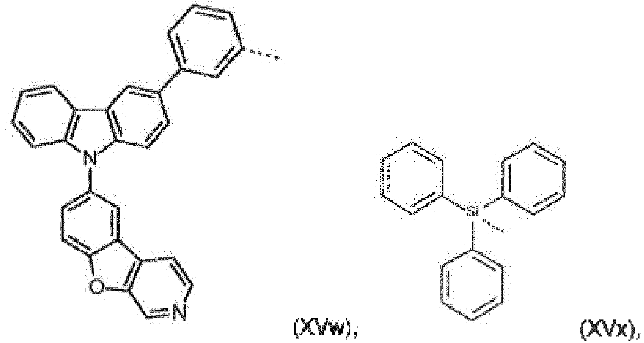 oder 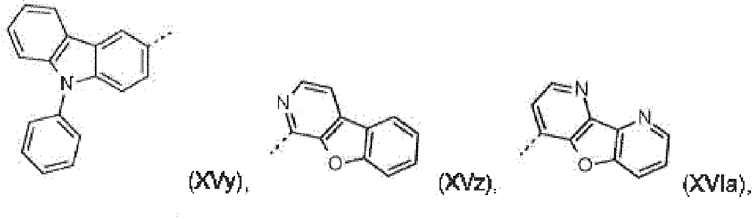 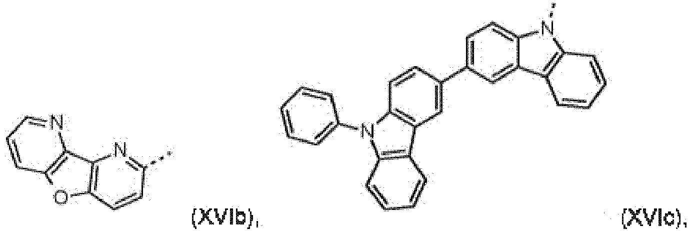 oder

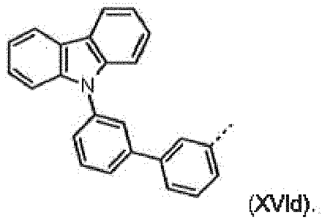

7. Verbindung nach einem der Ansprüche 2 bis 6, die Folgendes ist:
eine Verbindung mit der Formel (Ia), wobei R⁸³ eine Gruppe mit der Formel (XIIa), (XIIb), (XIIc), (XIId), (XIIe), (XIIf), (XIIg), (XIIh), (XIIi), (XIIj), (XIIk), ((XIIl)), (XIIm), (XIIn), (XIIo), (XIIp), (XIIq), (XIIr), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) oder (XIIIg), wie in Anspruch 5 definiert, ist, und
R⁸⁷ H, eine C₁-C₂₅-Alkylgruppe, eine Gruppe mit der Formel (XIIa), (XIIb), (XIIc), (XIId), (XIIe), (XIIf), (XIIg), (XIIh), (XIIi), (XIIj), (XIIk), ((XIIl)), (XIIm), (XIIn), (XIIo), (XIIp), (XIIq), (XIIr), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) oder (XIIIg), wie in Anspruch 5 definiert, oder eine Gruppe mit der Formel (XIVb), (XIVc), (XIVd), (XIVe), (XIVf), (XIVg), (XIVh), (XIVo), (XIVp), (XIVq), (XIVr), (XIVs), (XIVt), (XIVu), (XIVv), (XIVw), (XIVx), (XIVy), (XIVz), (XVa), (XVb), (XVc), (XVd), (XVe), (XVf), (XVg), (XVh), (XVi), (XVj), (XVk), (XVI), (XVm), (XVn), (XVo), (XVp), (XVq), (XVr), (XVs), (XVt), (XVu), (XVv), (XVw), (XVx), (XVy), (XVz), (XVIa), (XVIb), (XVIc) oder (XVId), wie in Anspruch 6 definiert, ist; oder
eine Verbindung mit der Formel (Ia), wobei R⁸⁷ eine Gruppe mit der Formel (XIIa), (XIIb), (XIIc), (XIId), (XIIe), (XIIf), (XIIg), (XIIh), (XIIi), (XIIj), (XIIk), ((XIIl)), (XIIm), (XIIn), (XIIo), (XIIp), (XIIq), (XIIr), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) oder (XIIIg), wie in Anspruch 5 definiert, ist, und
R⁸³ H, eine C₁-C₂₅-Alkylgruppe, eine Gruppe mit der Formel (XIIa), (XIIb), (XIIc), (XIId), (XIIe), (XIIf), (XIIg), (XIIh), (XIIi), (XIIj), (XIIk), ((XIIl)), (XIIm), (XIIn), (XIIo), (XIIp), (XIIq), (XIIr), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) oder (XIIIg), wie in Anspruch 5 definiert, oder eine Gruppe mit der Formel (XIVb), (XIVc), (XIVd), (XIVe), (XIVf), (XIVg), (XIVh), (XIVo), (XIVp), (XIVq), (XIVr), (XIVs), (XIVt), (XIVu), (XIVv), (XIVw), (XIVx), (XIVy), (XIVz), (XVa), (XVb), (XVc), (XVd), (XVe), (XVf), (XVg), (XVh), (XVi), (XVj), (XVk), (XVl), (XVm), (XVn), (XVo), (XVp), (XVq), (XVr), (XVs), (XVt), (XVu), (XVv), (XVw), (XVx), (XVy), (XVz), (XVIa), (XVIb), (XVIc) oder (XVId), wie in Anspruch 6 definiert, ist; oder
eine Verbindung mit der Formel (Ib), wobei R⁸² eine Gruppe mit der Formel (XIIa), (XIIb), (XIIc), (XIId), (XIIe), (XIIf), (XIIg), (XIIh), (XIIi), (XIIj), (XIIk), ((XIIl)), (XIIm), (XIIn), (XIIo), (XIIp), (XIIq), (XIIr), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) oder (XIIIg), wie in Anspruch 5 definiert, ist, und
R⁸⁷ H, eine Gruppe mit der Formel (XIIa), (XIIb), (XIIc), (XIId), (XIIe), (XIIf), (XIIg), (XIIh), (XIIi), (XIIj), (XIIk), ((XIIl)), (XIIm), (XIIn), (XIIo), (XIIp), (XIIq), (XIIr), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) oder (XIIIg), wie in Anspruch 5 definiert, oder eine Gruppe mit der Formel (XIVb), (XIVc), (XIVd), (XIVe), (XIVf), (XIVg), (XIVh), (XIVo), (XIVp), (XIVq), (XIVr), (XIVs), (XIVt), (XIVu), (XIVv), (XIVw), (XIVx), (XIVy), (XIVz), (XVa), (XVb), (XVc), (XVd), (XVe), (XVf), (XVg), (XVh), (XVi), (XVj), (XVk), (XVl), (XVm), (XVn), (XVo), (XVp), (XVq), (XVr), (XVs), (XVt), (XVu), (XVv), (XVw), (XVx), (XVy), (XVz), (XVIa), (XVIb), (XVIc) oder (XVId), wie in Anspruch 6 definiert, ist; oder
eine Verbindung mit der Formel (Ib), wobei R⁸⁷ eine Gruppe mit der Formel (XIIa), (XIIb), (XIIc), (XIId), (XIIe), (XIIf), (XIIg), (XIIh), (XIIi), (XIIj), (XIIk), ((XIIl)), (XIIm), (XIIn), (XIIo), (XIIp), (XIIq), (XIIr), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) oder (XIIIg), wie in Anspruch 5 definiert, ist, und
R⁸² H, eine Gruppe mit der Formel (XIIa), (XIIb), (XIIc), (XIId), (XIIe), (XIIf), (XIIg), (XIIh), (XIIi), (XIIj), (XIIk), ((XIIl)), (XIIm), (XIIn), (XIIo), (XIIp), (XIIq), (XIIr), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) oder (XIIIg), wie in Anspruch 5 definiert, oder eine Gruppe mit der Formel (XIVb), (XIVc), (XIVd), (XIVe), (XIVf), (XIVg), (XIVh), (XIVo), (XIVp), (XIVq), (XIVr), (XIVs), (XIVt), (XIVu), (XIVv), (XIVw), (XIVx), (XIVy), (XIVz), (XVa), (XVb), (XVc), (XVd), (XVe), (XVf), (XVg), (XVh), (XVi), (XVj), (XVk), (XVl), (XVm), (XVn), (XVo), (XVp), (XVq), (XVr), (XVs), (XVt), (XVu), (XVv), (XVw), (XVx), (XVy), (XVz), (XVIa), (XVIb), (XVIc) oder (XVId), wie in Anspruch 6 definiert, ist; oder
eine Verbindung mit der Formel (Ic), wobei R⁸⁵ eine Gruppe mit der Formel (XIIa), (XIIb), (XIIc), (XIId), (XIIe), (XIIf), (XIIg), (XIIh), (XIIi), (XIIj), (XIIk), ((XIIl)), (XIIm), (XIIn), (XIIo), (XIIp), (XIIq), (XIIr), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) oder (XIIIg), wie in Anspruch 5 definiert, ist, und
R⁸⁷ H, eine C₁-C₂₅-Alkylgruppe, eine Gruppe mit der Formel (XIIa), (XIIb), (XIIc), (XIId), (XIIe), (XIIf), (XIIg), (XIIh), (XIIi), (XIIj), (XIIk), ((XIIl)), (XIIm), (XIIn), (XIIo), (XIIp), (XIIq), (XIIr), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) oder (XIIIg), wie in Anspruch 5 definiert, oder eine Gruppe mit der Formel (XIVb), (XIVc), (XIVd), (XIVe), (XIVf), (XIVg), (XIVh), (XIVo), (XIVp), (XIVq), (XIVr), (XIVs), (XIVt), (XIVu), (XIVv), (XIVw), (XIVx), (XIVy), (XIVz), (XVa), (XVb), (XVc), (XVd), (XVe), (XVf), (XVg), (XVh), (XVi), (XVj), (XVk), (XVl), (XVm), (XVn), (XVo), (XVp), (XVq), (XVr), (XVs), (XVt), (XVu), (XVv), (XVw), (XVx), (XVy), (XVz), (XVIa), (XVIb), (XVIc) oder (XVId), wie in Anspruch 6 definiert, ist; oder
eine Verbindung mit der Formel (Ic), wobei R⁸⁷ eine Gruppe mit der Formel (XIIa), (XIIb), (XIIc), (XIId), (XIIe), (XIIf), (XIIg), (XIIh), (XIIi), (XIIj), (XIIk), ((XIIl)), (XIIm), (XIIn), (XIIo), (XIIp), (XIIq), (XIIr), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) oder (XIIIg), wie in Anspruch 5 definiert, ist; und
R⁸⁵ H, eine C₁-C₂₅-Alkylgruppe, eine Gruppe mit der Formel (XIIa), (XIIb), (XIIc), (XIId), (XIIe), (XIIf), (XIIg), (XIIh), (XIIi), (XIIj), (XIIk), ((XIIl)), (XIIm), (XIIn), (XIIo), (XIIp), (XIIq), (XIIr), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) oder (XIIIg), wie in Anspruch 5 definiert, oder eine Gruppe mit der Formel (XIVb), (XIVc), (XIVd), (XIVe), (XIVf), (XIVg), (XIVh), (XIVo), (XIVp), (XIVq), (XIVr), (XIVs), (XIVt), (XIVu), (XIVv), (XIVw), (XIVx), (XIVy), (XIVz), (XVa), (XVb), (XVc), (XVd), (XVe), (XVf), (XVg), (XVh), (XVi), (XVj), (XVk), (XVl), (XVm), (XVn), (XVo), (XVp), (XVq), (XVr), (XVs), (XVt), (XVu), (XVv), (XVw), (XVx), (XVy), (XVz), (XVIa), (XVIb), (XVIc) oder (XVId), wie in Anspruch 6 definiert, ist; oder
eine Verbindung mit der Formel (Id), wobei R⁸¹ eine Gruppe mit der Formel (XIIa), (XIIb), (XIIc), (XIId), (XIIe), (XIIf), (XIIg), (XIIh), (XIIi), (XIIj), (XIIk), ((XIIl)), (XIIm), (XIIn), (XIIo), (XIIp), (XIIq), (XIIr), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) oder (XIIIg), wie in Anspruch 5 definiert, ist, und
R⁸⁵ H, eine Gruppe mit der Formel (XIIa), (XIIb), (XIIc), (XIId), (XIIe), (XIIf), (XIIg), (XIIh), (XIIi), (XIIj), (XIIk), ((XIIl)), (XIIm), (XIIn), (XIIo), (XIIp), (XIIq), (XIIr), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) oder (XIIIg), wie in Anspruch 5 definiert; oder eine Gruppe mit der Formel (XIVb), (XIVc), (XIVd), (XIVe), (XIVf), (XIVg), (XIVh), (XIVo), (XIVp), (XIVq), (XIVr), (XIVs), (XIVt), (XIVu), (XIVv), (XIVw), (XIVx), (XIVy), (XIVz), (XVa), (XVb), (XVc), (XVd), (XVe), (XVf), (XVg), (XVh), (XVi), (XVj), (XVk), (XVl), (XVm), (XVn), (XVo), (XVp), (XVq), (XVr), (XVs), (XVt), (XVu), (XVv), (XVw), (XVx), (XVy), (XVz), (XVIa), (XVIb), (XVIc) oder (XVId), wie in Anspruch 6 definiert, ist; oder
eine Verbindung mit der Formel (Id), wobei R⁸⁵ eine Gruppe mit der Formel (XIIa), (XIIb), (XIIc), (XIId), (XIIe), (XIIf), (XIIg), (XIIh), (XIIi), (XIIj), (XIIk), ((XIIl)), (XIIm), (XIIn), (XIIo), (XIIp), (XIIq), (XIIr), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) oder (XIIIg), wie in Anspruch 5 definiert, ist; und
R⁸¹ H, eine Gruppe mit der Formel (XIIa), (XIIb), (XIIc), (XIId), (XIIe), (XIIf), (XIIg), (XIIh), (XIIi), (XIIj), (XIIk), ((XIIl)), (XIIm), (XIIn), (XIIo), (XIIp), (XIIq), (XIIr), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) oder (XIIIg), wie in Anspruch 5 definiert, oder eine Gruppe mit der Formel (XIVb), (XIVc), (XIVd), (XIVe), (XIVf), (XIVg), (XIVh), (XIVo), (XIVp), (XIVq), (XIVr), (XIVs), (XIVt), (XIVu), (XIVv), (XIVw), (XIVx), (XIVy), (XIVz), (XVa), (XVb), (XVc), (XVd), (XVe), (XVf), (XVg), (XVh), (XVi), (XVj), (XVk), (XVl), (XVm), (XVn), (XVo), (XVp), (XVq), (XVr), (XVs), (XVt), (XVu), (XVv), (XVw), (XVx), (XVy), (XVz), (XVIa), (XVIb), (XVIc) oder (XVId), wie in Anspruch 6 definiert, ist; oder
eine Verbindung mit der Formel (Ie), wobei R⁸³ eine Gruppe mit der Formel (XIIa), (XIIb), (XIIc), (XIId), (XIIe), (XIIf), (XIIg), (XIIh), (XIIi), (XIIj), (XIIk), ((XIIl)), (XIIm), (XIIn), (XIIo), (XIIp), (XIIq), (XIIr), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) oder (XIIIg), wie in Anspruch 5 definiert, ist, und
R⁸⁷ H, eine C₁-C₂₅-Alkylgruppe, eine Gruppe mit der Formel (XIIa), (XIIb), (XIIc), (XIId), (XIIe), (XIIf), (XIIg), (XIIh), (XIIi), (XIIj), (XIIk), ((XIIl)), (XIIm), (XIIn), (XIIo), (XIIp), (XIIq), (XIIr), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) oder (XIIIg), wie in Anspruch 5 definiert, oder eine Gruppe mit der Formel (XIVb), (XIVc), (XIVd), (XIVe), (XIVf), (XIVg), (XIVh), (XIVo), (XIVp), (XIVq), (XIVr), (XIVs), (XIVt), (XIVu), (XIVv), (XIVw), (XIVx), (XIVy), (XIVz), (XVa), (XVb), (XVc), (XVd), (XVe), (XVf), (XVg), (XVh), (XVi), (XVj), (XVk), (XVl), (XVm), (XVn), (XVo), (XVp), (XVq), (XVr), (XVs), (XVt), (XVu), (XVv), (XVw), (XVx), (XVy), (XVz), (XVIa), (XVIb), (XVIc) oder (XVId), wie in Anspruch 6 definiert, ist; oder
eine Verbindung mit der Formel (Ie), wobei R⁸⁷ eine Gruppe mit der Formel (XIIa), (XIIb), (XIIc), (XIId), (XIIe), (XIIf), (XIIg), (XIIh), (XIIi), (XIIj), (XIIk), ((XIIl)), (XIIm), (XIIn), (XIIo), (XIIp), (XIIq), (XIIr), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) oder (XIIIg), wie in Anspruch 5 definiert, ist, und
R⁸³ H, eine C₁-C₂₅-Alkylgruppe, eine Gruppe mit der Formel (XIIa), (XIIb), (XIIc), (XIId), (XIIe), (XIIf), (XIIg), (XIIh), (XIIi), (XIIj), (XIIk), ((XIIl)), (XIIm), (XIIn), (XIIo), (XIIp), (XIIq), (XIIr), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) oder (XIIIg), wie in Anspruch 5 definiert, oder eine Gruppe mit der Formel (XIVb), (XIVc), (XIVd), (XIVe), (XIVf), (XIVg), (XIVh), (XIVo), (XIVp), (XIVq), (XIVr), (XIVs), (XIVt), (XIVu), (XIVv), (XIVw), (XIVx), (XIVy), (XIVz), (XVa), (XVb), (XVc), (XVd), (XVe), (XVf), (XVg), (XVh), (XVi), (XVj), (XVk), (XVl), (XVm), (XVn), (XVo), (XVp), (XVq), (XVr), (XVs), (XVt), (XVu), (XVv), (XVw), (XVx), (XVy), (XVz), (XVIa), (XVIb), (XVIc) oder (XVId), wie in Anspruch 6 definiert, ist; oder
eine Verbindung mit der Formel (If), wobei R⁸³ eine Gruppe mit der Formel (XIIa), (XIIb), (XIIc), (XIId), (XIIe), (XIIf), (XIIg), (XIIh), (XIIi), (XIIj), (XIIk), ((XIIl)), (XIIm), (XIIn), (XIIo), (XIIp), (XIIq), (XIIr), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) oder (XIIIg), wie in Anspruch 5 definiert, ist, und
R⁸⁵ H, eine Gruppe mit der Formel (XIIa), (XIIb), (XIIc), (XIId), (XIIe), (XIIf), (XIIg), (XIIh), (XIIi), (XIIj), (XIIk), ((XIIl)), (XIIm), (XIIn), (XIIo), (XIIp), (XIIq), (XIIr), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) oder (XIIIg), wie in Anspruch 5 definiert, oder eine Gruppe mit der Formel (XIVb), (XIVc), (XIVd), (XIVe), (XIVf), (XIVg), (XIVh), (XIVo), (XIVp), (XIVq), (XIVr), (XIVs), (XIVt), (XIVu), (XIVv), (XIVw), (XIVx), (XIVy), (XIVz), (XVa), (XVb), (XVc), (XVd), (XVe), (XVf), (XVg), (XVh), (XVi), (XVj), (XVk), (XVl), (XVm), (XVn), (XVo), (XVp), (XVq), (XVr), (XVs), (XVt), (XVu), (XVv), (XVw), (XVx), (XVy), (XVz), (XVIa), (XVIb), (XVIc) oder (XVId), wie in Anspruch 6 definiert, ist; oder
eine Verbindung mit der Formel (If), wobei R⁸⁵ eine Gruppe mit der Formel (XIIa), (XIIb), (XIIc), (XIId), (XIIe), (XIIf), (XIIg), (XIIh), (XIIi), (XIIj), (XIIk), ((XIIl)), (XIIm), (XIIn), (XIIo), (XIIp), (XIIq), (XIIr), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) oder (XIIIg), wie in Anspruch 5 definiert, ist, und
R⁸³ H, eine Gruppe mit der Formel (XIIa), (XIIb), (XIIc), (XIId), (XIIe), (XIIf), (XIIg), (XIIh), (XIIi), (XIIj), (XIIk), ((XIIl)), (XIIm), (XIIn), (XIIo), (XIIp), (XIIq), (XIIr), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) oder (XIIIg), wie in Anspruch 5 definiert, oder eine Gruppe mit der Formel (XIVb), (XIVc), (XIVd), (XIVe), (XIVf), (XIVg), (XIVh), (XIVo), (XIVp), (XIVq), (XIVr), (XIVs), (XIVt), (XIVu), (XIVv), (XIVw), (XIVx), (XIVy), (XIVz), (XVa), (XVb), (XVc), (XVd), (XVe), (XVf), (XVg), (XVh), (XVi), (XVj), (XVk), (XVl), (XVm), (XVn), (XVo), (XVp), (XVq), (XVr), (XVs), (XVt), (XVu), (XVv), (XVw), (XVx), (XVy), (XVz), (XVIa), (XVIb), (XVIc) oder (XVId), wie in Anspruch 6 definiert, ist; oder
eine Verbindung mit der Formel (Ii), wobei R⁸³ eine Gruppe mit der Formel (XIIa), (XIIb), (XIIc), (XIId), (XIIe), (XIIf), (XIIg), (XIIh), (XIIi), (XIIj), (XIIk), ((XIIl)), (XIIm), (XIIn), (XIIo), (XIIp), (XIIq), (XIIr), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) oder (XIIIg), wie in Anspruch 5 definiert, ist, und
R⁸⁵ H, eine Gruppe mit der Formel (XIIa), (XIIb), (XIIc), (XIId), (XIIe), (XIIf), (XIIg), (XIIh), (XIIi), (XIIj), (XIIk), ((XIIl)), (XIIm), (XIIn), (XIIo), (XIIp), (XIIq), (XIIr), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) oder (XIIIg), wie in Anspruch 5 definiert, oder eine Gruppe mit der Formel (XIVb), (XIVc), (XIVd), (XIVe), (XIVf), (XIVg), (XIVh), (XIVo), (XIVp), (XIVq), (XIVr), (XIVs), (XIVt), (XIVu), (XIVv), (XIVw), (XIVx), (XIVy), (XIVz), (XVa), (XVb), (XVc), (XVd), (XVe), (XVf), (XVg), (XVh), (XVi), (XVj), (XVk), (XVl), (XVm), (XVn), (XVo), (XVp), (XVq), (XVr), (XVs), (XVt), (XVu), (XVv), (XVw), (XVx), (XVy), (XVz), (XVIa), (XVIb), (XVIc) oder (XVId), wie in Anspruch 6 definiert, ist; oder
eine Verbindung mit der Formel (Ii), wobei R⁸⁵ eine Gruppe mit der Formel (XIIa), (XIIb), (XIIc), (XIId), (XIIe), (XIIf), (XIIg), (XIIh), (XIIi), (XIIj), (XIIk), ((XIIl)), (XIIm), (XIIn), (XIIo), (XIIp), (XIIq), (XIIr), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) oder (XIIIg), wie in Anspruch 5 definiert, ist, und
R⁸³ H, eine Gruppe mit der Formel (XIIa), (XIIb), (XIIc), (XIId), (XIIe), (XIIf), (XIIg), (XIIh), (XIIi), (XIIj), (XIIk), ((XIIl)), (XIIm), (XIIn), (XIIo), (XIIp), (XIIq), (XIIr), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) oder (XIIIg), wie in Anspruch 5 definiert, oder eine Gruppe mit der Formel (XIVb), (XIVc), (XIVd), (XIVe), (XIVf), (XIVg), (XIVh), (XIVo), (XIVp), (XIVq), (XIVr), (XIVs), (XIVt), (XIVu), (XIVv), (XIVw), (XIVx), (XIVy), (XIVz), (XVa), (XVb), (XVc), (XVd), (XVe), (XVf), (XVg), (XVh), (XVi), (XVj), (XVk), (XVl), (XVm), (XVn), (XVo), (XVp), (XVq), (XVr), (XVs), (XVt), (XVu), (XVv), (XVw), (XVx), (XVy), (XVz), (XVIa), (XVIb), (XVIc) oder (XVId), wie in Anspruch 6 definiert, ist; oder
eine Verbindung mit der Formel (Ij), wobei R⁸³ eine Gruppe mit der Formel (XIIa), (XIIb), (XIIc), (XIId), (XIIe), (XIIf), (XIIg), (XIIh), (XIIi), (XIIj), (XIIk), ((XIIl)), (XIIm), (XIIn), (XIIo), (XIIp), (XIIq), (XIIr), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) oder (XIIIg), wie in Anspruch 5 definiert, ist, und
R⁸⁵ H, eine C₁-C₂₅-Alkylgruppe, eine Gruppe mit der Formel (XIIa), (XIIb), (XIIc), (XIId), (XIIe), (XIIf), (XIIg), (XIIh), (XIIi), (XIIj), (XIIk), ((XIIl)), (XIIm), (XIIn), (XIIo), (XIIp), (XIIq), (XIIr), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) oder (XIIIg), wie in Anspruch 5 definiert, oder eine Gruppe mit der Formel (XIVb), (XIVc), (XIVd), (XIVe), (XIVf), (XIVg), (XIVh), (XIVo), (XIVp), (XIVq), (XIVr), (XIVs), (XIVt), (XIVu), (XIVv), (XIVw), (XIVx), (XIVy), (XIVz), (XVa), (XVb), (XVc), (XVd), (XVe), (XVf), (XVg), (XVh), (XVi), (XVj), (XVk), (XVl), (XVm), (XVn), (XVo), (XVp), (XVq), (XVr), (XVs), (XVt), (XVu), (XVv), (XVw), (XVx), (XVy), (XVz), (XVIa), (XVIb), (XVIc) oder (XVId), wie in Anspruch 6 definiert, ist; oder
eine Verbindung mit der Formel (Ij), wobei R⁸⁵ eine Gruppe mit der Formel (XIIa), (XIIb), (XIIc), (XIId), (XIIe), (XIIf), (XIIg), (XIIh), (XIIi), (XIIj), (XIIk), ((XIIl)), (XIIm), (XIIn), (XIIo), (XIIp), (XIIq), (XIIr), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) oder (XIIIg), wie in Anspruch 5 definiert, ist, und
R⁸³ H, eine C₁-C₂₅-Alkylgruppe, eine Gruppe mit der Formel (XIIa), (XIIb), (XIIc), (XIId), (XIIe), (XIIf), (XIIg), (XIIh), (XIIi), (XIIj), (XIIk), ((XIIl)), (XIIm), (XIIn), (XIIo), (XIIp), (XIIq), (XIIr), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) oder (XIIIg), wie in Anspruch 5 definiert, oder eine Gruppe mit der Formel (XIVb), (XIVc), (XIVd), (XIVe), (XIVf), (XIVg), (XIVh), (XIVo), (XIVp), (XIVq), (XIVr), (XIVs), (XIVt), (XIVu), (XIVv), (XIVw), (XIVx), (XIVy), (XIVz), (XVa), (XVb), (XVc), (XVd), (XVe), (XVf), (XVg), (XVh), (XVi), (XVj), (XVk), (XVl), (XVm), (XVn), (XVo), (XVp), (XVq), (XVr), (XVs), (XVt), (XVu), (XVv), (XVw), (XVx), (XVy), (XVz), (XVIa), (XVIb), (XVIc) oder (XVId), wie in Anspruch 6 definiert, ist;
eine Verbindung mit der Formel (II), wobei R⁸⁵ eine Gruppe mit der Formel (XIIa), (XIIb), (XIIc), (XIId), (XIIe), (XIIf), (XIIg), (XIIh), (XIIi), (XIIj), (XIIk), ((XIIl)), (XIIm), (XIIn), (XIIo), (XIIp), (XIIq), (XIIr), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) oder (XIIIg), wie in Anspruch 5 definiert, ist, und
R⁸⁷ H, eine Gruppe mit der Formel (XIIa), (XIIb), (XIIc), (XIId), (XIIe), (XIIf), (XIIg), (XIIh), (XIIi), (XIIj), (XIIk), ((XIIl)), (XIIm), (XIIn), (XIIo), (XIIp), (XIIq), (XIIr), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) oder (XIIIg), wie in Anspruch 5 definiert, oder eine Gruppe mit der Formel (XIVb), (XIVc), (XIVd), (XIVe), (XIVf), (XIVg), (XIVh), (XIVo), (XIVp), (XIVq), (XIVr), (XIVs), (XIVt), (XIVu), (XIVv), (XIVw), (XIVx), (XIVy), (XIVz), (XVa), (XVb), (XVc), (XVd), (XVe), (XVf), (XVg), (XVh), (XVi), (XVj), (XVk), (XVl), (XVm), (XVn), (XVo), (XVp), (XVq), (XVr), (XVs), (XVt), (XVu), (XVv), (XVw), (XVx), (XVy), (XVz), (XVIa), (XVIb), (XVIc) oder (XVId), wie in Anspruch 6 definiert, ist; oder
eine Verbindung mit der Formel (II), wobei R⁸⁷ eine Gruppe mit der Formel (XIIa), (XIIb), (XIIc), (XIId), (XIIe), (XIIf), (XIIg), (XIIh), (XIIi), (XIIj), (XIIk), ((XIIl)), (XIIm), (XIIn), (XIIo), (XIIp), (XIIq), (XIIr), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) oder (XIIIg), wie in Anspruch 5 definiert, ist, und
R⁸⁵ H, eine Gruppe mit der Formel (XIIa), (XIIb), (XIIc), (XIId), (XIIe), (XIIf), (XIIg), (XIIh), (XIIi), (XIIj), (XIIk), ((XIIl)), (XIIm), (XIIn), (XIIo), (XIIp), (XIIq), (XIIr), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) oder (XIIIg), wie in Anspruch 5 definiert, oder eine Gruppe mit der Formel (XIVb), (XIVc), (XIVd), (XIVe), (XIVf), (XIVg), (XIVh), (XIVo), (XIVp), (XIVq), (XIVr), (XIVs), (XIVt), (XIVu), (XIVv), (XIVw), (XIVx), (XIVy), (XIVz), (XVa), (XVb), (XVc), (XVd), (XVe), (XVf), (XVg), (XVh), (XVi), (XVj), (XVk), (XVl), (XVm), (XVn), (XVo), (XVp), (XVq), (XVr), (XVs), (XVt), (XVu), (XVv), (XVw), (XVx), (XVy), (XVz), (XVIa), (XVIb), (XVIc) oder (XVId), wie in Anspruch 6 definiert, ist; oder
eine Verbindung mit der Formel (Im), wobei R⁸³ eine Gruppe mit der Formel (XIIa), (XIIb), (XIIc), (XIId), (XIIe), (XIIf), (XIIg), (XIIh), (XIIi), (XIIj), (XIIk), ((XIIl)), (XIIm), (XIIn), (XIIo), (XIIp), (XIIq), (XIIr), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) oder (XIIIg), wie in Anspruch 5 definiert, ist, und
R⁸⁵ H, eine C₁-C₂₅-Alkylgruppe, eine Gruppe mit der Formel (XIIa), (XIIb), (XIIc), (XIId), (XIIe), (XIIf), (XIIg), (XIIh), (XIIi), (XIIj), (XIIk), ((XIIl)), (XIIm), (XIIn), (XIIo), (XIIp), (XIIq), (XIIr), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) oder (XIIIg), wie in Anspruch 5 definiert, oder eine Gruppe mit der Formel (XIVb), (XIVc), (XIVd), (XIVe), (XIVf), (XIVg), (XIVh), (XIVo), (XIVp), (XIVq), (XIVr), (XIVs), (XIVt), (XIVu), (XIVv), (XIVw), (XIVx), (XIVy), (XIVz), (XVa), (XVb), (XVc), (XVd), (XVe), (XVf), (XVg), (XVh), (XVi), (XVj), (XVk), (XVl), (XVm), (XVn), (XVo), (XVp), (XVq), (XVr), (XVs), (XVt), (XVu), (XVv), (XVw), (XVx), (XVy), (XVz), (XVIa), (XVIb), (XVIc) oder (XVId), wie in Anspruch 6 definiert, ist; oder
eine Verbindung mit der Formel (Im), wobei R⁸⁵ eine Gruppe mit der Formel (XIIa), (XIIb), (XIIc), (XIId), (XIIe), (XIIf), (XIIg), (XIIh), (XIIi), (XIIj), (XIIk), ((XIIl)), (XIIm), (XIIn), (XIIo), (XIIp), (XIIq), (XIIr), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) oder (XIIIg), wie in Anspruch 5 definiert, ist, und
R⁸³ H, eine C₁-C₂₅-Alkylgruppe, eine Gruppe mit der Formel (XIIa), (XIIb), (XIIc), (XIId), (XIIe), (XIIf), (XIIg), (XIIh), (XIIi), (XIIj), (XIIk), ((XIIl)), (XIIm), (XIIn), (XIIo), (XIIp), (XIIq), (XIIr), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) oder (XIIIg), wie in Anspruch 5 definiert, oder eine Gruppe mit der Formel (XIVb), (XIVc), (XIVd), (XIVe), (XIVf), (XIVg), (XIVh), (XIVo), (XIVp), (XIVq), (XIVr), (XIVs), (XIVt), (XIVu), (XIVv), (XIVw), (XIVx), (XIVy), (XIVz), (XVa), (XVb), (XVc), (XVd), (XVe), (XVf), (XVg), (XVh), (XVi), (XVj), (XVk), (XVl), (XVm), (XVn), (XVo), (XVp), (XVq), (XVr), (XVs), (XVt), (XVu), (XVv), (XVw), (XVx), (XVy), (XVz), (XVIa), (XVIb), (XVIc) oder (XVId), wie in Anspruch 6 definiert, ist; oder
eine Verbindung mit der Formel (In), wobei R⁸³ eine Gruppe mit der Formel (XIIa), (XIIb), (XIIc), (XIId), (XIIe), (XIIf), (XIIg), (XIIh), (XIIi), (XIIj), (XIIk), ((XIIl)), (XIIm), (XIIn), (XIIo), (XIIp), (XIIq), (XIIr), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) oder (XIIIg), wie in Anspruch 5 definiert, ist, und
R⁸⁷ H, eine Gruppe mit der Formel (XIIa), (XIIb), (XIIc), (XIId), (XIIe), (XIIf), (XIIg), (XIIh), (XIIi), (XIIj), (XIIk), ((XIIl)), (XIIm), (XIIn), (XIIo), (XIIp), (XIIq), (XIIr), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) oder (XIIIg), wie in Anspruch 5 definiert, oder eine Gruppe mit der Formel (XIVb), (XIVc), (XIVd), (XIVe), (XIVf), (XIVg), (XIVh), (XIVo), (XIVp), (XIVq), (XIVr), (XIVs), (XIVt), (XIVu), (XIVv), (XIVw), (XIVx), (XIVy), (XIVz), (XVa), (XVb), (XVc), (XVd), (XVe), (XVf), (XVg), (XVh), (XVi), (XVj), (XVk), (XVl), (XVm), (XVn), (XVo), (XVp), (XVq), (XVr), (XVs), (XVt), (XVu), (XVv), (XVw), (XVx), (XVy), (XVz), (XVIa), (XVIb), (XVIc) oder (XVId), wie in Anspruch 6 definiert, ist.

8. Verbindung nach einem der Ansprüche 2 bis 7, die Folgendes ist: eine Verbindung mit der Formel (Ia), wobei R⁸³ eine Gruppe mit der Formel (XIIa), (XIIb), (XIIc), (XIId), (XIIe), (XIIf), (XIIg), (XIIj), (XIIk), (XIIn), (XIIo), (XIIq), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) oder (XIIIg), wie in Anspruch 5 definiert, ist, und
R⁸⁷ H, eine C₁-C₂₅-Alkylgruppe, eine Gruppe mit der Formel (XIIa), (XIIb), (XIIc), (XIId), (XIIe), (XIIf), (XIIg), (XIIj), (XIIk), (XIIn), (XIIo), (XIIq), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) oder (XIIIg), wie in Anspruch 5 definiert, oder eine Gruppe mit der Formel (XIVb), (XIVc), (XIVd), (XIVe), (XIVf), (XIVg), (XIVo), (XIVp), (XIVq), (XIVr), (XIVt), (XIVu), (XIVv), (XIVw), (XIVx), (XIVz), (XVa), (XVb), (XVc), (XVk), (XVl), (XVo), (XVp), (XVs), (XVw), (XVx), (XVy), (XVIa), (XVIb), (XVIc) oder (XVId), wie in Anspruch 6 definiert, ist; oder
eine Verbindung mit der Formel (Ia), wobei R⁸⁷ eine Gruppe mit der Formel (XIIa), (XIIb), (XIIc), (XIId), (XIIe), (XIIf), (XIIg), (XIIj), (XIIk), (XIIn), (XIIo), (XIIq), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) oder (XIIIg), wie in Anspruch 5 definiert, ist, und
R⁸³ H, eine C₁-C₂₅-Alkylgruppe, eine Gruppe mit der Formel (XIIa), (XIIb), (XIIc), (XIId), (XIIe), (XIIf), (XIIg), (XIIj), (XIIk), (XIIn), (XIIo), (XIIq), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) oder (XIIIg), wie in Anspruch 5 definiert, oder eine Gruppe mit der Formel (XIVb), (XIVc), (XIVd), (XIVe),
(XIVf), (XIVg), (XIVo), (XIVp), (XIVq), (XIVr), (XIVt), (XIVu), (XIVv), (XIVw), (XIVx), (XIVz), (XVa), (XVb), (XVc), (XVk), (XVl), (XVo), (XVp), (XVs), (XVw), (XVx), (XVy), (XVIa), (XVIb), (XVIc) oder (XVId), wie in Anspruch 6 definiert, ist;
eine Verbindung mit der Formel (Ib), wobei R⁸² eine Gruppe mit der Formel (XIIa), (XIIb), (XIIc), (XIId), (XIIe), (XIIf), (XIIg), (XIIj), (XIIk), (XIIn), (XIIo), (XIIq), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) oder (XIIIg), wie in Anspruch 5 definiert, ist, und
R⁸⁷ H, eine Gruppe mit der Formel (XIIa), (XIIb), (XIIc), (XIId), (XIIe), (XIIf), (XIIg), (XIIj), (XIIk), (XIIn), (XIIo), (XIIq), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) oder (XIIIg), wie in Anspruch 5 definiert, oder eine Gruppe mit der Formel (XIVb), (XIVc), (XIVd), (XIVe), (XIVf), (XIVg), (XIVo), (XIVp), (XIVq), (XIVr), (XIVt), (XIVu), (XIVv), (XIVw), (XIVx), (XIVz), (XVa), (XVb), (XVc), (XVk), (XVl), (XVo), (XVp), (XVs), (XVw), (XVx), (XVy), (XVIa), (XVIb), (XVIc) oder (XVId), wie in Anspruch 6 definiert, ist; oder
eine Verbindung mit der Formel (Ib), wobei R⁸⁷ eine Gruppe mit der Formel (XIIa), (XIIb), (XIIc), (XIId), (XIIe), (XIIf), (XIIg), (XIIj), (XIIk), (XIIn), (XIIo), (XIIq), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) oder (XIIIg), wie in Anspruch 5 definiert, ist, und
R⁸² H, eine Gruppe mit der Formel (XIIa), (XIIb), (XIIc), (XIId), (XIIe), (XIIf), (XIIg), (XIIj), (XIIk), (XIIn), (XIIo), (XIIq), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) oder (XIIIg), wie in Anspruch 5 definiert, oder eine Gruppe mit der Formel (XIVb), (XIVc), (XIVd), (XIVe), (XIVf), (XIVg), (XIVo),
(XIVp), (XIVq), (XIVr), (XIVt), (XIVu), (XIVv), (XIVw), (XIVx), (XIVz), (XVa), (XVb), (XVc), (XVk), (XVl), (XVo), (XVp), (XVs), (XVw), (XVx), (XVy), (XVIa), (XVIb), (XVIc) oder (XVId), wie in Anspruch 6 definiert, ist;
eine Verbindung mit der Formel (Ic), wobei R⁸⁵ eine Gruppe mit der Formel (XIIa), (XIIb), (XIIc), (XIId), (XIIe), (XIIf), (XIIg), (XIIj), (XIIk), (XIIn), (XIIo), (XIIq), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) oder (XIIIg), wie in Anspruch 5 definiert, ist, und
R⁸⁷ H, eine C₁-C₂₅-Alkylgruppe, eine Gruppe mit der Formel (XIIa), (XIIb), (XIIc), (XIId), (XIIe), (XIIf), (XIIg), (XIIj), (XIIk), (XIIn), (XIIo), (XIIq), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) oder (XIIIg), wie in Anspruch 5 definiert, oder eine Gruppe mit der Formel (XIVb), (XIVc), (XIVd), (XIVe), (XIVf), (XIVg), (XIVo), (XIVp), (XIVq), (XIVr), (XIVt), (XIVu), (XIVv), (XIVw), (XIVx), (XIVz), (XVa), (XVb), (XVc), (XVk), (XVl), (XVo), (XVp), (XVs), (XVw), (XVx), (XVy), (XVIa), (XVIb), (XVIc) oder (XVId), wie in Anspruch 6 definiert, ist; oder
eine Verbindung mit der Formel (Ic), wobei R⁸⁷ eine Gruppe mit der Formel (XIIa), (XIIb), (XIIc), (XIId), (XIIe), (XIIf), (XIIg), (XIIj), (XIIk), (XIIn), (XIIo), (XIIq), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) oder (XIIIg), wie in Anspruch 5 definiert, ist, und
R⁸⁵ H, eine C₁-C₂₅-Alkylgruppe, eine Gruppe mit der Formel (XIIa), (XIIb), (XIIc), (XIId), (XIIe), (XIIf), (XIIg), (XIIj), (XIIk), (XIIn), (XIIo), (XIIq), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) oder (XIIIg), wie in Anspruch 5 definiert, oder eine Gruppe mit der Formel (XIVb), (XIVc), (XIVd), (XIVe),
(XIVf), (XIVg), (XIVo), (XIVp), (XIVq), (XIVr), (XIVt), (XIVu), (XIVv), (XIVw), (XIVx), (XIVz), (XVa), (XVb), (XVc), (XVk), (XVl), (XVo), (XVp), (XVs), (XVw), (XVx), (XVy), (XVIa), (XVIb), (XVIc) oder (XVId), wie in Anspruch 6 definiert, ist; oder
eine Verbindung mit der Formel (Id), wobei R⁸¹ eine Gruppe mit der Formel (XIIa), (XIIb), (XIIc), (XIId), (XIIe), (XIIf), (XIIg), (XIIj), (XIIk), (XIIn), (XIIo), (XIIq), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) oder (XIIIg), wie in Anspruch 5 definiert, ist, und
R⁸⁵ H, eine Gruppe mit der Formel (XIIa), (XIIb), (XIIc), (XIId), (XIIe), (XIIf), (XIIg), (XIIj), (XIIk), (XIIn), (XIIo), (XIIq), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) oder (XIIIg), wie in Anspruch 5 definiert, oder eine Gruppe mit der Formel (XIVb), (XIVc), (XIVd), (XIVe), (XIVf), (XIVg), (XIVo), (XIVp), (XIVq), (XIVr), (XIVt), (XIVu), (XIVv), (XIVw), (XIVx), (XIVz), (XVa), (XVb), (XVc), (XVk), (XVl), (XVo), (XVp), (XVs), (XVw), (XVx), (XVy), (XVIa), (XVIb), (XVIc) oder (XVId), wie in Anspruch 6 definiert, ist;
eine Verbindung mit der Formel (Ie), wobei R⁸³ eine Gruppe mit der Formel (XIIa), (XIIb), (XIIc), (XIId), (XIIe), (XIIf), (XIIg), (XIIj), (XIIk), (XIIn), (XIIo), (XIIq), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) oder (XIIIg), wie in Anspruch 5 definiert, ist, und
R⁸⁷ H, eine C₁-C₂₅-Alkylgruppe, eine Gruppe mit der Formel (XIIa), (XIIb), (XIIc), (XIId), (XIIe), (XIIf), (XIIg), (XIIj), (XIIk), (XIIn), (XIIo), (XIIq), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) oder (XIIIg), wie in Anspruch 5 definiert, oder eine Gruppe mit der Formel (XIVb), (XIVc), (XIVd), (XIVe), (XIVf), (XIVg), (XIVo), (XIVp), (XIVq), (XIVr), (XIVt), (XIVu), (XIVv), (XIVw), (XIVx), (XIVz), (XVa), (XVb), (XVc), (XVk), (XVl), (XVo), (XVp), (XVs), (XVw), (XVx), (XVy), (XVIa), (XVIb), (XVIc) oder (XVId), wie in Anspruch 6 definiert, ist; oder
eine Verbindung mit der Formel (Ie), wobei R⁸⁷ eine Gruppe mit der Formel (XIIa), (XIIb), (XIIc), (XIId), (XIIe), (XIIf), (XIIg), (XIIj), (XIIk), (XIIn), (XIIo), (XIIq), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) oder (XIIIg), wie in Anspruch 5 definiert, ist, und
R⁸³ H, eine C₁-C₂₅-Alkylgruppe, eine Gruppe mit der Formel (XIIa), (XIIb), (XIIc), (XIId), (XIIe), (XIIf), (XIIg), (XIIj), (XIIk), (XIIn), (XIIo), (XIIq), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) oder (XIIIg), wie in Anspruch 5 definiert, oder eine Gruppe mit der Formel (XIVb), (XIVc), (XIVd), (XIVe), (XIVf), (XIVg), (XIVo), (XIVp), (XIVq), (XIVr), (XIVt), (XIVu), (XIVv), (XIVw), (XIVx), (XIVz), (XVa), (XVb), (XVc), (XVk), (XVl), (XVo), (XVp), (XVs), (XVw), (XVx), (XVy), (XVIa), (XVIb), (XVIc) oder (XVId), wie in Anspruch 6 definiert, ist;
eine Verbindung mit der Formel (If), wobei R⁸³ eine Gruppe mit der Formel (XIIa), (XIIb), (XIIc), (XIId), (XIIe), (XIIf), (XIIg), (XIIj), (XIIk), (XIIn), (XIIo), (XIIq), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) oder (XIIIg), wie in Anspruch 5 definiert, ist, und
R⁸⁵ H, eine Gruppe mit der Formel (XIIa), (XIIb), (XIIc), (XIId), (XIIe), (XIIf), (XIIg), (XIIj), (XIIk), (XIIn), (XIIo), (XIIq), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) oder (XIIIg), wie in Anspruch 5 definiert, oder eine Gruppe mit der Formel (XIVb), (XIVc), (XIVd), (XIVe), (XIVf), (XIVg), (XIVo), (XIVp), (XIVq), (XIVr), (XIVt), (XIVu), (XIVv), (XIVw), (XIVx), (XIVz), (XVa), (XVb), (XVc), (XVk), (XVl), (XVo), (XVp), (XVs), (XVw), (XVx), (XVy), (XVIa), (XVIb), (XVIc) oder (XVId), wie in Anspruch 6 definiert, ist; oder
eine Verbindung mit der Formel (If), wobei R⁸⁵ eine Gruppe mit der Formel (XIIa), (XIIb), (XIIc), (XIId), (XIIe), (XIIf), (XIIg), (XIIj), (XIIk), (XIIn), (XIIo), (XIIq), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) oder (XIIIg), wie in Anspruch 5 definiert, ist, und
R⁸³ H, eine Gruppe mit der Formel (XIIa), (XIIb), (XIIc), (XIId), (XIIe), (XIIf), (XIIg), (XIIj), (XIIk), (XIIn), (XIIo), (XIIq), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) oder (XIIIg), wie in Anspruch 5 definiert, oder eine Gruppe mit der Formel (XIVb), (XIVc), (XIVd), (XIVe), (XIVf), (XIVg), (XIVo), (XIVp), (XIVq), (XIVr), (XIVt), (XIVu), (XIVv), (XIVw), (XIVx), (XIVz), (XVa), (XVb), (XVc), (XVk), (XVl), (XVo), (XVp), (XVs), (XVw), (XVx), (XVy), (XVIa), (XVIb), (XVIc) oder (XVId), wie in Anspruch 6 definiert, ist; oder
eine Verbindung mit der Formel (Ij), wobei R⁸³ eine Gruppe mit der Formel (XIIa), (XIIb), (XIIc), (XIId), (XIIe), (XIIf), (XIIg), (XIIj), (XIIk), (XIIn), (XIIo), (XIIq), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) oder (XIIIg), wie in Anspruch 5 definiert, ist, und
R⁸⁵ H, eine C₁-C₂₅-Alkylgruppe, eine Gruppe mit der Formel (XIIa), (XIIb), (XIIc), (XIId), (XIIe), (XIIf), (XIIg), (XIIj), (XIIk), (XIIn), (XIIo), (XIIq), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) oder (XIIIg), wie in Anspruch 5 definiert, oder eine Gruppe mit der Formel (XIVb), (XIVc), (XIVd), (XIVe), (XIVf), (XIVg), (XIVo), (XIVp), (XIVq), (XIVr), (XIVt), (XIVu), (XIVv), (XIVw), (XIVx), (XIVz), (XVa), (XVb), (XVc), (XVk), (XVl), (XVo), (XVp), (XVs), (XVw), (XVx), (XVy), (XVIa), (XVIb), (XVIc) oder (XVId), wie in Anspruch 6 definiert, ist; oder
eine Verbindung mit der Formel (Ij), wobei R⁸⁵ eine Gruppe mit der Formel (XIIa), (XIIb), (XIIc), (XIId), (XIIe), (XIIf), (XIIg), (XIIj), (XIIk), (XIIn), (XIIo), (XIIq), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) oder (XIIIg), wie in Anspruch 5 definiert, ist, und
R⁸³ H, eine C₁-C₂₅-Alkylgruppe, eine Gruppe mit der Formel (XIIa), (XIIb), (XIIc), (XIId), (XIIe), (XIIf), (XIIg), (XIIj), (XIIk), (XIIn), (XIIo), (XIIq), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) oder (XIIIg), wie in Anspruch 5 definiert, oder eine Gruppe mit der Formel (XIVb), (XIVc), (XIVd), (XIVe), (XIVf), (XIVg), (XIVo), (XIVp), (XIVq), (XIVr), (XIVt), (XIVu), (XIVv), (XIVw), (XIVx), (XIVz), (XVa), (XVb), (XVc), (XVk), (XVl), (XVo), (XVp), (XVs), (XVw), (XVx), (XVy), (XVIa), (XVIb), (XVIc) oder (XVId), wie in Anspruch 6 definiert, ist; oder
eine Verbindung mit der Formel (Im), wobei R⁸³ eine Gruppe mit der Formel (XIIa), (XIIb), (XIIc), (XIId), (XIIe), (XIIf), (XIIg), (XIIj), (XIIk), (XIIn), (XIIo), (XIIq), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) oder (XIIIg), wie in Anspruch 5 definiert, ist, und
R⁸⁵ H, eine C₁-C₂₅-Alkylgruppe, eine Gruppe mit der Formel (XIIa), (XIIb), (XIIc), (XIId), (XIIe), (XIIf), (XIIg), (XIIj), (XIIk), (XIIn), (XIIo), (XIIq), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) oder (XIIIg), wie in Anspruch 5 definiert, oder eine Gruppe mit der Formel (XIVb), (XIVc), (XIVd), (XIVe), (XIVf), (XIVg), (XIVo), (XIVp), (XIVq), (XIVr), (XIVt), (XIVu), (XIVv), (XIVw), (XIVx), (XIVz), (XVa), (XVb), (XVc), (XVk), (XVl), (XVo), (XVp), (XVs), (XVw), (XVx), (XVy), (XVIa), (XVIb), (XVIc) oder (XVId), wie in Anspruch 6 definiert, ist; oder
eine Verbindung mit der Formel (Im), wobei R⁸⁷ eine Gruppe mit der Formel (XIIa), (XIIb), (XIIc), (XIId), (XIIe), (XIIf), (XIIg), (XIIj), (XIIk), (XIIn), (XIIo), (XIIq), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) oder (XIIIg), wie in Anspruch 5 definiert, ist, und
R⁸³ H, eine C₁-C₂₅-Alkylgruppe, eine Gruppe mit der Formel (XIIa), (XIIb), (XIIc), (XIId), (XIIe), (XIIf), (XIIg), (XIIj), (XIIk), (XIIn), (XIIo), (XIIq), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) oder (XIIIg), wie in Anspruch 5 definiert, oder eine Gruppe mit der Formel (XIVb), (XIVc), (XIVd), (XIVe), (XIVf), (XIVg), (XIVo), (XIVp), (XIVq), (XIVr), (XIVt), (XIVu), (XIVv), (XIVw), (XIVx), (XIVz), (XVa), (XVb), (XVc), (XVk), (XVl), (XVo), (XVp), (XVs), (XVw), (XVx), (XVy), (XVIa), (XVIb), (XVIc) oder (XVId), wie in Anspruch 6 definiert, ist; oder
eine Verbindung mit der Formel (In), wobei R⁸³ eine Gruppe mit der Formel (XIIa), (XIIb), (XIIc), (XIId), (XIIe), (XIIf), (XIIg), (XIIj), (XIIk), (XIIn), (XIIo), (XIIq), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) oder (XIIIg), wie in Anspruch 5 definiert, ist, und
R⁸⁷ H, eine Gruppe mit der Formel (XIIa), (XIIb), (XIIc), (XIId), (XIIe), (XIIf), (XIIg), (XIIj), (XIIk), (XIIn), (XIIo), (XIIq), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) oder (XIIIg), wie in Anspruch 5 definiert, oder eine Gruppe mit der Formel (XIVb), (XIVc), (XIVd), (XIVe), (XIVf), (XIVg), (XIVo), (XIVp), (XIVq), (XIVr), (XIVt), (XIVu), (XIVv), (XIVw), (XIVx), (XIVz), (XVa), (XVb), (XVc), (XVk), (XVl), (XVo), (XVp), (XVs), (XVw), (XVx), (XVy), (XVIa), (XVIb), (XVIc) oder (XVId), wie in Anspruch 6 definiert, ist.

9. Verbindung nach einem der Ansprüche 1 bis 8, die Folgendes ist: eine Verbindung mit der Formel (Ia), wobei R⁸³ eine Gruppe mit der Formel (XIIa), (XIIb), (XIIc), (XIId), (XIIe), (XIIf), (XIIg), (XIIj), (XIIk), (XIIn), (XIIo), (XIIq), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) oder (XIIIg), wie in Anspruch 5 definiert, ist und R⁸⁷ H oder eine C₁-C₂₅-Alkylgruppe ist; oder
eine Verbindung mit der Formel (Im), wobei R⁸⁷ eine Gruppe mit der Formel (XIIa), (XIIb), (XIIc), (XIId), (XIIe), (XIIf), (XIIg), (XIIj), (XIIk), (XIIn), (XIIo), (XIIq), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) oder (XIIIg), wie in Anspruch 5 definiert, ist und R⁸³ H, eine C₁-C₂₅-Alkylgruppe ist.

10. Elektronische Vorrichtung, die eine Verbindung nach einem der Ansprüche 1 bis 9 umfasst.

11. Elektronische Vorrichtung nach Anspruch 10, die eine Elektrolumineszenzvorrichtung ist.

12. Lochtransportschicht, Elektrontransportschicht, Lochsperrschicht Elektronsperrschicht oder Emissionsschicht, die eine Verbindung nach einem der Ansprüche 1 bis 9 umfasst.

13. Emissionsschicht nach Anspruch 12, die eine Verbindung nach einem der Ansprüche 1 bis 9 als Hostmaterial in Verbinung mit einem Phosphoreszenzemitter umfasst.

14. Einrichtung, die aus der Gruppe ausgewählt ist, die aus stationären visuellen Anzeigeeinheiten; mobilen visuellen Anzeigeeinheiten; Beleuchtungseinheiten, Tastaturen; Kleidungsstücken; Möbeln; Tapeten besteht, und die die organische elektronische Vorrichtung nach Anspruch 10 oder 11 oder die Lochtransportschicht oder die Emissionsschicht nach Anspruch 12 umfasst.

15. Verwendung der Verbindungen mit der Formel I nach einem der Ansprüche 1 bis 9 für elektrofotografische Fotorezeptoren, fotoelektrische Wandler, organische Solarzellen, Schaltelemente, organische lichtemittierende Feldeffekttransistoren, Bildsensoren, Farbstofflaser und Elektrolumineszenzvorrichtungen.

## Revendications

1. Composé de formule 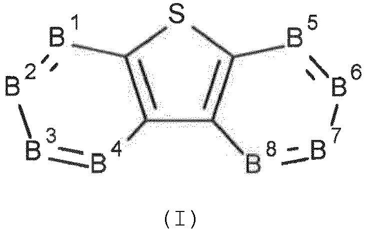 dans lequel
B¹ est N ou CR⁸¹,
B² est N ou CR⁸²,
B³ est N ou CR⁸³,
B⁴ est N ou CR⁸⁴,
B⁵ est N ou CR⁸⁵,
B⁶ est N ou CR⁸⁶,
B⁷ est N ou CR⁸⁷,
B⁸ est N ou CR⁸⁸,
R⁸¹ R⁸², R⁸³, R⁸⁴, R⁸⁵, R⁸⁶, R⁸⁷ et R⁸⁸ sont chacun indépendamment des autres H, F, un groupe cyano, un groupe alkyle en C₁-C₂₅, qui peut éventuellement être substitué par E et/ou interrompu par D ; un groupe alcoxy en C₁-C₂₅ ; un groupe cycloalkyle en C₃-C₁₈, qui peut éventuellement être substitué par E ; un groupe aryle en C₆-C₂₄, qui peut éventuellement être substitué par G, un groupe hétéroaryle en C₂-C₃₀, qui peut éventuellement être substitué par G ; ou un groupe de formule -(A)ₒ-(A²)ₚ-(A³)_{q}-(A⁴)ᵣR¹⁶,
o vaut 0 ou 1, p vaut 0 ou 1, q vaut 0 ou 1, r vaut 0 ou 1,
A¹, A², A³ et A⁴ sont chacun indépendamment des autres un groupe arylène en C₆-C₂₄, qui peut éventuellement être substitué par G, ou un groupe hétéroarylène en C₂-C₃₀, qui peut éventuellement être substitué par G ;
R¹⁶ est -NR¹⁰R¹¹ ou -Si(R¹²)(R¹³)(R¹⁴), un groupe aryle en C₆-C₂₄, qui peut éventuellement être substitué par G ; ou un groupe hétéroaryle en C₂-C₃₀, qui peut éventuellement être substitué par G ;
R¹⁰ et R¹¹ sont chacun indépendamment de l'autre un groupe aryle en C₆-C₂₄, qui peut éventuellement être substitué par G ; ou un groupe hétéroaryle en C₂-C₃₀, qui peut éventuellement être substitué par G ;
R¹², R¹³ et R¹⁴ sont chacun indépendamment des autres un groupe alkyle en C₁-C₂₅, qui peut éventuellement être substitué par E et/ou interrompu par D ; un groupe aryle en C₆-C₂₄, qui peut éventuellement être substitué par G ; ou un groupe hétéroaryle en C₂-C₃₀, qui peut éventuellement être substitué par G ;
D est -CO-, -COO-, -S-, -SO-, -SO₂-, -O-, -NR⁶⁵-, -SiR⁷⁰R⁷¹-, -POR⁷²-, -CR⁶³=CR⁶⁴- ou -C≡C-,
E est -OR⁶⁹, -SR⁶⁹, -NR⁶⁵R⁶⁶, -COR⁶⁸, -COOR⁶⁷, CONR⁶⁵R⁶⁶, -CN ou F,
G est E ou un groupe alkyle en C₁-C₁₈, un groupe cycloalkyle en C₃-C₁₈, un groupe aryle en C₆-C₂₄, un groupe aryle en C₆-C₂₄ qui est substitué par F, alkyle en C₁-C₁₈ ou alkyle en C₁-C₁₈ qui est interrompu par O ; un groupe hétéroaryle en C₂-C₃₀ ou un groupe hétéroaryle en C₂-C₃₀ qui est substitué par F, alkyle en C₁-C₁₈ ou alkyle en C₁-C₁₈ qui est interrompu par O ;
R⁶³ et R⁶⁴ sont chacun indépendamment de l'autre H ou un groupe aryle en C₆-C₁₈ ; aryle en C₆-C₁₈ qui est substitué par alkyle en C₁-C₁₈ ou alcoxy en C₁-C₁₈ ; alkyle en C₁-C₁₈ ; ou alkyle en C₁-C₁₈ qui est interrompu par -O- ;
R⁶⁵ et R⁶⁶ sont chacun indépendamment de l'autre un groupe aryle en C₆-C₁₈ ; un groupe aryle en C₆-C₁₈ qui est substitué par alkyle en C₁-C₁₈ ou alcoxy en C₁-C₁₈ ; un groupe alkyle en C₁-C₁₈ ; ou un groupe alkyle en C₁-C₁₈ qui est interrompu par -O- ; ou
R⁶⁵ et R⁶⁶ forment ensemble un cycle à cinq ou six chaînons,
R⁶⁷ est un groupe aryle en C₆-C₁₈ ; un groupe aryle en C₆-C₁₈ qui est substitué par alkyle en C₁-C₁₈ ou alcoxy en C₁-C₁₈ ; un groupe alkyle en C₁-C₁₈ ; ou un groupe alkyle en C₁-C₁₈ qui est interrompu par -O-,
R⁶⁸ est H ; un groupe aryle en C₆-C₁₈ ; un groupe aryle en C₆-C₁₈ qui est substitué par alkyle en C₁-C₁₈ ou alcoxy en C₁-C₁₈ ; un groupe alkyle en C₁-C₁₈ ; ou un groupe alkyle en C₁-C₁₈ qui est interrompu par -O-,
R⁶⁹ est un groupe aryle en C₆-C₁₈ ; un groupe aryle en C₆-C₁₈ qui est substitué par alkyle en C₁-C₁₈ ou alcoxy en C₁-C₁₈ ; un groupe alkyle en C₁-C₁₈ ; ou un groupe alkyle en C₁-C₁₈ qui est interrompu par -O-,
R⁷⁰ et R⁷¹ sont chacun indépendamment de l'autre un groupe alkyle en C₁-C₁₈, un groupe aryle en C₆-C₁₈ ou un groupe aryle en C₆-C₁₈ qui est substitué par alkyle en C₁-C₁₈ et
R⁷² est un groupe alkyle en C₁-C₁₈, un groupe aryle en C₆-C₁₈ ou un groupe aryle en C₆-C₁₈ qui est substitué par alkyle en C₁-C₁₈,
à condition que
au moins l'un des substituants B¹, B², B³, B⁴, B⁵, B⁶, B⁷ et B⁸ représente N ;
pas plus de deux des groupes B¹, B², B³ et B⁴ ne représentent N ; et
pas plus de deux des groupes B⁵, B⁶, B⁷ et B⁸ ne représentent N ; et
à condition en outre qu'au moins l'un des substituants R⁸¹, R⁸², R⁸³, R⁸⁴, R⁸⁵, R⁸⁶, R⁸⁷ et R⁸⁸ représente un groupe de formule -(A¹)ₒ-(A²)ₚ-(A³)_{q}-(A⁴)ᵣR¹⁶, dans lequel R¹⁶ représente un groupe benzimidazo[1,2-a]benzimidazo-5-yle, qui peut éventuellement être substitué par G ; et/ou au moins l'un des groupes A¹, A², A³ et A⁴ représente un groupe benzimidazo[1,2-a]benzimidazo-2,5-ylène, qui peut éventuellement être substitué par G.

2. Composé selon la revendication 1, qui est un composé de formule 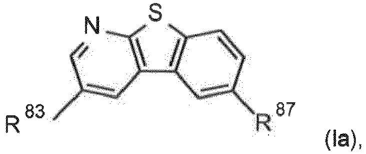 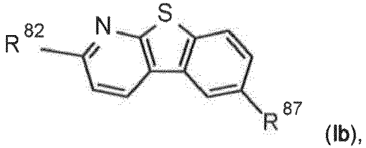 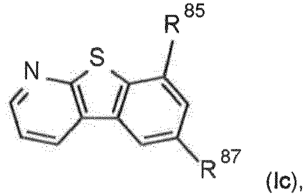 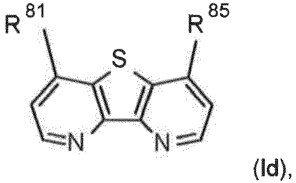 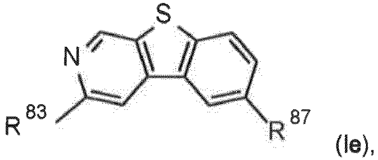 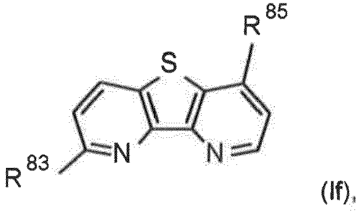 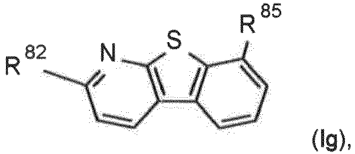 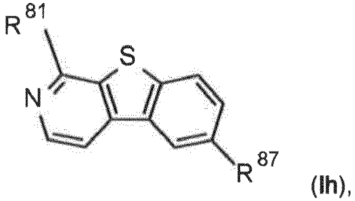 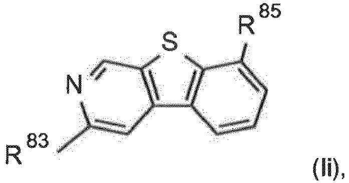 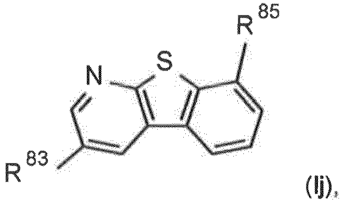 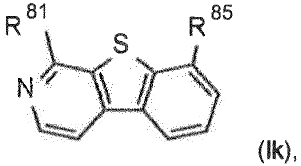 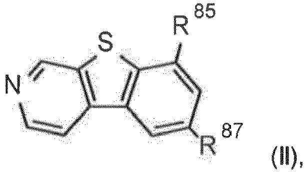 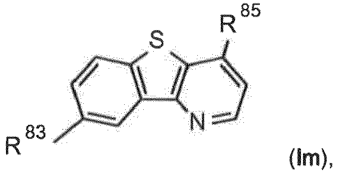 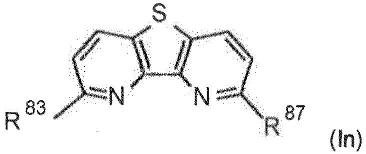 ou 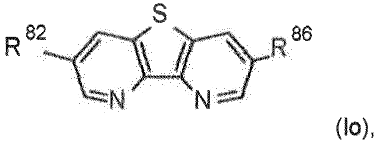 dans lequel R⁸¹, R⁸², R⁸³, R⁸⁵, R⁸⁶ et R⁸⁷ sont tels que définis dans la revendication 1.

3. Composé selon la revendication 2, dans lequel dans les composés de formule (Ia)
R⁸³ est un groupe de formule -(A¹)ₒ-(A²)_{P}-(A³)_{q}-(A⁴)ᵣR¹⁶ ; et
R⁸⁷ est H, un groupe alkyle en C₁-C₂₅ ou un groupe de formule -(A¹)ₒ-(A²)_{P}-(A³)_{q}-(A⁴)ᵣR^{16'}; ou
R⁸³ est H, un groupe alkyle en C₁-C₂₅ ou un groupe de formule -(A¹)ₒ-(A²)_{P}-(A³)_{q}-(A⁴)ᵣR^{16'} ; et
R⁸⁷ est un groupe de formule -(A¹)ₒ(A²)_{P}-(A³)_{q}-(A⁴)ᵣR¹⁶ ;
dans les composés de formule (Ib)
R⁸² est un groupe de formule -(A¹)ₒ(A²)_{P}-(A³)_{q}-(A⁴)ᵣR¹⁶ ; et
R⁸⁷ est H, un groupe alkyle en C₁-C₂₅ ou un groupe de formule -(A¹)ₒ-(A²)_{P}-(A³)_{q}-(A⁴)ᵣR^{16'} ; ou
R⁸² est H, un groupe alkyle en C₁-C₂₅ ou un groupe de formule -(A¹)ₒ⁻(A²)_{P}-(A³)_{q}-(A⁴)ᵣR^{16'}; et
R⁸⁷ est un groupe de formule -(A¹)ₒ-(A²)ₚ-(A³)_{q}-(A⁴)ᵣR¹⁶ ;
dans les composés de formule (Ic)
R⁸⁵ est un groupe de formule -(A¹)ₒ-(A²)ₚ-(A³)_{q}-(A⁴)ᵣR¹⁶ ; et
R⁸⁷ est H, un groupe alkyle en C₁-C₂₅ ou un groupe de formule -(A¹)ₒ-(A²)_{P}-(A³)_{q}-(A⁴)ᵣR^{16'}; ou
R⁸⁵ est H, un groupe alkyle en C₁-C₂₅ ou un groupe de formule -(A¹)ₒ-(A²)_{P}-(A³)_{q}-(A⁴)ᵣR^{16'}; et
R⁸⁷ est un groupe de formule -(A¹)ₒ-(A²)_{P}-(A³)_{q}-(A⁴)ᵣR¹⁶ ;
dans les composés de formule (Id)
R⁸¹ est un groupe de formule -(A¹)ₒ-(A²)_{P}-(A³)_{q}-(A⁴)ᵣR¹⁶ ; et
R⁸⁵ est H, un groupe alkyle en C₁-C₂₅ ou un groupe de formule -(A¹)ₒ-(A²)_{P}-(A³)_{q}-(A⁴)ᵣR^{16'} ; ou
R⁸¹ est H, un groupe alkyle en C₁-C₂₅ ou un groupe de formule -(A¹)ₒ-(A²)_{P}-(A³)_{q}-(A⁴)ᵣR^{16'} ; et
R⁸⁵ est un groupe de formule -(A¹)ₒ-(A²)_{P}-(A³)_{q}-(A⁴)ᵣR¹⁶ ;
dans les composés de formule (Ie)
R⁸³ est un groupe de formule -(A¹)ₒ-(A²)_{P}-(A³)_{q}-(A⁴)ᵣR¹⁶ ; et
R⁸⁷ est H, un groupe alkyle en C₁-C₂₅ ou un groupe de formule -(A¹)ₒ-(A²)_{P}-(A³)_{q}-(A⁴)ᵣR^{16'} ; ou
R⁸³ est H, un groupe alkyle en C₁-C₂₅ ou un groupe de formule - (A¹)ₒ-(A²)_{P}-(A³)_{q}-(A⁴)ᵣR^{16'} ; et
R⁸⁷ est un groupe de formule -(A¹)ₒ-(A²)_{P}-(A³)_{q}-(A⁴)ᵣR¹⁶ ;
dans les composés de formule (If)
R⁸³ est un groupe de formule -(A¹)ₒ-(A²)_{P}-(A³)_{q}-(A⁴)ᵣR¹⁶ ; et
R⁸⁵ est H, un groupe alkyle en C₁-C₂₅ ou un groupe de formule -(A¹)ₒ-(A²)_{P}-(A³)_{q}-(A⁴)ᵣR^{16'} ; ou
R⁸³ est H, un groupe alkyle en C₁-C₂₅ ou un groupe de formule -(A¹)ₒ-(A²)_{P}-(A³)_{q}-(A⁴)ᵣR^{16'} ; et
R⁸⁵ est un groupe de formule -(A¹)ₒ-(A²)_{P}-(A³)_{q}-(A⁴)ᵣR¹⁶ ;
dans les composés de formule (Ig)
R⁸² est un groupe de formule -(A¹)ₒ-(A²)_{P}-(A³)_{q}-(A⁴)ᵣR¹⁶ ; et
R⁸⁵ est H, un groupe alkyle en C₁-C₂₅ ou un groupe de formule -(A¹)ₒ-(A²)_{P}-(A³)_{q}-(A⁴)ᵣR^{16'} ; ou
R⁸² est H, un groupe alkyle en C₁-C₂₅ ou un groupe de formule -(A¹)ₒ-(A²)_{P}-(A³)_{q}-(A⁴)ᵣR^{16'} ; et
R⁸⁵ est un groupe de formule -(A¹)ₒ-(A²)_{P}-(A³)_{q}-(A⁴)ᵣR¹⁶ ;
dans les composés de formule (Ih)
R⁸¹ est un groupe de formule -(A¹)ₒ-(A²)_{P}-(A³)_{q}-(A⁴)ᵣR¹⁶ ; et
R⁸⁷ est H, un groupe alkyle en C₁-C₂₅ ou un groupe de formule -(A¹)ₒ-(A²)_{P}-(A³)_{q}-(A⁴)ᵣR^{16'} ; ou
R⁸¹ est H, un groupe alkyle en C₁-C₂₅ ou un groupe de formule -(A¹)ₒ-(A²)_{P}-(A³)_{q}-(A⁴)ᵣR^{16'} ; et
R⁸⁷ est un groupe de formule -(A¹)ₒ-(A²)_{P}-(A³)_{q}-(A⁴)ᵣR¹⁶ ;
dans les composés de formule (Ii)
R⁸³ est un groupe de formule -(A¹)ₒ-(A²)_{P}-(A³)_{q}-(A⁴)ᵣR¹⁶ ; et
R⁸⁵ est H, un groupe alkyle en C₁-C₂₅ ou un groupe de formule -(A¹)ₒ-(A²)_{P}-(A³)_{q}-(A⁴)ᵣR^{16'} ; ou
R⁸³ est H, un groupe alkyle en C₁-C₂₅ ou un groupe de formule -(A¹)ₒ-(A²)_{P}-(A³)_{q}-(A⁴)ᵣR^{16'} ; et
R⁸⁵ est un groupe de formule -(A¹)ₒ-(A²)_{P}-(A³)_{q}-(A⁴)ᵣR¹⁶ ;
dans les composés de formule (Ij)
R⁸³ est un groupe de formule -(A¹)ₒ-(A²)_{P}-(A³)_{q}-(A⁴)ᵣR¹⁶ ; et
R⁸⁵ est H, un groupe alkyle en C₁-C₂₅ ou un groupe de formule -(A¹)ₒ-(A²)_{P}-(A³)_{q}-(A⁴)ᵣR^{16'} ; ou
R⁸³ est H, un groupe alkyle en C₁-C₂₅ ou un groupe de formule -(A¹)ₒ-(A²)_{P}-(A³)_{q}-(A⁴)ᵣR^{16'} ; et
R⁸⁵ est un groupe de formule -(A¹)ₒ-(A²)_{P}-(A³)_{q}-(A⁴)ᵣR¹⁶ ;
dans les composés de formule (Ik)
R⁸¹ est un groupe de formule -(A¹)ₒ-(A²)_{P}-(A³)_{q}-(A⁴)ᵣR¹⁶ ; et
R⁸⁵ est H, un groupe alkyle en C₁-C₂₅ ou un groupe de formule -(A¹)ₒ-(A²)_{P}-(A³)_{q}-(A⁴)ᵣR^{16'} ; ou
R⁸¹ est H, un groupe alkyle en C₁-C₂₅ ou un groupe de formule -(A¹)ₒ-(A²)_{P}-(A³)_{q}-(A⁴)ᵣR^{16'} ; et
R⁸⁵ est un groupe de formule -(A¹)ₒ-(A²)_{P}-(A³)_{q}-(A⁴)ᵣR¹⁶ ;
dans les composés de formule (Il)
R⁸⁵ est un groupe de formule -(A¹)ₒ-(A²)_{P}-(A³)_{q}-(A⁴)ᵣR¹⁶ ; et
R⁸⁷ est H, un groupe alkyle en C₁-C₂₅ ou un groupe de formule -(A¹)ₒ-(A²)_{P}-(A³)_{q}-(A⁴)ᵣR^{16'} ; ou
R⁸⁵ est H, un groupe alkyle en C₁-C₂₅ ou un groupe de formule -(A¹)ₒ-(A²)_{P}-(A³)_{q}-(A⁴)ᵣR^{16'} ; et
R⁸⁷ est un groupe de formule -(A¹)ₒ-(A²)_{P}-(A³)_{q}-(A⁴)ᵣR¹⁶ ;
dans les composés de formule (Im)
R⁸³ est un groupe de formule -(A¹)ₒ-(A²)_{P}-(A³)_{q}-(A⁴)ᵣR¹⁶ ; et
R⁸⁵ est H, un groupe alkyle en C₁-C₂₅ ou un groupe de formule -(A¹)ₒ-(A²)_{P}-(A³)_{q}-(A⁴)ᵣR^{16'} ; ou
R⁸³ est H, un groupe alkyle en C₁-C₂₅ ou un groupe de formule -(A¹)ₒ-(A²)_{P}-(A³)_{q}-(A⁴)ᵣR^{16'} ; et
R⁸⁵ est un groupe de formule -(A¹)ₒ-(A²)_{P}-(A³)_{q}-(A⁴)ᵣR¹⁶ ;
dans les composés de formule (In)
R⁸³ est un groupe de formule -(A¹)ₒ-(A²)_{P}-(A³)_{q}-(A⁴)ᵣR¹⁶ ; et
R⁸⁷ est H, un groupe alkyle en C₁-C₂₅ ou un groupe de formule -(A¹)ₒ-(A²)_{P}-(A³)_{q}-(A⁴)ᵣR^{16'} ; ou
R⁸³ est H, un groupe alkyle en C₁-C₂₅ ou un groupe de formule -(A¹)ₒ-(A²)_{P}-(A³)_{q}-(A⁴)ᵣR^{16'} ; et
R⁸⁷ est un groupe de formule -(A¹)ₒ-(A²)_{P}-(A³)_{q}-(A⁴)ᵣR¹⁶ ; et
dans les composés de formule (Io)
R⁸² est un groupe de formule -(A¹)ₒ-(A²)_{P}-(A³)_{q}-(A⁴)ᵣR¹⁶ ; et
R⁸⁶ est H, un groupe alkyle en C₁-C₂₅ ou un groupe de formule -(A¹)ₒ-(A²)_{P}-(A³)_{q}-(A⁴)ᵣR^{16'} ; ou
R⁸² est H, un groupe alkyle en C₁-C₂₅ ou un groupe de formule -(A¹)ₒ-(A²)_{P}-(A³)_{q}-(A⁴)ᵣR^{16'} ; et
R⁸⁶ est un groupe de formule -(A¹)ₒ-(A²)_{P}-(A³)_{q}-(A⁴)ᵣR¹⁶ ;
o valant 0 ou 1, p valant 0 ou 1, q valant 0 ou 1, r valant 0 ou 1 ;
A¹, A², A³ et A⁴ étant chacun indépendamment des autres un groupe de formule 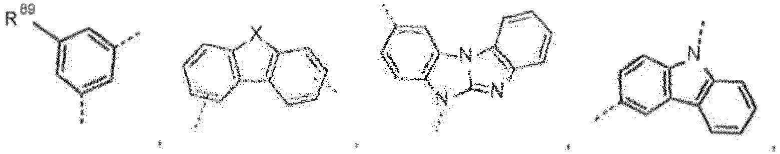 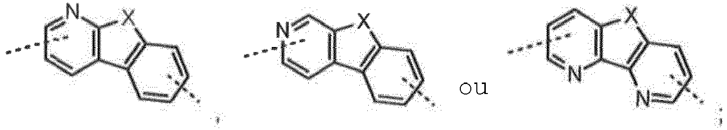 R¹⁶ étant un groupe de formule 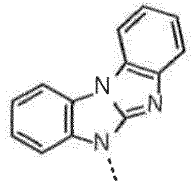 ou R¹⁶ ayant la signification de R^{16'}, si au moins l'un des groupes A¹, A², A³ et A⁴ représente un groupe de formule 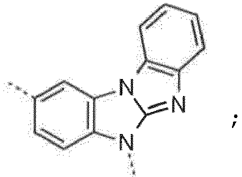 R^{16'} étant H ou un groupe de formule -Si(R¹²)(R¹³)(R¹⁴), 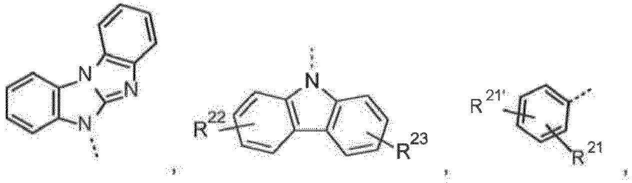 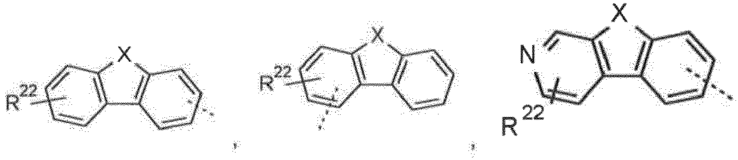 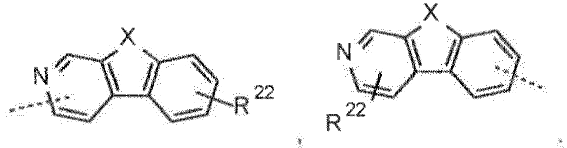 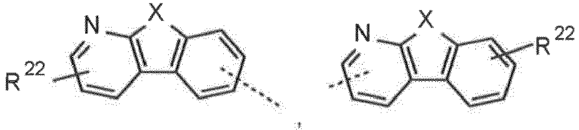 ou 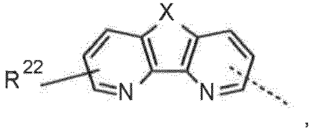 dans lequel
R¹², R¹³ et R¹⁴ sont chacun indépendamment des autres un groupe phényle, qui peut éventuellement être substitué par un ou plusieurs groupes alkyle ;
R²¹ et R^{21'} sont chacun indépendamment de l'autre H, un groupe phényle ou un groupe alkyle en C₁-C₁₈ ;
R²² et R²³ sont chacun indépendamment de l'autre H ou un groupe de formule 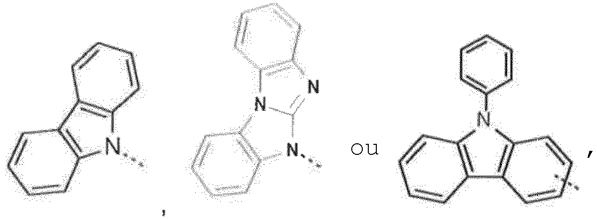
X est O, S ou NR²⁴,
R²⁴ est un groupe aryle en C₆-C₂₄ ou un groupe hétéroaryle en C₂-C₃₀, qui peut éventuellement être substitué par G, G étant tel que défini dans la revendication 1 ; et
R⁸⁹ est H, un groupe de formule

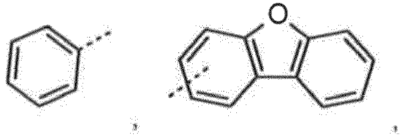

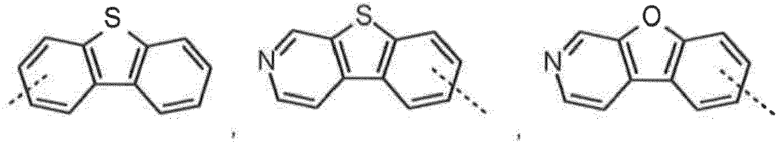

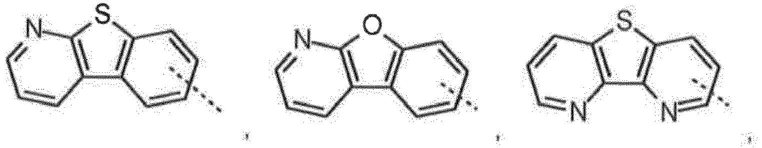

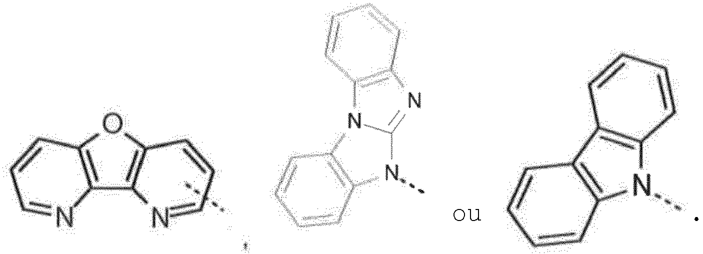

4. Composé selon les revendications 2 ou 3, dans lequel
o vaut 0 ou 1, p vaut 0 ou 1, q vaut 0 ou 1, r vaut 0 ou 1,
A¹, A², A³ et A⁴ sont chacun indépendamment des autres un groupe de formule 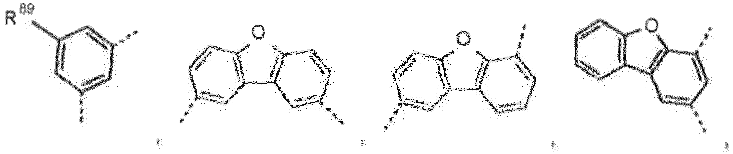 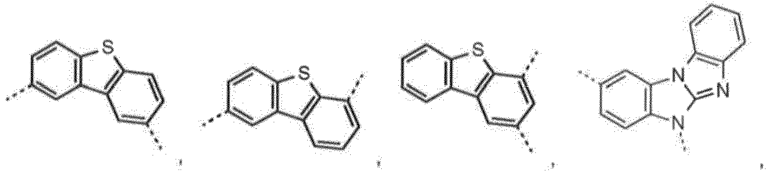 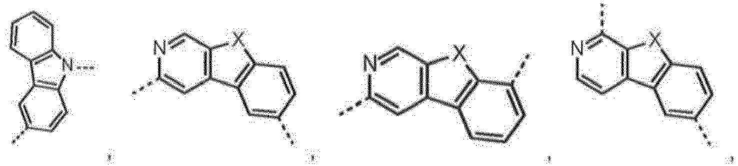 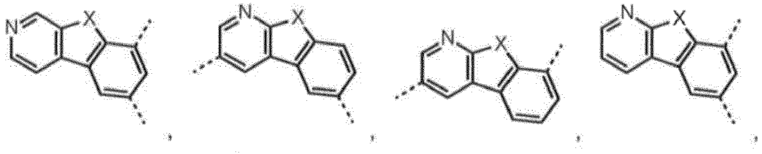 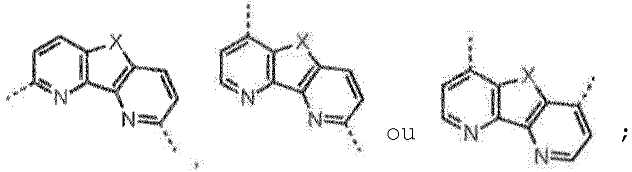 R¹⁶ est un groupe de formule 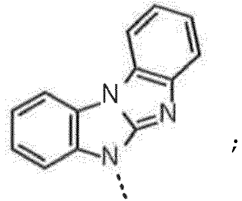 ou R¹⁶ a la signification de R^{16'}, si au moins l'un des groupes A¹, A², A³ et A⁴ représente un groupe de formule 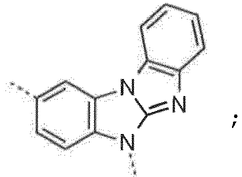 R^{16'} est H ou un groupe de formule 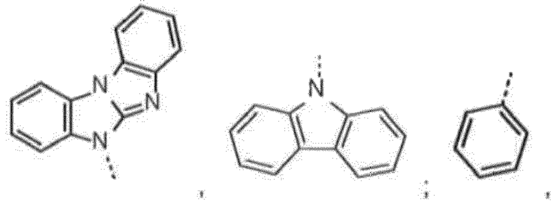 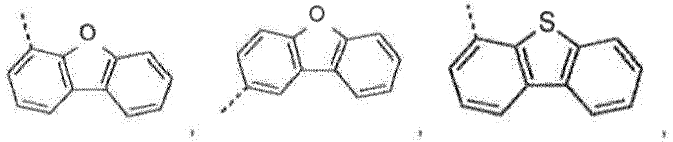 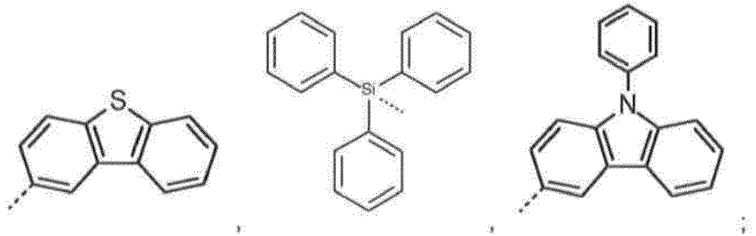 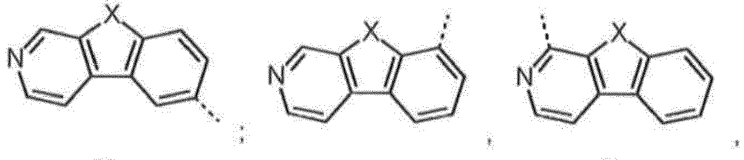 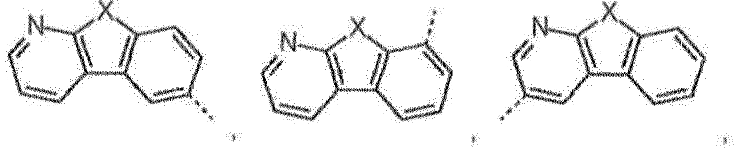 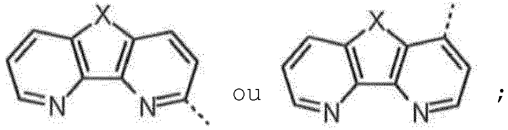 et
R⁸⁹ est H, un groupe de formule 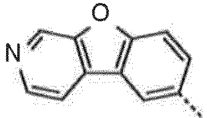 ou 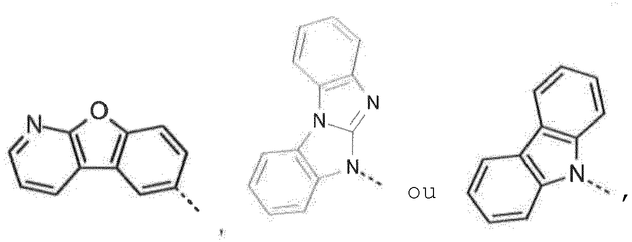 X étant O, S ou NR²⁴,
R²⁴ étant

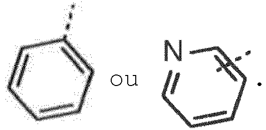

5. Composé selon l'une quelconque des revendications 2 à 4, dans lequel le groupe de formule -(A¹)ₒ-(A²)ₚ-(A³)_{q}-(A⁴)ᵣR¹⁶ est un groupe de formule 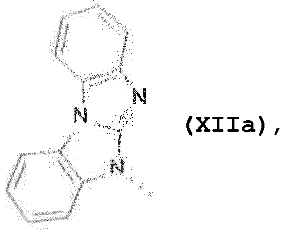 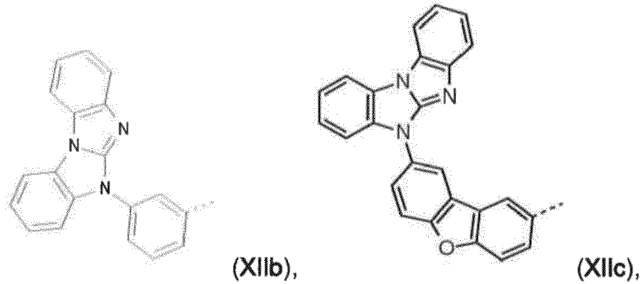 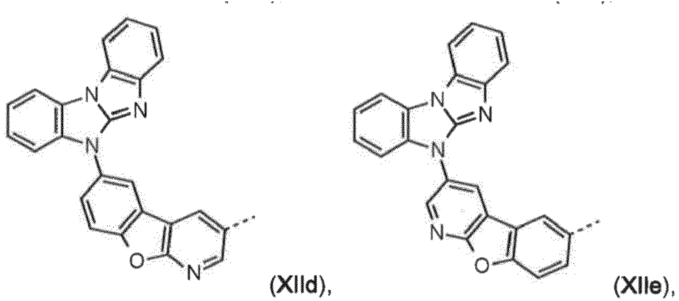 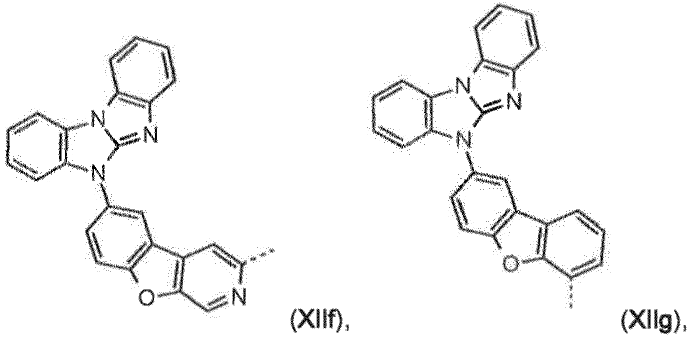 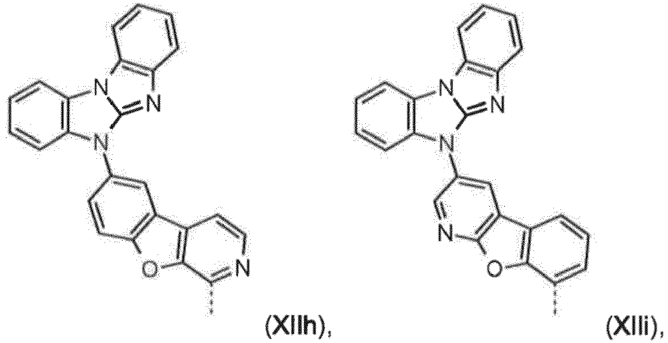 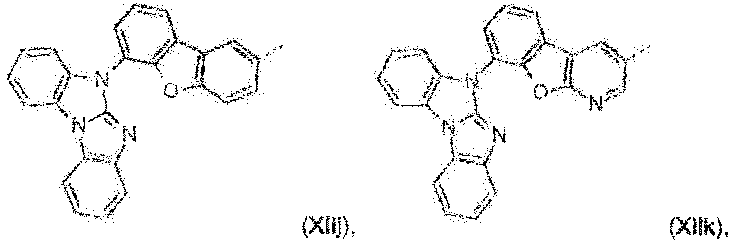 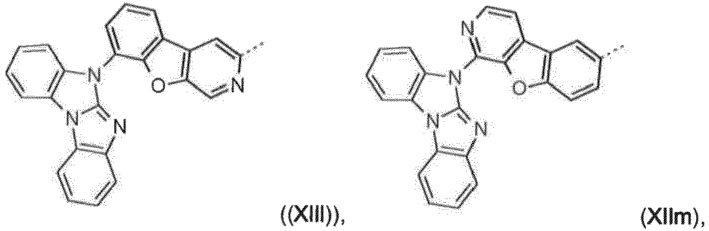 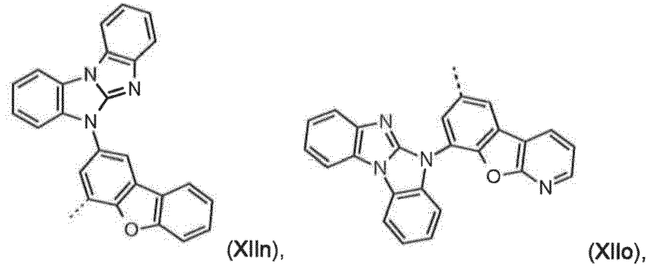 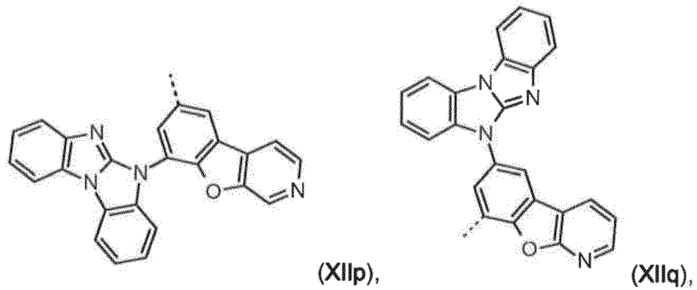 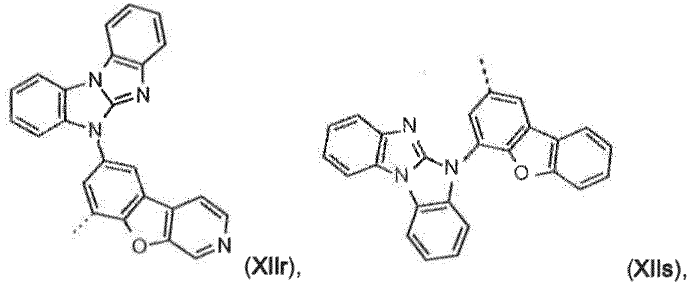 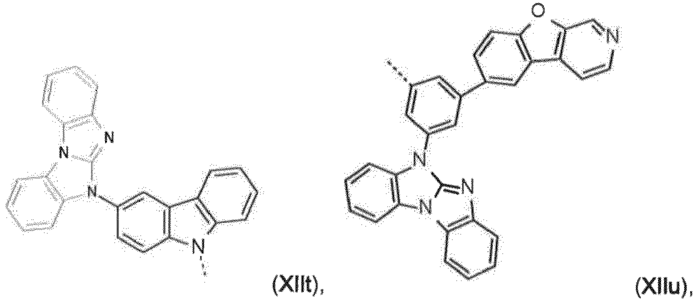 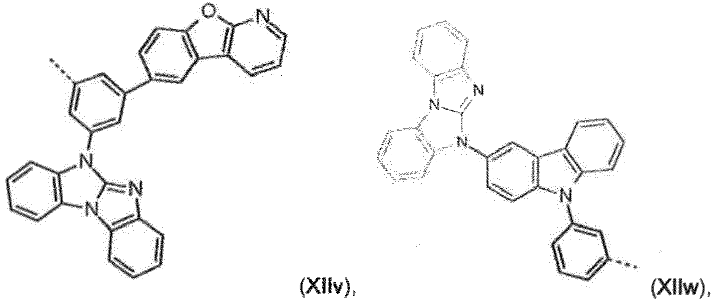 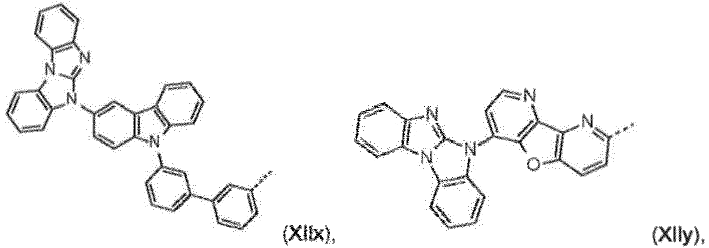 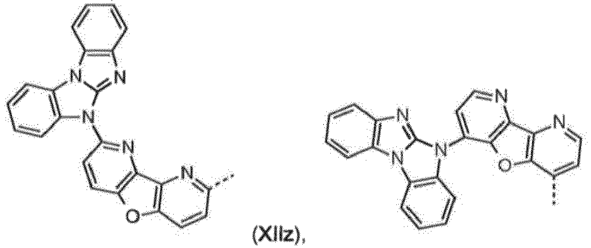 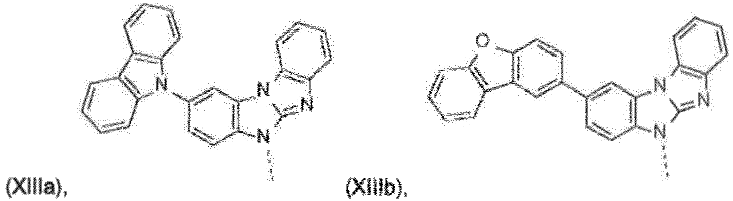 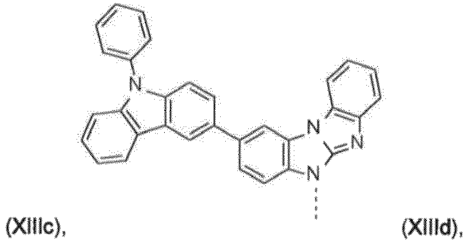 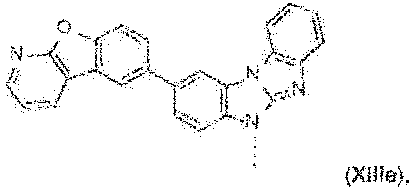 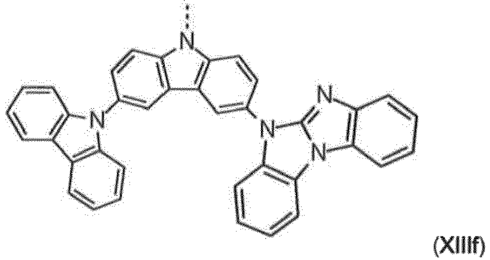 ou

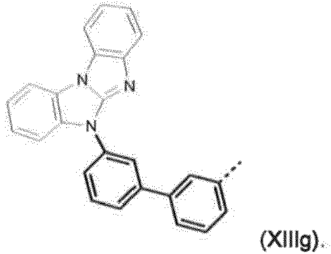

6. Composé selon l'une quelconque des revendications 2 à 5, dans lequel le groupe de formule -(A¹)ₒ-(A²)ₚ-(A³)_{q}-(A⁴)ᵣR^{16'} est H, un groupe alkyle en C₁-C₂₅ ou un groupe de formule (XIIa), (XIIb), (XIIc), (XIId), (XIIe), (XIIf), (XIIg), (XIIh), (XIIi), (XIIj), (XIIk), ((XIIl)), (XIIm), (XIIn), (XIIo), (XIIp), (XIIq), (XIIr), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) ou (XIIIg), tel que défini dans la revendication 5 ; ou un groupe de formule 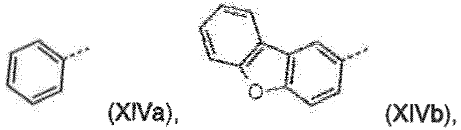 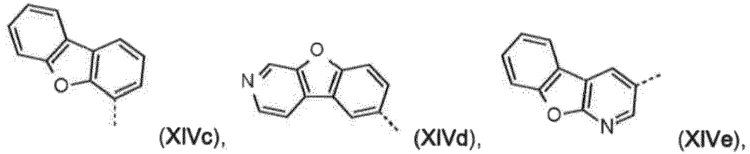 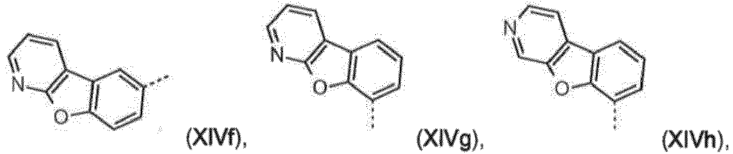 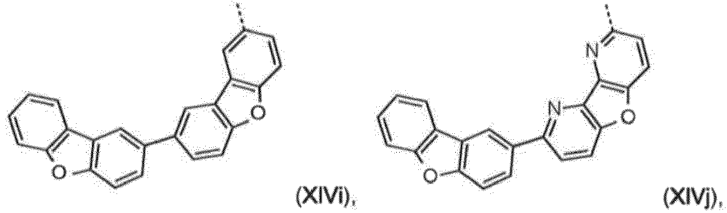 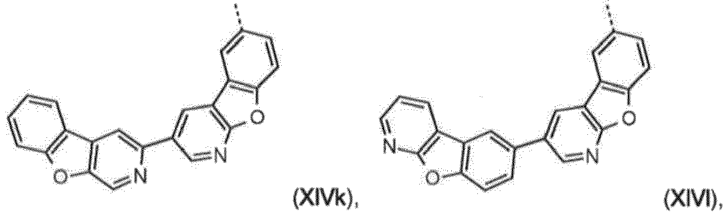 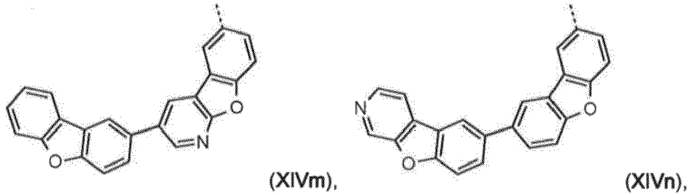 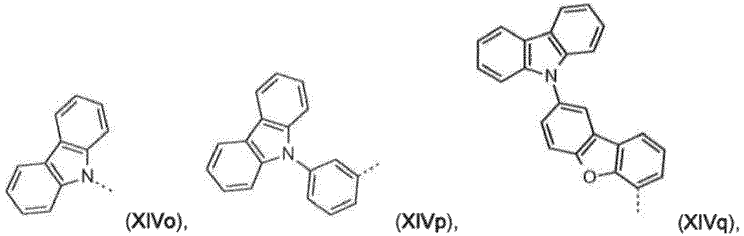 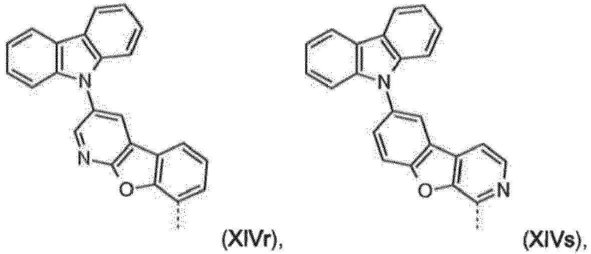 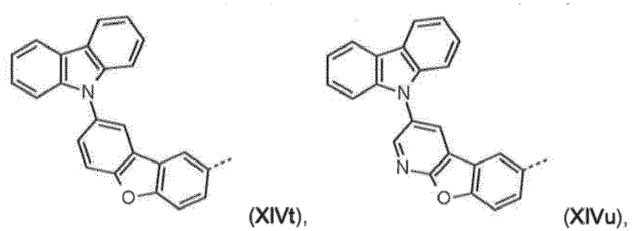 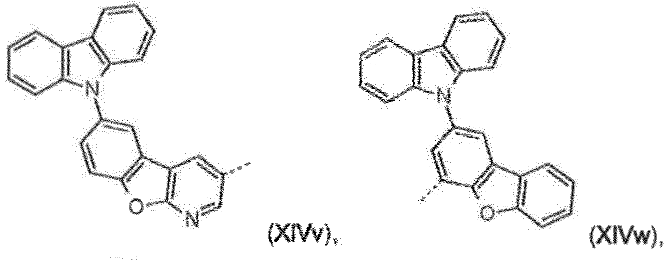 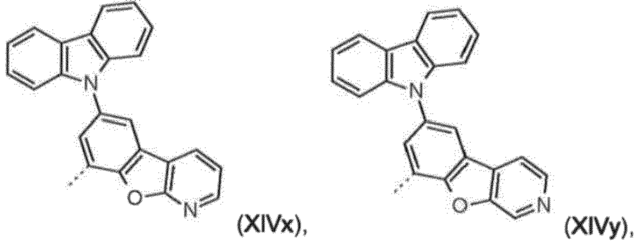 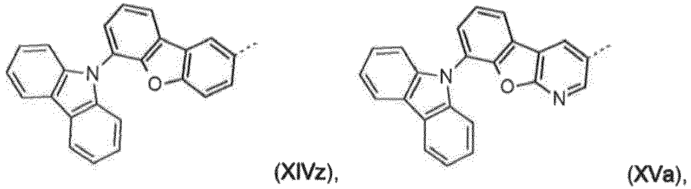 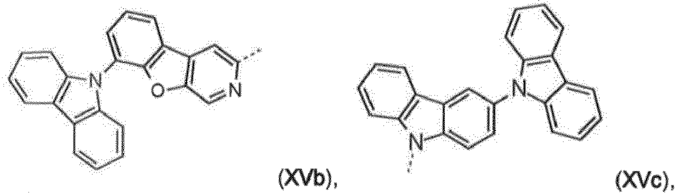 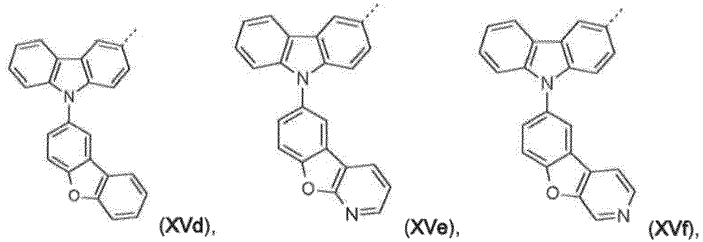 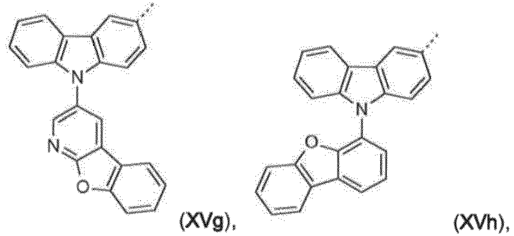 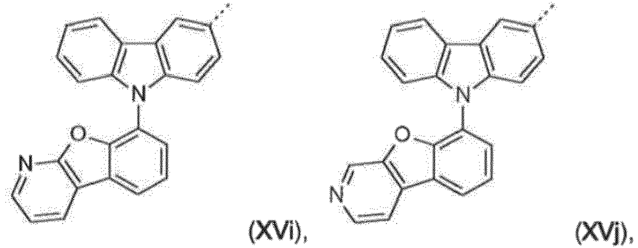 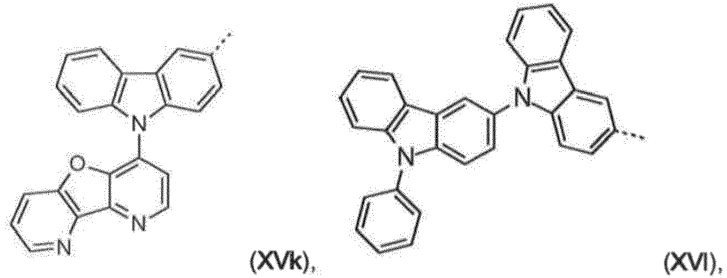 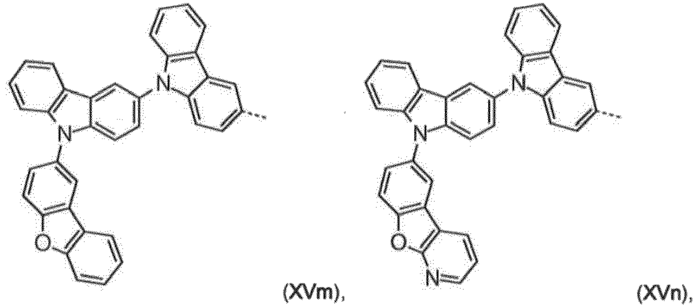 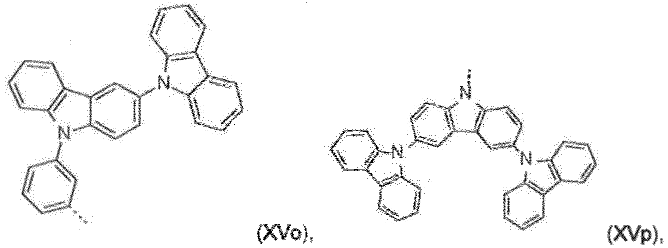 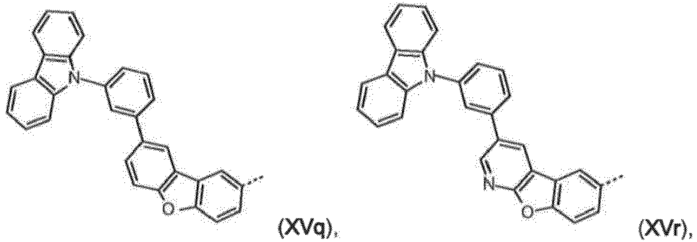 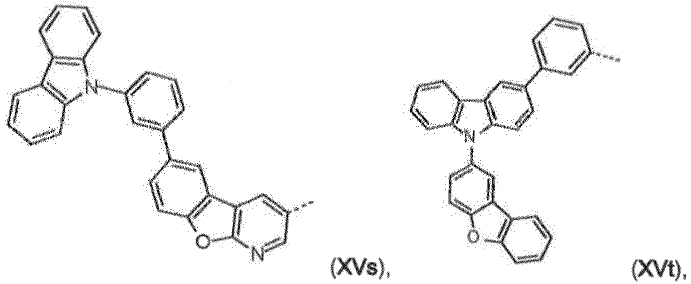 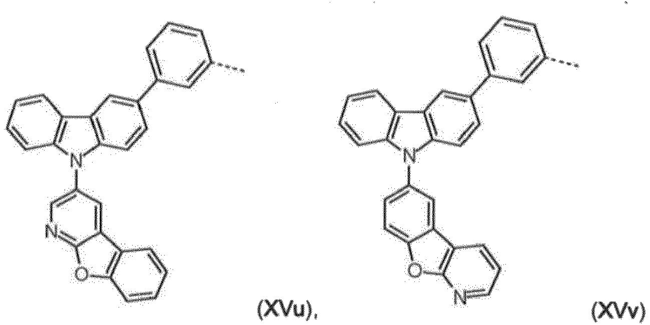 ou 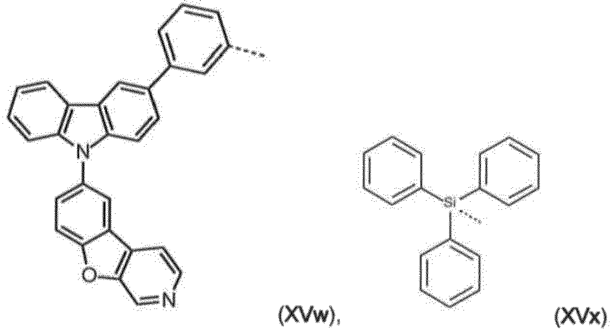 ou 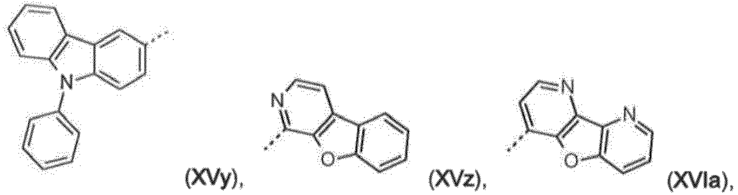 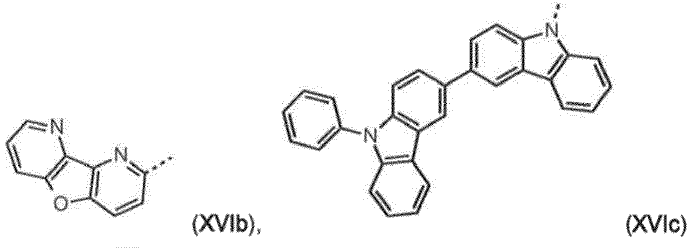 ou

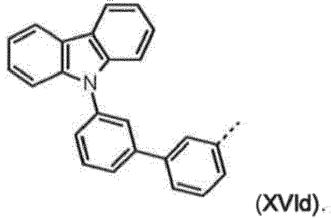

7. Composé selon l'une quelconque des revendications 2 à 6, qui est un composé de formule (Ia), dans lequel
R⁸³ est un groupe de formule (XIIa), (XIIb), (XIIc), (XIId), (XIIe), (XIIf), (XIIg), (XIIh), (XIIi), (XIIj), (XIIk), ((XIIl)), (XIIm), (XIIn), (XIIo), (XIIp), (XIIq), (XIIr), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) ou (XIIIg) tel que défini dans la revendication 5 et
R⁸⁷ est H, un groupe alkyle en C₁-C₂₅, un groupe de formule (XIIa), (XIIb), (XIIc), (XIId), (XIIe), (XIIf), (XIIg), (XIIh), (XIIi), (XIIj), (XIIk), ((XIIl)), (XIIm), (XIIn), (XIIo), (XIIp), (XIIq), (XIIr), (XIIs), (XIIt), (XIIu), (XIIv) (XIIw), (XIIx), (XIIy), (XIIz), (XIIIa), (XIIIb), (XIIIc), (XIIId), (XIIIe), (XIIIf) ou (XIIIg) tel que défini dans la revendication 5 ou un groupe de formule (XIVb), (XIVc), (XIVd), (XIVe), (XIVf), (XIVg), (XIVh), (XIVo), (XIVp), (XIVq), (XIVr), (XIVs), (XIVt), (XIVu), (XIVv), (XIVw), (XIVx), (XIVy), (XIVz), (XVa), (XVb), (XVc), (XVd), (XVe), (XVf), (XVg), (XVh), (XVi), (XVj), (XVk), (XVl), (XVm), (XVn), (XVo), (XVp), (XVq), (XVr), (XVs), (XVt), (XVu), (XVv), (XVw), (XVx), (XVy), (XVz), (XVIa), (XVIb), (XVIc) ou (XVId) tel que défini dans la revendication 6 ; ou
un composé de formule (Ia), dans lequel
R⁸⁷ est un groupe de formule (XIIa), (XIIb), (XIIc), (XIId), (XIIe), (XIIf), (XIIg), (XIIh), (XIIi), (XIIj), (XIIk), ((XIIl)), (XIIm), (XIIn), (XIIo), (XIIp), (XIIq), (XIIr), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) ou (XIIIg) tel que défini dans la revendication 5 et
R⁸³ est H, un groupe alkyle en C₁-C₂₅, un groupe de formule (XIIa), (XIIb), (XIIc), (XIId), (XIIe), (XIIf), (XIIg), (XIIh), (XIIi), (XIIj), (XIIk), ((XIIl)), (XIIm), (XIIn), (XIIo), (XIIp), (XIIq), (XIIr), (XIIs), (XIIt), (XIIu), (XIIv) (XIIw), (XIIx), (XIIy), (XIIz), (XIIIa), (XIIIb), (XIIIc), (XIIId), (XIIIe), (XIIIf) ou (XIIIg) tel que défini dans la revendication 5 ou un groupe de formule (XIVb), (XIVc), (XIVd), (XIVe), (XIVf), (XIVg), (XIVh), (XIVo), (XIVp), (XIVq), (XIVr), (XIVs), (XIVt), (XIVu), (XIVv), (XIVw), (XIVx), (XIVy), (XIVz), (XVa), (XVb), (XVc), (XVd), (XVe), (XVf), (XVg), (XVh), (XVi), (XVj), (XVk), (XVl), (XVm), (XVn), (XVo), (XVp), (XVq), (XVr), (XVs), (XVt), (XVu), (XVv), (XVw), (XVx), (XVy), (XVz), (XVIa), (XVIb), (XVIc) ou (XVId) tel que défini dans la revendication 6 ; ou
un composé de formule (Ib), dans lequel
R⁸² est un groupe de formule (XIIa), (XIIb), (XIIc), (XIId), (XIIe), (XIIf), (XIIg), (XIIh), (XIIi), (XIIj), (XIIk), ((XIIl)), (XIIm), (XIIn), (XIIo), (XIIp), (XIIq), (XIIr), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) ou (XIIIg) tel que défini dans la revendication 5 et
R⁸⁷ est H, un groupe de formule (XIIa), (XIIb), (XIIc), (XIId), (XIIe), (XIIf), (XIIg), (XIIh), (XIIi), (XIIj), (XIIk), ((XIIl)), (XIIm), (XIIn), (XIIo), (XIIp), (XIIq), (XIIr), (XIIs), (XIIt), (XIIu), (XIIv) (XIIw), (XIIx), (XIIy), (XIIz), (XIIIa), (XIIIb), (XIIIc), (XIIId), (XIIIe), (XIIIf) ou (XIIIg) tel que défini dans la revendication 5 ou un groupe de formule (XIVb), (XIVc), (XIVd), (XIVe), (XIVf), (XIVg), (XIVh), (XIVo), (XIVp), (XIVq), (XIVr), (XIVs), (XIVt), (XIVu), (XIVv), (XIVw), (XIVx), (XIVy), (XIVz), (XVa), (XVb), (XVc), (XVd), (XVe), (XVf), (XVg), (XVh), (XVi), (XVj), (XVk), (XVl), (XVm), (XVn), (XVo), (XVp), (XVq), (XVr), (XVs), (XVt), (XVu), (XVv), (XVw), (XVx), (XVy), (XVz), (XVIa), (XVIb), (XVIc) ou (XVId) tel que défini dans la revendication 6 ; ou
un composé de formule (Ib), dans lequel
R⁸⁷ est un groupe de formule (XIIa), (XIIb), (XIIc), (XIId), (XIIe), (XIIf), (XIIg), (XIIh), (XIIi), (XIIj), (XIIk), ((XIIl)), (XIIm), (XIIn), (XIIo), (XIIp), (XIIq), (XIIr), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) ou (XIIIg) tel que défini dans la revendication 5 et
R⁸² est H, un groupe de formule (XIIa), (XIIb), (XIIc), (XIId), (XIIe), (XIIf), (XIIg), (XIIh), (XIIi), (XIIj), (XIIk), ((XIIl)), (XIIm), (XIIn), (XIIo), (XIIp), (XIIq), (XIIr), (XIIs), (XIIt), (XIIu), (XIIv) (XIIw), (XIIx), (XIIy), (XIIz), (XIIIa), (XIIIb), (XIIIc), (XIIId), (XIIIe), (XIIIf) ou (XIIIg) tel que défini dans la revendication 5 ou un groupe de formule (XIVb), (XIVc), (XIVd), (XIVe), (XIVf), (XIVg), (XIVh), (XIVo), (XIVp), (XIVq), (XIVr), (XIVs), (XIVt), (XIVu), (XIVv), (XIVw), (XIVx), (XIVy), (XIVz), (XVa), (XVb), (XVc), (XVd), (XVe), (XVf), (XVg), (XVh), (XVi), (XVj), (XVk), (XVl), (XVm), (XVn), (XVo), (XVp), (XVq), (XVr), (XVs), (XVt), (XVu), (XVv), (XVw), (XVx), (XVy), (XVz), (XVIa), (XVIb), (XVIc) ou (XVId) tel que défini dans la revendication 6 ; ou
un composé de formule (Ic), dans lequel
R⁸⁵ est un groupe de formule (XIIa), (XIIb), (XIIc), (XIId), (XIIe), (XIIf), (XIIg), (XIIh), (XIIi), (XIIj), (XIIk), ((XIIl)), (XIIm), (XIIn), (XIIo), (XIIp), (XIIq), (XIIr), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) ou (XIIIg) tel que défini dans la revendication 5 et
R⁸⁷ est H, un groupe alkyle en C₁-C₂₅, un groupe de formule (XIIa), (XIIb), (XIIc), (XIId), (XIIe), (XIIf), (XIIg), (XIIh), (XIIi), (XIIj), (XIIk), ((XIIl)), (XIIm), (XIIn), (XIIo), (XIIp), (XIIq), (XIIr), (XIIs), (XIIt), (XIIu), (XIIv) (XIIw), (XIIx), (XIIy), (XIIz), (XIIIa), (XIIIb), (XIIIc), (XIIId), (XIIIe), (XIIIf) ou (XIIIg) tel que défini dans la revendication 5 ou un groupe de formule (XIVb), (XIVc), (XIVd), (XIVe), (XIVf), (XIVg), (XIVh), (XIVo), (XIVp), (XIVq), (XIVr), (XIVs), (XIVt), (XIVu), (XIVv), (XIVw), (XIVx), (XIVy), (XIVz), (XVa), (XVb), (XVc), (XVd), (XVe), (XVf), (XVg), (XVh), (XVi), (XVj), (XVk), (XVl), (XVm), (XVn), (XVo), (XVp), (XVq), (XVr), (XVs), (XVt), (XVu), (XVv), (XVw), (XVx), (XVy), (XVz), (XVIa), (XVIb), (XVIc) ou (XVId) tel que défini dans la revendication 6 ; ou
un composé de formule (Ic), dans lequel
R⁸⁷ est un groupe de formule (XIIa), (XIIb), (XIIc), (XIId), (XIIe), (XIIf), (XIIg), (XIIh), (XIIi), (XIIj), (XIIk), ((XIIl)), (XIIm), (XIIn), (XIIo), (XIIp), (XIIq), (XIIr), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) ou (XIIIg) tel que défini dans la revendication 5 et
R⁸⁵ est H, un groupe alkyle en C₁-C₂₅, un groupe de formule (XIIa), (XIIb), (XIIc), (XIId), (XIIe), (XIIf), (XIIg), (XIIh), (XIIi), (XIIj), (XIIk), ((XIIl)), (XIIm), (XIIn), (XIIo), (XIIp), (XIIq), (XIIr), (XIIs), (XIIt), (XIIu), (XIIv) (XIIw), (XIIx), (XIIy), (XIIz), (XIIIa), (XIIIb), (XIIIc), (XIIId), (XIIIe), (XIIIf) ou (XIIIg) tel que défini dans la revendication 5 ou un groupe de formule (XIVb), (XIVc), (XIVd), (XIVe), (XIVf), (XIVg), (XIVh), (XIVo), (XIVp), (XIVq), (XIVr), (XIVs), (XIVt), (XIVu), (XIVv), (XIVw), (XIVx), (XIVy), (XIVz), (XVa), (XVb), (XVc), (XVd), (XVe), (XVf), (XVg), (XVh), (XVi), (XVj), (XVk), (XVl), (XVm), (XVn), (XVo), (XVp), (XVq), (XVr), (XVs), (XVt), (XVu), (XVv), (XVw), (XVx), (XVy), (XVz), (XVIa), (XVIb), (XVIc) ou (XVId) tel que défini dans la revendication 6 ; ou
un composé de formule (Id), dans lequel
R⁸¹ est un groupe de formule (XIIa), (XIIb), (XIIc), (XIId), (XIIe), (XIIf), (XIIg), (XIIh), (XIIi), (XIIj), (XIIk), ((XIIl)), (XIIm), (XIIn), (XIIo), (XIIp), (XIIq), (XIIr), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) ou (XIIIg) tel que défini dans la revendication 5 et
R⁸⁵ est H, un groupe de formule (XIIa), (XIIb), (XIIc), (XIId), (XIIe), (XIIf), (XIIg), (XIIh), (XIIi), (XIIj), (XIIk), ((XIIl)), (XIIm), (XIIn), (XIIo), (XIIp), (XIIq), (XIIr), (XIIs), (XIIt), (XIIu), (XIIv) (XIIw), (XIIx), (XIIy), (XIIz), (XIIIa), (XIIIb), (XIIIc), (XIIId), (XIIIe), (XIIIf) ou (XIIIg) tel que défini dans la revendication 5 ou un groupe de formule (XIVb), (XIVc), (XIVd), (XIVe), (XIVf), (XIVg), (XIVh), (XIVo), (XIVp), (XIVq), (XIVr), (XIVs), (XIVt), (XIVu), (XIVv), (XIVw), (XIVx), (XIVy), (XIVz), (XVa), (XVb), (XVc), (XVd), (XVe), (XVf), (XVg), (XVh), (XVi), (XVj), (XVk), (XVl), (XVm), (XVn), (XVo), (XVp), (XVq), (XVr), (XVs), (XVt), (XVu), (XVv), (XVw), (XVx), (XVy), (XVz), (XVIa), (XVIb), (XVIc) ou (XVId) tel que défini dans la revendication 6 ; ou
un composé de formule (Id), dans lequel
R⁸⁵ est un groupe de formule (XIIa), (XIIb), (XIIc), (XIId), (XIIe), (XIIf), (XIIg), (XIIh), (XIIi), (XIIj), (XIIk), ((XIIl)), (XIIm), (XIIn), (XIIo), (XIIp), (XIIq), (XIIr), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) ou (XIIIg) tel que défini dans la revendication 5 et
R⁸¹ est H, un groupe de formule (XIIa), (XIIb), (XIIc), (XIId), (XIIe), (XIIf), (XIIg), (XIIh), (XIIi), (XIIj), (XIIk), ((XIIl)), (XIIm), (XIIn), (XIIo), (XIIp), (XIIq), (XIIr), (XIIs), (XIIt), (XIIu), (XIIv) (XIIw), (XIIx), (XIIy), (XIIz), (XIIIa), (XIIIb), (XIIIc), (XIIId), (XIIIe), (XIIIf) ou (XIIIg) tel que défini dans la revendication 5 ou un groupe de formule (XIVb), (XIVc), (XIVd), (XIVe), (XIVf), (XIVg), (XIVh), (XIVo), (XIVp), (XIVq), (XIVr), (XIVs), (XIVt), (XIVu), (XIVv), (XIVw), (XIVx), (XIVy), (XIVz), (XVa), (XVb), (XVc), (XVd), (XVe), (XVf), (XVg), (XVh), (XVi), (XVj), (XVk), (XVl), (XVm), (XVn), (XVo), (XVp), (XVq), (XVr), (XVs), (XVt), (XVu), (XVv), (XVw), (XVx), (XVy), (XVz), (XVIa), (XVIb), (XVIc) ou (XVId) tel que défini dans la revendication 6 ; ou
un composé de formule (Ie), dans lequel
R⁸³ est un groupe de formule (XIIa), (XIIb), (XIIc), (XIId), (XIIe), (XIIf), (XIIg), (XIIh), (XIIi), (XIIj), (XIIk), ((XIIl)), (XIIm), (XIIn), (XIIo), (XIIp), (XIIq), (XIIr), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) ou (XIIIg) tel que défini dans la revendication 5 et
R⁸⁷ est H, un groupe alkyle en C₁-C₂₅, un groupe de formule (XIIa), (XIIb), (XIIc), (XIId), (XIIe), (XIIf), (XIIg), (XIIh), (XIIi), (XIIj), (XIIk), ((XIIl)), (XIIm), (XIIn), (XIIo), (XIIp), (XIIq), (XIIr), (XIIs), (XIIt), (XIIu), (XIIv) (XIIw), (XIIx), (XIIy), (XIIz), (XIIIa), (XIIIb), (XIIIc), (XIIId), (XIIIe), (XIIIf) ou (XIIIg) tel que défini dans la revendication 5 ou un groupe de formule (XIVb), (XIVc), (XIVd), (XIVe), (XIVf), (XIVg), (XIVh), (XIVo), (XIVp), (XIVq), (XIVr), (XIVs), (XIVt), (XIVu), (XIVv), (XIVw), (XIVx), (XIVy), (XIVz), (XVa), (XVb), (XVc), (XVd), (XVe), (XVf), (XVg), (XVh), (XVi), (XVj), (XVk), (XVl), (XVm), (XVn), (XVo), (XVp), (XVq), (XVr), (XVs), (XVt), (XVu), (XVv), (XVw), (XVx), (XVy), (XVz), (XVIa), (XVIb), (XVIc) ou (XVId) tel que défini dans la revendication 6 ; ou un composé de formule (Ie), dans lequel
R⁸⁷ est un groupe de formule (XIIa), (XIIb), (XIIc), (XIId), (XIIe), (XIIf), (XIIg), (XIIh), (XIIi), (XIIj), (XIIk), ((XIIl)), (XIIm), (XIIn), (XIIo), (XIIp), (XIIq), (XIIr), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) ou (XIIIg) tel que défini dans la revendication 5 et
R⁸³ est H, un groupe alkyle en C₁-C₂₅, un groupe de formule (XIIa), (XIIb), (XIIc), (XIId), (XIIe), (XIIf), (XIIg), (XIIh), (XIIi), (XIIj), (XIIk), ((XIIl)), (XIIm), (XIIn), (XIIo), (XIIp), (XIIq), (XIIr), (XIIs), (XIIt), (XIIu), (XIIv) (XIIw), (XIIx), (XIIy), (XIIz), (XIIIa), (XIIIb), (XIIIc), (XIIId), (XIIIe), (XIIIf) ou (XIIIg) tel que défini dans la revendication 5 ou un groupe de formule (XIVb), (XIVc), (XIVd), (XIVe), (XIVf), (XIVg), (XIVh), (XIVo), (XIVp), (XIVq), (XIVr), (XIVs), (XIVt), (XIVu), (XIVv), (XIVw), (XIVx), (XIVy), (XIVz), (XVa), (XVb), (XVc), (XVd), (XVe), (XVf), (XVg), (XVh), (XVi), (XVj), (XVk), (XVl), (XVm), (XVn), (XVo), (XVp), (XVq), (XVr), (XVs), (XVt), (XVu), (XVv), (XVw), (XVx), (XVy), (XVz), (XVIa), (XVIb), (XVIc) ou (XVId) tel que défini dans la revendication 6 ; ou
un composé de formule (If), dans lequel
R⁸³ est un groupe de formule (XIIa), (XIIb), (XIIc), (XIId), (XIIe), (XIIf), (XIIg), (XIIh), (XIIi), (XIIj), (XIIk), ((XIIl)), (XIIm), (XIIn), (XIIo), (XIIp), (XIIq), (XIIr), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) ou (XIIIg) tel que défini dans la revendication 5 et
R⁸⁵ est H, un groupe de formule (XIIa), (XIIb), (XIIc), (XIId), (XIIe), (XIIf), (XIIg), (XIIh), (XIIi), (XIIj), (XIIk), ((XIIl)), (XIIm), (XIIn), (XIIo), (XIIp), (XIIq), (XIIr), (XIIs), (XIIt), (XIIu), (XIIv) (XIIw), (XIIx), (XIIy), (XIIz), (XIIIa), (XIIIb), (XIIIc), (XIIId), (XIIIe), (XIIIf) ou (XIIIg) tel que défini dans la revendication 5 ou un groupe de formule (XIVb), (XIVc), (XIVd), (XIVe), (XIVf), (XIVg), (XIVh), (XIVo), (XIVp), (XIVq), (XIVr), (XIVs), (XIVt), (XIVu), (XIVv), (XIVw), (XIVx), (XIVy), (XIVz), (XVa), (XVb), (XVc), (XVd), (XVe), (XVf), (XVg), (XVh), (XVi), (XVj), (XVk), (XVl), (XVm), (XVn), (XVo), (XVp), (XVq), (XVr), (XVs), (XVt), (XVu), (XVv), (XVw), (XVx), (XVy), (XVz), (XVIa), (XVIb), (XVIc) ou (XVId) tel que défini dans la revendication 6 ; ou
un composé de formule (If), dans lequel
R⁸⁵ est un groupe de formule (XIIa), (XIIb), (XIIc), (XIId), (XIIe), (XIIf), (XIIg), (XIIh), (XIIi), (XIIj), (XIIk), ((XIIl)), (XIIm), (XIIn), (XIIo), (XIIp), (XIIq), (XIIr), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) ou (XIIIg) tel que défini dans la revendication 5 et
R⁸³ est H, un groupe de formule (XIIa), (XIIb), (XIIc), (XIId), (XIIe), (XIIf), (XIIg), (XIIh), (XIIi), (XIIj), (XIIk), ((XIIl)), (XIIm), (XIIn), (XIIo), (XIIp), (XIIq), (XIIr), (XIIs), (XIIt), (XIIu), (XIIv) (XIIw), (XIIx), (XIIy), (XIIz), (XIIIa), (XIIIb), (XIIIc), (XIIId), (XIIIe), (XIIIf) ou (XIIIg) tel que défini dans la revendication 5 ou un groupe de formule (XIVb), (XIVc), (XIVd), (XIVe), (XIVf), (XIVg), (XIVh), (XIVo), (XIVp), (XIVq), (XIVr), (XIVs), (XIVt), (XIVu), (XIVv), (XIVw), (XIVx), (XIVy), (XIVz), (XVa), (XVb), (XVc), (XVd), (XVe), (XVf), (XVg), (XVh), (XVi), (XVj), (XVk), (XVl), (XVm), (XVn), (XVo), (XVp), (XVq), (XVr), (XVs), (XVt), (XVu), (XVv), (XVw), (XVx), (XVy), (XVz), (XVIa), (XVIb), (XVIc) ou (XVId) tel que défini dans la revendication 6 ; ou
un composé de formule (Ii), dans lequel
R⁸³ est un groupe de formule (XIIa), (XIIb), (XIIc), (XIId), (XIIe), (XIIf), (XIIg), (XIIh), (XIIi), (XIIj), (XIIk), ((XIIl)), (XIIm), (XIIn), (XIIo), (XIIp), (XIIq), (XIIr), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) ou (XIIIg) tel que défini dans la revendication 5 et
R⁸⁵ est H, un groupe de formule (XIIa), (XIIb), (XIIc), (XIId), (XIIe), (XIIf), (XIIg), (XIIh), (XIIi), (XIIj), (XIIk), ((XIIl)), (XIIm), (XIIn), (XIIo), (XIIp), (XIIq), (XIIr), (XIIs), (XIIt), (XIIu), (XIIv) (XIIw), (XIIx), (XIIy), (XIIz), (XIIIa), (XIIIb), (XIIIc), (XIIId), (XIIIe), (XIIIf) ou (XIIIg) tel que défini dans la revendication 5 ou un groupe de formule (XIVb), (XIVc), (XIVd), (XIVe), (XIVf), (XIVg), (XIVh), (XIVo), (XIVp), (XIVq), (XIVr), (XIVs), (XIVt), (XIVu), (XIVv), (XIVw), (XIVx), (XIVy), (XIVz), (XVa), (XVb), (XVc), (XVd), (XVe), (XVf), (XVg), (XVh), (XVi), (XVj), (XVk), (XVl), (XVm), (XVn), (XVo), (XVp), (XVq), (XVr), (XVs), (XVt), (XVu), (XVv), (XVw), (XVx), (XVy), (XVz), (XVIa), (XVIb), (XVIc) ou (XVId) tel que défini dans la revendication 6 ; ou
un composé de formule (Ii), dans lequel
R⁸⁵ est un groupe de formule (XIIa), (XIIb), (XIIc), (XIId), (XIIe), (XIIf), (XIIg), (XIIh), (XIIi), (XIIj), (XIIk), ((XIIl)), (XIIm), (XIIn), (XIIo), (XIIp), (XIIq), (XIIr), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) ou (XIIIg) tel que défini dans la revendication 5 et
R⁸³ est H, un groupe de formule (XIIa), (XIIb), (XIIc), (XIId), (XIIe), (XIIf), (XIIg), (XIIh), (XIIi), (XIIj), (XIIk), ((XIIl)), (XIIm), (XIIn), (XIIo), (XIIp), (XIIq), (XIIr), (XIIs), (XIIt), (XIIu), (XIIv) (XIIw), (XIIx), (XIIy), (XIIz), (XIIIa), (XIIIb), (XIIIc), (XIIId), (XIIIe), (XIIIf) ou (XIIIg) tel que défini dans la revendication 5 ou un groupe de formule (XIVb), (XIVc), (XIVd), (XIVe), (XIVf), (XIVg), (XIVh), (XIVo), (XIVp), (XIVq), (XIVr), (XIVs), (XIVt), (XIVu), (XIVv), (XIVw), (XIVx), (XIVy), (XIVz), (XVa), (XVb), (XVc), (XVd), (XVe), (XVf), (XVg), (XVh), (XVi), (XVj), (XVk), (XVl), (XVm), (XVn), (XVo), (XVp), (XVq), (XVr), (XVs), (XVt), (XVu), (XVv), (XVw), (XVx), (XVy), (XVz), (XVIa), (XVIb), (XVIc) ou (XVId) tel que défini dans la revendication 6 ; ou
un composé de formule (Ij), dans lequel
R⁸³ est un groupe de formule (XIIa), (XIIb), (XIIc), (XIId), (XIIe), (XIIf), (XIIg), (XIIh), (XIIi), (XIIj), (XIIk), ((XIIl)), (XIIm), (XIIn), (XIIo), (XIIp), (XIIq), (XIIr), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) ou (XIIIg) tel que défini dans la revendication 5 et
R⁸⁵ est H, un groupe alkyle en C₁-C₂₅, un groupe de formule (XIIa), (XIIb), (XIIc), (XIId), (XIIe), (XIIf), (XIIg), (XIIh), (XIIi), (XIIj), (XIIk), ((XIIl)), (XIIm), (XIIn), (XIIo), (XIIp), (XIIq), (XIIr), (XIIs), (XIIt), (XIIu), (XIIv) (XIIw), (XIIx), (XIIy), (XIIz), (XIIIa), (XIIIb), (XIIIc), (XIIId), (XIIIe), (XIIIf) ou (XIIIg) tel que défini dans la revendication 5 ou un groupe de formule (XIVb), (XIVc), (XIVd), (XIVe), (XIVf), (XIVg), (XIVh), (XIVo), (XIVp), (XIVq), (XIVr), (XIVs), (XIVt), (XIVu), (XIVv), (XIVw), (XIVx), (XIVy), (XIVz), (XVa), (XVb), (XVc), (XVd), (XVe), (XVf), (XVg), (XVh), (XVi), (XVj), (XVk), (XVl), (XVm), (XVn), (XVo), (XVp), (XVq), (XVr), (XVs), (XVt), (XVu), (XVv), (XVw), (XVx), (XVy), (XVz), (XVIa), (XVIb), (XVIc) ou (XVId) tel que défini dans la revendication 6 ; ou
un composé de formule (Ij), dans lequel
R⁸⁵ est un groupe de formule (XIIa), (XIIb), (XIIc), (XIId), (XIIe), (XIIf), (XIIg), (XIIh), (XIIi), (XIIj), (XIIk), ((XIIl)), (XIIm), (XIIn), (XIIo), (XIIp), (XIIq), (XIIr), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) ou (XIIIg) tel que défini dans la revendication 5 et
R⁸³ est H, un groupe alkyle en C₁-C₂₅, un groupe de formule (XIIa), (XIIb), (XIIc), (XIId), (XIIe), (XIIf), (XIIg), (XIIh), (XIIi), (XIIj), (XIIk), ((XIIl)), (XIIm), (XIIn), (XIIo), (XIIp), (XIIq), (XIIr), (XIIs), (XIIt), (XIIu), (XIIv) (XIIw), (XIIx), (XIIy), (XIIz), (XIIIa), (XIIIb), (XIIIc), (XIIId), (XIIIe), (XIIIf) ou (XIIIg) tel que défini dans la revendication 5 ou un groupe de formule (XIVb), (XIVc), (XIVd), (XIVe), (XIVf), (XIVg), (XIVh), (XIVo), (XIVp), (XIVq), (XIVr), (XIVs), (XIVt), (XIVu), (XIVv), (XIVw), (XIVx), (XIVy), (XIVz), (XVa), (XVb), (XVc), (XVd), (XVe), (XVf), (XVg), (XVh), (XVi), (XVj), (XVk), (XVl), (XVm), (XVn), (XVo), (XVp), (XVq), (XVr), (XVs), (XVt), (XVu), (XVv), (XVw), (XVx), (XVy), (XVz), (XVIa), (XVIb), (XVIc) ou (XVId) tel que défini dans la revendication 6 ;
un composé de formule (Il), dans lequel
R⁸⁵ est un groupe de formule (XIIa), (XIIb), (XIIc), (XIId), (XIIe), (XIIf), (XIIg), (XIIh), (XIIi), (XIIj), (XIIk), ((XIIl)), (XIIm), (XIIn), (XIIo), (XIIp), (XIIq), (XIIr), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) ou (XIIIg) tel que défini dans la revendication 5 et
R⁸⁷ est H, un groupe de formule (XIIa), (XIIb), (XIIc), (XIId), (XIIe), (XIIf), (XIIg), (XIIh), (XIIi), (XIIj), (XIIk), ((XIIl)), (XIIm), (XIIn), (XIIo), (XIIp), (XIIq), (XIIr), (XIIs), (XIIt), (XIIu), (XIIv) (XIIw), (XIIx), (XIIy), (XIIz), (XIIIa), (XIIIb), (XIIIc), (XIIId), (XIIIe), (XIIIf) ou (XIIIg) tel que défini dans la revendication 5 ou un groupe de formule (XIVb), (XIVc), (XIVd), (XIVe), (XIVf), (XIVg), (XIVh), (XIVo), (XIVp), (XIVq), (XIVr), (XIVs), (XIVt), (XIVu), (XIVv), (XIVw), (XIVx), (XIVy), (XIVz), (XVa), (XVb), (XVc), (XVd), (XVe), (XVf), (XVg), (XVh), (XVi), (XVj), (XVk), (XVl), (XVm), (XVn), (XVo), (XVp), (XVq), (XVr), (XVs), (XVt), (XVu), (XVv), (XVw), (XVx), (XVy), (XVz), (XVIa), (XVIb), (XVIc) ou (XVId) tel que défini dans la revendication 6 ; ou
un composé de formule (Il), dans lequel
R⁸⁷ est un groupe de formule (XIIa), (XIIb), (XIIc), (XIId), (XIIe), (XIIf), (XIIg), (XIIh), (XIIi), (XIIj), (XIIk), ((XIIl)), (XIIm), (XIIn), (XIIo), (XIIp), (XIIq), (XIIr), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) ou (XIIIg) tel que défini dans la revendication 5 et
R⁸⁵ est H, un groupe de formule (XIIa), (XIIb), (XIIc), (XIId), (XIIe), (XIIf), (XIIg), (XIIh), (XIIi), (XIIj), (XIIk), ((XIIl)), (XIIm), (XIIn), (XIIo), (XIIp), (XIIq), (XIIr), (XIIs), (XIIt), (XIIu), (XIIv) (XIIw), (XIIx), (XIIy), (XIIz), (XIIIa), (XIIIb), (XIIIc), (XIIId), (XIIIe), (XIIIf) ou (XIIIg) tel que défini dans la revendication 5 ou un groupe de formule (XIVb), (XIVc), (XIVd), (XIVe), (XIVf), (XIVg), (XIVh), (XIVo), (XIVp), (XIVq), (XIVr), (XIVs), (XIVt), (XIVu), (XIVv), (XIVw), (XIVx), (XIVy), (XIVz), (XVa), (XVb), (XVc), (XVd), (XVe), (XVf), (XVg), (XVh), (XVi), (XVj), (XVk), (XVl), (XVm), (XVn), (XVo), (XVp), (XVq), (XVr), (XVs), (XVt), (XVu), (XVv), (XVw), (XVx), (XVy), (XVz), (XVIa), (XVIb), (XVIc) ou (XVId) tel que défini dans la revendication 6 ; ou
un composé de formule (Im), dans lequel
R⁸³ est un groupe de formule (XIIa), (XIIb), (XIIc), (XIId), (XIIe), (XIIf), (XIIg), (XIIh), (XIIi), (XIIj), (XIIk), ((XIIl)), (XIIm), (XIIn), (XIIo), (XIIp), (XIIq), (XIIr), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) ou (XIIIg) tel que défini dans la revendication 5 et
R⁸⁵ est H, un groupe alkyle en C₁-C₂₅, un groupe de formule (XIIa), (XIIb), (XIIc), (XIId), (XIIe), (XIIf), (XIIg), (XIIh), (XIIi), (XIIj), (XIIk), ((XIIl)), (XIIm), (XIIn), (XIIo), (XIIp), (XIIq), (XIIr), (XIIs), (XIIt), (XIIu), (XIIv) (XIIw), (XIIx), (XIIy), (XIIz), (XIIIa), (XIIIb), (XIIIc), (XIIId), (XIIIe), (XIIIf) ou (XIIIg) tel que défini dans la revendication 5 ou un groupe de formule (XIVb), (XIVc), (XIVd), (XIVe), (XIVf), (XIVg), (XIVh), (XIVo), (XIVp), (XIVq), (XIVr), (XIVs), (XIVt), (XIVu), (XIVv), (XIVw), (XIVx), (XIVy), (XIVz), (XVa), (XVb), (XVc), (XVd), (XVe), (XVf), (XVg), (XVh), (XVi), (XVj), (XVk), (XVl), (XVm), (XVn), (XVo), (XVp), (XVq), (XVr), (XVs), (XVt), (XVu), (XVv), (XVw), (XVx), (XVy), (XVz), (XVIa), (XVIb), (XVIc) ou (XVId) tel que défini dans la revendication 6 ; ou
un composé de formule (Im), dans lequel
R⁸⁵ est un groupe de formule (XIIa), (XIIb), (XIIc), (XIId), (XIIe), (XIIf), (XIIg), (XIIh), (XIIi), (XIIj), (XIIk), ((XIIl)), (XIIm), (XIIn), (XIIo), (XIIp), (XIIq), (XIIr), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) ou (XIIIg) tel que défini dans la revendication 5 et
R⁸³ est H, un groupe alkyle en C₁-C₂₅, un groupe de formule (XIIa), (XIIb), (XIIc), (XIId), (XIIe), (XIIf), (XIIg), (XIIh), (XIIi), (XIIj), (XIIk), ((XIIl)), (XIIm), (XIIn), (XIIo), (XIIp), (XIIq), (XIIr), (XIIs), (XIIt), (XIIu), (XIIv) (XIIw), (XIIx), (XIIy), (XIIz), (XIIIa), (XIIIb), (XIIIc), (XIIId), (XIIIe), (XIIIf) ou (XIIIg) tel que défini dans la revendication 5 ou un groupe de formule (XIVb), (XIVc), (XIVd), (XIVe), (XIVf), (XIVg), (XIVh), (XIVo), (XIVp), (XIVq), (XIVr), (XIVs), (XIVt), (XIVu), (XIVv), (XIVw), (XIVx), (XIVy), (XIVz), (XVa), (XVb), (XVc), (XVd), (XVe), (XVf), (XVg), (XVh), (XVi), (XVj), (XVk), (XVl), (XVm), (XVn), (XVo), (XVp), (XVq), (XVr), (XVs), (XVt), (XVu), (XVv), (XVw), (XVx), (XVy), (XVz), (XVIa), (XVIb), (XVIc) ou (XVId) tel que défini dans la revendication 6 ; ou
un composé de formule (In), dans lequel
R⁸³ est un groupe de formule (XIIa), (XIIb), (XIIc), (XIId), (XIIe), (XIIf), (XIIg), (XIIh), (XIIi), (XIIj), (XIIk), ((XIIl)), (XIIm), (XIIn), (XIIo), (XIIp), (XIIq), (XIIr), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) ou (XIIIg) tel que défini dans la revendication 5 et
R⁸⁷ est H, un groupe de formule (XIIa), (XIIb), (XIIc), (XIId), (XIIe), (XIIf), (XIIg), (XIIh), (XIIi), (XIIj), (XIIk), ((XIIl)), (XIIm), (XIIn), (XIIo), (XIIp), (XIIq), (XIIr), (XIIs), (XIIt), (XIIu), (XIIv) (XIIw), (XIIx), (XIIy), (XIIz), (XIIIa), (XIIIb), (XIIIc), (XIIId), (XIIIe), (XIIIf) ou (XIIIg) tel que défini dans la revendication 5 ou un groupe de formule (XIVb), (XIVc), (XIVd), (XIVe), (XIVf), (XIVg), (XIVh), (XIVo), (XIVp), (XIVq), (XIVr), (XIVs), (XIVt), (XIVu), (XIVv), (XIVw), (XIVx), (XIVy), (XIVz), (XVa), (XVb), (XVc), (XVd), (XVe), (XVf), (XVg), (XVh), (XVi), (XVj), (XVk), (XVl), (XVm), (XVn), (XVo), (XVp), (XVq), (XVr), (XVs), (XVt), (XVu), (XVv), (XVw), (XVx), (XVy), (XVz), (XVIa), (XVIb), (XVIc) ou (XVId) tel que défini dans la revendication 6.

8. Composé selon l'une quelconque des revendications 2 à 7, qui est
un composé de formule (Ia), dans lequel
R⁸³ est un groupe de formule (XIIa), (XIIb), (XIIc), (XIId), (XIIe), (XIIf), (XIIg), (XIIj), (XIIk), (XIIn), (XIIo), (XIIq), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) ou (XIIIg) tel que défini dans la revendication 5 et
R⁸⁷ est H, un groupe alkyle en C₁-C₂₅, un groupe de formule (XIIa), (XIIb), (XIIc), (XIId), (XIIe), (XIIf), (XIIg), (XIIj), (XIIk), (XIIn), (XIIo), (XIIq), (XIIs), (XIIt), (XIIu), (XIIv) (XIIw), (XIIx), (XIIy), (XIIz), (XIIIa), (XIIIb), (XIIIc), (XIIId), (XIIIe), (XIIIf) ou (XIIIg) tel que défini dans la revendication 5 ou un groupe de formule (XIVb), (XIVc), (XIVd), (XIVe), (XIVf), (XIVg), (XIVo), (XIVp), (XIVq), (XIVr), (XIVt), (XIVu), (XIVv), (XIVw), (XIVx), (XIVz), (XVa), (XVb), (XVc), (XVk), (XVl), (XVo), (XVp), (XVs), (XVw), (XVx), (XVy), (XVIa), (XVIb), (XVIc) ou (XVId) tel que défini dans la revendication 6 ; ou
un composé de formule (Ia), dans lequel
R⁸⁷ est un groupe de formule (XIIa), (XIIb), (XIIc), (XIId), (XIIe), (XIIf), (XIIg), (XIIj), (XIIk), (XIIn), (XIIo), (XIIq), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) ou (XIIIg) tel que défini dans la revendication 5 et
R⁸³ est H, un groupe alkyle en C₁-C₂₅, un groupe de formule (XIIa), (XIIb), (XIIc), (XIId), (XIIe), (XIIf), (XIIg), (XIIj), (XIIk), (XIIn), (XIIo), (XIIq), (XIIs), (XIIt), (XIIu), (XIIv) (XIIw), (XIIx), (XIIy), (XIIz), (XIIIa), (XIIIb), (XIIIc), (XIIId), (XIIIe), (XIIIf) ou (XIIIg) tel que défini dans la revendication 5 ou un groupe de formule (XIVb), (XIVc), (XIVd), (XIVe), (XIVf), (XIVg), (XIVo), (XIVp), (XIVq), (XIVr), (XIVt), (XIVu), (XIVv), (XIVw), (XIVx), (XIVz), (XVa), (XVb), (XVc), (XVk), (XVl), (XVo), (XVp), (XVs), (XVw), (XVx), (XVy), (XVIa), (XVIb), (XVIc) ou (XVId) tel que défini dans la revendication 6 ;
un composé de formule (Ib), dans lequel
R⁸² est un groupe de formule (XIIa), (XIIb), (XIIc), (XIId), (XIIe), (XIIf), (XIIg), (XIIj), (XIIk), (XIIn), (XIIo), (XIIq), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) ou (XIIIg) tel que défini dans la revendication 5 et
R⁸⁷ est H, un groupe de formule (XIIa), (XIIb), (XIIc), (XIId), (XIIe), (XIIf), (XIIg), (XIIj), (XIIk), (XIIn), (XIIo), (XIIq), (XIIs), (XIIt), (XIIu), (XIIv) (XIIw), (XIIx), (XIIy), (XIIz), (XIIIa), (XIIIb), (XIIIc), (XIIId), (XIIIe), (XIIIf) ou (XIIIg) tel que défini dans la revendication 5 ou un groupe de formule (XIVb), (XIVc), (XIVd), (XIVe), (XIVf), (XIVg), (XIVo), (XIVp), (XIVq), (XIVr), (XIVt), (XIVu), (XIVv), (XIVw), (XIVx), (XIVz), (XVa), (XVb), (XVc), (XVk), (XVl), (XVo), (XVp), (XVs), (XVw), (XVx), (XVy), (XVIa), (XVIb), (XVIc) ou (XVId) tel que défini dans la revendication 6 ; ou
un composé de formule (Ib), dans lequel
R⁸⁷ est un groupe de formule (XIIa), (XIIb), (XIIc), (XIId), (XIIe), (XIIf), (XIIg), (XIIj), (XIIk), (XIIn), (XIIo), (XIIq), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) ou (XIIIg) tel que défini dans la revendication 5 et
R⁸² est H, un groupe de formule (XIIa), (XIIb), (XIIc), (XIId), (XIIe), (XIIf), (XIIg), (XIIj), (XIIk), (XIIn), (XIIo), (XIIq), (XIIs), (XIIt), (XIIu), (XIIv) (XIIw), (XIIx), (XIIy), (XIIz), (XIIIa), (XIIIb), (XIIIc), (XIIId), (XIIIe), (XIIIf) ou (XIIIg) tel que défini dans la revendication 5 ou un groupe de formule (XIVb), (XIVc), (XIVd), (XIVe), (XIVf), (XIVg), (XIVo), (XIVp), (XIVq), (XIVr), (XIVt), (XIVu), (XIVv), (XIVw), (XIVx), (XIVz), (XVa), (XVb), (XVc), (XVk), (XVl), (XVo), (XVp), (XVs), (XVw), (XVx), (XVy), (XVIa), (XVIb), (XVIc) ou (XVId) tel que défini dans la revendication 6 ;
un composé de formule (Ic), dans lequel
R⁸⁵ est un groupe de formule (XIIa), (XIIb), (XIIc), (XIId), (XIIe), (XIIf), (XIIg), (XIIj), (XIIk), (XIIn), (XIIo), (XIIq), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) ou (XIIIg) tel que défini dans la revendication 5 et
R⁸⁷ est H, un groupe alkyle en C₁-C₂₅, un groupe de formule (XIIa), (XIIb), (XIIc), (XIId), (XIIe), (XIIf), (XIIg), (XIIj), (XIIk), (XIIn), (XIIo), (XIIq), (XIIs), (XIIt), (XIIu), (XIIv) (XIIw), (XIIx), (XIIy), (XIIz), (XIIIa), (XIIIb), (XIIIc), (XIIId), (XIIIe), (XIIIf) ou (XIIIg) tel que défini dans la revendication 5 ou un groupe de formule (XIVb), (XIVc), (XIVd), (XIVe), (XIVf), (XIVg), (XIVo), (XIVp), (XIVq), (XIVr), (XIVt), (XIVu), (XIVv), (XIVw), (XIVx), (XIVz), (XVa), (XVb), (XVc), (XVk), (XVl), (XVo), (XVp), (XVs), (XVw), (XVx), (XVy), (XVIa), (XVIb), (XVIc) ou (XVId) tel que défini dans la revendication 6 ; ou
un composé de formule (Ic), dans lequel
R⁸⁷ est un groupe de formule (XIIa), (XIIb), (XIIc), (XIId), (XIIe), (XIIf), (XIIg), (XIIj), (XIIk), (XIIn), (XIIo), (XIIq), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) ou (XIIIg) tel que défini dans la revendication 5 et
R⁸⁵ est H, un groupe alkyle en C₁-C₂₅, un groupe de formule (XIIa), (XIIb), (XIIc), (XIId), (XIIe), (XIIf), (XIIg), (XIIj), (XIIk), (XIIn), (XIIo), (XIIq), (XIIs), (XIIt), (XIIu), (XIIv) (XIIw), (XIIx), (XIIy), (XIIz), (XIIIa), (XIIIb), (XIIIc), (XIIId), (XIIIe), (XIIIf) ou (XIIIg) tel que défini dans la revendication 5 ou un groupe de formule (XIVb), (XIVc), (XIVd), (XIVe), (XIVf), (XIVg), (XIVo), (XIVp), (XIVq), (XIVr), (XIVt), (XIVu), (XIVv), (XIVw), (XIVx), (XIVz), (XVa), (XVb), (XVc), (XVk), (XVl), (XVo), (XVp), (XVs), (XVw), (XVx), (XVy), (XVIa), (XVIb), (XVIc) ou (XVId) tel que défini dans la revendication 6 ; ou
un composé de formule (Id), dans lequel
R⁸¹ est un groupe de formule (XIIa), (XIIb), (XIIc), (XIId), (XIIe), (XIIf), (XIIg), (XIIj), (XIIk), (XIIn), (XIIo), (XIIq), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) ou (XIIIg) tel que défini dans la revendication 5 et
R⁸⁵ est H, un groupe de formule (XIIa), (XIIb), (XIIc), (XIId), (XIIe), (XIIf), (XIIg), (XIIj), (XIIk), (XIIn), (XIIo), (XIIq), (XIIs), (XIIt), (XIIu), (XIIv) (XIIw), (XIIx), (XIIy), (XIIz), (XIIIa), (XIIIb), (XIIIc), (XIIId), (XIIIe), (XIIIf) ou (XIIIg) tel que défini dans la revendication 5 ou un groupe de formule (XIVb), (XIVc), (XIVd), (XIVe), (XIVf), (XIVg), (XIVo), (XIVp), (XIVq), (XIVr), (XIVt), (XIVu), (XIVv), (XIVw), (XIVx), (XIVz), (XVa), (XVb), (XVc), (XVk), (XVl), (XVo), (XVp), (XVs), (XVw), (XVx), (XVy), (XVIa), (XVIb), (XVIc) ou (XVId) tel que défini dans la revendication 6 ;
un composé de formule (Ie), dans lequel
R⁸³ est un groupe de formule (XIIa), (XIIb), (XIIc), (XIId), (XIIe), (XIIf), (XIIg), (XIIj), (XIIk), (XIIn), (XIIo), (XIIq), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) ou (XIIIg) tel que défini dans la revendication 5 et
R⁸⁷ est H, un groupe alkyle en C₁-C₂₅, un groupe de formule (XIIa), (XIIb), (XIIc), (XIId), (XIIe), (XIIf), (XIIg), (XIIj), (XIIk), (XIIn), (XIIo), (XIIq), (XIIs), (XIIt), (XIIu), (XIIv) (XIIw), (XIIx), (XIIy), (XIIz), (XIIIa), (XIIIb), (XIIIc), (XIIId), (XIIIe), (XIIIf) ou (XIIIg) tel que défini dans la revendication 5 ou un groupe de formule (XIVb), (XIVc), (XIVd), (XIVe), (XIVf), (XIVg), (XIVo), (XIVp), (XIVq), (XIVr), (XIVt), (XIVu), (XIVv), (XIVw), (XIVx), (XIVz), (XVa), (XVb), (XVc), (XVk), (XVl), (XVo), (XVp), (XVs), (XVw), (XVx), (XVy), (XVIa), (XVIb), (XVIc) ou (XVId) tel que défini dans la revendication 6 ; ou
un composé de formule (Ie), dans lequel
R⁸⁷ est un groupe de formule (XIIa), (XIIb), (XIIc), (XIId), (XIIe), (XIIf), (XIIg), (XIIj), (XIIk), (XIIn), (XIIo), (XIIq), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) ou (XIIIg) tel que défini dans la revendication 5 et
R⁸³ est H, un groupe alkyle en C₁-C₂₅, un groupe de formule (XIIa), (XIIb), (XIIc), (XIId), (XIIe), (XIIf), (XIIg), (XIIj), (XIIk), (XIIn), (XIIo), (XIIq), (XIIs), (XIIt), (XIIu), (XIIv) (XIIw), (XIIx), (XIIy), (XIIz), (XIIIa), (XIIIb), (XIIIc), (XIIId), (XIIIe), (XIIIf) ou (XIIIg) tel que défini dans la revendication 5 ou un groupe de formule (XIVb), (XIVc), (XIVd), (XIVe), (XIVf), (XIVg), (XIVo), (XIVp), (XIVq), (XIVr), (XIVt), (XIVu), (XIVv), (XIVw), (XIVx), (XIVz), (XVa), (XVb), (XVc), (XVk), (XVl), (XVo), (XVp), (XVs), (XVw), (XVx), (XVy), (XVIa), (XVIb), (XVIc) ou (XVId) tel que défini dans la revendication 6 ;
un composé de formule (If), dans lequel
R⁸³ est un groupe de formule (XIIa), (XIIb), (XIIc), (XIId), (XIIe), (XIIf), (XIIg), (XIIj), (XIIk), (XIIn), (XIIo), (XIIq), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) ou (XIIIg) tel que défini dans la revendication 5 et
R⁸⁵ est H, un groupe de formule (XIIa), (XIIb), (XIIc), (XIId), (XIIe), (XIIf), (XIIg), (XIIj), (XIIk), (XIIn), (XIIo), (XIIq), (XIIs), (XIIt), (XIIu), (XIIv) (XIIw), (XIIx), (XIIy), (XIIz), (XIIIa), (XIIIb), (XIIIc), (XIIId), (XIIIe), (XIIIf) ou (XIIIg) tel que défini dans la revendication 5 ou un groupe de formule (XIVb), (XIVc), (XIVd), (XIVe), (XIVf), (XIVg), (XIVo), (XIVp), (XIVq), (XIVr), (XIVt), (XIVu), (XIVv), (XIVw), (XIVx), (XIVz), (XVa), (XVb), (XVc), (XVk), (XVl), (XVo), (XVp), (XVs), (XVw), (XVx), (XVy), (XVIa), (XVIb), (XVIc) ou (XVId) tel que défini dans la revendication 6 ; ou
un composé de formule (If), dans lequel
R⁸⁵ est un groupe de formule (XIIa), (XIIb), (XIIc), (XIId), (XIIe), (XIIf), (XIIg), (XIIj), (XIIk), (XIIn), (XIIo), (XIIq), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) ou (XIIIg) tel que défini dans la revendication 5 et
R⁸³ est H, un groupe de formule (XIIa), (XIIb), (XIIc), (XIId), (XIIe), (XIIf), (XIIg), (XIIj), (XIIk), (XIIn), (XIIo), (XIIq), (XIIs), (XIIt), (XIIu), (XIIv) (XIIw), (XIIx), (XIIy), (XIIz), (XIIIa), (XIIIb), (XIIIc), (XIIId), (XIIIe), (XIIIf) ou (XIIIg) tel que défini dans la revendication 5 ou un groupe de formule (XIVb), (XIVc), (XIVd), (XIVe), (XIVf), (XIVg), (XIVo), (XIVp), (XIVq), (XIVr), (XIVt), (XIVu), (XIVv), (XIVw), (XIVx), (XIVz), (XVa), (XVb), (XVc), (XVk), (XVl), (XVo), (XVp), (XVs), (XVw), (XVx), (XVy), (XVIa), (XVIb), (XVIc) ou (XVId) tel que défini dans la revendication 6 ; ou
un composé de formule (Ij), dans lequel
R⁸³ est un groupe de formule (XIIa), (XIIb), (XIIc), (XIId), (XIIe), (XIIf), (XIIg), (XIIj), (XIIk), (XIIn), (XIIo), (XIIq), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) ou (XIIIg) tel que défini dans la revendication 5 et
R⁸⁵ est H, un groupe alkyle en C₁-C₂₅, un groupe de formule (XIIa), (XIIb), (XIIc), (XIId), (XIIe), (XIIf), (XIIg), (XIIj), (XIIk), (XIIn), (XIIo), (XIIq), (XIIs), (XIIt), (XIIu), (XIIv) (XIIw), (XIIx), (XIIy), (XIIz), (XIIIa), (XIIIb), (XIIIc), (XIIId), (XIIIe), (XIIIf) ou (XIIIg) tel que défini dans la revendication 5 ou un groupe de formule (XIVb), (XIVc), (XIVd), (XIVe), (XIVf), (XIVg), (XIVo), (XIVp), (XIVq), (XIVr), (XIVt), (XIVu), (XIVv), (XIVw), (XIVx), (XIVz), (XVa), (XVb), (XVc), (XVk), (XVl), (XVo), (XVp), (XVs), (XVw), (XVx), (XVy), (XVIa), (XVIb), (XVIc) ou (XVId) tel que défini dans la revendication 6 ; ou
un composé de formule (Ij), dans lequel
R⁸⁵ est un groupe de formule (XIIa), (XIIb), (XIIc), (XIId), (XIIe), (XIIf), (XIIg), (XIIj), (XIIk), (XIIn), (XIIo), (XIIq), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) ou (XIIIg) tel que défini dans la revendication 5 et
R⁸³ est H, un groupe alkyle en C₁-C₂₅, un groupe de formule (XIIa), (XIIb), (XIIc), (XIId), (XIIe), (XIIf), (XIIg), (XIIj), (XIIk), (XIIn), (XIIo), (XIIq), (XIIs), (XIIt), (XIIu), (XIIv) (XIIw), (XIIx), (XIIy), (XIIz), (XIIIa), (XIIIb), (XIIIc), (XIIId), (XIIIe), (XIIIf) ou (XIIIg) tel que défini dans la revendication 5 ou un groupe de formule (XIVb), (XIVc), (XIVd), (XIVe), (XIVf), (XIVg), (XIVo), (XIVp), (XIVq), (XIVr), (XIVt), (XIVu), (XIVv), (XIVw), (XIVx), (XIVz), (XVa), (XVb), (XVc), (XVk), (XVl), (XVo), (XVp), (XVs), (XVw), (XVx), (XVy), (XVIa), (XVIb), (XVIc) ou (XVId) tel que défini dans la revendication 6 ; ou
un composé de formule (Im), dans lequel
R⁸³ est un groupe de formule (XIIa), (XIIb), (XIIc), (XIId), (XIIe), (XIIf), (XIIg), (XIIj), (XIIk), (XIIn), (XIIo), (XIIq), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) ou (XIIIg) tel que défini dans la revendication 5 et
R⁸⁵ est H, un groupe alkyle en C₁-C₂₅, un groupe de formule (XIIa), (XIIb), (XIIc), (XIId), (XIIe), (XIIf), (XIIg), (XIIj), (XIIk), (XIIn), (XIIo), (XIIq), (XIIs), (XIIt), (XIIu), (XIIv) (XIIw), (XIIx), (XIIy), (XIIz), (XIIIa), (XIIIb), (XIIIc), (XIIId), (XIIIe), (XIIIf) ou (XIIIg) tel que défini dans la revendication 5 ou un groupe de formule (XIVb), (XIVc), (XIVd), (XIVe), (XIVf), (XIVg), (XIVo), (XIVp), (XIVq), (XIVr), (XIVt), (XIVu), (XIVv), (XIVw), (XIVx), (XIVz), (XVa), (XVb), (XVc), (XVk), (XVl), (XVo), (XVp), (XVs), (XVw), (XVx), (XVy), (XVIa), (XVIb), (XVIc) ou (XVId) tel que défini dans la revendication 6 ; ou
un composé de formule (Im), dans lequel
R⁸⁷ est un groupe de formule (XIIa), (XIIb), (XIIc), (XIId), (XIIe), (XIIf), (XIIg), (XIIj), (XIIk), (XIIn), (XIIo), (XIIq), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) ou (XIIIg) tel que défini dans la revendication 5 et
R⁸³ est H, un groupe alkyle en C₁-C₂₅, un groupe de formule (XIIa), (XIIb), (XIIc), (XIId), (XIIe), (XIIf), (XIIg), (XIIj), (XIIk), (XIIn), (XIIo), (XIIq), (XIIs), (XIIt), (XIIu), (XIIv) (XIIw), (XIIx), (XIIy), (XIIz), (XIIIa), (XIIIb), (XIIIc), (XIIId), (XIIIe), (XIIIf) ou (XIIIg) tel que défini dans la revendication 5 ou un groupe de formule (XIVb), (XIVc), (XIVd), (XIVe), (XIVf), (XIVg), (XIVo), (XIVp), (XIVq), (XIVr), (XIVt), (XIVu), (XIVv), (XIVw), (XIVx), (XIVz), (XVa), (XVb), (XVc), (XVk), (XVl), (XVo), (XVp), (XVs), (XVw), (XVx), (XVy), (XVIa), (XVIb), (XVIc) ou (XVId) tel que défini dans la revendication 6 ; ou
un composé de formule (In), dans lequel
R⁸³ est un groupe de formule (XIIa), (XIIb), (XIIc), (XIId), (XIIe), (XIIf), (XIIg), (XIIj), (XIIk), (XIIn), (XIIo), (XIIq), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) ou (XIIIg) tel que défini dans la revendication 5 et
R⁸⁷ est H, un groupe de formule (XIIa), (XIIb), (XIIc), (XIId), (XIIe), (XIIf), (XIIg), (XIIj), (XIIk), (XIIn), (XIIo), (XIIq), (XIIs), (XIIt), (XIIu), (XIIv) (XIIw), (XIIx), (XIIy), (XIIz), (XIIIa), (XIIIb), (XIIIc), (XIIId), (XIIIe), (XIIIf) ou (XIIIg) tel que défini dans la revendication 5 ou un groupe de formule (XIVb), (XIVc), (XIVd), (XIVe), (XIVf), (XIVg), (XIVo), (XIVp), (XIVq), (XIVr), (XIVt), (XIVu), (XIVv), (XIVw), (XIVx), (XIVz), (XVa), (XVb), (XVc), (XVk), (XVl), (XVo), (XVp), (XVs), (XVw), (XVx), (XVy), (XVIa), (XVIb), (XVIc) ou (XVId) tel que défini dans la revendication 6.

9. Composé selon l'une quelconque des revendications 1 à 8, qui est
un composé de formule (Ia), dans lequel
R⁸³ est un groupe de formule (XIIa), (XIIb), (XIIc), (XIId), (XIIe), (XIIf), (XIIg), (XIIj), (XIIk), (XIIn), (XIIo), (XIIq), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) ou (XIIIg) tel que défini dans la revendication 5 et
R⁸⁷ est H ou un groupe alkyle en C₁-C₂₅ ; ou
un composé de formule (Im), dans lequel
R⁸⁷ est un groupe de formule (XIIa), (XIIb), (XIIc), (XIId), (XIIe), (XIIf), (XIIg), (XIIj), (XIIk), (XIIn), (XIIo), (XIIq), (XIIs), (XIIt), (XIIu), (XIIv), (XIIw), (XIIx), (XIIy), (XIIz), (XIIIa), (XIIIb), (XIIIc), (XIIId), (XIIIe), (XIIIf) ou (XIIIg) tel que défini dans la revendication 5 et
R⁸³ est H, un groupe alkyle en C₁-C₂₅.

10. Dispositif électronique, comprenant un composé selon l'une quelconque des revendications 1 à 9.

11. Dispositif électronique selon la revendication 10, qui est un dispositif électroluminescent.

12. Couche de transport de trous, couche de transport d'électrons, couche de blocage de trous, couche de blocage d'électrons ou couche émettrice comprenant un composé selon l'une quelconque des revendications 1 à 9.

13. Couche émettrice selon la revendication 12, comprenant un composé selon l'une quelconque des revendications 1 à 9 en tant que matériau hôte en association avec un émetteur phosphorescent.

14. Appareil choisi dans le groupe constitué par les écrans de visualisation fixes ; les écrans de visualisation mobiles ; les unités d'éclairage ; les claviers ; les vêtements ; les meubles ; le papier peint, comprenant le dispositif électronique organique selon la revendication 10 ou 11 ou la couche de transport de trous ou la couche émettrice selon la revendication 12.

15. Utilisation des composés de formule I selon l'une quelconque des revendications 1 à 9 pour des photorécepteurs électrophotographiques, des convertisseurs photoélectriques, des photopiles organiques, des éléments de commutation, des transistors à effet de champ organiques émettant de la lumière, des capteurs d'image, des lasers à colorant et des dispositifs électroluminescents.
